# EUROPEAN PATENT APPLICATION

(11) **EP 4 545 097 A1**
(43) Date of publication of application: **30.04.2025**
(21) Application number: 23831821.6
(22) Date of filing: 23.06.2023
(51) Int. Cl.: A61K 47/54, A61K 47/68, C07D 409/14, C07H 17/02, C07H 17/00, C07H 17/04, C07J 43/00, A61K 49/00, A61P 35/00

(54) **NOVEL LINKER COMPOUND AND LIGAND-DRUG CONJUGATE THEREFOR**

(30) Priority: 27.06.2022 KR 20220078513
(71) Applicant: Trioar, Inc., Daejeon, 34037 (KR)
(72) Inventor: WOO, Sung Ho, Daejeon, 34037 (KR); PARK, Su Ho, Daejeon, 34037 (KR); CHO, Jong Un, Daejeon, 34037 (KR); YUN, Sang Hyeon, Daejeon, 34037 (KR); MIN, Gyoung Wook, Daejeon, 34037 (KR); PARK, Ok Ku, Daejeon, 34037 (KR); LEE, Hyun Mi, Daejeon, 34037 (KR)
(74) Representative: HGF
(86) International application number: PCT/KR2023/008747
(87) International publication number: WO 2024/005461

(57) **Abstract**

The present invention provides a novel linker compound and a ligand-drug conjugate thereof. This can stably deliver an active ingredient to a target site and rapidly release the active ingredient at the target site, and thus can increase efficacy of the active ingredient and can prevent the active ingredient from causing side effects at locations other than the target location.

## Description

### Technical Field

The present invention relates to a novel linker compound and a ligand-drug conjugate thereof, and specifically, relates to a novel linker compound of Formula 1 and a ligand-drug conjugate of Formula 2.

### Background Art

Bioactive substances such as drugs and diagnostic substances have target-specific activity *in vivo.* For example, drugs have an inhibitory or therapeutic effect on specific target cells, and diagnostic substances react with specific proteins in the body to perform diagnosis.

Meanwhile, since bioactive substances can be toxic to off-target biomaterials, technologies have been proposed to suppress the side effects of the drug while allowing the drug effect to selectively appear on target cells.

For example, an antibody-drug conjugate (ADC) is a target-directed technology that binds a drug or toxin to an antibody that binds to an *in vivo* receptor and then selectively releases the drug or toxin from target cells to exhibit the desired drug effect. Because it releases a drug or toxin only under specific conditions and selectively delivers it to target cells while minimizing side effects on normal cells, it has better efficacy than antibody therapeutic agents and can greatly reduce the risk of side effects.

These antibody-drug conjugates consist of a general "antibody-linker-drug (toxin)" structure. The linker not only simply connects the antibody and the drug, but also allows the antibody-drug conjugate to stably reach the target cell during circulation in the body, and then the drug is separated by dissociation of the antibody-drug in the target cell (e.g., as a result of hydrolysis by enzymes), thereby selectively exerting its effect on the target cell. Therefore, the stability of the linker has a significant impact on the efficacy and systemic toxicity of the antibody-drug conjugate (Discovery Medicine 2010, 10(53): 329-39).

Linkers of antibody-drug conjugates can generally be classified into non-cleavable and cleavable types.

As a non-cleavable linker, thioether is mainly used. Instead of the bond between the drug and the linker dissociating within the cell, the bond between the linker and the antibody is dissociated, and the drug bound to the linker is separated from the antibody. A thiol-maleimide linker is mainly used, but has the disadvantages of low chemical and plasma stability and low potency.

As a cleavable linker, a linker that can be separated by a chemical method or hydrolyzed by an enzymatic reaction is mainly used.

As a linker having a chemical separation mechanism, a linker consisting of a disulfide, hydrazone, or oxime bond is typically used. However, a chemically separated linker can dissociate the drug at a location unrelated to the target site depending on conditions in the blood or cells, resulting in toxic side effects.

In order to solve this problem, linkers that are selectively hydrolyzed within target cells by enzymatic reactions are being developed. For example, a linker that is hydrolyzed by an enzymatic reaction is not directly linked to a drug, but is linked through a self-immolative group (SIG) interposed between the drug and the linker, and the drug is dissociated through a mechanism such as 1,6-elimination or cyclization after hydrolysis by an enzymatic reaction (Clinical Cancer Res. 2005, 11, 843-852).

However, there is still a need in the art for the development of a linker that has excellent plasma stability and chemical stability, can rapidly release the drug selectively within target cells, and has excellent versatility to form conjugates with various antibodies and drugs.

### Detailed Description of Invention

### Technical Problem

One object of the present invention is to provide a linker compound of Formula 1, Formula 1-1 or Formula B, and a ligand-drug conjugate of Formula 2 or Formula 2-1.

Another object of the present invention is to provide a pharmaceutical composition, an imaging composition, or a composition for detection comprising a compound of Formula 1, Formula 1-1 or Formula B, or a ligand-drug conjugate of Formula 2 or Formula 2-1.

### Solution to Problem

Each description and embodiment disclosed in the present application may also be applied to each other description and embodiment. That is, all combinations of said various elements disclosed in the present application fall within the scope of the present application. In addition, the scope of the present application cannot be considered limited by the specific description described below.

In one aspect of the present invention, there is provided a linker compound represented by Formula 1 below, or a pharmaceutically acceptable salt thereof:

[Formula 1] **A-(L¹)k-Uⱼ**

In addition, in another aspect of the present invention, there is provided a ligand-drug conjugate represented by Formula 2 below, or a pharmaceutically acceptable salt thereof:

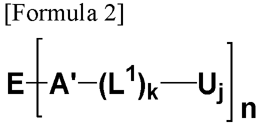

In Formula 1 or Formula 2 of the present invention, L¹ is a divalent or multivalent linking group, k is 0 or 1, and j is 1 to 10.

In Formula 1 or Formula 2 of the present invention, A is absent, H or a binding functional group, and A' is a divalent linking group derived from the binding functional group of A.

In Formula 2 of the present invention, n is a real number from 1 to 10, and E is a ligand or protein having a receptor binding property.

In Formula 1 or Formula 2 of the present invention, U is a moiety represented by Formula A below: 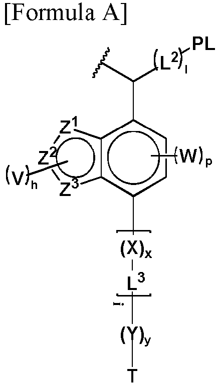 in Formula A,
PL is an active agent linked to L² or the carbon atom to which the benzene ring and A-(L¹)ₖ- are bound, via a heteroatom selected from N, O and S;
L² is a self-eliminating linker selected such that cleavage of the bond between L² and the carbon atom to which the benzene ring and A-(L¹)ₖ- are bound promotes cleavage of the bond between L² and PL;
W is an optional substituent on the benzene ring;
one of Z¹ and Z³ is selected from the group consisting of NR³, O, S and Se, and the other one of Z¹ and Z³ and Z² are each independently CH or N, and -(V)ₕ, if present, each independently replaces H of NH or CH; 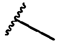
represents a bond with A-(L₁)ₖ-;
R³ is H or C₁-C₈ hydrocarbyl;
V is an electron withdrawing group, an electron donating group, or -CH₂-(L²)₁-PL-;
T is a triggering group capable of initiating the release of PL and L₂, if present, through a 1,6-elimination reaction upon cleavage;
L³ is an optional self-immolative spacer group that, if present, is cleaved sequentially upon cleavage of T;
X and Y are each independently selected from -O-, -NH-, and S; and
h, i, l, x and y are each independently 0 or 1, and p is an integer from 0 to 2.

### Linker compounds of Formula 1

### Fused-ring Containing Core Unit

U in Formula 1 (A-(L¹)ₖ-Uⱼ) is represented by Formula A below:

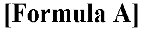

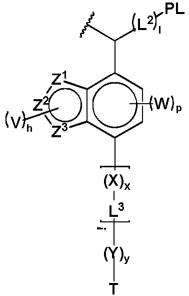

In Formula 1, if L¹ is a divalent linking group, then one side of L¹ binds to A and the other side binds to U. In this case, one U is bound to L¹. Alternatively, if L¹ comprises a branched structure or a dendrimeric structure (i.e., if L¹ is a multivalent linking group), multiple U's may bind to L¹. In this case, the number j of U's bound to L¹ may be 1 to 10. In one embodiment, j is 1 to 5. In one embodiment, j is 1.

In Formula A above, one of Z¹ and Z³ is selected from the group consisting of NR³, O, S and Se, and the other one of Z¹ and Z³ and Z² are each independently CH or N. -(V)ₕ, if present, each independently replace H of NH or CH. In this case, R³ is H or C₁-C₈ hydrocarbyl.

In Formula A above, 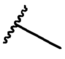 linked to the carbon atom to which the benzene ring and - (L²)₁-PL represents a bond with A-(L¹)ₖ- of Formula 1.

In one embodiment, one of Z¹ and Z³ is selected from the group consisting of NR³, O, S and Se, and the other one of Z¹ and Z³ and Z² are each independently C bound to V, or N. The R³ is H or C₁-C₈ alkyl. In one embodiment, the R³ may be H, C₁₋C₄ alkyl or C₁₋C₃ alkyl.

In one embodiment, any one of Z¹ to Z³ may comprise a heteroatom selected from the group consisting of N, O, S and Se. In one embodiment, two or more of Z¹ to Z³ may each independently comprise a heteroatom selected from the group consisting of N, O, S and Se.

In one embodiment, one of Z¹ and Z³ may be selected from the group consisting of NR³, O, S and Se, and the other one and Z² may be CH. In this case, either of the CH may be substituted with V.

In one embodiment, one of Z¹ and Z³ may be selected from the group consisting of NR³, O, S and Se, while one of the other of Z¹ and Z³ and Z² may be N, and the remaining one of Z¹ to Z³ may be CH or C bound to V.

In one embodiment, when any one or more of Z¹ to Z³ is CH, V may be substituted on the carbon atom of the CH. For example, when Z² is CH, V may be substituted on Z².

In one embodiment, the compound represented by Formula 1 or Formula 2 may be a derivative of indole, benzothiophene, benzofuran, benzoselenophene, indazole, benzimidazole, benzoxazole, benzisoxazole or benzothiazole.

In one embodiment, the ring 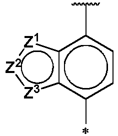 in Formula A may be selected from the following group:

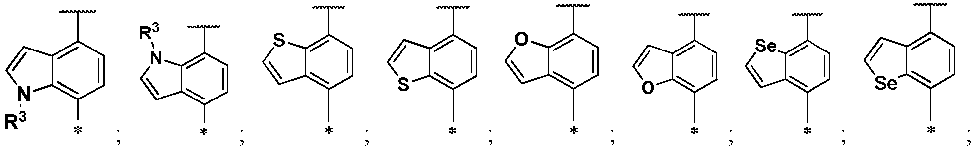

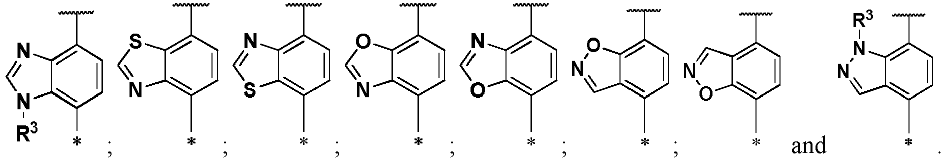

The benzene ring in the fused ring structure of Formula A above may be substituted with an optional substituent as long as it does not have an undesirable effect on the 1,6-elimination reaction initiated from a triggering group T. In one embodiment, the substituent W on the benzene ring may be selected from the group consisting of H, C₁-C₁₂ saturated or unsaturated hydrocarbyl, halogen, halo-C₁-C₈ alkyl, CN, NO₂, OH, C₁-C₈ alkoxy, hydroxy-C₁-C₈ alkyl, C₁-C₈ alkoxy- C₁-C₈ alkyl, SH, C₁-C₈ alkylthio, mercapto-C₁-C₈ alkyl, amino, mono-C₁-C₈ alkylamino, di-C₁-C₈ alkylamino, amino-C₁-C₈ alkyl, C₁-C₈ monoalkylamino-C₁-C₈ alkyl, C₁-C₈ dialkylamino-C₁-C₈ alkyl, carboxy, C₁-C₈ alkoxycarbonyl, C₁-C₈ alkoxycarbonyloxy, carboxy-C₁-C₈ alkyl, C₁-C₈ alkoxycarbonyl-C₁-C₈ alkyl, carbamoyl, mono-C₁-C₈ alkylcarbamoyl, di-C₁-C₈ alkylcarbamoyl, carbamoyl-C₁-C₈ alkyl, mono-C₁-C₈ alkylcarbamoyl-C₁-C₈ alkyl and di-C₁-C₈ alkylcarbamoyl-C₁-C₈ alkyl. In Formula 1 and Formula 2, p may be an integer from 0 to 2. In one embodiment, p may be 0.

The 5-membered ring comprising Z¹, Z² and Z³ in the fused ring of Formula A above may be optionally substituted with V.

In Formula A, V is an electron withdrawing group, an electron donating group, -CH₂-(L²)₁-PL-. h and l are each 0 or 1. For example, V may be an electron withdrawing group. For example, V may be -CH₂-(L²)₁-PL-.

In some embodiments, said V may be halogen, CN, NO₂, formyl, C₁-C₈ alkylcarbonyl, carboxy, C₁-C₈ alkoxycarbonyl, carboxy-C₁-C₈ alkyl, carbamoyl, mono-C₁-C₈ alkylcarbamoyl, di-C₁-C₈ alkylcarbamoyl, C₁-C₈ alkyl, C₁-C₈ alkenyl, OH, C₁-C₈ alkoxy, SH, C₁-C₈ alkylsulfanyl, NH₂, mono-C₁-C₈ alkylamino, di-C₁-C₈ alkylamino or C₆-C₁₈ aryl. In one embodiment, the V may be -CH₂-PL or -CH₂-L²-PL.

In some embodiments, the V may be carboxy, C₁-C₈ alkoxycarbonyl, carboxy-C₁-C₈ alkyl, NH₂, mono-C₁-C₈ alkylamino, or di-C₁-C₈ alkylamino. For example, the V may be carboxy, methoxycarbonyl, ethoxycarbonyl, propoxycarbonyl, carboxymethyl, carboxyethyl, carboxypropyl, amino, aminomethyl, aminoethyl, or aminopropyl.

In one embodiment, when the V is -CH₂-(L²)₁-PL, L₂, PL and l may each independently have the same definitions as described below for L₂, PL and l in connection with Formula A. In this case, Formula A comprises two instances of -(L²)₁-PL, each of which may the same as or different from each other.

### Self-eliminating Linker

In Formula A of the present invention, L² is a self-eliminating linker selected such that cleavage of the bond between L² and the carbon atom to which the benzene ring and A-(L¹)ₖ- are bound promotes cleavage of the bond between L² and PL. l may be 0 or 1. When l is 0, the PL may be directly bound to the carbon atom to which the benzene ring and A-(L¹)ₖ- are bound.

When a compound of Formula 1 comprises a self-eliminating linker, the L² group is cleaved from the carbon atom to which the benzene ring and A-(L¹)ₖ- are bound through a 1,6-elimination reaction triggered by the triggering group T, and PL⁻ or PL-H may be released from L².

In one embodiment, the L² may be at least one linker selected from the group consisting of -OC(=O)-, -S(=O)₂-, 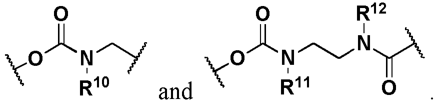 In this case, R¹⁰ to R¹² may be each independently H, C₁-C₈ alkyl, amino-C₁-C₈ alkyl, C₁-C₈ alkyl substituted with mono- or di-(C₁-C₈ alkyl)amino, or -(CH₂CH₂O)_{g}R¹³. In this case, R¹³ may be H or C₁₋₄ alkyl, and g may be an integer from 1 to 10. In one embodiment, the C₁-C₈ alkyl may be C₁-C₆ alkyl, C₁-C₄ alkyl or C₁₋C₃ alkyl. For example, R¹⁰ to R¹² may each independently include H, methyl, ethyl, propyl, 2-aminoethyl, 2-(N-methylamino)ethyl, 2-(N,N-dimethylamino)ethyl, 2-(N-ethylamino)ethyl or 2-(N,N-diethylamino)ethyl. In one embodiment, the moiety of Formula A may include -OC(=O)-as L².

### Active agent (PL)

In Formula A of the present invention, PL is an active agent that exhibits the desired biological activity in target cells. PL may be linked to L² or the carbon atom to which the benzene ring and A-(L¹)ₖ- are bound, via a heteroatom selected from N, O and S in the active agent. In addition, when V is -CH₂-(L²)₁-PL, the heteroatom selected from N, O and S in the active agent is linked to -CH₂-(L²)₁-. In this case, the moietyof Formula A may carry two active agent moieties. PL is released from the compound of Formula 1 through a 1,6-elimination reaction when T is cleaved through an enzymatic reaction or a chemical reaction.

In one embodiment, PL may be at least one active agent selected from the group consisting of a drug, a toxin, a fluorophore, an affinity ligand, a diagnostic substance and a detection probe. Specifically, a heteroatom (N, O or S) included in the drug, toxin, fluorophore, affinity ligand, diagnostic substance and detection probe may be bound to -CH₂-(L²)₁-. Therefore, as long as it contains a heteroatom which is capable of forming the aforementioned binding, or it can further introduce a functional group containing such a heteroatom, it can be used as the PL of the present invention, and it should be noted that the PL of the present invention is not limited to the specific active agents exemplified herein. For example, the PL may include a functional group where H of a primary or secondary amine group, H of a hydroxy group, or H of a carboxyl group is removed, a functional group where the lone pair of electrons of the nitrogen atom of a tertiary amine group is donated, or a functional group linked to a nitrogen atom through an addition reaction of an imine group.

The drug may be selected from, but is not limited thereto, the group consisting of erlotinib (TARCEVA; Genentech/OSI Pharm.); bortezomib (VELCADE; MilleniumPharm.); fulvestrant (FASLODEX; AstraZeneca); sutent (SU11248; Pfizer); letrozole (FEMARA; Novartis); imatinib mesylate (GLEEVEC; Novartis); PTK787/ZK 222584(Novartis); oxaliplatin (Eloxatin; Sanofi); 5-fluorouracil (5-FU); leucovorin; rapamycin (Sirolimus, RAPAMUNE; Wyeth); lapatinib (TYKERB, GSK572016; GlaxoSmithKline); lonafarnib (SCH 66336); sorafenib (BAY43-9006; Bayer Labs.); gefitinib (IRESSA; Astrazeneca); AG1478, AG1571 (SU 5271; Sugen); alkylating agents (e.g., thiotepa or CYTOXAN^{®}; cyclophosphamide); alkyl sulfonates (e.g., busulfan, improsulfan or piposulfan); aziridine (e.g., benzodopa, carboquone, meturedopa or uredopa); ethylenimine, methylmelamine, altretamine, triethylenemelamine, triethylenephosphoramide, triethylenethiophosphoramide, trimethylolmelamine; acetogenins (e.g., bullatacin or bullatacinone); camptothecin including synthetic analogue topotecan; bryostatin; callystatin; CC-1065 (including adozelesin, carzelesin, or bizelesin synthetic analogues thereof); cryptophycins (e.g., cryptophycin 1 or cryptophycin 8); dolastatin; duocarmycin (including synthetic analogues, KW-2189 and CB1-TM1); eleutherobin; pancratistatin; sarcodictyin; spongistatin; nitrogen mustard (e.g., chlorambucil, chlornaphazine, cholophosphamide, estramustine, ifosfamide, mechlorethamine, mechlorethamine oxide hydrochloride, melphalan, novembichin, phenesterine, prednimustine, trofosfamide or uracil mustard); nitrousurea (e.g., carmustine, chlorozotocin, fotemustine, lomustine, nimustine or ranimnustine); antibiotics (e.g., as enediyne antibiotics, calicheamycin selected from calicheamycin gamma1 I and calicheamycin omegaI1 or dynemicin including dynemicin A); bisphosphonates (e.g., clodronate); esperamicin, neocarzinostatin chromophore or related chromoprotein enediyne antibiotic chromophores, aclacinomysins, actinomycin, antrmycin, azaserine, bleomycins, cactinomycin, carabicin, carninomycin, carzinophilin, chromomycins, dactinomycin, daunorubicin, detorubucin, 6-diazo-5-oxo-L-norleucine, ADRLIMYCIN; doxorubicin (ADRLIMYCIN) (e.g., morpholino-doxorubicin, cyanomorpholino-doxorubicin, 2-pyrrolino-doxorubucin, liposomal doxorubicin or deoxydoxorubicin), epirubicin, esorubicin, marcellomycin, mitomycins (e.g., mitomycin C, mycophenolic acid, nogalamycin, olivomycins, peplomycin, potfiromycin, puromycin, quelamycin, rodorubicin, streptomigrin, streptozocin, tubercidin, ubenimex, zinostatin or zorubicin); anti-metabolites (e.g., 5-fluorouracil (5-FU)); folic acid analogues (e.g., denopterin, methotrexate, pteropterin or trimetrexate); purine analogs (e.g., fludarabine, 6-mercaptopurine, thiamiprine or thiguanine); pyrimidine analogs (e.g., ancitabine, azacitidine, 6-azauridine, carmofur, cytarabine, dideoxyuridine, doxifluridine, enocitabine or floxuridine); androgens (e.g., calusterone, dromostanolone propionate, epitiostanol, mepitiostane or testolactone); anti-adrenals (e.g., aminoglutethimide, mitotane or trilostane); folic acid replenishers (e.g., folinic acid); aceglatone; aldophosphamide glycoside; aminolevulinic acid; eniluracil; amsacrine; bestrabucil; bisantrene; edatraxate; defofamine; demecolcine; diaziquone; elfornithine; elliptinium acetate; epothilone; etoglucid; gallium nitrate; hydroxyurea; lentinan; lonidainine; maytansinoids (e.g., maytansine or ansamitocins); trichothecenes (e.g., T-2 toxin, verracurin A, roridin A or anguidine); mitoguazone; mitoxantrone; mopidanmol; nitraerine; pentostatin; phenamet; pirarubicin; losoxantrone; 2-ethylhydrazide; procarbazine; PSK^{®}; polysaccharides; razoxane; rhizoxin; sizofiran; spirogermanium; tenuazonic acid; triaziquone; 2,2',2"-trichlorotriethylamine; trichothecenes (particularly, T-2 toxin, verracurin A, roridin A and anguidine); urethane; vindesine; dacarbazine; mannomustine; mitobronitol; mitolactol; pipobroman; gacytosine; arabinoside ('Ara-C'); cyclophosphamide; thiotepa; taxoids (e.g., TAXOL; paclitaxel (TAXOL; Bristol-Myers Squibb Oncology, Princeton, N. J.), ABRAXANETM cremophor-free, albumin-engineered nanoparticle formulation of paclitaxel (American Pharmaceutical Partners, Schaumber, I11.) or TAXOTERE; doxetaxel (TAXOTERE); chloranbucil; gemcitabine; 6-thioguanine; mercaptopurine; platinum analogs (e.g., cisplatin or carboplatin); vinblastine; platinum; etoposide, ifosfamide; mitoxantrone; vincristine; vinorelbine (NAVELBINE); novantrone; teniposide; edatrexate; daunomycin; aminopterin; xeloda; ibandronate; CPT-11; topoisomerase inhibitors RFS 2000; difluorometlhylornithine (DFMO); retinoids (e.g., retinoic acid); capecitabine; and a pharmaceutically acceptable salt, solvate, acid, or derivative thereof.

Additional drugs other than the aforementioned drug comprise, but are not limited thereto, (i) anti-hormones which modulates or inhibits hormone actions on tumors such as anti-estrogens and selective estrogen receptor modulators (SERM), including tamoxifen (NOLVADEX; including tamoxifen), raloxifene, droloxifene, 4-hydroxytamoxifen, trioxifene, keoxifene, LY117018, onapristone and FAREATON; toremifene; (ii) aromatase inhibitors which inhibits aromatase enzymes to modulate estrogen production in the adrenal glands, such as 4(5)-imidazole, aminoglutethimide, MEGASE; megestrol acetate, AROMASIN; exemestane, FEMARA; letrozole and ARIMIDEX; anastrozole; (iii) anti-androgens, such as flutamide, nilutamide, bicalutamide, leuprolid and goserelin; as well as troxacitabine (1,3-dioxolane nucleoside cytosine analog); (iv) aromatase inhibitors; (v) protein kinase inhibitors; (vi) lipid kinase inhibitors; (vii) antisense oligonucleotides, particularly those which inhibit gene expression in the signaling pathway associated with adherent cells, such as PKC-α, Raf, H-Ras; (viii) ribozymes, such as VEGF inhibitors, such as ANGIOZYME ribozymes and HER2 expression inhibitors; (ix) vaccines, such as gene therapy vaccines; ALLOVECTIN; vaccine, LEUVECTIN vaccine and VAXID vaccine; PROLEUKIN; rlL-2; LURTOTECAN; topoisomerase 1 inhibitors; ABARELIX; rmRH; (x) anti-angiogenic agents, such as bevacizumab (AVASTIN, Genentech); and (xi) a pharmaceutically acceptable salt, solvate, acid, or derivative thereof.

In one embodiment, the drug may be selected from cytokines, immunomodulatory compounds, anticancer agents, antiviral agents, antibacterial agents, antifungal agents, anthelmintic agents or a combination thereof.

The cytokines are small cell-signaling protein molecules that are secreted by numerous cells, which may be signaling molecules used extensively in intercellular communication. The cytokines include monokine, lympokine, traditional polypeptidehormone, etc. Exemplary cytokines may comprise, but are not limited to, growth hormone (e.g., human growth hormone, N-methionyl human growth hormone or bovine growth hormone); parathyroid hormone; thyroxine; insulin; proinsulin; relaxin; prorelaxin; glycoprotein hormone (e.g., follicle stimulating hormone (FSH), thyroid stimulating hormone (TSH) or luteinizing hormone (LH)); hepatic growth factor; fibroblast growth factor; prolactin; placental lactogen; tumor necrosis factor-α, tumor necrosis factor-β; mullerian-inhibiting substance; mouse gonadotropin-associated peptide; inhibin; activin; vascular endothelial growth factor; integrin, thrombopoietin (TPO); nerve growth factor (e.g., NGF-β); platelet-growth factor; transforming growth factor (TGF) (e.g., TGF-α or TGF-β); insulin-likegrowth factor-I, insulin-like growth factor-II; erythropoietin (EPO); osteoinductive factor; interferon (e.g., interferon-α, interferon-β or interferon-γ); colony stimulating factor (CSF) (e.g., macrophage-CSF (M-CSF), granulocyte-macrophage-CSF (GM-CSF) or granulocyte-CSF (G-CSF)); interleukin (IL) (e.g., IL-1, IL-1α, IL-2, IL-3, IL-4, IL-5, IL-6, IL-7, IL-8, IL-9, IL-10, IL-11 or IL-12); tumor necrosis factor (e.g., TNF-α or TNF-β); and polypeptide factor (e.g., LIF or kit ligand (KL)). In addition, the term "cytokine" also includes proteins from natural sources or from recombinant cell culture and biologically active equivalents of the native sequence cytokines.

The immunomodulatory compounds may be selected from the group consisting of aminocaproic acid, azathioprine, bromocriptine, chloroquine, chlorambucil, cyclosporine, cyclosporine A, danazol, DHEA (dehydroepiandrosterone), dexamethasone, etanercept, hydroxychloroquine, hydrocortisone, infliximab, meloxicam, methotrexate, cyclophosphamide, mycophenylate mofetil, prednisone, sirolimus and tacrolimus.

The anticancer agents may be selected from the group consisting of methotrexate, taxol, L-asparaginase, mercaptopurine, thioguanine, hydroxyurea, cytarabine, cyclophosphamide, ifosfamide, nitrosourea, cisplatin, carboplatin, mitomycin, dacarbazine, procarbazine, topotecan, nitrogen mustard, cytoxan, etoposide, 5-fluorouracil, BCNU (bis-chloroethylnitrosourea), irinotecan, camptothecin, exatecan, belotecan, bleomycin, doxorubicin, idarubicin, daunorubicin, dactinomycin, plicamycin, mitoxantrone, asparaginase, vinblastine, vincristine, vinorelbine, paclitaxel, docetaxel, chlorambucil, melphalan, carmustine, lomustine, busulfan, treosulfan, decarbazine, etoposide, teniposide, topotecan, 9-aminocamptothecin, crisnatol, mitomycin C, trimetrexate, mycophenolic acid, tiazofurin, ribavirin, EICAR (5-ethynyl-1-beta-D-ribofuranosylimidazole-4-carboxamide), hydroxyurea, deferoxamine, floxuridine, doxifluridine, raltitrexed, cytarabine (ara C), cytosine arabinoside, fludarabine, tamoxifen, raloxifene, megestrol, goserelin, leuprolide acetate, flutamide, bicalutamide, EB1089, CB1093, KH1060, verteporfin, phthalocyanine, photosensitizer Pe4, demethoxy-hypocrellin A, Interferon-α, Interferon-γ, tumor necrosis factor, Gemcitabine, velcade, revamid, thalamid, lovastatin, 1-methyl-4-phenylpyridinium ion, staurosporine, actinomycin D, dactinomycin, bleomycin A2, bleomycin B2, peplomycin, epirubicin, pirarubicin, zorubicin, mitoxantrone, verapamil and thapsigargin. The antiviral agents may be selected from the group consisting of pencicyclovir, valacyclovir, gancicyclovir, foscarnet, rivavirin, idoxuridine, vidarabine, trifluridine, acyclovir, famcicyclovir, amantadine, rimantadine, cidofovir, antisense oligonucleotide, immunoglobulin and interferon. The antibacterial agents may be selected from the group consisting of chloramphenicol, vancomycin, metronidazole, trimethoprin, sulfamethazole, quinupristin, dalfopristin, rifampin, spectinomycin and nitrofurantoin. The antifungal agents may be selected from the group consisting of amphotericin B, Candicidin, filipin, hamycin, natamycin, nystatin, rimocidin, Bifonazole, Butoconazole, Clotrimazole, Econazole, Fenticonazole, Isoconazole, Ketoconazole, Luliconazole, Miconazole, Omoconazole, Oxiconazole, Sertaconazole, Sulconazole, Tioconazole, Albaconazole, Fluconazole, Isavuconazole, Itraconazole, Posaconazole, Ravuconazole, Terconazole, Voriconazole, Abafungin, Amorolfin, Butenafine, Naftifine, Terbinafine, Anidulafungin, Caspofungin, Micafungin, benzoic acid, ciclopirox, flucytosine, griseofulvin, haloprogin, tolnaftate, undecylenic acid, crystal violet, balsam of peru, Ciclopirox olamine, Piroctone olamine, Zinc pyrithione and Selenium sulfide. The anthelmintic agents may be selected from the group consisting of mebendazole, pyrantel pamoate, thiabendazole, diethylcarbamazine, ivermectin, niclosamide, praziquantel, albendazole, rifampin, amphotericin B, melarsoprol, eflornithine, metronidazole, tinidazole and miltefosine.

The "toxin" refers to a poisonous substance produced within living cells or organisms. Toxins can be small molecules, peptides or proteins that are capable of causing disease on contact with or absorption by body tissue interacting with biological macromolecules such as enzyme or cellular receptors. In addition, the "toxin" comprises plant toxins and animal toxins. Exemplary animal toxins include, but are not limited to, diphtheria toxin, botulium toxin, tetanus toxin, dysentery toxin, cholera toxin, tetrodotoxin, brevetoxin, ciguatoxin. Exemplary plant toxins include, but are not limited to, ricin and AM-toxin.

For example, small molecule toxins may include, but are not limited to, auristatin, tubulysin, geldanamycin (Kerr et al., 1997, Bioconjugate Chem. 8(6):781-784), maytansinoid (EP 1391213, ACR 2008, 41, 98-107), calicheamycin (US 2009105461, Cancer Res. 1993, 53, 3336-3342), daunomycin, doxorubicin, methotrexate, vindesine, SG2285 (Cancer Res. 2010, 70(17), 6849-6858), dolastatin, dolastatin analog's auristatin (US563548603), cryptophycin, camptothecin, rhizoxin derivative, CC-1065 analogues or derivatives, duocarmycin, enediyne antibiotics, esperamicin, epothilone, PBD (pyrrolobenzodiazepine) derivatives, α-amanitin and toxoid. Toxins may exhibit cytotoxicity and cell growth-inhibiting activity by tubulin binding, DNA binding, and topoisomerase inhibition, etc.

The affinity ligand may include a molecule capable of forming a complex with a target biomolecule. The affinity ligand may be a molecule that transmits a signal by binding to a predetermined position on the target protein. The affinity ligand may be a substrate, an inhibitor, a stimulant, a neurotransmitter, or a radioisotope.

The term "detection probe" may refer to a substance or a portion of a substance that can be detected by spectroscopic, photochemical, biochemical, immunochemical, radioactive or chemical means. For example, useful detection probes may include 32P, 35S, fluorescent dyes, electron-dense reagents, enzymes (e.g., those commonly used in ELISA), biotin-streptavidin, dioxigenin, haptens, and proteins for which antisera or monoclonal antibodies are available, or nucleic acid molecules with a sequence complementary to a target. Detection probes are often capable of generating a measurable signal, such as a radioactive, chromogenic or fluorescent signal, which can be used to quantify the amount of bound detectable moiety in a sample. Quantification of the signal can be achieved, for example, by scintillation counting, densitometry, flow cytometry, ELISA, or direct analysis by mass spectrometry of intact or subsequently digested peptides (one or more peptides can be assayed).

The above probe may include (i) a substance capable of providing a detectable signal, (ii) a substance capable of altering a detectable signal provided by the first or second probe, such as fluorescence resonance energy transfer (FRET), by causing the first or second probe to react with each other, (iii) a substance capable of stabilizing an interaction with an antigen or a ligand or increasing binding affinity, (iv) a substance capable of affecting electrophoretic mobility or cell-invasiveness by physical parameters such as charge, hydrophobicity, and the like, and (v) a substance capable of modulating ligand affinity, antigen-antibody binding, or ionic complex formation.

In one embodiment, PL may be a moiety of an active agent selected from the group consisting of MMAF (monomethyl auristatin F), auristatin F, MMAE (monomethyl auristatin E), SN-38, p-nitrophenol, xanthenecarboxylic acid, abiraterone, gefitinib, PBD dimer, α-amanitin, seco-DUBA, doxorubicin, lapatinib, imatinib, erlotinib, exatecan, belotecan, and a compound represented by one of the following formulas:

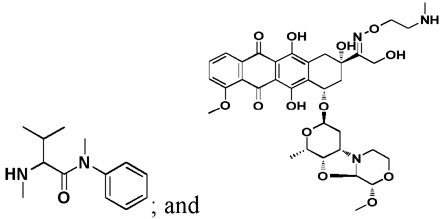

In one embodiment, PL may be selected from the group consisting of moieties represented by the following formulas:

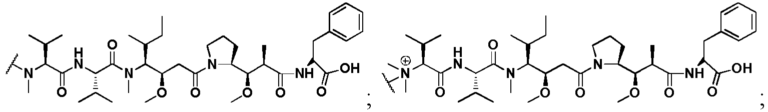

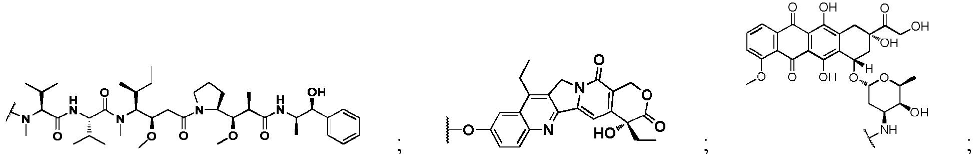

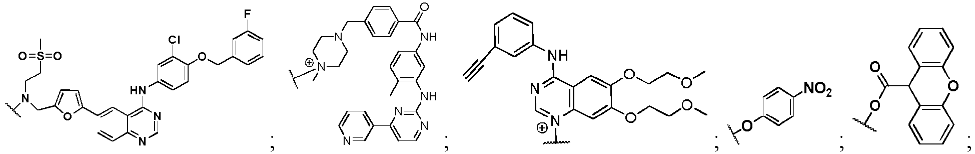

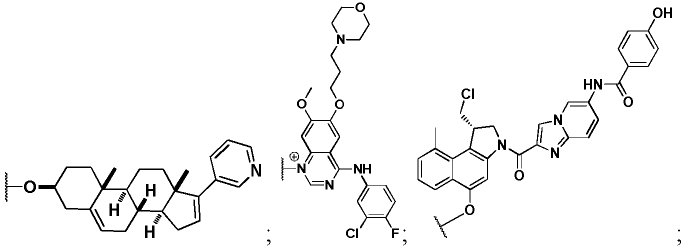

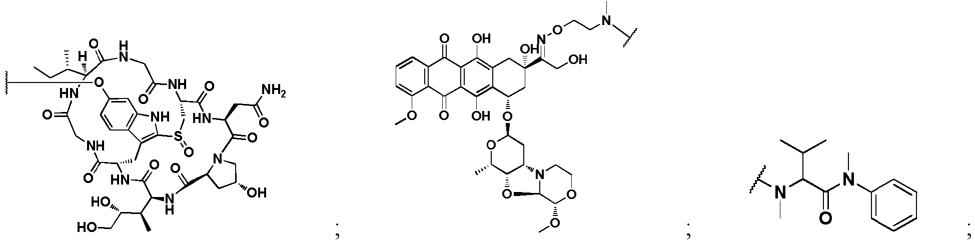

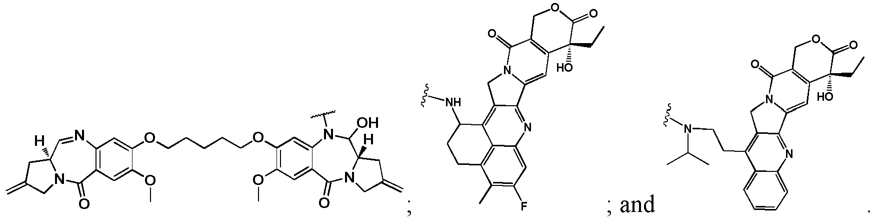

In one embodiment, the PL may be selected from moieties represented by the following formulas:

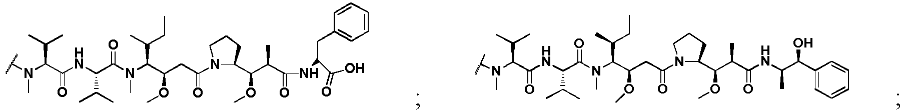

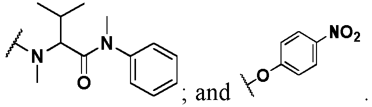

### Triggering Unit

In Formula A of the present invention, T is a triggering group capable of initiating the release of PL and, if present, L², through a 1,6-elimination reaction upon cleavage.

T can be selectively cleaved in vivo by chemical reaction or enzymatic reaction. That is, T (or the bond between Y and T if Y is present) can be selectively cleaved under specific conditions *in vivo,* thereby stably delivering PL to the target site, and selectively releasing PL at the target site.

In one embodiment, T may be linked to the benzene ring via a heteroatom such as an oxygen atom, a sulfur atom, or a nitrogen atom. That is, in Formula A, Y connecting the triggering group T to the compound moiety may be -O-, -NH-, and S.

In one embodiment, -(Y)_{y-}T may be selected from the group consisting of -β-galactoside, -β-glucuronide, -O-SO₃⁻, -NO₂, -valine-citrulline derivative, -valine-alanine derivative, - OC(O)(CH₂)ᵣCOR^{t1}, -O(CH₂)-Ar¹-NO₂, -S-C(O)(CH₂)ₛCOR^{t2}, -S(CH₂)-Ar²-NO₂ and -BR^{t3}R^{t4}.

The β-galactoside and β-glucuronide may be linked to the benzene ring via the oxygen atom attached to C1 position of the β-galactoside moiety and the β-glucuronide moiety. The valine-citrulline derivative and the valine-alanine derivative may be linked to the benzene ring via the nitrogen atom of the valine moiety. That is, the -β-galactoside, -β-glucuronide, -valine-citrulline derivative and -valine-alanine derivative herein may be moieties having the structure below:

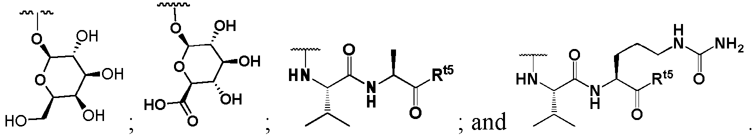

R^{t1} and R^{t2} may be each C₁-C₈ alkyl, Ar¹ and Ar² may be each C₅-C₂₀ arylene or heteroarylene. In addition, R^{t3} and R^{t4} may be each independently hydrogen, C₁-C₈ alkoxy or hydroxy. r and s may be each an integer from 1 to 5. In one embodiment, the arylene may be phenyl or naphthyl. In one embodiment, the heteroarylene may contain 1 to 3 heteroatoms (N, O or S). For example, the heteroarylene may be furanyl, thiophenyl, or pyrrolyl.

R^{t5} may be OH, mono-C₁-C₈ alkylamino, di-C₁-C₈ alkylamino or -NH(CH₂CH₂O)_{f}R^{t6}, wherein R^{t6} may be H or C₁-C₄ alkyl, and f may be an integer from 1 to 10.

In one embodiment, -(Y)_{y}-T includes saccharides such as 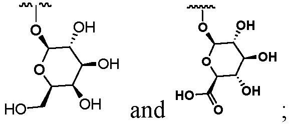 peptides such as 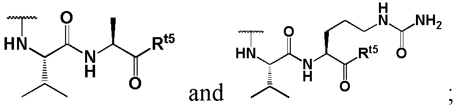 and -O-SO₃⁻ functional group, which can be selectively hydrolyzed by specific enzymes in lysosomes. The above saccharides may include those in which the -OH is protected with a protecting group (e.g., acetyl) or substituted with an optional substituent. In addition, -SC(O)(CH₂)ₛCOR^{t2}, -S(CH₂)-Ar²-NO₂, - OC(O)(CH₂)ᵣCOR^{t1} and -O(CH₂)-Ar¹-NO₂ can be cleaved under reducing conditions.

For example, if Y is absent (y = 0) and T is -NO₂ or -BR^{t3}R^{t4}, this can be cleaved under reducing conditions and protonolysis conditions, respectively.

### Optional self-immolative spacer unit

In Formula A of the present invention, L³ is an optional self-immolative spacer group that, if present, is cleaved sequentially upon cleavage of T. The 1,6-elimination reaction triggered by the cleavage of the T under specific conditions *in vivo* separates L³ from the moiety of the compound. Therefore, L³ has a structure that can transfer electrons generated upon cleavage of T to the benzene ring of Formula A and PL.

In one embodiment, -(X)ₓ-L³- may be 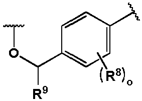 . Here, R⁸ and R⁹ may be each independently selected from the group consisting of H, halogen, C₁-C₈ alkyl, CN and NO₂, and o may be an integer from 0 to 2. For example, -(X)ₓ-L³- may be derived from benzylhydroxide (PhCH₂OH) and a derivative thereof.

In one embodiment, one of Z¹ and Z³ may be O, and -(X)ₓ-L³- may be

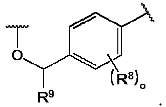

In Formula A of the present invention, i may be 0 or 1. In one embodiment, if i is 0, the self-immolative spacer group may be absent, and the -(Y)y-T may be directly linked to the benzene ring.

In one embodiment, in Formula A of the present invention, i may be 0 (i.e., the self-immolative spacer group is absent), and the self-eliminating linker L² may be -O(C=O)-. In such case, Formula A of the present invention may be represented by the following Formula A-1.

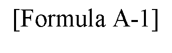

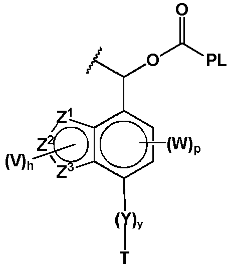

In Formula A-1, the definitions of Z¹, Z², Z³, V, Y, T, W, PL, h, y and p are as described above with respect to Formula A.

### Binding Unit

In Formula 1 of the present invention, A is absent, H or a binding functional group. If L¹ is present (k=1), A is H or a binding functional group, and when L¹ is absent (k=0), A is also absent.

The binding functional group may refer to, for example, a functional group that can be linked to, through reactions such as addition or substitution, a functional group included in a ligand or protein (E of Formula 2) or a functional group included in a linker precursor forming L¹ of Formula 1. That is, the binding functional group is any functional group that can provide binding between the ligand or protein (E) and L¹ of Formula 1, or (if L¹ is not present) U of Formula 1 and another linker. In the field of ligand-drug conjugates, various functional groups for binding between a ligand and a linker are known in the art. Therefore, those of ordinary skill in the art will be able to select an appropriate binding functional group considering the structure and properties of the ligand and linker, and it should be noted that the binding functional group of the present invention is not limited to the specific functional groups exemplified herein. For example, the binding functional group may bind to a ligand or protein (E) having a receptor binding property, or a linker precursor through a click chemistry reaction. In one embodiment, when L¹ in Formula 1 is absent (i.e., k is 0), the binding functional group is further bound to a linker precursor and then may be bound to a ligand or protein (E) having a receptor binding property through the functional group included in the linker precursor. In this case, the compound of Formula 1 has the structure of "A-U", and the ligand-drug conjugate of Formula 2 has the structure of "E-linker-A-U". In another embodiment, even when L¹ in Formula 1 is present (i.e., k is 1), the binding functional group may be bound to an additional linker precursor to form an extended linker, which may then be bound to a ligand having a receptor binding property through the functional group included in the additional linker precursor.

Therefore, when A is a binding functional group, the compound according to Formula 1 of the present invention is intended for binding to an additional linker precursor or a ligand or protein (E) having a receptor binding property, and may be, for example, an intermediate for providing a ligand-drug conjugate. On the other hand, when A is absent or H, the compound according to Formula 1 of the present invention may be a complex comprising an active agent (PL) that is not supposed to bind to a ligand having a receptor binding property. The complex can be used for various purposes, such as changing the properties (e.g., water solubility) of the active agent, targeting, and the like.

In one embodiment, the binding functional group may comprise or consist of a functional group selected from the group consisting of halogen, OH, C₁-C₈ alkoxy, hydroxylamino, COH, C₁-C₈ alkylcarbonyl, carboxy, C₁-C₈ alkoxycarbonyl, tosyl, tosylate, amino, mono-C₁-C₈ alkylamino, di-C₁-C₈ alkylamino, NHNH₂, N₃, haloacetamide, maleimidyl, succinimidyl, SH, SO₃H, C₁-C₈ alkylsulfonyl, 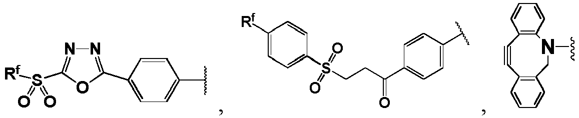 , C₁-C₈ alkoxysulfonyl, 2-pyridyl disulfide, PO₃H₂, OPO₃H₂, -N≡C, -NCS, C₄-C₁₀ dienyl, C₂-C₈ alkenyl, C₂-C₈ alkynyl, C₄-C₁₀ cycloalkynyl and C₂-C₈ alkynylcarbonyl. R^{f} may be each independently H or C₁-C₈ alkyl. In one embodiment, A may be maleimidyl, hydroxylamino, carboxy, amino, N₃, C₂-C₈ alkynyl, or 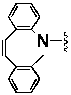 . For example, maleimidyl may refer to a monovalent functional group in which the nitrogen atom of maleimide is linked to L¹ or U of Formula 1. In one embodiment, the C₁-C₈ alkyl may be C₁-C₆ alkyl, C₁-C₄ alkyl or C₁₋C₃ alkyl. In one embodiment, C₂-C₈ alkynyl may be ethynyl, propynyl (propargyl) or butynyl.

In Formula 1 of the present invention, L¹ is a linking group connecting A with the moiety U represented by Formula A, and k is 0 or 1. The position where L¹ is attached to the moiety U is indicated by 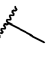 in Formula A.

In one embodiment, when k in Formula 1 is 0, A may be directly bound to the moiety U represented by Formula A. In such case, the position where A is attached to the moiety U is indicated by 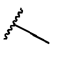 in Formula A.

In one embodiment, A-L¹ in Formula 1 may be formed by binding between the precursor of A and the precursor of L¹. The binding between the precursor of A and the precursor of L¹ may be, but is not limited to, achieved through bond through a click chemistry reaction, amide bond, urea bond, ester bond, carbamate bond, disulfide bond, or maleimide bond.

For example, the precursor of A may include at least one functional group selected from the group consisting of hydroxy, amino, azido, alkynyl, conjugated dienyl, alkenyl, cyclooctynyl, maleimidyl, SO₂N₃, alkoxysulfinyl, oxiranyl, aziridinyl, oxo, hydrazinyl, hydroxyamino, mercapto and 1,3-dicarbonyl. In addition, the precursor of L¹ may include a functional group that chemically reacts with the precursor of A to form an A-L¹ bond. Alternatively, a linker precursor having the structure A-L¹ may be bound to the remaining moiety of the compound of Formula 1.

In one embodiment, L¹ may be a C₁-C₂₀₀ alkylene optionally comprising a divalent or multivalent functional group selected from the group consisting of amide, sulfonamide, amino, ether, carbonyl, triazole, tetrazole, sugar-derived group, sulfo ester and dendrimer in the middle of the chain. The C₁-C₂₀₀ alkylene group may be C₁-C₁₅₀ alkylene group, C₁-C₁₀₀ alkylene group, C₁-C₈₀ alkylene group, C₁-C₆₀ alkylene group, C₁-C₅₀ alkylene group, C₁-C₄₀ alkylene group, C₁-C₃₀ alkylene group, C₁-C₂₀ alkylene group or C₁-C₁₀ alkylene group. The sugar-derived group refers to any chemical structure formed by a covalent bond between a sugar molecule and another group. For example, the sugar-derived group may include a glycosidic bond. The dendrimer refers to a well-ordered three-dimensional molecular structure having a branching unit centered on the core. In the field of ligand-drug conjugates, linkers of various dendrimeric structures are known *(see,* e.g., Lee, et al, Nat. Biotechnol. 2005, 23, 1517-26; Almutairi, et al; Proc. Natl. Acad. Sci. 2009, 106, 685-90), which may be advantageous, for example, to increase the ratio of ligand to drug.

In one embodiment, when L¹ comprises a multivalent functional group, that is, when L¹ comprises a branched structure or a dendrimeric structure, multiple U's may bind to L¹. In this case, the number of U bound to L¹ may be 1 to 10. In one embodiment, j may be 1 to 5. For example, j may be 1.

In one embodiment, a structure for the L¹-U bond (e.g., carboxyl group, aminocarbonyl group, amino group, etc.) may be required as a substituent for the carbon atom at the position where L¹ is attached to the moiety U represented by Formula A.

In one embodiment, L¹ may include C₁-C₁₀ alkylene, oxyethylene, amide, triazole ring, tetrazole ring, ether, carbonyl or a combination thereof.

In one embodiment, L¹ may include any one selected from the group consisting of - (CH₂)ₙₐ-; -(CH₂CH₂O)ₘₐ-; -(CH₂OCH₂)_{mb}-; -(OCH₂CH₂)_{mc}-; -C(=O)-; 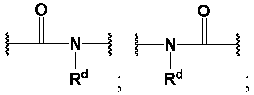 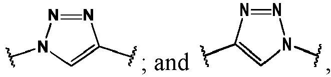 or a combination thereof. Here, R^{d} may be H or C₁-C₈ alkyl, and na and ma to mc may be each independently an integer from 0 to 10. In one embodiment, na and ma to mc may be each independently an integer from 1 to 8, an integer from 1 to 6, or an integer from 1 to 4. When two or more types of functional groups described above are combined with each other, the order of the functional groups is not limited.

In one embodiment, in Formula 1, k may be 1, and A-L¹- may be selected from the group consisting of R^{a1}C≡C-(CH₂)ₙ₁-; N₃-(CH₂)ₙ₂-; 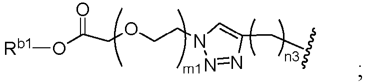 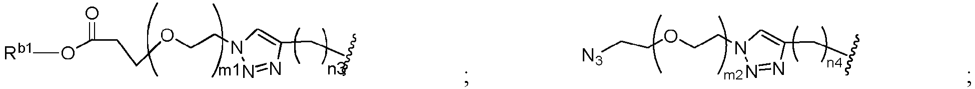 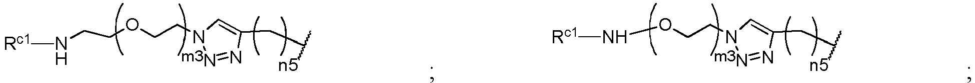 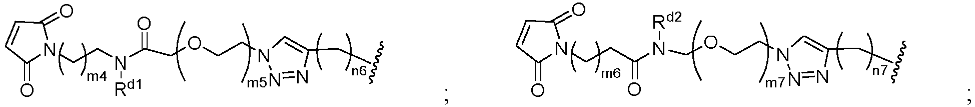 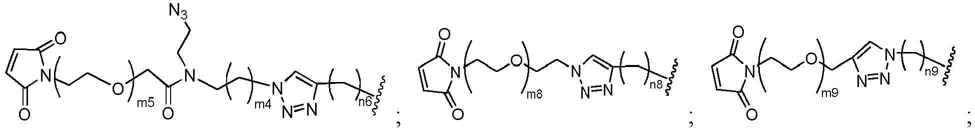 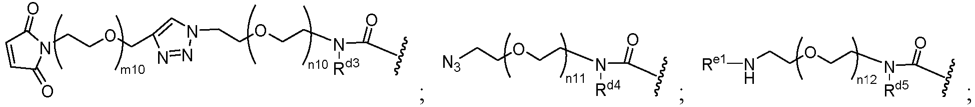 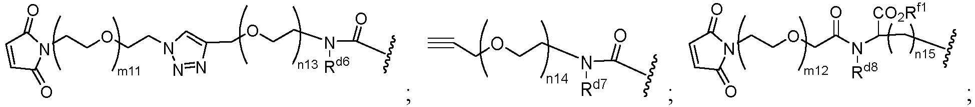 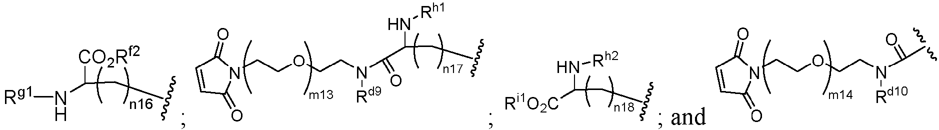 . In this case, R^{a1}, R^{b1}, R^{f1}, R^{f2}, Rⁱ¹ and R^{d1} to R^{d10} may be each independently H or C₁-C₈ alkyl. In addition, R^{c1}, R^{e1}, R^{g1}, R^{h1} and R^{h2} may be each independently H, C₁-C₈ alkyl, C₁-C₈ alkylcarbonyl (e.g., acetyl), C₁-C₈ alkoxycarbonyl (e.g., tert-butoxycarbonyl) or C₆-C₁₂ aryl-C₁-C₄ alkoxycarbonyl (e.g., benzyloxycarbonyl). n1 to n18 and m1 to m14 may be each independently an integer from 0 to 10. In one embodiment, n1 to n18 and m1 to m14 may be each independently an integer from 0 to 8, an integer from 0 to 6, or an integer from 0 to 5. In one embodiment, n1 to n18 and m1 to m14 may be each independently an integer from 1 to 8, an integer from 1 to 6, or an integer from 1 to 5.

### Exemplary compounds of Formula 1

In one embodiment, the compound represented by Formula 1 of the present invention may be a compound represented by the following formula.

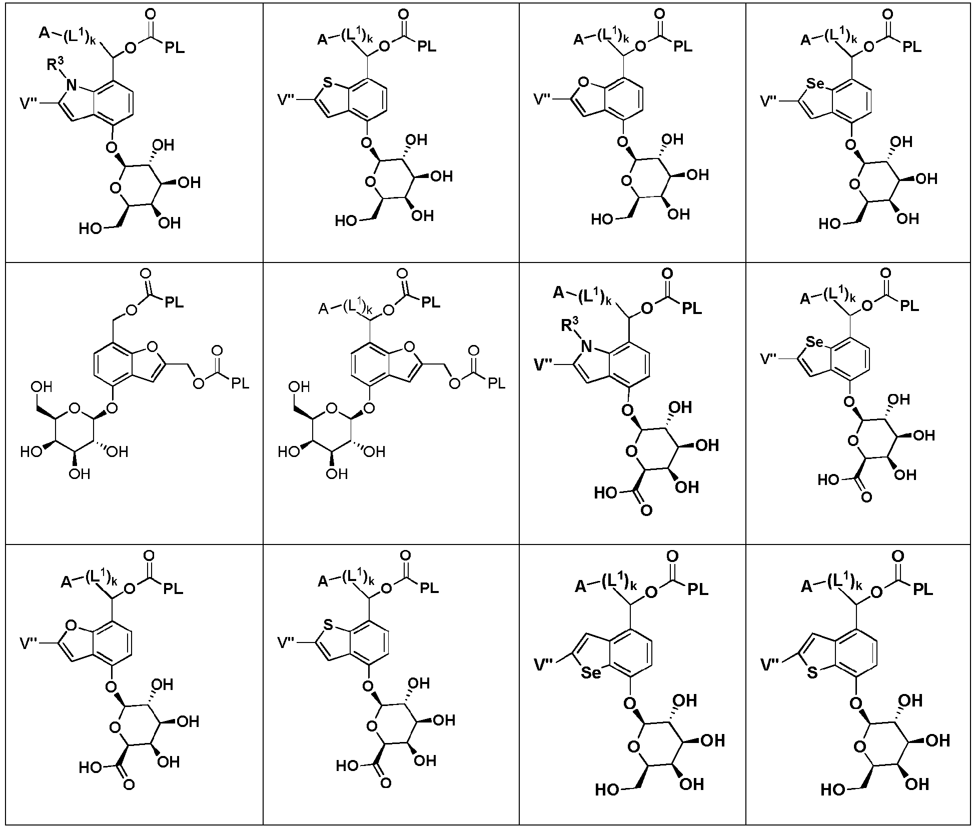

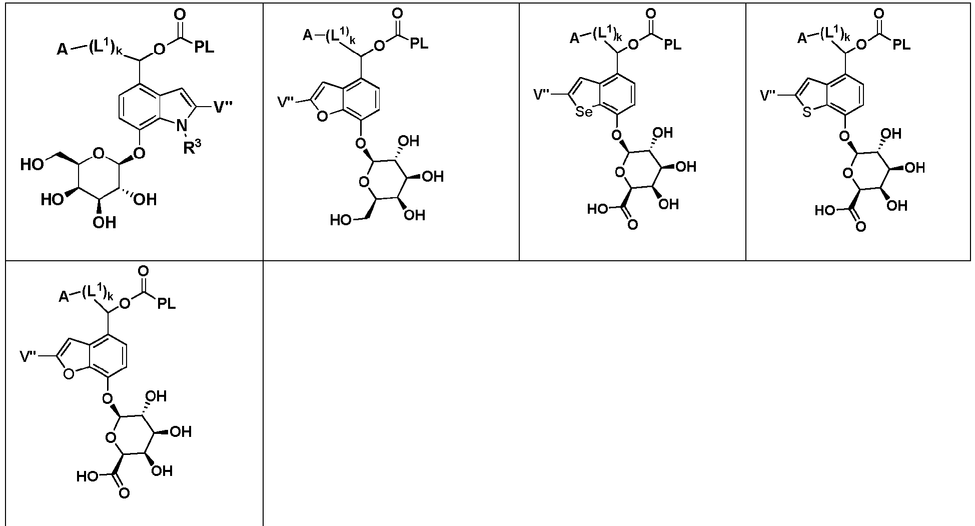

In the above formulas, A, L¹, k, R³, and PL are as described above with respect to Formula 1. In the above formulas, V" indicates an electron withdrawing group. For example, V" may be carboxy, carboxy-C₁-C₈ alkyl, or C₁-C₈ alkoxycarbonyl. For example, the V" may be carboxy, carboxymethyl, carboxyethyl, carboxypropyl, methoxycarbonyl, ethoxycarbonyl, or propoxycarbonyl.

The combinations of the structures A-(L¹)ₖ-, V", L₂, and -(Y)_{y}-T- shown in the above formula are merely exemplary, and the compounds of Formula 1 having various combinations thereof can be easily prepared based on the examples described below and the disclosure of the present application. It should be understood that all such compounds fall within the scope of the present application.

In one embodiment, the compound represented by Formula 1 of the present invention may carry one PL. In this case, for example, the compound represented by Formula 1 may be selected from the group consisting of the compounds shown in Table A attached.

In one embodiment, a DAR2 type compound may include a functional group capable of forming a bond with a ligand, such as an antibody, for the preparation of a ligand-drug conjugate, such as a maleimide functional group. Examples of these compounds are shown in Table B attached.

### DAR4 type compound (Formula 1-1)

In one embodiment, when the linking group L1 of Formula 1 according to the present invention has a branched structure or a dendrimeric structure (i.e., when L1 is a multivalent linking group), a compound of Formula 1 to which two or more active agents are bound can be provided. In one embodiment, the compound represented by Formula 1 of the present invention may be a compound represented by Formula 1-1 below.

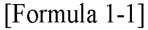

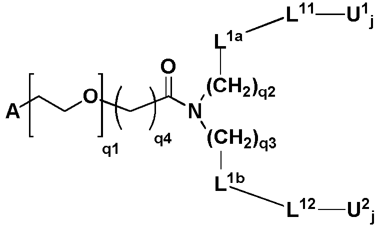

In Formula 1-1, A has the same meaning as in Formula 1.

In Formula 1-1, U¹ and U² each have the same meaning as U in Formula 1, wherein U¹ and U² may be the same as or different from each other.

In Formula 1-1, L¹¹ and L¹² each have the same meaning as L¹ in Formula 1, wherein L¹¹ and L¹² may be the same as or different from each other.

In Formula 1-1, j is 1 to 10. In one embodiment, j is 1 to 5. For example, j may be 1.

With respect to Formula A, L¹ and U, the matters described in Formula 1 may be equally applied to A, U¹ and U², and L¹¹ and L¹² in Formula 1-1, respectively, if applicable.

In Formula 1-1, L^{1a} and L^{1b} may be each independently selected from a direct bond; 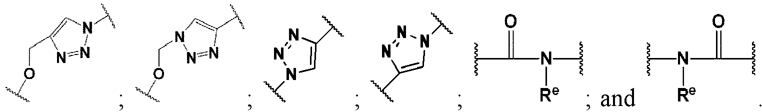 In this case, R^{e} may be H or C₁-C₈ alkyl.

In Formula 1-1, q1, q2 and q3 may be each independently an integer from 0 to 10. In Formula 1-1, q4 may be an integer from 1 to 10. Further, if L^{1a} is 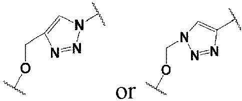 , q2 is not 0, and if L^{1b} is 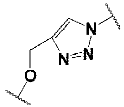 or 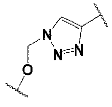 , q3 is not 0. In one embodiment, if L^{1a} and/or L^{1b} are a direct bond and q2 and q3 are 0, then a linker structure of L¹¹ and L¹², such as a C₁-C₂₀₀ alkylene optionally comprising a divalent or multivalent functional group selected from amide, sulfonamide, amino, ether, carbonyl, triazole, tetrazole, sugar-derived group, sulfoester and dendrimer in the middle of the chain may be directly bound to the central N atom.

In one embodiment, q1 may be an integer from 0 to 8, an integer from 1 to 8, or an integer from 1 to 6. In one embodiment, q2 and q3 may be each independently an integer from 0 to 8, an integer from 0 to 6, or an integer from 0 to 4. In one embodiment, q4 may be an integer from 1 to 8, an integer from 1 to 6, or an integer from 1 to 4.

In one embodiment, 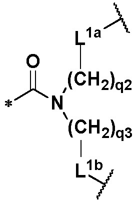 in Formula 1-1 above may be selected from the following structures: 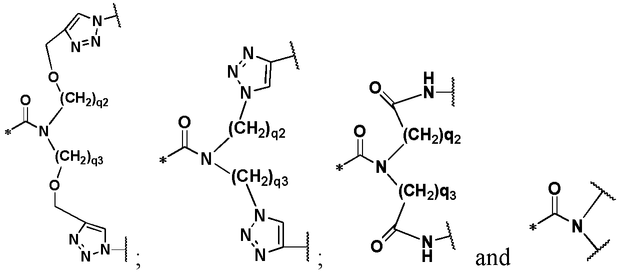 (where the definitions of q2 and q3 are as described above.)

In one embodiment, A in Formula 1-1 above may comprise or consist of a functional group selected from the group consisting of halogen, OH, C₁-C₈ alkoxy, hydroxylamino, COH, C₁-C₈ alkylcarbonyl, carboxy, C₁-C₈ alkoxycarbonyl, tosyl, tosylate, amino, mono-C₁-C₈ alkylamino, di-C₁-C₈ alkylamino, NHNH₂, N₃, haloacetamide, maleimidyl, succinimidyl, SH, SO₃H, C₁-C₈ alkylsulfonyl, 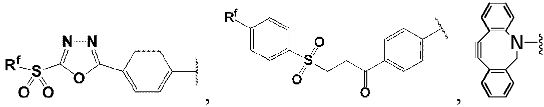 , C₁-C₈ alkoxysulfonyl, 2-pyridyl disulfide, PO₃H₂, OPO₃H₂, -N≡C, -NCS, C₄-C₁₀ dienyl, C₂-C₈ alkenyl, C₂-C₈ alkynyl, C₄-C₁₀ cycloalkynyl and C₂-C₈ alkynylcarbonyl. R^{f} may be each independently H or C₁-C₈ alkyl. For example, in Formula 1-1, A may be maleimidyl, hydroxylamino, carboxy, amino, N₃, C₂-C₈ alkynyl, or

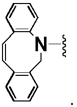

In one embodiment, L¹¹ and L¹² may be each independently a C₁-C₂₀₀ alkylene optionally comprising a divalent or multivalent functional group selected from the group consisting of amide, sulfonamide, amino, ether, carbonyl, triazole, tetrazole, sugar-derived group, sulfo ester and dendrimer in the middle of the chain. The C₁-C₂₀₀ alkylene group may be a C₁-C₁₅₀ alkylene group, a C₁-C₁₀₀ alkylene group, a C₁-C₈₀ alkylene group, a C₁-C₆₀ alkylene group, a C₁-C₅₀ alkylene group, a C₁-C₄₀ alkylene group, a C₁-C₃₀ alkylene group, a C₁-C₂₀ alkylene group or a C₁-C₁₀ alkylene group.

In one embodiment, L¹¹ and L¹² may each independently include any one selected from the group consisting of -(CH₂)ₙₐ-; -(CH₂CH₂O)ₘₐ-; -(CH₂OCH₂)_{mb}-; -(OCH₂CH₂)_{mc}-; -C(=O)-; 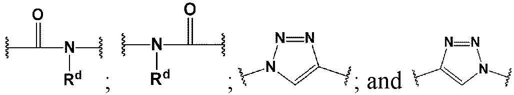 , or a combination thereof. Here, R^{d}, na, ma to mc are as described above with respect to Formula 1.

In one embodiment, L¹¹ and L¹² may be each independently selected from -(CH₂)ₙ₁-; - (CH₂CH₂O)ₘ₁-(CH₂)ₙ₂-; -(CH₂CH₂O)ₘ₂-(CH₂)ₙ₃-NR^{d1}CO-(CH₂)ₙ₄-; -(CH₂CH₂O)ₘ₁₀-(CH₂)ₙ₁₅-CONR^{d5}-(CH₂)ₙ₁₄-; and 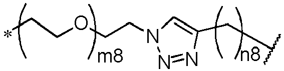 . Here, n1, n2, n3, n4, n8, n14 and n15, and m1, m2, m8 and m10 may be each independently an integer from 1 to 8, an integer from 1 to 6 or an integer from 1 to 5. In addition, R^{d1} and R^{d5} are each independently H or C₁-C₈ alkyl.

In one embodiment, the compound represented by Formula 1-1 (where j is 1) may be a compound represented by the following formula:

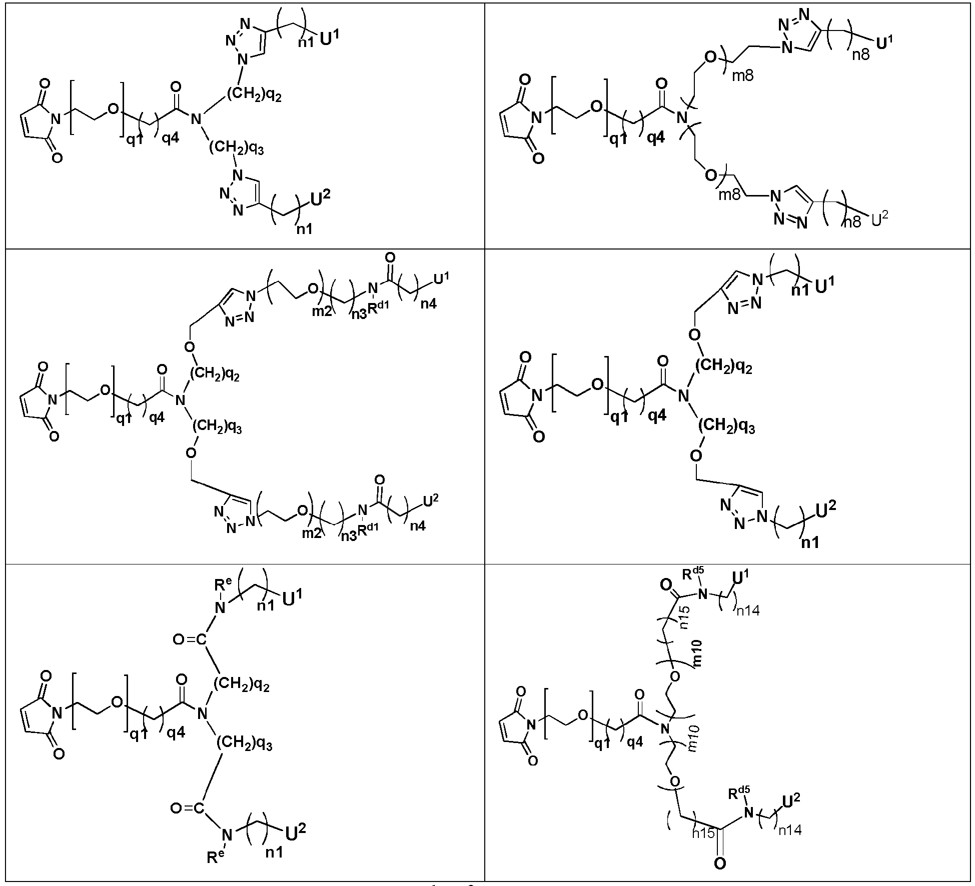

(in the above formulas, q1 to q4, U¹, U², n1, n3, n4, n8, n14 and n15, and m2, m8 and m10 are as described above with respect to Formula 1-1. In addition, R^{d1}, R^{d5} and R^{e} are each independently H or C₁-C₈ alkyl.)

In one embodiment, the compound represented by Formula 1-1 may be a compound represented by the following formula:

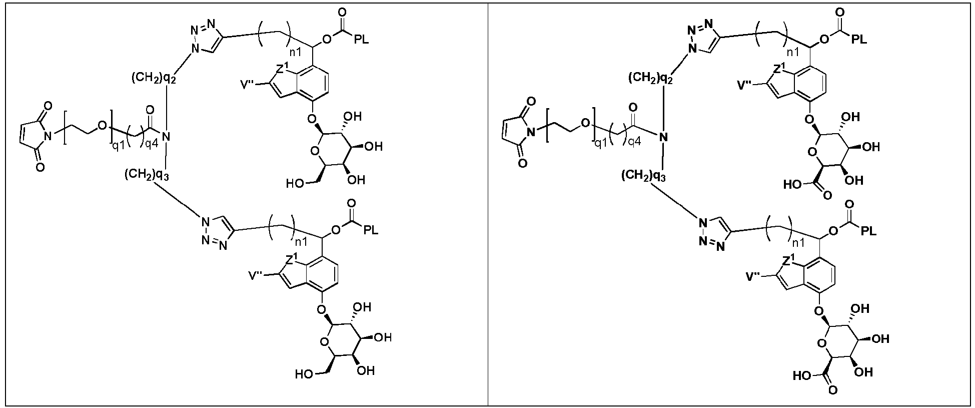

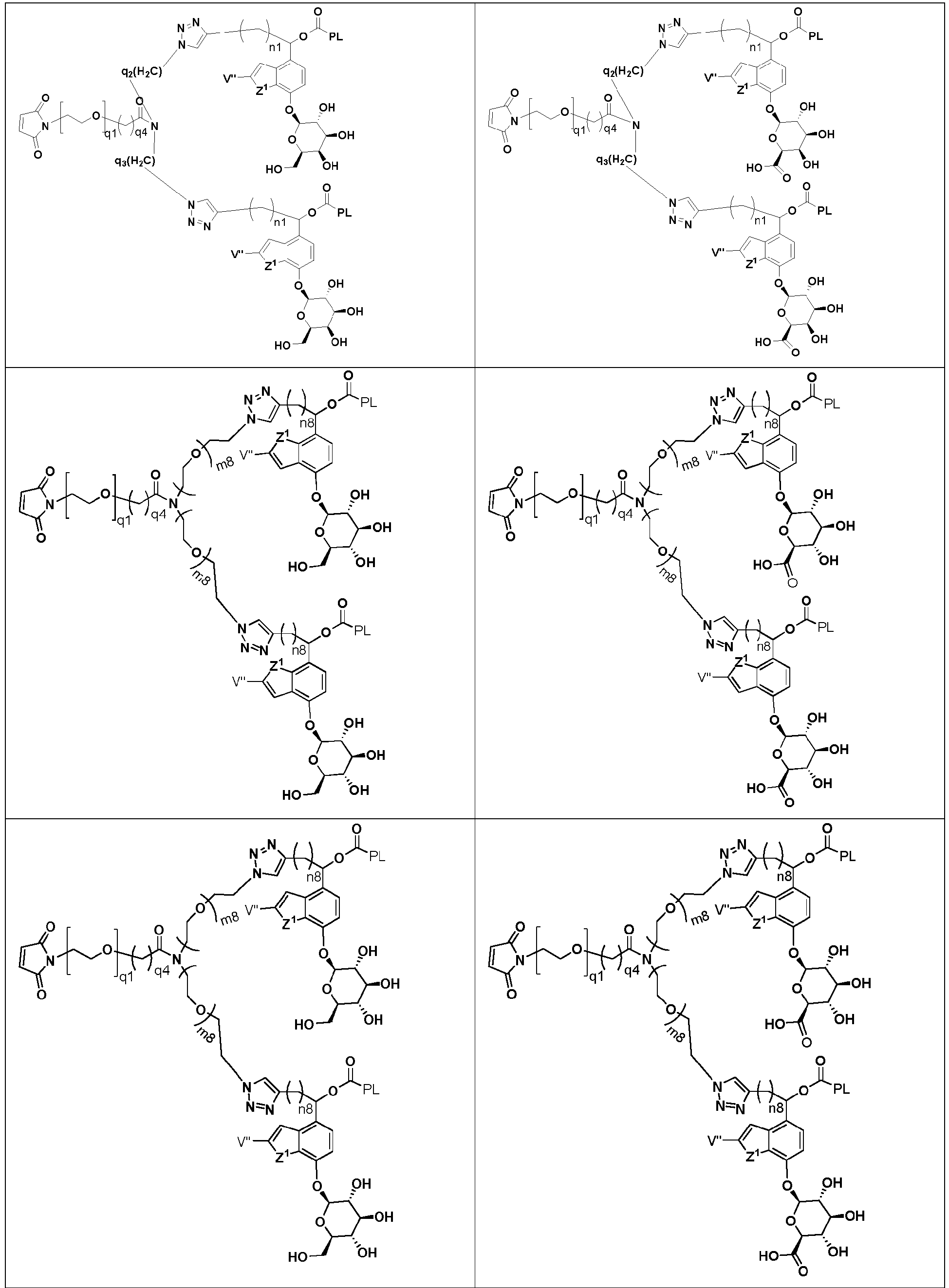

In the above formulas, q1 to q4, n1, n8 and m8 are as described above with respect to Formula 1-1. In the above formulas, PL has the same meaning as PL in Formula 1. In the above formulas, Z¹ is a heteroatom selected from NR³, O, S and Se. R³ is H or C₁-C₈ hydrocarbyl. In the above formulas, V" indicates an electron withdrawing group. For example, V" may be carboxy, carboxy-C₁-C₈ alkyl, or C₁-C₈ alkoxycarbonyl. For example, the V" may be carboxy, carboxymethyl, carboxyethyl, carboxypropyl, methoxycarbonyl, ethoxycarbonyl, or propoxycarbonyl. In the above formulas, the -O-CO- group in the -O-CO-PL group is an optional self-immolative spacer group, and may be absent (i.e., the -PL group is directly linked to the -CH₂-group) or may be replaced with the functional groups described above with respect to an "optional self-immolative spacer group".

In one embodiment, the compound represented by Formula 1-1 may be selected from the compounds listed in Table C attached.

### Method for preparing a compound of Formula 1

The novel linker compound of Formula 1 according to the present invention can be easily prepared by selecting an appropriate solvent, starting material, intermediate, reaction conditions, and the like based on the examples herein and the technical knowledge of those of ordinary skill in the art of organic synthesis.

In one embodiment, a compound of Formula 1 having a benzothiophene core and a β-galactoside triggering group where A-(L¹)ₖ- is propagyl (HCCCH₂-) can be prepared according to Reaction Scheme 1 below.

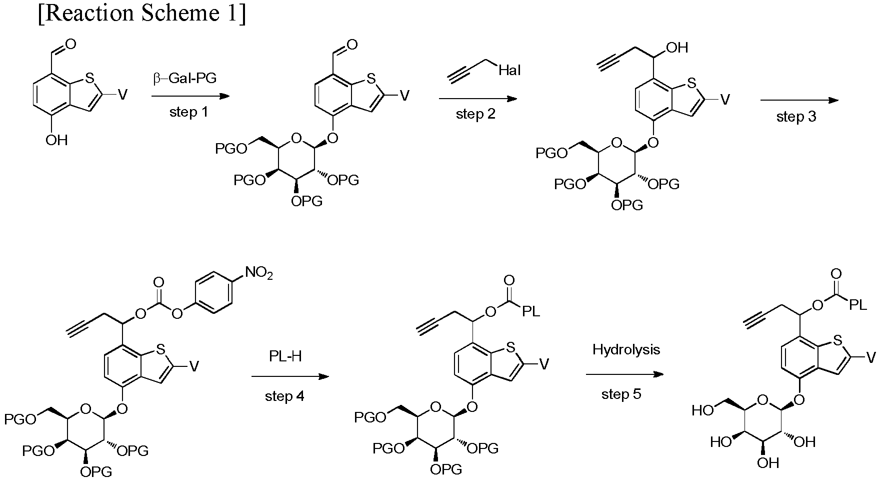

In Step 1 of Reaction Scheme 1, a benzothiophene-based starting material with COH and OH substituted at the para positions of the benzene ring may be prepared, and the starting material may be reacted with galactose where the hydroxy groups are protected with protecting groups (-PG). For example, a protected galactoside group such as an acetogalactoside group can be introduced through reaction with galactose into which a protecting group has been introduced, such as acetylated galactose or acetobromo-alpha-D-galactose. In the reaction of Step 1, additives such as benzyltributylammonium chloride, HOBt, pyridine, DIPEA, and the like may be used. In addition, additives such as silver oxide and molecular sieve may be used. Step 1 may be performed under temperature conditions of -40°C to 40°C, -40°C to 30°C, -20°C to 40°C, -20°C to 30°C, - 10°C to 40°C, or -10°C to 30°C.

In Step 2, a propargyl group may be introduced to COH attached to the benzene ring. For example, the product of Step 1 may be reacted with propargyl halide (HCCCH₂-Hal; Hal is halogen). In this case, additives such as 1,2-diiodoethane, zinc powder, and the like may be used. The reaction in Step 2 may be conducted with agitated sonication under the temperature conditions of -10°C to 40°C, -10°C to 30°C, 0°C to 40°C or 0°C to 30°C.

In Step 3, the self-eliminating linker -OCO- may be introduced. As a precursor for the self-eliminating linker -OCO-, bis(4-nitrophenyl) carbonate, 4-nitrophenyl chloroformate, and the like may be used. In the reaction of Step 3, additives such as DIPEA, pyridine, and the like may be used. Step 3 may be performed under low temperature conditions of -40°C to 10°C, -40°C to 0°C, -30°C to 10°C, -30°C to 0°C, -20°C to 10°C or -20°C to 0°C.

In Step 4, PL may be introduced. For example, PL may be introduced by replacing a precursor such as PL-H for a leaving group (e.g., p-nitrophenyl) linked to -OCO-. In the reaction of Step 4, additives such as HOBt, pyridine, DIPEA, and the like may be used depending on the type of PL. Step 4 may be performed under low temperature conditions of -40°C to 10°C, -40°C to 0°C, -30°C to 10°C, -30°C to 0°C, -20°C to 10°C or -20°C to 0°C.

In Step 5, the protecting group of the protected galactoside can be deprotected through hydrolysis, thereby converting it to galactoside. Hydrolysis may be performed with an acid such as hydrochloric acid or a base such as potassium carbonate, sodium hydroxide, or lithium hydroxide. Step 5 may be performed under temperature conditions of -40°C to 40°C, -40°C to 30°C, -20°C to 40°C, -20°C to 30°C, -10°C to 40°C, or -10°C to 30°C.

Each step in Reaction Scheme 1 may be performed in an appropriate solvent selected from the group consisting of organic solvents such as methanol, DMF, MC, ACN, THF, EA, and distilled water. In addition, after the completion of the reaction at each step, the product may be purified through dilution, extraction, and chromatography using the appropriate solvent. In one embodiment, each step of Reaction Scheme 1 may be performed under a nitrogen atmosphere.

In one embodiment, depending on PL, A, L¹ and V, some steps in Reaction Scheme 1 may be omitted. For example, if PL can react with a hydroxymethyl group directly linked to the benzene ring even in the absence of -OCO- or can be bound to a methyl group, Step 3 may be omitted.

### Linker Compound of Formula B

In one aspect of the present invention, there is provided a novel linker compound represented by Formula B below, or a pharmaceutically acceptable salt thereof:

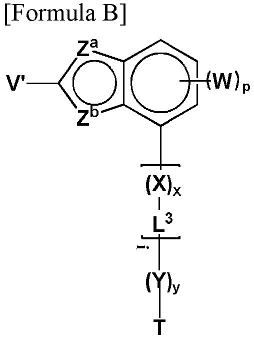

In the above Formula B, V' is -CH₂-(L²)₁-PL.

In the above Formula B, L² is a self-eliminating linker selected such that cleavage of the bond between the -CH₂- of V' and L² promotes cleavage of the bond between L² and PL.

In the above Formula B, PL is an active agent linked to L² or -CH₂- of V' via a heteroatom selected from N, O and S.

In the above Formula B, one of Z^{a} and Z^{b} is selected from the group consisting of N, NR³, O, S and Se, the other one of Z^{a} and Z^{b} is CH or N, R³ is H or C₁-C₈ hydrocarbyl.

In the above Formula B, T is a triggering group capable of initiating the release of PL and L₂, if present, through a 1,6-elimination reaction upon cleavage.

In the above Formula B, L³ is an optional self-immolative spacer group that, if present, is cleaved sequentially upon cleavage of T.

In the above Formula B, X and Y are each independently selected from -O-, -NH-, and - S-, and i, l, x and y are each independently 0 or 1, and p is an integer of 0 to 2.

The particular embodiments of L₂, PL, X, Y, L³ and T in the above Formula B are as described above with respect to Formula 1, the matters therefor described above with respect to Formula 1 may be equally applied to Formula B, if applicable.

In one embodiment, the compound represented by the above Formula B may be selected from the group consisting of the compounds represented by the following formulas:

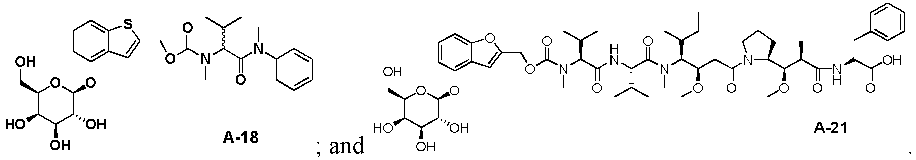

The novel linker compound represented by the above Formula B is a complex comprising an active agent (PL) that is not supposed to bind to a ligand having a receptor binding property. The complex can be used for various purposes, such as changing the properties (e.g., water solubility) of the active agent, targeting, and the like.

### Ligand-drug conjugate represented by Formula 2

In one aspect of the present invention, there is provided a ligand-drug conjugate represented by Formula 2 below:

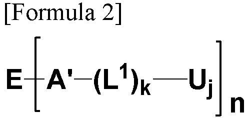

In Formula 2 of the present invention, E is a ligand or protein having a receptor binding property.

The compound of Formula 1 according to the present invention can be bound to a ligand or protein (E) having a receptor binding property to provide a ligand-drug complex of Formula 2. The ligand or protein (E) may be optionally modified for binding to the binding functional group of the compound of Formula 1. In one embodiment, when Ligand E is an antibody, a moiety such as -SH of cysteine, -NH₂ of lysine, -C(=O)NH₂ of glutamine, -C₆H₄-OH of tyrosine, -SeH of selenocysteine , and -N₃ and 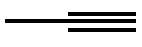 of unnatural amino acids present at specific positions of the antibody and the binding functional group of the compound of Formula 1 may be bound to each other. In the field of ligand-drug complexes, the types and modifications of functional groups of a ligand for binding a linker-drug moiety to a ligand or protein are known in the art.

The ligand may be selected from the group consisting of peptides, tumor cell-specific peptides, tumor cell-specific aptamers, tumor cell-specific carbohydrates, tumor cell-specific monoclonal or polyclonal antibodies, and antibody fragments.

In one embodiment, the protein may be selected from the group consisting of C₁-C₂₀ hydrocarbyl, oligopeptide, polypeptide, antibody, fragment of antigenic polypeptide, and artificial antibody (Repebody). In one embodiment, the C-terminus of the protein may be the light or heavy chain of an antibody.

In one embodiment, the antibody may be selected from the group consisting of an intact polyclonal antibody, an intact monoclonal antibody, an antibody fragment, a single chain Fv (scFv) mutant, a multispecific antibody, a bispecific antibody, a chimeric antibody, a humanized antibody, a human antibody, a fusion protein comprising an antigen determination portion of an antibody, and modified immunoglobulin molecules comprising an antigen recognition site.

In one embodiment, the antibody may be selected from the group consisting of Muromonab-CD3, Abciximab, Rituximab, Daclizumab, Palivizumab, Infliximab, Trastuzumab (herceptin), Etanercept, Basiliximab, Gemtuzumab, Alemtuzumab, Ibritumomab, Adalimumab, Alefacept, Omalizumab, Efalizumab, Tositumomob-I131, Cetuximab, Bevacizumab, Natalizumab, Ranibizumab, Panitumumab, Eculizumab, Rilonacept, Certolizumab pegol, Romiplostim, AMG-531 (Romiplostim), CNTO-148 (Golimumab), CNTO-1275 (Ustekinumab), ABT874 (Briakinumab), LEA-29Y (Belatacept), Belimumab, TACI-Ig (transmembrane active agent and calcium modulator and cyclophilin ligand interactor-immunoglobulin), second generation anti-CD20, ACZ-885 (Canakinumab), Tocilizumab, Atlizumab, Mepolizumab, Pertuzumab, Humax CD20 (Ofatumumab), Tremelimumab (CP-675 206), Ticilimumab, MDX-010 (Ipilimumab), IDEC-114 (Galiximab), Inotuzumab, HuMax EGFR (Zalutumumab), Aflibercept (VEGF Trap-Eye), HuMax-CD4 (Zanolimumab), Ala-Ala (hOKT3gamma1), Otelixizumab (ChAglyCD3; TRX4), Catumaxomab, MT-201 (Adecatumumab), Pregovomab, CH-14.18 (Dinutuximab), WXG250 (Girentuximab), AMG-162 (Denosumab), AAB-001 (Bapineuzumab), Motavizumab, MEDI524 (Motavizumab), Efumgumab, Aurograb^{®}, Raxibacumab, third generation anti-CD20, LY2469298 (Ocaratuzumab), and Veltuzumab.

In one embodiment, the antibody may be a monoclonal antibody (mAb).

In Formula 2 above, A' is a divalent linking group derived from the binding functional group (A) of Formula 1. For example, A' may include a functional group formed by an addition reaction of a double bond included in the binding functional group. In one embodiment, when the binding functional group is maleimidyl, A' may be a functional group formed by participating in an addition reaction of a double bond in the 5-membered ring of the maleimidyl. The matters regarding the binding functional group (A) of Formula 1 described above may be equally applied to A', if applicable.

In one embodiment, Ligand E may be an antibody. For example, the antibody may include a functional group that binds to A in Formula 1 to form the E-A' binding structure in Formula 2. If necessary, a functional group for the above binding can be introduced into the antibody, and the types of such functional groups and methods of introducing the functional groups are known in the art.

In one embodiment, the binding structure of Ligand E and A' may be represented by a moiety represented by one of the following formulas. In the formulas below, * may be the remaining moiety of the antibody. In the formulas below, the S, NH, CONH, C₆H₃-OH, Se and triazole ring moieties directly linked to * may be derived from a moiety such as -SH of cysteine, - NH₂ of lysine, -C(=O)NH₂ of glutamine, -C₆H₄-OH of tyrosine, -SeH of selenocysteine , and -N₃ and 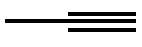 of unnatural amino acids present at specific positions of Ligand E.

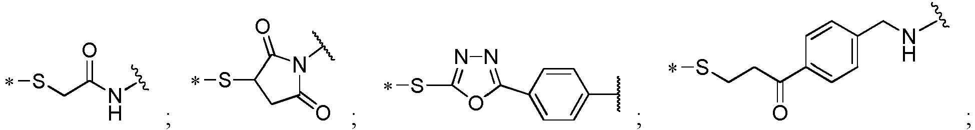

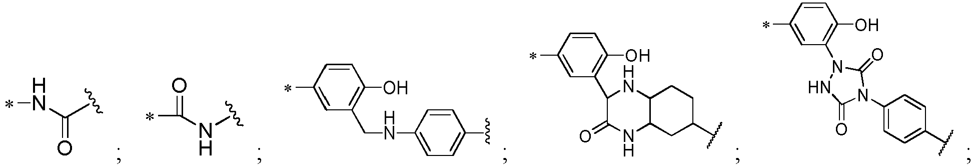

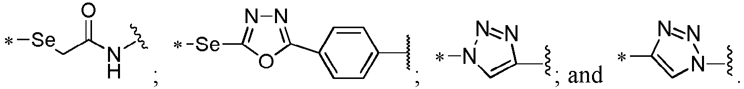

In Formula 2, n may be a real number from 1 to 10. For example, n may be a real number from 1 to 6, a real number from 1 to 4, or a real number from 1 to 2.

In Formula 2, U, L¹, k and j may be the same as U, L¹, k and j in Formula 1. The matters regarding U, L¹, k and j in Formula 1 described above may be equally applied to U, L¹, k and j in Formula 2, if applicable. In one embodiment, when E of Formula 2 above is an antibody and PL of U is a drug, the compound represented by Formula 2 may be provided as an antibody-drug conjugate.

In one embodiment, the compound represented by the above Formula 2 may be selected from the group consisting of the compounds represented by the following formulas:

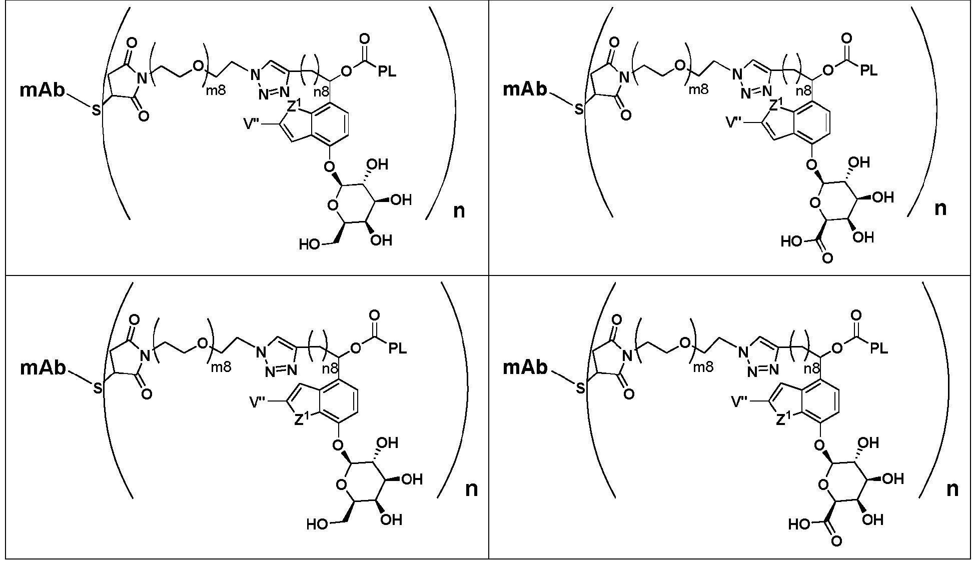

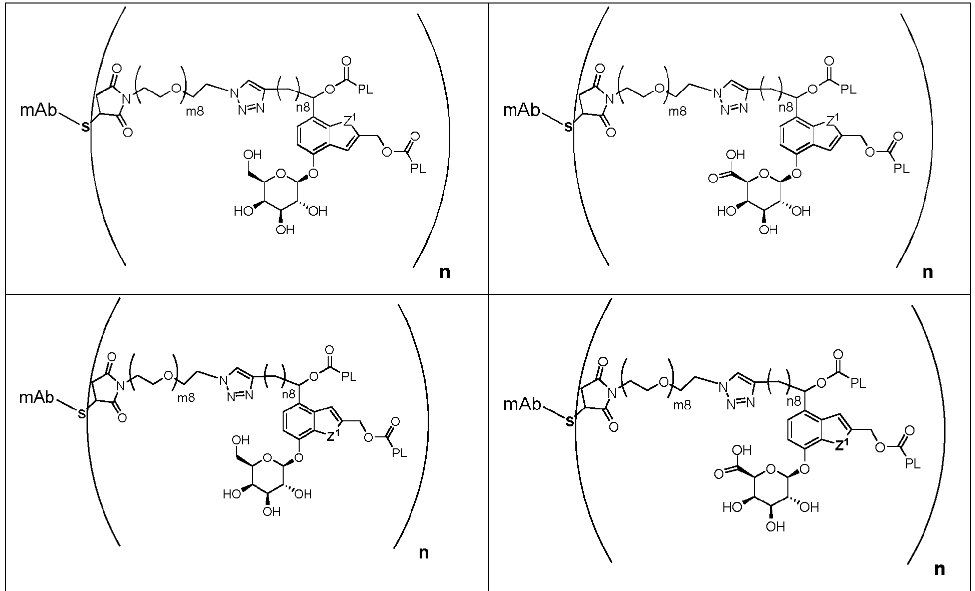

In the above formulas, mAb represents the moiety of the antibody. In the above formulas, m8 and n8 may be each independently an integer from 1 to 10. In one embodiment, m8 and n8 may be each independently an integer from 1 to 8, an integer from 1 to 6, an integer from 1 to 5, an integer from 1 to 4, or an integer from 1 to 3. In the above formulas, Z¹ is a heteroatom selected from NR³, O, S and Se. R³ is H or C₁-C₈ hydrocarbyl. In the above formulas, PL has the same meaning as PL in Formula 1. V" indicates an electron withdrawing group. For example, V" may be carboxy, carboxy-C₁-C₈ alkyl, or C₁-C₈ alkoxycarbonyl. For example, the V" may be carboxy, carboxymethyl, carboxyethyl, carboxypropyl, methoxycarbonyl, ethoxycarbonyl, or propoxycarbonyl. In the above formulas, the -O-CO- group in the -O-CO-PL group is an optional self-immolative spacer group, and may be absent (i.e., the -PL group is directly linked to the -CH₂-group) or may be replaced with -S(=O)₂-, 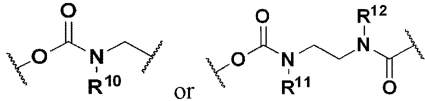 (in the above formulas, R¹⁰ to R¹² are each independently H, C₁-C₈ alkyl, amino-C₁-C₈ alkyl, C₁-C₈ alkyl substituted with mono- or di-(C₁-C₈ alkyl)amino, or -(CH₂CH₂O)_{g}R¹³, R¹³ is H or C₁-C₄ alkyl, and g is an integer from 1 to 10.)

In the above formulas, n is a real number from 1 to 10. In one embodiment, n may be a real number from 1 to 8, a real number from 1 to 6, a real number from 1 to 4, or a real number from 1 to 2.

In one embodiment, the conjugate represented by Formula 2 may be selected from the group consisting of conjugates represented by the following formulas:

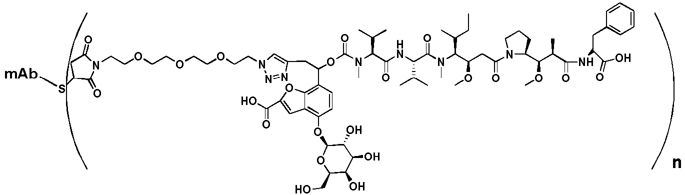

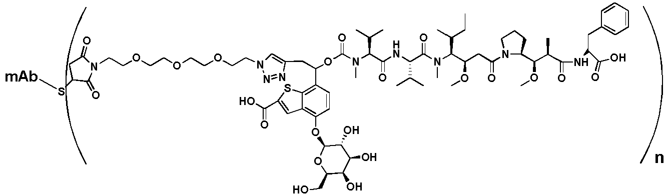

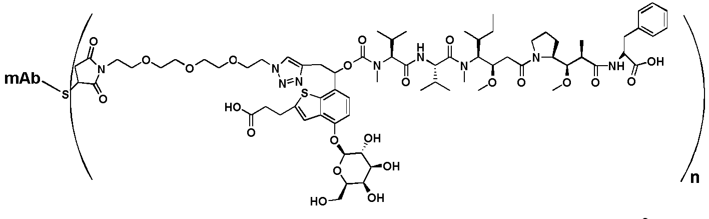

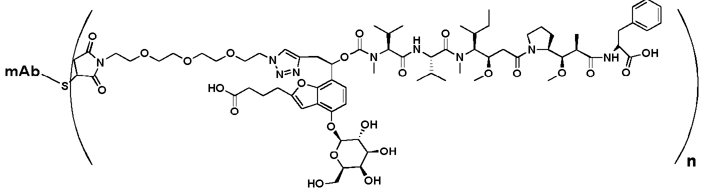

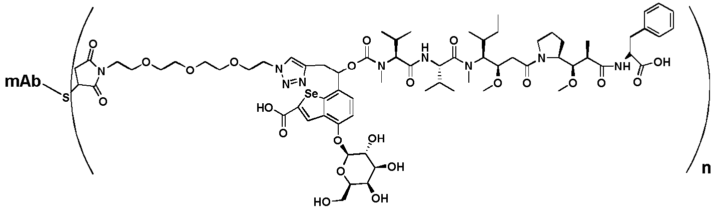

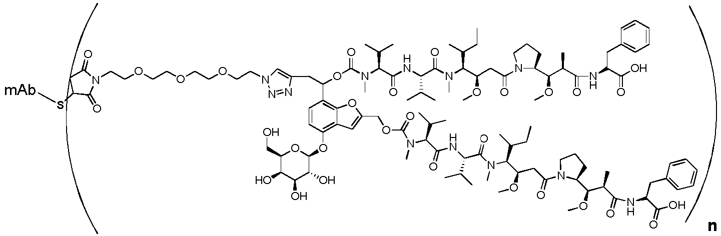

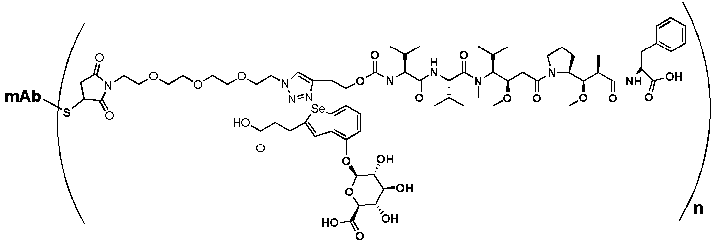

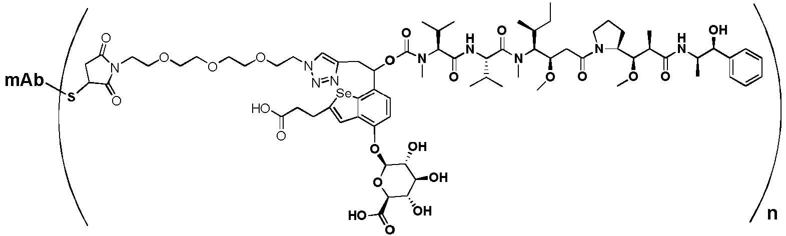

In the above formulas, mAb represents the moiety of the antibody. In the above formulas, n is a real number from 1 to 10. In one embodiment, n may be a real number from 1 to 8, a real number from 1 to 6, a real number from 1 to 4, or a real number from 1 to 2.

### Exemplary DAR2 type ligand-drug conjugates

In one embodiment, the compound represented by Formula 2 may be selected from the compounds listed in Table D attached. The specific n values listed in Table D may vary in the real number range of 1 to 10. In one embodiment, n may be a real number from 1 to 8, a real number from 1 to 6, a real number from 1 to 4, or a real number from 1 to 2.

### DAR4 type ligand-drug conjugate (Formula 2-1)

In one embodiment, when the linking group L¹ of Formula 2 according to the present invention has a branched structure or a dendrimeric structure (i.e., when L¹ is a multivalent linking group), a ligand-drug conjugate of Formula 2 to which a plurality of active agents are bound can be provided. In one embodiment, the compound represented by Formula 2 may be a ligand-drug conjugate represented by Formula 2-1 below:

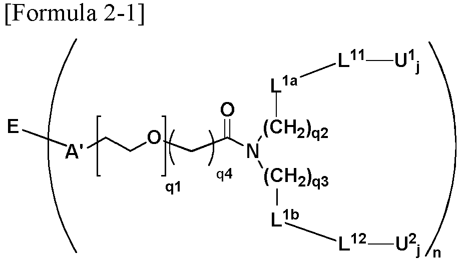

In Formula 2-1, E is a ligand or protein having a receptor binding property, and A' is a divalent linking group derived from the binding functional group (A) of Formula 1. With respect to E and A', the matters described in Formula 2 may also be equally applied to Formula 2-1.

In Formula 2-1, U¹ and U² each have the same meaning as U in Formula 1, wherein U¹ and U² may be the same or different from each other. In addition, L¹¹ and L¹² each have the same meaning as L¹ in Formula 1, wherein L¹¹ and L¹² may be the same or different from each other.

In Formula 2-1, j is 1 to 10. In one embodiment, j is 1 to 5. For example, j is 1.

With respect to Formula A, L¹ and U, the matters described in Formula 1 may be equally applied to A, U¹ and U², and L¹¹ and L¹² in Formula 2-1, respectively. In addition, with respect to U¹ and U², and L¹¹ and L¹², the matters described in Formula 1-1 may be equally applied in Formula 2-1, respectively.

In Formula 2-1, L^{1a} and L^{1b} may be each independently selected from a direct bond; 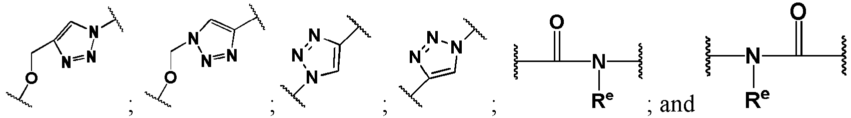 . In this case, R^{e} may be H or C₁-C₈ alkyl.

In Formula 2-1, q1, q2 and q3 may be each independently an integer from 0 to 10. In Formula 2-1, q4 may be an integer from 1 to 10. Further, if L^{1a} is 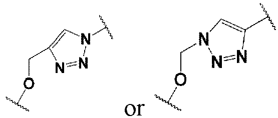 , q2 is not 0, and if L^{1b} is 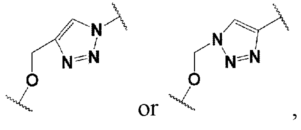 q3 is not 0.

In one embodiment, q1 may be an integer from 0 to 8, an integer from 1 to 8, or an integer from 1 to 6. In one embodiment, q2 and q3 may be each independently an integer from 0 to 8, an integer from 0 to 6, or an integer from 0 to 4. In one embodiment, q4 may be an integer from 1 to 8, an integer from 1 to 6, or an integer from 1 to 4.

With respect to L^{1a} and L^{1b}, q1, q2, q3 and q4, the matters described in Formula 1-1 may be equally applied in Formula 2-1, respectively.

In Formula 2-1, n is a real number from 1 to 10. In one embodiment, n may be a real number from 1 to 6, a real number from 1 to 4, or a real number from 1 to 2.

In one embodiment, the ligand-drug conjugate represented by Formula 2-1 may be conjugates represented by the following formulas:

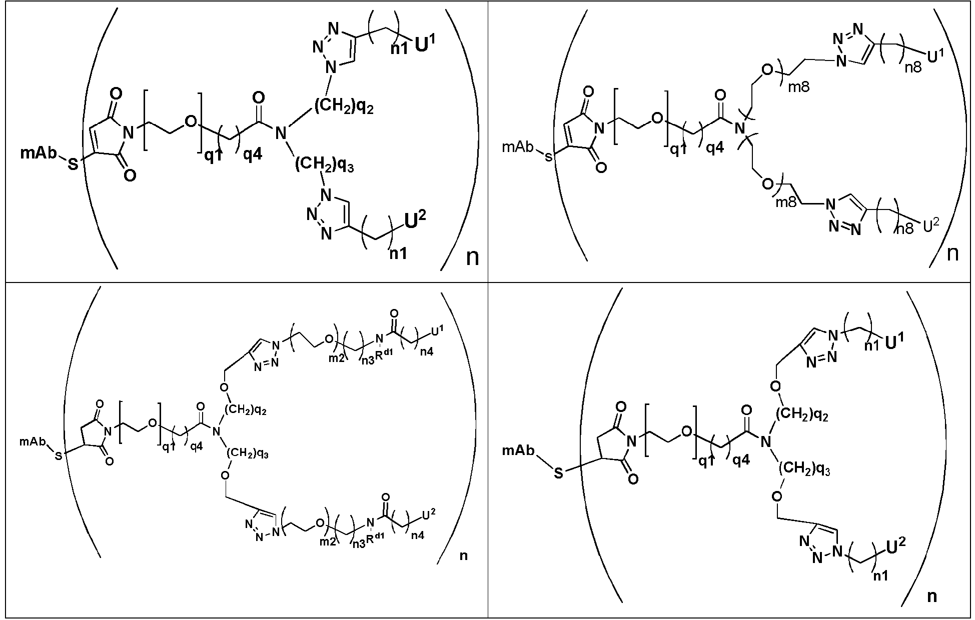

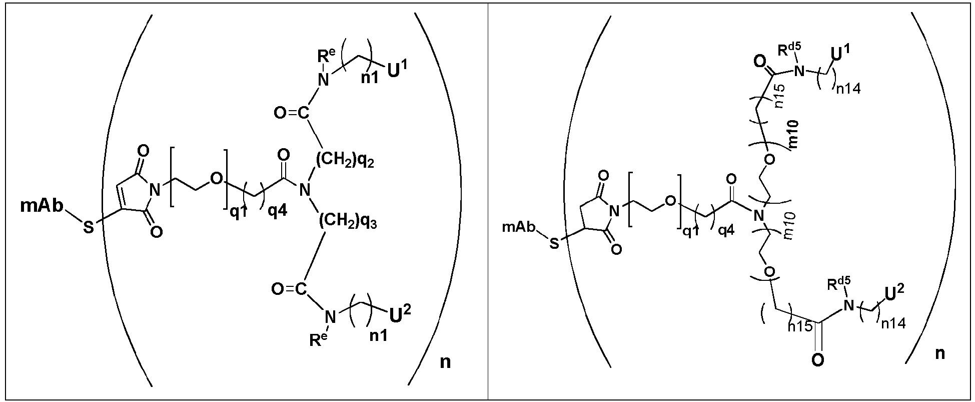

(In the above formulas, mAb is the moiety of the antibody. In the above formulas, q1 to q4, n, U¹ and U², R^{d1}, R^{d5}, R^{e}, n1, n3, n4, n8, n14 and n15, m2, m8 and m10 are as described above with respect to Formula 1-1 or Formula 2-1.)

In one embodiment, the ligand-drug conjugate represented by Formula 2-1 may be conjugates represented by the following formulas:

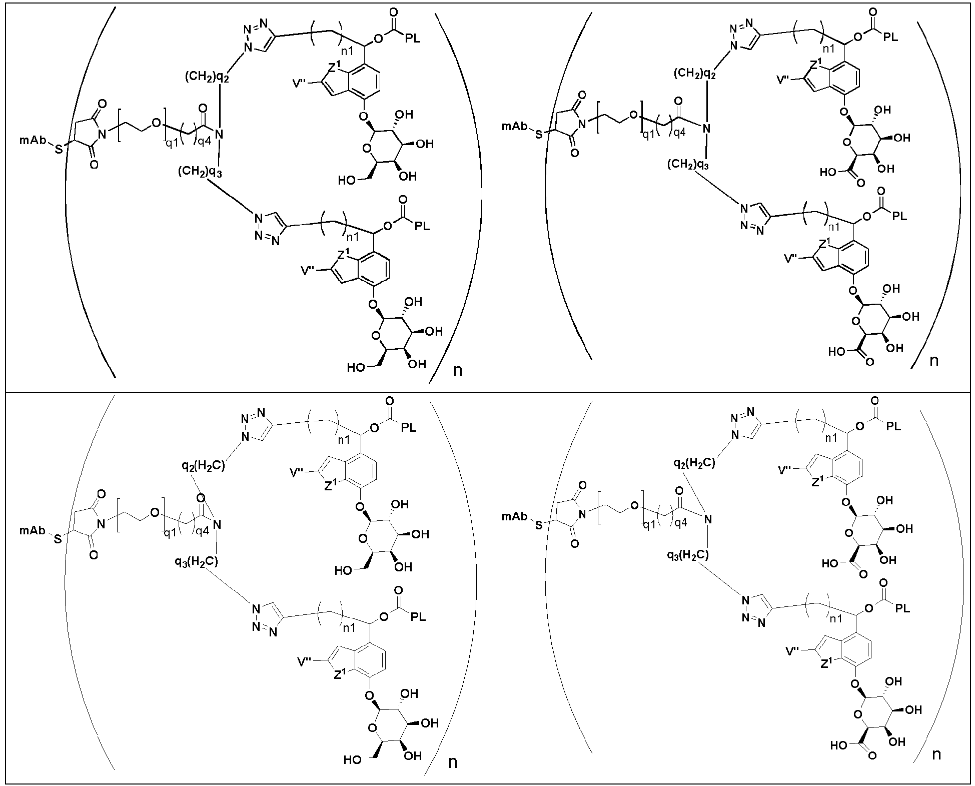

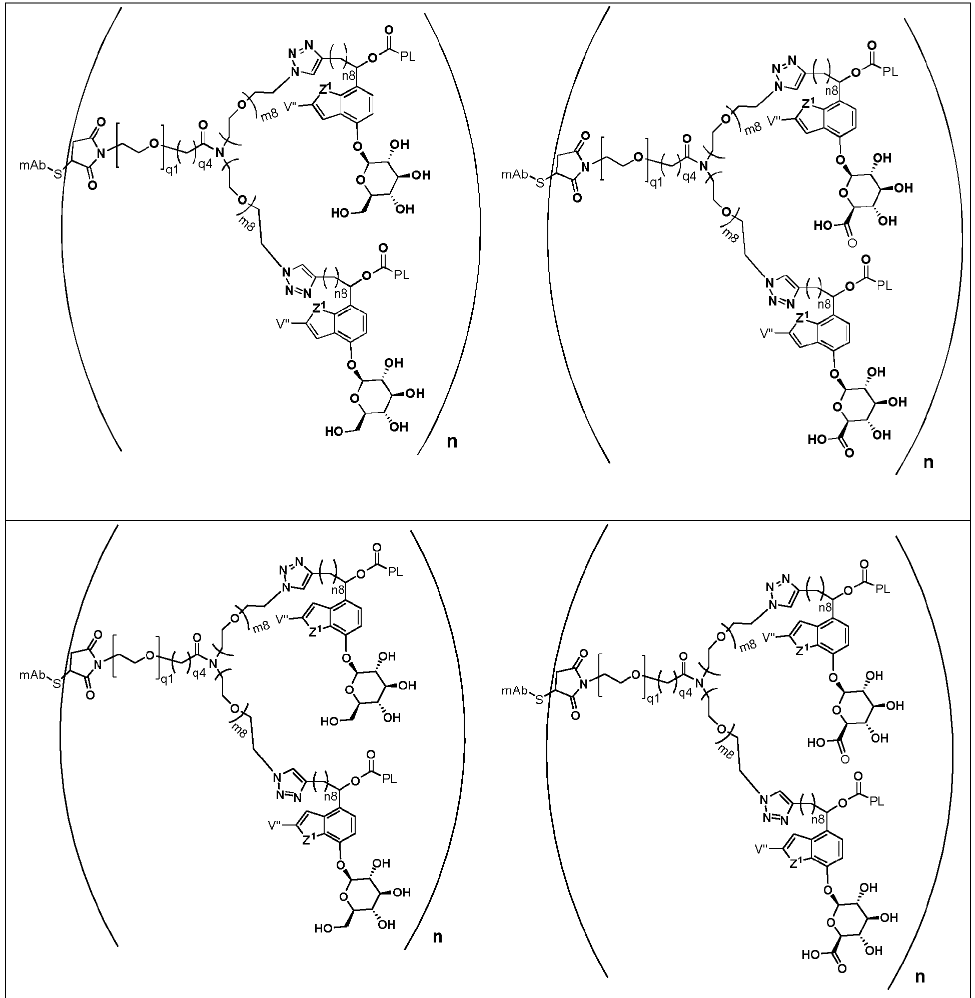

In the above formulas, mAb is the moiety of the antibody. In the above formulas, q1 to q4, n1, n8 and m8 are as described above with respect to Formula 1-1. In the above formulas, PL has the same meaning as PL in Formula 1. In the above formulas, Z¹ is a heteroatom selected from NR³, O, S and Se. R³ is H or C₁-C₈ hydrocarbyl. In the above formulas, V" indicates an electron withdrawing group. For example, V" may be carboxy, carboxy-C₁-C₈ alkyl, or C₁-C₈ alkoxycarbonyl. For example, the V" may be carboxy, carboxymethyl, carboxyethyl, carboxypropyl, methoxycarbonyl, ethoxycarbonyl, or propoxycarbonyl. In the above formulas, the -O-CO- group in the -O-CO-PL group is an optional self-immolative spacer group, and may be absent (i.e., the -PL group is directly linked to the -CH₂- group) or may be replaced with the functional groups described above with respect to an "optional self-immolative spacer group".

In one embodiment, the ligand-drug conjugate represented by Formula 2-1 may be selected from the compounds listed in Table E attached. The specific n values listed in Table E may vary in the real number range of 1 to 10. For example, n may be a real number from 1 to 6, a real number from 1 to 4, or a real number from 1 to 2.

### Definition

As used herein, the term "hydrocarbyl" refers to a functional group consisting of carbon and hydrogen, and refers to a saturated, partially unsaturated or fully unsaturated straight-chain, branched or cyclic hydrocarbon. The hydrocarbyl may include alkyl, alkenyl, alkynyl, cycloalkyl, cycloalkenyl, cycloalkynyl, and the like. Non-limiting examples of the hydrocarbyl may include methyl, ethyl, propyl, butyl, ethenyl, propenyl, butenyl, ethynyl, propynyl, butynyl, and the like.

The term "alkyl" refers to a fully saturated branched or unbranched (or straight-chain or linear) hydrocarbon. The alkyl may be substituted or unsubstituted alkyl. The C₁-C₈ alkyl may be C₁ to C₆, C₁ to C₅, C₁ to C₄, C₁ to C₃, or C₁ to C₂ alkyl. Non-limiting examples of the alkyl may include methyl, ethyl, n-propyl, isopropyl, n-butyl, isobutyl, sec-butyl, n-pentyl, isopentyl, neopentyl, iso-amyl, or n-hexyl.

The term "alkenyl" includes straight-chain or branched alkenyl having 2 to 6 carbon atoms, 2 to 5 carbon atoms, or 2 to 4 carbon atoms with one or more double bonds at any position. For example, the alkenyl may include vinyl, propenyl, isopropenyl, butenyl, isobutenyl, prenyl, butadienyl, pentenyl, isopentenyl, pentadienyl, hexenyl, isohexenyl, hexadienyl, and the like.

The term "alkynyl" refers to a straight or branched hydrocarbon chain having at least one triple bond. The alkynyl is preferably a straight or branched chain having 2 to 8 carbon atoms, and examples of the alkynyl may include 2-propynyl, 3-butynyl, 2-butynyl, 4-pentynyl, 3-pentynyl, 2-hexynyl, 3-hexynyl, 2-heptynyl, 3-heptynyl, 4-heptynyl, 3-octynyl, and the like.

The term "alkoxy" refers to alkyl bound to an oxygen atom. For example, the C₁ to C₈ alkoxy may be C₁ to C₆, C₁ to C₅, C₁ to C₄, C₁ to C₃, or C₁ to C₂ alkoxy. The alkoxy may be methoxy, ethoxy, or propoxy.

The term "cycloalkyl" includes monocyclic or polycyclic saturated carbocycles containing 3 to 8 carbon atoms. Examples may include cyclopropyl, cyclobutyl, cyclopentyl, cyclohexyl, cycloheptyl, cyclooctyl, and the like.

The term "cycloalkenyl" includes non-aromatic monocyclic or polycyclic rings having 3 to 8 carbon atoms and containing at least one carbon-carbon double bond. Examples may include cyclopentenyl, cyclohexenyl, cycloheptenyl, cyclooctenyl, and the like.

The term "cycloalkynyl" refers to a monocyclic or polycyclic unsaturated hydrocarbon ring having 4 to 10 carbon atoms and containing at least one triple bond. Examples may include monocyclic alkynyl groups, such as cyclooctynyl group and cyclodecynyl group. "Cycloalkynyl" in its broadest sense includes structures in which one or more carbon atoms of a hydrocarbon ring containing at least one triple bond between carbon atoms are replaced by a heteroatom, such as N.

The term "dienyl" refers to an unsaturated branched or unbranched C₄-C₁₀ aliphatic substituent having two double bonds between two adjacent carbon atoms. Examples include 2,4-pentadienyl, 2,4-hexadienyl, 4-methyl-2,4-pentadienyl, and the like, but are not limited thereto.

The term "halogen" atom refers to an atom belonging to group 17 of the periodic table. The halogen atom includes fluorine, chlorine, bromine, and iodine, etc.

The term "haloalkyl" refers to alkyl substituted with one or more halogen atoms.

The term "hydroxy" refers to the OH functional group (hydroxyl group).

The term "mercapto" refers to the SH functional group.

The term "cyano" is CN and refers to a functional group consisting of a triple bond between a carbon atom and a nitrogen atom.

The term "oxo" refers to =O, and "substituted with oxo" means that the carbon atom has a =O substituent in the form of -C(=O)-.

The term "nitro" refers to NO₂.

The term "amino" refers to -NH₂.

The term "alkylamino" refers to a functional group in which one or two hydrogen atoms of amino (-NH₂) are substituted with one or two of the alkyls mentioned above, and includes both mono-alkylamino and di-alkylamino, and the two alkyls in di-alkylamino may be the same or different. Specifically, mono-C₁-C₈ alkylamino may be one in which one hydrogen atom of the amino (-NH₂) group is substituted with C₁-C₈ alkyl, and di-C₁-C₈ alkylamino may be one in which two hydrogen atoms of the amino (-NH₂) group are substituted with the same or different C₁-C₈ alkyl. For example, mono-C₁-C₈ alkylamino (-NH(C₁-C₈ alkyl)) may include methylamino, ethylamino, propylamino, isopropylamino, butylamino, isobutylamino, sec-butylamino, tert-butylamino, pentylamino, hexylamino, and the like. Di-C₁-C₈ alkylamino (-N(C₁-C₈ alkyl)₂) may include, for example, dimethylamino, diethylamino, dipropylamino, methylethylamino, methylpropylamino, methylisopropylamino, methylbutylamino, methylisobutylamino, ethylpropylamino, ethylisopropylamino, ethylisobutylamino, isopropylisobutylamino, methylhexylamino, ethylhexylamino, and the like.

The term "carboxy" refers to -COOH.

The term "carbamoyl" refers to -CONH₂.

The terms "N-mono-C₁-C₈ alkylcarbamoyl" and "N,N-di-C₁-C₈ alkylcarbamoyl" refer to one in which one hydrogen atom or two hydrogen atoms bound to the nitrogen atom of carbamoyl (-CONH₂) are substituted with C₁-C₈ alkyl. In N,N-di-C₁-C₈ alkylcarbamoyl, the two C₁-C₈ alkyls may be the same or different from each other.

The term "alkanoyl" refers to alkyl as defined above, having the specified number of carbon atoms attached through a carbonyl bridge (i.e., -(C=O)-alkyl). For example, alkanoyl includes methanoyl (formyl: -COH), ethanoyl (acetyl: -COCH₃), propanoyl (-COCH₂CH₃), butanoyl (-CO(CH₂)₂CH₃), and the like.

The term "alkanoylamino" refers to an amino substituted with an alkanoyl group (i.e., - NH(C=O)-alkyl). The nitrogen atom of the alkanoylamino may be substituted with an additional substituent, for example an alkyl group. For example, alkanoylamino includes formylamino (-NHCOH), acetylamino (-NHCOCH₃), propanoylamino (-NHCOCH₂CH₃), butanoylamino (-NHCO(CH₂)₂CH₃), and the like.

The terms "cyano-C₁-C₈ alkyl", "halo-C₁-C₈ alkyl", "hydroxy-C₁-C₈ alkyl", "C₁-C₈ alkoxy-C₁-C₈ alkyl", "C₁-C₈ alkoxy-C₁-C₈ alkoxy-C₁-C₈ alkyl", "carboxy-C₁-C₈ alkyl", "amino-C₁-C₈ alkyl", "carbamoyl-C₁-C₈ alkyl", "N-mono-C₁-C₈ alkylcarbamoyl-C₁-C₈ alkyl" and "N,N-di-C₁-C₈ alkylcarbamoyl-C₁-C₈ alkyl" refer to C₁-C₈ alkyl substituted at the end or in the middle with cyano, halogen, hydroxy, alkoxy, carboxy, amino, carbamoyl, N-mono-C₁-C₈ alkylcarbamoyl and N,N-di-C₁-C₈ alkylcarbamoyl, respectively.

The term "glycosidyl" refers to a functional group formed by the condensation reaction of sugar molecules.

The term "heterocyclyl" refers to a saturated or partially unsaturated cyclic hydrocarbon containing at least one heteroatom. The heterocyclyl ring group may be monocyclic or bicyclic. The bicyclic heterocyclyl may be a spiro, bridged, or fused ring group. The heterocyclyl may contain 3 to 20 ring atoms, 3 to 10 ring atoms, 3 to 8 ring atoms, 3 to 7 ring atoms, 3 to 6 ring atoms, 4 to 9 ring atoms, 4 to 8 ring atoms, 4 to 7 ring atoms, or 4 to 6 ring atoms. The heteroatom may be any one or more selected from the group consisting of N, O and S. The heteroatom may be 1 to 3, 1, or 2 heteroatoms selected from the group consisting of N, O, and S.

Non-limiting examples of heterocyclyl may include aziridinyl, azetidinyl, pyrrolidinyl, piperidinyl, piperazinyl, dihydropyridinyl, tetrahydropyridinyl, oxetanyl, tetrahydrofuranyl, tetrahydropyranyl, dihydrofuranyl, dihydropyranyl, tetrahydrothiophenyl, morpholinyl, thiomorpholinyl, azepanyl, diazepanyl, oxazepanyl, thiazepanyl, and the like.

The term "heterocyclyloxy" refers to a functional group in which an oxygen atom is directly linked to the ring of the heterocycle.

The term "heteroaryl" or "heteroarylene" refers to a monocyclic or bicyclic aromatic moiety that contains one or more heteroatoms selected from the group consisting of N, O, and S, and the remaining ring atoms being carbon. The heteroaryl group may contain, for example, 1 to 4 heteroatoms, 1 to 3 heteroatoms, or 1 or 2 heteroatoms. The heteroaryl group may contain 5 to 10 ring elements, 5 to 7 ring elements, or 5 or 6 ring elements. The heteroaryl may be a 5- to 6-membered heteroaryl containing one or two N, O or S. The heteroaryl group may be a one-ring group, a two-ring group, or a three-ring group. The two-ring group may be a spiro-ring group, a bridged-ring group, and a fused-ring group.

Non-limiting examples of "heteroaryl" may include pyrrolyl, imidazolyl, pyrazolyl, pyridazinyl, furanyl, pyranyl, thienyl, thiophenyl, 1,2,3-oxadiazolyl, 1,2,4-oxadiazolyl, 1,2,5-oxadiazolyl, 1,3,4-oxadiazolyl, 1,2,3-thiadiazolyl, 1,2,4-thiadiazolyl, 1,2,5-thiadiazolyl, 1,3,4-thiadiazolyl, isothiazol-3-yl, isothiazol-4-yl, isothiazol-5-yl, oxazol-2-yl, oxazol-4-yl, oxazol-5-yl, isooxazol-3-yl, isooxazol-4-yl, isooxazol-5-yl, 1,2,4-triazol-3-yl, 1,2,4-triazol-5-yl, 1,2,3-triazol-4-yl, 1,2,3-triazol-5-yl, tetrazolyl, pyrid-2-yl, pyrid-3-yl, 2-pyrazin-2-yl, pyrazin-4-yl, pyrazin-5-yl, 2-pyrimidin-2-yl, 4-pyrimidin-2-yl, 5-pyrimidin-2-yl, indolyl, and the like.

The term "binding functional group" refers to a functional group that can form a covalent bond through addition, substitution, condensation reactions, and the like with a functional group included in a ligand or protein, or a functional group included in a linker precursor.

As used herein, the term "moiety" refers to a portion of the compound that corresponds to the parent compound of the moiety when the parent compound is bound to the compound of Formula 1 or the conjugate of Formula 2. Even when a portion of an entire compound is referred to herein as a compound or an active agent, it will be understood from the context that it refers to a "moiety" of the compound or active agent.

In the present invention, "precursor" refers to a compound as a reactant that ultimately provides the desired moiety through a chemical reaction, etc.

In the present invention, "linker precursor" refers to a compound that forms the desired linker structure or a part thereof through a chemical reaction. For example, as a PEG linker, various substances such as hydroxy-PEG linker, alkynyl-PEG linker, bromo-PEG linker, DBCO-PEG linker, azido-PEG linker, amino-PEG linker, maleimide-PEG linker are known. In addition, linker compounds such as aminooxy-PEG linker, tetrazine-PEG linker, tosylate-PEG linker, thiol-PEG linker, aldehyde-PEG linker, phosphonate-PEG linker, hydrazide-PEG linker, iodo-PEG linker, carboxyl-PEG linker are also widely used in the related art. As another example, as a linker having a DBCO (dibenzocyclooctyne) group, various substances such as amine reactive DBCO (DBCO-NHS, DBCO-sulfo-NHS ester, DBCO-PEG-NHS ester, DBCO-NHCO-PEG-NHS ester, etc.), carboxyl/carbonyl reactive DBCO (DBCO-amine, DBCO-PEG-amine, etc.), -SH group reactive DBCO (DBCO-maleimide, DBCO-PEG-maleimide, etc.), DBCO-PEG-t-butyl ester, DBCO-alcohol, DBCO-PEG-alcohol, DBCO-PEG-DBCO, Bis-DBCO-PEG are known. The linker precursor of the present invention includes a variety of linkers known in the field of ligand-conjugates and is not limited to the linker structures exemplified herein. Available linker precursors, preparation methods, reaction conditions, and the like are well known in the related art.

The term "dendrimer" refers to a well-ordered three-dimensional molecular structure with branching units centered on a core.

The term "1,6-elimination reaction" refers to a reaction in which cleavage of a covalent bond occurs at a specific position in the molecular structure of a compound, leading to cleavage of the covalent bond at a position 5 atoms away (1,6-position relationship).

In one embodiment, a compound of Formula 1 having a benzothiophene core and a β-galactoside triggering group can release PL- or PL-H through a 1,6-elimination reaction when β-galactose is separated by galactosidase.

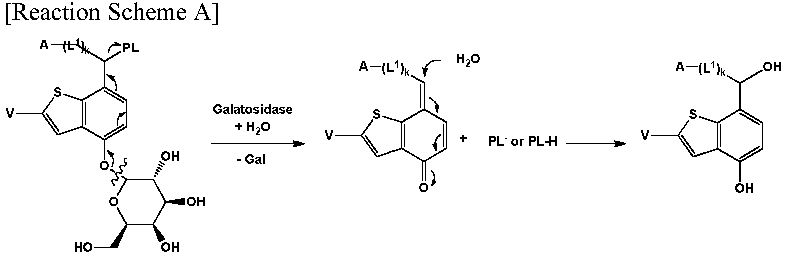

In addition, when the compound of Formula 1 comprises an optional self-immolative group T, β-galactose and 4-hydroxybenzyl alcohol can be separated to release PL⁻ or PL-H, for example, by a 1,6-elimination reaction as shown in Reaction Scheme B below.

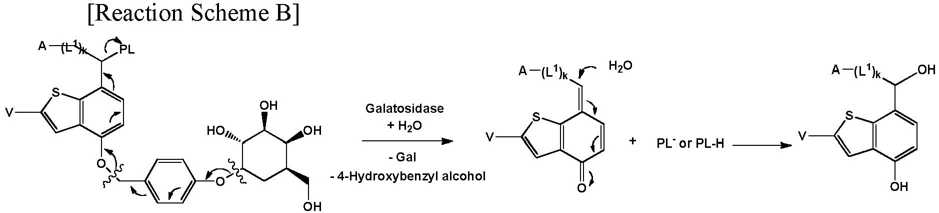

In addition, when the compound of Formula 1 comprises a self-eliminating linker, such as -OCO-, β-galactose and CO₂ can be separated to release PL⁻ or PL-H, for example, by a 1,6-elimination reaction as shown in Reaction Scheme C below.

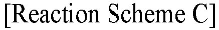

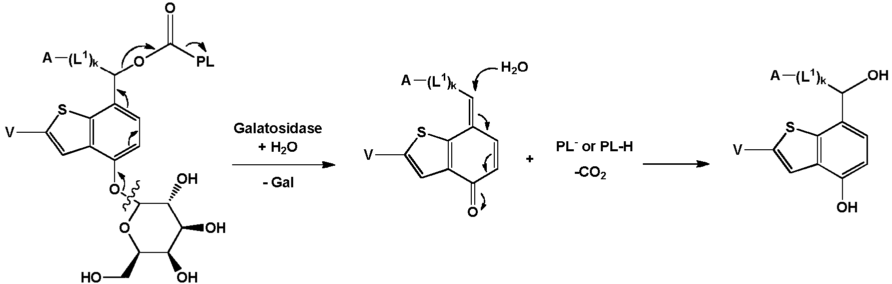

In one embodiment, when the compound of Formula B containing a benzothiophene core and a β-galactoside triggering group comprises a self-eliminating linker (L²) of -OCO-, β-galactose and CO₂ can be separated to release PL⁻ or PL-H, for example, through a 1,6-elimination reaction as shown in Reaction Scheme D below.

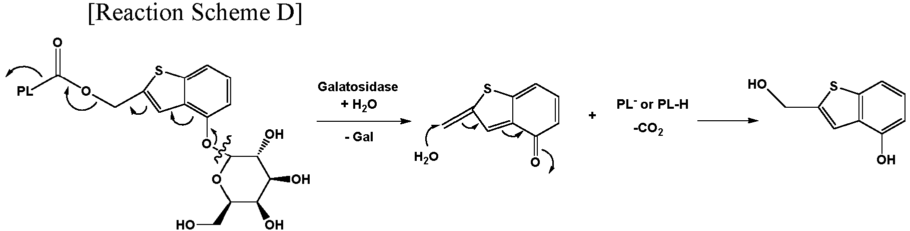

In the present application, the term "click chemistry reaction" refers to a general term for molecular assembly reactions using modular reactants that specifically react with each other even under mild conditions such as room temperature and room pressure, and the types of click chemistry reactions and the functional groups involved in click chemistry reactions are generally well known. For example, the click chemistry reaction includes [3+2] cycloadditions, thiol-ene reaction, Diels-Alder reaction, inverse electron demand Diels-Alder reaction, [4+1] cycloadditions, and the like, but is not limited thereto. More specifically, the click chemistry reaction includes copper (I)-catalyzed azide-alkyne cycloaddition (CuAAC), strain-promoted azide-alkyne cycloaddition (SPAAC), strain-promoted alkyne-nitrone cycloaddition (SPANC), [3+2] cycloaddition of alkenes and azides, inverse-demand Diels-Alder of alkenes and tetrazines, photoclick reaction of alkenes and tetrazoles, and Huisgen cycloaddition of azides and alkynes, but is not limited thereto. The click chemistry functional group includes alkyne, cycloalkyne, cyclooctyne and cyclononyne (e.g., cycloalkyne such as bicyclo[6.1.0]-non-4-yn-9-yl methanol), trans-cyclooctene, nitrone, nitrile oxide, azide, Conjugated Diene, dienophile, and cycloalkyne such as cyclooctyne, cyclononyne, dibenzocyclooctyne (DIBO), BARAC (biarylazacyclooctynone), ALO (aryl-less octyne), DIFO (difluorinated cyclooctyne), MOFO (monofluorinated), DIBAC (dibenzo-aza-cyclooctyne) and DIMAC (dimethoxyazacylooctyne), but is not limited thereto. For example, the click chemistry functional group may include acetylene, transcyclooctene, cyclooctyne, diarylcyclooctyne, methyl ester phosphine, norbornene, tetrazine, methylcyclopropene, azetine, cyanide, azide, dibenzocyclooctyne, and the like.

### Medical uses and pharmaceutical compositions

The compound of Formula 1 or the ligand-drug conjugate of Formula 2 of the present invention can have excellent stability in various blood environments. In other words, the active agent can maintain a stable binding state in the plasma environment of mice, rats, dogs, and humans, thereby minimizing blood toxicity.

In addition, the compound of Formula 1 or the ligand-drug conjugate of Formula 2 of the present invention can exhibit excellent target cell selectivity and excellent active agent-releasing property. The compound of the present invention can rapidly dissociate and release the active agent by reacting with enzymes such as galactosidase and glucuronidase under pH 4 to 5 conditions. Therefore, the compound of the present invention can effectively release the active agent in the environment within the target cells (e.g., tumor lysosomes).

In one aspect of the present invention, there are provided a pharmaceutical composition comprising a linker compound of Formula 1, Formula 1-1 or Formula B or a ligand-drug conjugate of Formula 2 or Formula 2-1, or a pharmaceutically acceptable salt thereof, and a pharmaceutically acceptable carrier or excipient.

In another aspect of the present invention, there is provided an imaging composition or a composition for detection comprising a linker compound of Formula 1, Formula 1-1 or Formula B or a ligand-drug conjugate of Formula 2 or Formula 2-1, or a pharmaceutically acceptable salt thereof.

The linker compound of Formula 1, Formula 1-1 or Formula B and the ligand-drug conjugate of Formula 2 or Formula 2-1 according to the present invention may be mixed with a solvent and provided as a composition.

The composition may be prepared in an injectable form as a liquid solution or as a suspension. In addition, the composition may be prepared in a solid form suitable for injection as an emulsion or a polypeptide encapsulated in liposomes. The compound or ligand-drug conjugate of the present invention can be combined with a pharmaceutically acceptable carrier, including any carrier that does not induce the production of antibodies harmful to the subject receiving the carrier. Suitable carriers may typically include slowly metabolized macromolecules such as proteins, polysaccharides, polylactic acid, polyglycolic acid, polymeric amino acids, amino acid copolymers, lipid aggregates, and the like. Proteins may be formulated into a vaccine in a neutral or salt form.

The composition may contain a diluent such as water, saline, glycerol, ethanol, and the like. Auxiliary substances such as wetting or emulsifying agents, pH buffers, and the like may be added to the composition. The composition may be administered parenterally by injection, or may be administered via subcutaneous or intramuscular injection. Additional formulations may be provided, for example, as suppositories or oral preparations. Oral compositions may be provided as solutions, suspensions, tablets, pills, capsules, or sustained-release formulations.

The composition may be administered in a manner that is compatible with the dosage form. The composition comprises a therapeutically effective amount of the compound or the ligand-drug conjugate according to the present invention. The therapeutically effective amount refers to a single dose or a dose in a multiple dose schedule that is effective in treating or preventing a disease or disorder. The administered dose will be determined depending on the type of an active agent included in the compound or ligand-drug conjugate of the present invention, and/or the type of a ligand or protein that binds to a receptor. In addition, the administered dose may vary depending on the health and physical condition of the subject being treated, the desired degree of protection, and other related factors.

For example, a therapeutically effective amount of the compound or ligand-drug conjugate of the present invention or a pharmaceutical composition comprising the same can be used for the treatment or prevention of proliferative diseases, autoimmune diseases, or infectious diseases.

For example, the composition can be used for the treatment of cancer or tumors. For example, the composition can be administered to a patient to treat or prevent infection by pathogens (e.g., viruses, bacteria, fungi, parasites, etc.). These methods comprise administering to a mammal a therapeutic or prophylactic amount of a compound or conjugate sufficient to treat the disease or disorder or symptoms thereof, under conditions such that the disease or disorder is prevented or treated.

In the above composition, the compound or conjugate of the present invention may be administered in the form of a pharmaceutically acceptable salt, hydrate, or solvate thereof. In one embodiment, it may be administered with a pharmaceutically acceptable carrier, a pharmaceutically acceptable excipient and/or a pharmaceutically acceptable additive. The pharmaceutically effective amount and the types of pharmaceutically acceptable salts or solvates, excipients and additives can be determined using standard methods (*see* Remington's Pharmaceutical Sciences, Mack Publishing Co., Easton, PA, 18th edition, 1990).

As used herein, the term "pharmaceutically acceptable salt" includes organic salts and inorganic salts. Examples may include, but are not limited to, hydrochloride, hydrobromide, hydroiodide, sulfate, citrate, acetate, oxalate, chloride, bromide, iodide, nitrate, bisulfate, phosphate, acid phosphate, isonicotinate, lactate, salicylate, acid citrate, tartrate, oleate, tannate, pantonate, bitartrate, ascorbate, succinate, maleate, genticinate, fumarate, gluconate, glucuronate, saccharate, formate, benzoate, glutamate, methane sulfonate, ethane sulfonate, benzene sulfonate, p-toluene sulfonate and pamoate (i.e., 1,1'-methylene-bis(2-hydroxy-3-naphthoate)). Pharmaceutically acceptable salts may comprise another molecule (e.g., acetate ion, succinate ion, and other counterions, etc.), and may also comprise one or more charged atoms or one or more counter ions.

Exemplary solvates that can be used for pharmaceutically acceptable solvates of the above compounds include, but are not limited to, solvates of water, isopropanol, ethanol, methanol, DMSO, ethyl acetate, acetic acid, or ethanol amine.

### Effects of Invention

The compound or ligand-drug conjugate of the present invention can stably deliver an active agent such as a drug, a toxin, a fluorophore, an affinity ligand, a diagnostic substance, or a detection probe to a target site, and can rapidly release the active agent in a specific environment of the target site.

The compound or ligand-drug conjugate of the present invention can have excellent stability at blood temperature and neutral conditions, and can rapidly release the active agent under acidic conditions, for example, under the tumor microenvironment.

### Brief Description of Drawings

Figures 1 to 3 show the results of measuring the enzyme cleavage rate using E. coli-β-galactosidase or E. coli-β-glucuronidase for Compounds A-12, A-16, and A-36, respectively.
Figure 4 shows the results of measuring the chemical stability and plasma stability for Compound A-36.
Figures 5 and 6 show the results of *in vivo* activity analysis for ADC-1 to ADC-4 and ADC-10 to ADC-13.
Figure 7 shows the results of an *in vivo* pharmacokinetic test for ADC-3 in rats.

### Mode for Carrying out the Invention

Hereinafter, the present invention will be described in more detail through examples. However, these examples are provided for illustrative purposes only and the scope of the present invention is not limited to these examples.

The abbreviations used in this specification are as follows, and abbreviations not listed in the abbreviation list below have meanings commonly used in the field of organic synthesis:
Ac : acetyl
AcOH : acetic acid
EA : ethyl acetate
MC : methylene chloride
DMF : dimethylformamide
EDCI : 1-ethyl-3-(3-dimethylaminopropyl)carbodiimide
EDC : N-(3-dimethylaminopropyl)-N'-ethylcarbodiimide hydrochloride
HOBt : 1-hydroxybenzotriazole hydrate
ACN : acetonitrile
THF : tetrahydrofuran
DCC : N,N'-dicyclohexylcarbodiimide
DMAP : 4-dimethylaminopyridine
NHS : N-hydroxysuccinimide
DIPEA : diisopropylethylamine
TEA : triethylamine
Boc : tert-butyloxycarbonyl
LAH : lithium aluminium hydride
TFA : trifluoroacetic acid
AgOTf : silver trifluoromethanesulfonate
KO^{t}Bu : potassium tert-butoxide
MMAF-OMe : monomethyl auristatin F methyl ester
PPTS : pyridinium p-toluenesulfonate
TBAI : tetrabutylammonium iodide
DIBAL-H : Diisobutylaluminum hydride
TEMPO : 2,2,6,6-Tetramethylpiperidine 1-oxyl, 2,2,6,6-Tetramethyl-1-piperidinyloxy, free radical
MMAE : monomethyl auristatin E
NCS : *N*-chlorosuccinimide

### Preparation Example 1: Preparation of Linker P-1

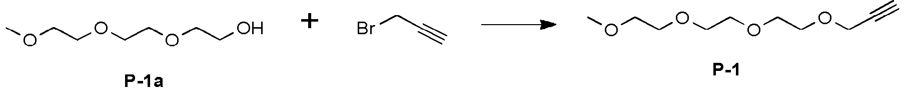

Compound P-1a (triethylene glycol monomethyl ether, TCI, CAS No. 112-35-6, 1 g, 6.09 mmol) was dissolved in THF (10 mL) under a nitrogen atmosphere at 0°C, and then KO^{t}Bu (1 g, 9.13 mmol) was added, and the mixture was stirred at 0°C for 20 minutes. Propargyl bromide (1.3 mL, 12.18 mmol) was added to the reaction solution, and the mixture was stirred for 16 hours while slowly raising the temperature from 0°C to room temperature. After completion of the reaction, the reaction solution was diluted with EA (200 mL), filtered using Celite, and extracted by adding distilled water (250 mL). The obtained organic layer was dried over anhydrous magnesium sulfate, filtered, and then concentrated under reduced pressure. The resulting residue was subjected to column chromatography to obtain Linker P-1 (820 mg, 66 %).

¹H-NMR (400 MHz, CDCl₃) δ 4.21 (d, *J =* 2.4 Hz, 2H), 3.71 - 3.63 (m, 10H), 3.56 - 3.54 (m, 2H), 3.38 (s, 3H), 2.42 (t, *J =* 2.4 Hz, 1H).

### Preparation Example 2: Preparation of Linker P-2

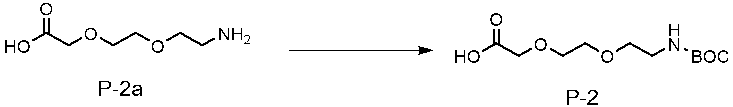

Compound P-2a (2-[2-(2-aminoethoxy)ethoxy]acetic acid, TCI, CAS No. 134978-97-5, 1.28 g, 7.87 mmol) was dissolved in 1,4-dioxane (30 mL) under a nitrogen atmosphere at 0°C, and then sodium bicarbonate (1.32 g, 15.74 mmol) and di-tert-butyl dicarbonate (Boc anhydride, 2.06 g, 9.44 mmol), which were dissolved in distilled water (15 mL), were added, and the mixture was stirred for 16 hours at room temperature. After completion of the reaction, EA (300 mL), distilled water (250 mL), and 2N-hydrochloric acid aqueous solution (50 mL) were added to extract the organic layer 5 times. The obtained organic layer was dried over anhydrous magnesium sulfate, filtered, and then concentrated under reduced pressure to obtain Linker P-2 (2.32 g, quantitatively obtained). The obtained Linker P-2 was used in the next reaction without an additional purification process.

¹H-NMR (400 MHz, CDCl₃) δ 4.93 (brs, 1H), 4.17 (s, 2H), 3.78 - 3.76 (m, 2H), 3.68 - 3.65 (m, 2H), 3.62 - 3.56 (m, 2H), 3.38 - 3.32 (m, 2H), 1.45 (s, 9H).

### Preparation Example 3: Preparation of Linker P-3

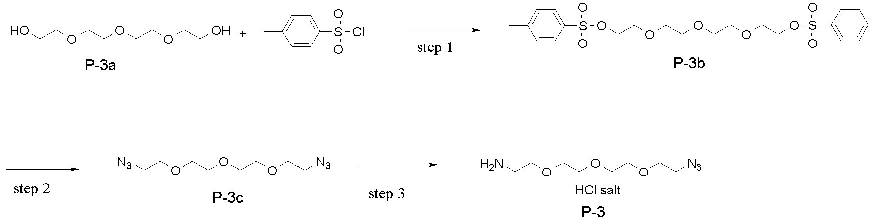

### Step 1: Preparation of Compound P-3b

Compound P-3a (tetraethylene glycol, Daejung Chemicals & Metals, CAS NO. 112-60-7, 10 g, 51.49 mmol) was dissolved in MC (200 mL) under a nitrogen atmosphere at 0°C, and then 4-methylbenzenesulfonyl chloride (24.54 g, 128.72 mmol) and potassium hydroxide (8.67 g, 154.46 mmol) were added, and the mixture was stirred at room temperature for 15 hours. After completion of the reaction, MC (100 mL) and distilled water (200 mL) were added to extract the organic layer twice. The obtained organic layer was dried over anhydrous magnesium sulfate, filtered, and then concentrated under reduced pressure. The residue was subjected to column chromatography to obtain Compound P-3b in the form of a colorless oil (18.98 g, 73.3 %).

¹H-NMR (400 MHz, CDCl₃) δ 7.79 (d, *J =* 8.4 Hz, 4H), 7.34 (d, *J =* 8.0 Hz, 4H), 4.16 - 4.14 (m, 4H), 3.69 - 3.66 (m, 4H), 3.57 - 3.55 (m, 8H), 2.44 (s, 6H); MS m/z: 503[M+H]⁺.

### Step 2: Preparation of Compound P-3c

Compound P-3b (18.98 g, 37.76 mmol) was dissolved in DMF (95 mL) under a nitrogen atmosphere at room temperature, and then sodium azide (7.36 g, 113.36 mmol) was added, and the mixture was stirred at 60°C for 16 hours. After completion of the reaction, the mixture was cooled to room temperature, and EA (200 mL) and saturated aqueous sodium bicarbonate solution (200 mL) were added to extract the organic layer twice. The obtained organic layer was dried over anhydrous magnesium sulfate, filtered, and then concentrated under reduced pressure. The residue was subjected to column chromatography to obtain Compound P-3c in the form of a colorless liquid (8.96 g, 97.1 %).

¹H-NMR (400 MHz, CDCl₃) δ 3.69 - 3.66 (m, 12H), 3.40 - 3.37 (m, 4H); MS m/z: 267[M+Na]⁺.

### Step 3: Preparation of Linker P-3

Compound P-3c (4.17 g, 17.06 mmol) was dissolved in EA (32 mL), diethyl ether (32 mL) and 5% hydrochloric acid aqueous solution (64 mL) under a nitrogen atmosphere at 0°C, and then triphenylphosphine (4.47 g, 17.06 mmol) was added, and the mixture was stirred at room temperature for 16 hours. After completion of the reaction, the mixture was concentrated under reduced pressure to remove the organic layer, MC (100 mL) was added to the aqueous layer to wash the aqueous layer three times. The obtained aqueous layer was concentrated under reduced pressure to obtain Linker P-3 in the form of a colorless liquid (4.12 g, 95 %).

¹H-NMR (400 MHz, CDCl₃) δ 8.27 (brs, 2H), 3.86 - 3.83 (m, 2H), 3.72 - 3.68 (m, 10H), 3.47 - 3.45 (m, 2H), 3.26 - 3.24 (m, 2H).

### Preparation Example 4: Preparation of Linker P-4

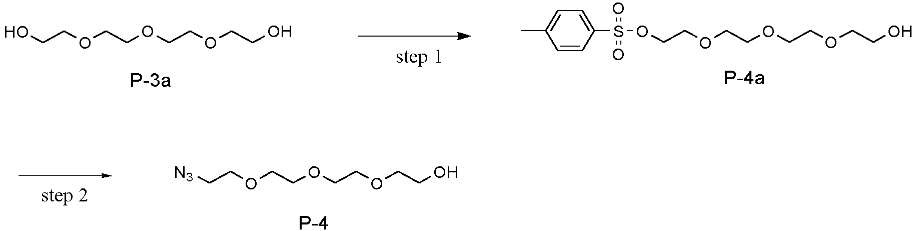

### Step 1: Preparation of Compound P-4a

Compound P-3a (tetraethylene glycol, Daejung Chemicals & Metals, 10 g, 51.49 mmol) was dissolved in MC (200 mL) under a nitrogen atmosphere at 0°C, and then 4-methylbenzenesulfonyl chloride (3.27 g, 17.16 mmol) and potassium hydroxide (963 mg, 17.16 mmol) were added, and the mixture was stirred for 15 hours at room temperature. After completion of the reaction, MC (100 mL) and distilled water (200 mL) were added to extract the organic layer twice. The obtained organic layer was dried over anhydrous magnesium sulfate, filtered, and then concentrated under reduced pressure. The residue was subjected to column chromatography to obtain Compound P-4a in the form of a colorless oil (2.46 g, 41.1 %).

¹H-NMR (400 MHz, CDCl₃) δ 7.80 (d, *J =* 8.4 Hz, 2H), 7.34 (d, *J =* 8.0 Hz, 2H), 4.18 - 4.15 (m, 2H), 3.72 - 3.59 (m, 14H), 2.45 (s, 3H); MS m/z: 349[M+H]⁺, 371[M+Na]⁺.

### Step 2: Preparation of Linker P-4

Compound P-4a (2.46 g, 7.05 mmol) was dissolved in DMF (15 mL) under a nitrogen atmosphere at room temperature, and then sodium azide (690 mg, 10.58 mmol) was added, and the mixture was stirred at 60°C for 4 hours. After completion of the reaction, the mixture was cooled to room temperature, and EA (50 mL) and distilled water (100 mL) were added to extract the organic layer twice. The obtained organic layer was dried over anhydrous magnesium sulfate, filtered, and then concentrated under reduced pressure. The residue was subjected to column chromatography to obtain Linker P-4 in the form of a colorless liquid (1.37 g, 88.4 %).

¹H-NMR (400 MHz, CDCl₃) δ 3.73 - 3.66 (m, 12H), 3.62 - 3.60 (m, 2H), 3.41 - 3.39 (m, 2H), 2.42 (t, *J =* 6.0 Hz, 1H); MS m/z: 220[M+H]⁺, 242[M+Na]⁺.

### Preparation Example 5: Preparation of Linker P-5

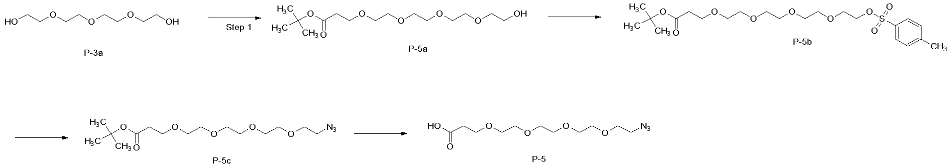

### Step 1: Preparation of Compound P-5

Compound P-3a (tetraethylene glycol, Daejung Chemicals & Metals, 5 g, 25.74 mmol) was dissolved in THF (60 mL) under a nitrogen atmosphere at room temperature, and then sodium hydride (NaH 60 % dispersion in mineral oil, 16.5 mg, 0.41 mmol) was added, and the mixture was stirred at room temperature for 30 minutes. Tert-butyl acrylate (Merck, CAS NO. 1663-39-4, 1.5 mL, 10.30 mmol) was slowly added to the reaction solution for 2 hours, and the mixture was stirred at room temperature for 3 hours. After completion of the reaction, a saturated aqueous sodium chloride solution (20 mL) was added to terminate the reaction, and the organic solvent was removed by concentration under reduced pressure. MC (60 mL) was added to the remaining aqueous sodium chloride solution to extract the organic layer, and then the obtained organic layer was dried over anhydrous magnesium sulfate, filtered, and concentrated under reduced pressure. The residue was subjected to column chromatography to obtain Compound P-5a in the form of a pinkish oil (1.39 g, 42 %).

¹H-NMR (400 MHz, CDCl₃) δ 3.75 - 3.60 (m, 18H), 2.69 (t, *J =* 6.4 Hz, 1H), 2.50 (t, *J =* 6.4 Hz, 2H), 1.45 (s, 9H); EI-MS m/z: 345 [M+Na]⁺.

### Step 2: Preparation of Compound P-5b

Compound P-5a (1.39 g, 4.31 mmol) was dissolved in ACN (7.5 mL) under a nitrogen atmosphere at 0°C, and then pyridine (4 mL) was slowly added. Then, a solution of 4-methylbenzenesulfonyl chloride (*p*-toluenesulfonyl chloride, 1.1 g, 5.77 mmol) dissolved in ACN (10.5 mL) was slowly added for 30 minutes, and the mixture was stirred at room temperature for 6.5 hours. After completion of the reaction, EA (200 mL) and distilled water (150 mL) and 2N-hydrochloric acid aqueous solution (50 mL) were added to extract the organic layer twice. The obtained organic layer was dried over anhydrous magnesium sulfate, filtered, and then concentrated under reduced pressure. The residue was subjected to column chromatography to obtain Compound P-5b in the form of a colorless oil (1.78 g, 86 %).

¹H-NMR (400 MHz, CDCl₃) δ 7.80 (d, *J =* 8 Hz, 2H), 7.34 (d, *J =* 8 Hz, 2H), 4.16 (t, *J =* 4.8 Hz, 2H), 3.73 - 3.65 (m, 4H), 3.65 - 3.57 (m, 12H), 2.50 (t, *J =* 6.4 Hz, 2H), 2.45 (s, 3H), 1.44 (s, 9H); EI-MS m/z: 499 [M+Na]⁺.

### Step 3: Preparation of Compound P-5c

Compound P-5b (1.78 g, 3.73 mmol) was dissolved in DMF (20 mL) under a nitrogen atmosphere at room temperature, and then sodium azide (NaN₃, 364 mg, 5.60 mmol) was added, and the mixture was stirred at 60°C for 16 hours. After completion of the reaction, the mixture was cooled to room temperature, and EA (200 mL) and distilled water (200 mL) were added to extract the organic layer twice. The obtained organic layer was washed three times by adding a saturated aqueous sodium chloride solution (200 mL). The obtained organic layer was dried over anhydrous magnesium sulfate, filtered, and then concentrated under reduced pressure. The residue was subjected to column chromatography to obtain Compound P-5c in the form of a colorless oil (1.25 g, 96 %).

¹H-NMR (400 MHz, CDCl₃) δ 3.73 - 3.60 (m, 16H), 3.39 (t, *J =* 4.8 Hz, 2H), 2.50 (t, *J*= 6.4 Hz, 2H), 1.44 (s, 9H); EI-MS m/z: 348 [M+H]⁺.

### Step 4: Preparation of Linker P-5

Compound P-5c (80.5 mg, 0.232 mmol) was dissolved in MC (2.5 mL) under a nitrogen atmosphere at 0°C, and then TFA (2.5 mL) was slowly added, and the mixture was stirred for 1.5 hours at room temperature. After completion of the reaction, the mixture was diluted by adding MC (20 mL), and then concentrated under reduced pressure to obtain Linker P-5 in the form of oil (67 mg, 99%).

¹H-NMR (400 MHz, CDCl₃) δ 7.13 (brs, 1H), 3.78 (t, *J =* 6.0 Hz, 2H), 3.75 - 3.60 (m, 14H), 3.40 (t, *J =* 4.8 Hz, 2H), 2.64 (t, *J =* 6.0 Hz, 2H); EI-MS m/z: 314[M+Na]⁺.

### Preparation Example 6: Preparation of Linker P-6

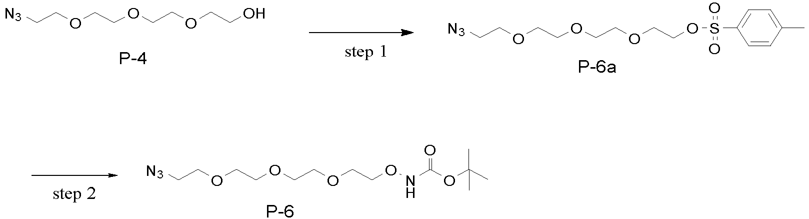

### Step 1: Preparation of Compound P-6a

Compound P-4 (648 mg, 2.96 mmol) was dissolved in MC (11.5 mL) under a nitrogen atmosphere at 0°C, and then 4-methylbenzenesulfonyl chloride (1.13 g, 5.91 mmol) and potassium hydroxide (332 mg, 5.91 mmol) were added, and the mixture was stirred for 16 hours at room temperature. After completion of the reaction, MC (50 mL) and distilled water (200 mL) were added to extract the organic layer three times. The obtained organic layer was dried over anhydrous magnesium sulfate, filtered, and then concentrated under reduced pressure. The residue was subjected to column chromatography to obtain Compound P-6a in the form of a colorless oil (963 mg, 87.5 %).

¹H-NMR (400 MHz, CDCl₃) δ 7.80 (d, *J =* 8.4 Hz, 2H), 7.34 (d, *J =* 8.4 Hz, 2H), 4.16 (t, *J* = 4.8 Hz, 2H), 3.70 - 3.59 (m, 12H), 3.38 (t, *J =* 4.8 Hz, 2H), 2.44 (s, 3H); MS m/z: 374[M+H]⁺, 396[M+Na]⁺.

### Step 2: Preparation of Linker P-6

Compound P-6a (487.2 mg, 1.30 mmol) was dissolved in ACN (20 mL) under a nitrogen atmosphere at room temperature, and then N-Boc-hydroxylamine(Merck, CAS NO. 36016-38-3, 208 mg, 1.56 mmol) and DBU (0.39 mL, 2.60 mmol) were sequentially added, and the mixture was stirred for 16 hours at 80°C. After completion of the reaction, MC (50 mL) and distilled water (50 mL) were added to extract the organic layer twice. The obtained organic layer was washed by adding aqueous sodium chloride solution. The washed organic layer was dried over anhydrous magnesium sulfate, filtered, and then concentrated under reduced pressure. The resulting residue was subjected to column chromatography to obtain Linker P-6 in the form of a colorless liquid (83.0 mg, 19.0 %).

¹H-NMR (400 MHz, CDCl₃) δ 7.53 (brs, 1H), 4.02 (t, *J =* 4.8 Hz, 2H), 3.72 (t, *J =* 4.8 Hz, 2H), 3.71 - 3.65 (m, 10H), 3.39 (t, *J =* 4.8 Hz, 2H), 1.48 (s, 9H); EI-MS m/z: 357[M+Na]⁺, 235[M-BOC]⁺.

### Preparation Example 7: Preparation of Compound PL-1

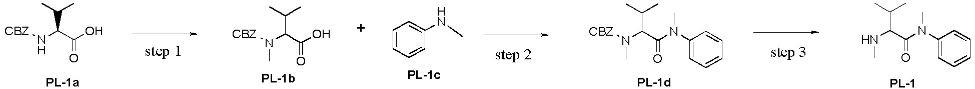

### Step 1: Preparation of Compound PL-1b

A solution of Compound PL-1a (carbobenzyloxy-L-valine, Z-Val-OH, Merck, CAS No. 1149-26-4, 5 g, 19.9 mmol) dissolved in THF (20 mL) was slowly added to a reaction solution in which THF (50 mL) was added to 60% sodium hydride (60% dispersion in mineral oil, 3.4 g, 85.56 mmol) under a nitrogen atmosphere at 0°C, and the mixture was stirred at 0°C for 20 minutes. Then, iodomethane (12.38 mL, 198.98 mmol) was slowly added, and the mixture was stirred at room temperature for 20 hours. The reaction solution was cooled to 0°C, and then distilled water (150 mL) was added to terminate the reaction, and EA (200 mL) and 2N-hydrochloric acid aqueous solution (50 mL) were added to extract the organic layer twice. The obtained organic layer was dried over anhydrous magnesium sulfate, filtered, and then concentrated under reduced pressure. The residue was subjected to column chromatography to obtain Compound PL-1b (3.7 g, 70 %).

¹H-NMR (400 MHz, DMSO-d₆) δ 7.36-7.30 (m, 5H), 5.11 (s, 2H), 4.22-412 (m, 1H), 2.84-81 (m, 3H), 2.11 (m, 1H), 0.95 (m, 3H), 0.82 (m, 3H).

### Step 2: Preparation of Compound PL-1d

Compound PL-1b (600 mg, 2.26 mmol) and Compound PL-1c (N-methylaniline, Merck, CAS No. 100-61-8, 361 mg, 3.39 mmol) were dissolved in MC (12 mL) under a nitrogen atmosphere at 0°C, and then DCC (700 mg, 3.39 mmol), DMAP (55.2 mg, 0.46 mmol) and DIPEA (0.78 mL, 4.52 mmol) were sequentially added, and the mixture was stirred at 0°C for 20 minutes. The reaction mixture was stirred for additional 16 hours. After completion of the reaction, the reaction solution was diluted with EA (100 mL) and extracted by adding 2N-hydrochloric acid aqueous solution (100 mL) to the filtered solution. The obtained organic layer was extracted once more by adding 2N-sodium hydroxide aqueous solution (100 mL), and then washed by adding a saturated aqueous sodium chloride solution (100 mL). The obtained organic layer was dried over anhydrous magnesium sulfate, filtered, and then concentrated under reduced pressure. The resulting residue was subjected to column chromatography to obtain Compound PL-1d in the form of a colorless oil (429 mg, 53 %).

¹H-NMR (400 MHz, CDCl₃) δ 7.41 - 7.24 (m, 6H), 7.09 - 7.02 (m, 4H), 5.01-4.96 (m, 2H), 4.40-4.28 (m, 1H), 3.25 (s, 3H), 2.91-2.87 (m, 3H), 2.33 (m, 1H), 0.90 (d, *J =* 6.8 Hz, 3H), 0.74-0.65 (m, 3H).

### Step 3: Preparation of Compound PL-1

Compound PL-1d (429 mg, 1.21 mmol) was dissolved in methanol (50 mL) under a nitrogen atmosphere at room temperature, and then 5% palladium charcoal (5% Pd/C, 150 mg) was added, and the mixture was stirred for 2 hours under a hydrogen environment. After completion of the reaction, the reaction solution was diluted with EA (100 mL) and filtered using a Celite filter, and the solution was concentrated under reduced pressure to obtain Compound PL-1 in the form of a colorless oil (264.3 mg, 99 %).

¹H-NMR (400 MHz, CDCl₃) δ 7.45 - 4.41 (m, 2H), 7.37 - 7.34 (m, 1H), 7.02 - 7.18 (m, 2H), 3.31 (s, 3H), 2.85 (d, *J =* 6.4 Hz, 1H), 2.31 (s, 3H), 1.71 (m, 1H), 0.85 (d, *J =* 6.8 Hz, 3H), 0.80 (d, *J* = 6.8 Hz, 3H).

### Preparation Example 8: Preparation of Core C-1

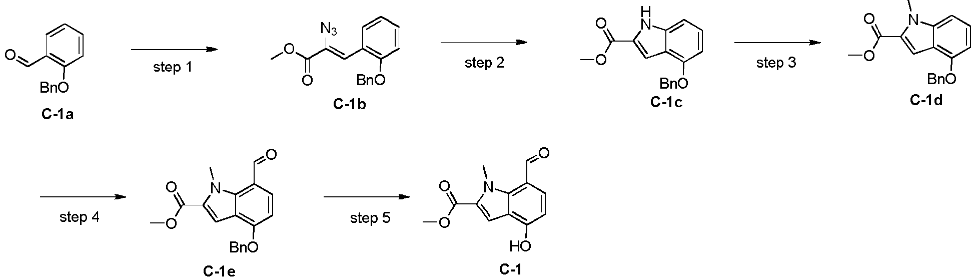

### Step 1: Preparation of Compound C-1b

Compound C-1a (2-benzyloxybenzaldehyde, CAS NO. 5896-17-3, 5.48 g, 25.8 mmol) was dissolved in methanol (55 mL) under a nitrogen atmosphere at -30°C, and then ethyl azidoacetate (CAS NO. 637-81-0, TCI, 28.2 mL, 258.20 mmol) and sodium methoxide (25 wt% in MeOH, 44.6 mL, 206.4 mmol) were slowly added. The mixture was stirred at -30°C for 3 hours, and then distilled water (100 mL) was slowly added dropwise to terminate the reaction, and EA (100 mL) was added to extract the organic layer. The obtained organic layer was dried over anhydrous magnesium sulfate, filtered, and then concentrated under reduced pressure to obtain Compound C-1b (7.74 g, 97 %). The obtained Compound C-1b was used in the next reaction without an additional purification process.

### Step 2: Preparation of Compound C-1c

Compound C-1b (7.74 g, 25.02 mmol) was dissolved in xylene (200 mL) under a nitrogen atmosphere at room temperature, and then the mixture was stirred at 150°C for 3 hours. After completion of the reaction, the mixture was cooled to room temperature and concentrated under reduced pressure. The resulting residue was subjected to column chromatography to obtain Compound C-1c (510.8 mg, 22.7 %).

¹H-NMR (400 MHz, CDCl₃) δ 8.82 (brs, 1H), 7.51-7.49 (m, 2H), 7.42-7.33 (m, 4H), 7.22 (t, *J =* 8 Hz, 1H), 7.02 (d, *J =* 8 Hz, 1H), 6.58 (d, *J =* 8 Hz, 1H), 5.22 (s, 2H), 3.92 (s, 3H).

### Step 3: Preparation of Compound C-1d

Compound C-1c (1.14 g, 4.05 mmol) was dissolved in DMF (35 mL) under a nitrogen atmosphere at room temperature, and then potassium carbonate (1.68 g, 12.15 mmol) and iodomethane (0.65 mL, 10.53 mmol) were sequentially added, and the mixture was stirred for 12 hours. After completion of the reaction, EA (100 mL) and distilled water (100 mL) were added to extract the organic layer. Distilled water (300 mL) was added to extract the obtained organic layer again, and the organic layer was dried over anhydrous magnesium sulfate, filtered, and then concentrated under reduced pressure. The resulting residue was subjected to column chromatography to obtain Compound C-1d (912.7 mg, 76 %).

¹H-NMR (400 MHz, CDCl₃) δ 7.50-7.47 (m, 3H), 7.42-7.33 (m, 3H), 7.25 (m, 1H), 6.99 (d, *J* = 8 Hz, 1H), 6.57 (d, *J =* 8 Hz, 1H), 5.22 (s, 2H), 4.06 (s, 3H), 3.89 (s, 3H).

### Step 4: Preparation of Compound C-1e

C-1d (800 mg, 2.70 mmol) was dissolved in MC (40 mL) under a nitrogen atmosphere at -50°C, and then dichloromethyl methyl ether (735 µL, 8.10 mmol) and titanium tetrachloride solution (1M-TiCl₄ in MC, 8.1 mL, 8.1 mmol) were sequentially and slowly added, and the mixture was stirred for 2 hours while maintaining the temperature. After completion of the reaction, cooled distilled water (100 mL) was slowly added dropwise to terminate the reaction, and EA (100 mL) was added to extract the organic layer. The obtained organic layer was dried over anhydrous magnesium sulfate, filtered, and then concentrated under reduced pressure. The resulting residue was subjected to column chromatography to obtain Compound C-1e (392 mg, 44.8 %).

¹H-NMR (400 MHz, CDCl₃) δ 10.10 (s, 1H), 7.81 (d, *J =* 8.4 Hz, 1H), 7.44 (s, 1H), 7.49-7.47 (m, 2H), 7.44-7.36 (m, 3H), 6.69 (d, *J* = 8.4 Hz, 1H), 5.39 (s, 2H), 4.36 (s, 3H), 3.90 (s, 3H).

### Step 5: Preparation of Core C-1

Compound C-1e (393.2 mg, 1.22 mmol) was dissolved in MC (20 mL) under a nitrogen atmosphere at -50°C, and then boron trichloride solution (1M-BCl₃ in MC, 6 mL, 6.08 mmol) was slowly added, and the mixture was stirred for 1.5 hours while slowly raising the temperature to - 30°C. After completion of the reaction, distilled water (100 mL) was slowly added dropwise to terminate the reaction, and 2N-sodium hydroxide aqueous solution was added at 0°C to neutralize it. MC (100 mL) was added to the mixture to extract the organic layer four times. The obtained organic layer was dried over anhydrous magnesium sulfate, filtered, and then concentrated under reduced pressure. The resulting residue was subjected to column chromatography to obtain Core C-1 in the form of a pale orange solid (239.7 mg, 84 %).

¹H-NMR (400 MHz, DMSO-d₆) δ 9.99 (s, 1H), 7.79 (m, 1H), 7.43 (s, 1H), 6.55 (m, 1H), 4.22 (s, 3H), 3.84 (s, 3H).

### Preparation Example 9: Preparation of Core C-2

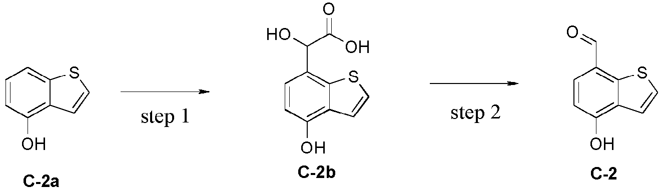

### Step 1: Preparation of Compound C-2b

Compound C-2a (benzo[b]thiophene-4-ol, Ambeed, CAS NO. 3610-02-4, 500 mg, 3.35 mmol) was dissolved in 10% potassium hydroxide aqueous solution (410 mg in 4 mL H₂O) under a nitrogen atmosphere at 0°C, and then glyoxylic acid monohydrate (300 mg) was added, and the mixture was stirred for 7 hours. After completion of the reaction, the mixture was diluted by adding distilled water (2 mL), and 2N-hydrochloric acid aqueous solution was slowly added dropwise to adjust the pH of the reaction solution to 7. The organic layer was extracted and removed using diethyl ether (10 mL), and 2N-hydrochloric acid aqueous solution was slowly added dropwise to the aqueous layer to adjust the pH to 2, and extracted three times using diethyl ether (100 mL). The organic layer was collected, dried over anhydrous magnesium sulfate, filtered, and then concentrated under reduced pressure to obtain Compound C-2b (460 mg, 61.6 %).

¹H-NMR (400 MHz, CDCl₃) δ 7.51 (d, *J =* 5.6 Hz, 1H), 7.36 (d, *J =* 5.6 Hz, 1H), 7.24 (d, *J =* 8 Hz, 1H), 6.72 (d, *J =* 8 Hz, 1H), 5.41 (s, 1H).

### Step 2: Preparation of Core C-2

Compound C-2b (450 mg, 2.0 mmol) was dissolved in ethanol (0.8 mL) under a nitrogen atmosphere at room temperature, and then ferric sulfate hydrate (923 mg, 2.3 mmol) and 0.4N-sulfuric acid aqueous solution (3.8 mL) were sequentially added, and the mixture was stirred at 60°C for 1 hour. After completion of the reaction, the reaction temperature was lowered to room temperature, and the reaction solution was filtered using EA (100 mL). Distilled water (100 mL) was added to the filtrate to extract the organic layer, and the organic layer was dried over anhydrous magnesium sulfate, filtered, and then concentrated under reduced pressure. The resulting residue was subjected to column chromatography to obtain Core C-2 (210 mg, 58 %).

¹H-NMR (400 MHz, CDCl₃) δ 10.01 (s, 1H), 7.74 (d, *J =* 8 Hz, 1H), 7.60 (d, *J =* 5.6 Hz, 1H), 7.52 (d, *J =* 5.6 Hz, 1H), 6.88 (d, *J =* 8 Hz, 1H).

### Preparation Example 10: Preparation of Core C-3

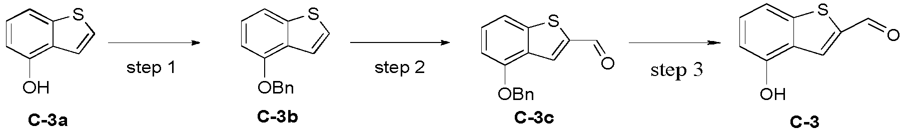

### Step 1: Preparation of Compound C-3b

Compound C-3a (benzo[b]thiophene-4-ol, Ambeed, CAS NO. 3610-02-4, 1.14 g, 7.63 mmol) was dissolved in ACN (50 mL) under a nitrogen atmosphere at 0°C, and then potassium carbonate (2.63 g, 19.07 mmol) and benzyl bromide (1 mL, 8.39 mmol) were slowly added, and the mixture was stirred at 0°C for 30 minutes. The reaction temperature was raised to room temperature, and the mixture was stirred for additional 16 hours. After completion of the reaction, EA (350 mL) and distilled water (350 mL) were added to extract the organic layer. The obtained organic layer was dried over anhydrous magnesium sulfate, filtered, and then concentrated under reduced pressure. The residue was subjected to column chromatography to obtain Compound C-3b in the form of a light orange oil (1.87 g, 99 %).

¹H-NMR (400 MHz, CDCl₃) δ 7.57 (d, *J =* 5.6 Hz, 1H), 7.50 - 7.47 (m, 3H), 7.42 - 7.38 (m, 2H), 7.35 - 7.32 (m, 2H), 7.27 - 7.23 (m, 1H), 6.81 (d, *J =* 8 Hz, 1H), 5.22 (s, 2H).

### Step 2: Preparation of Compound C-3c

Compound C-3b (1.87 g, 7.8 mmol) was dissolved in THF (60 mL) under a nitrogen atmosphere at room temperature. The solution was cooled to -78°C, and then n-butyl lithium solution (2.5M n-BuLi in hexane, 1.42 mL, 3.56 mmol) was slowly added dropwise, and the mixture was stirred at the same temperature for 30 minutes. DMF (0.78 mL) was slowly added to the mixture, and the mixture was stirred at the same temperature for additional 30 minutes. Distilled water (300 mL) was added to the mixture at the same temperature to terminate the reaction, and then EA (300 mL) was added to extract the organic layer. The obtained organic layer was dried over anhydrous magnesium sulfate, filtered, and then concentrated under reduced pressure. The residue was subjected to column chromatography to obtain Compound C-3c in the form of a light yellow oil (1.36 g, 65 %).

¹H-NMR (400 MHz, CDCl₃) δ 10.06 (s, 1H), 8.25 (s, 1H), 7.50 - 7.37 (m, 7H), 6.85 (d, *J* = 7.6 Hz, 1H), 5.24 (s, 2H).

### Step 3: Preparation of Core C-3

Compound C-3c (300 mg, 1.11 mmol) was dissolved in MC (20 mL) under a nitrogen atmosphere at -78°C, and then boron trichloride solution (1M-BCl₃ in MC, 2.2 mL, 2.20 mmol) was slowly added, and the mixture was stirred for 30 minutes. After completion of the reaction, EA (10 mL) and distilled water (10 mL) were slowly added dropwise at -50°C to terminate the reaction, and EA (100 mL) and distilled water (100 mL) were added at room temperature to extract the organic layer. The obtained organic layer was dried over anhydrous magnesium sulfate, filtered, and then concentrated under reduced pressure. The resulting residue was subjected to column chromatography to obtain Core C-3 in the form of a light yellowish brown solid (174 mg, 87.4 %).

¹H-NMR (400 MHz, DMSO-d₆) δ 10.62 (brs, 1H), 8.41 (s, 1H), 7.46 (d, *J =* 8 Hz, 1H), 7.39 (t, *J =* 8 Hz, 1H), 6.82 (d, *J =* 8 Hz, 1H).

### Preparation Example 11: Preparation of Core C-4

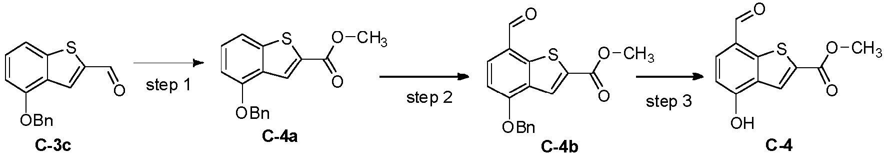

### Step 1: Preparation of Compound C-4a

Compound C-3c (1.36 g, 5.09 mmol) prepared in Step 2 of Preparation Example 10 was dissolved in methanol (65 mL) under a nitrogen atmosphere at 0°C, and then potassium hydroxide (713 mg, 12.72 mmol) dissolved in methanol (15 mL) and iodine (1.67 g, 6.61 mmol) dissolved in methanol (15 mL) were sequentially and slowly added, and then the mixture was stirred at room temperature for 1 hour. After completion of the reaction, the organic layer was extracted with EA (500 mL) and distilled water (500 mL). The obtained organic layer was dried over anhydrous magnesium sulfate, filtered, and then concentrated under reduced pressure. The resulting residue was subjected to column chromatography to obtain Compound C-4a in the form of an ivory solid (1.43 g, 94 %).

¹H-NMR (400 MHz, CDCl₃) δ 8.28 (s, 1H), 7.49 - 7.47 (m, 2H), 7.44 - 7.34 (m, 5H), 6.82 (d, *J =* 7.6 Hz, 1H), 5.21 (s, 2H), 3.93 (s, 3H).

### Step 2: Preparation of Compound C-4b

Compound C-4a (240 mg, 0.80 mmol) was dissolved in MC (15 mL) under a nitrogen atmosphere at -78°C, and then dichloromethyl methyl ether (218 µL, 2.41 mmol) and titanium tetrachloride solution (1M-TiCl₄ in MC, 2.41 mL, 2.41 mmol) were sequentially and slowly added, and the mixture was stirred for 1 hour while maintaining the temperature. After completion of the reaction, distilled water (100 mL) was slowly added dropwise to terminate the reaction, and EA (100 mL) was added to extract the organic layer twice. The obtained organic layer was dried over anhydrous magnesium sulfate, filtered, and then concentrated under reduced pressure. The resulting residue was subjected to column chromatography to obtain Compound C-4b (206.7 mg, 79 %).

¹H-NMR (400 MHz, CDCl₃) δ 10.07 (s, 1H), 8.34 (s, 1H), 7.93 (d, *J =* 8 Hz, 1H), 7.50 - 7.39 (m, 5H), 7.00 (d, *J =* 8 Hz, 1H), 5.34 (s, 2H), 3.95 (s, 3H).

### Step 3: Preparation of Core C-4

Compound C-4b (82.5 mg, 0.25 mmol) was dissolved in MC (5 mL) under a nitrogen atmosphere at -78°C, and then boron trichloride solution (1M-BCl₃ in MC, 2.78 mL, 2.78 mmol) was slowly added, and the mixture was stirred for 5 hours while slowly raising the temperature to -30°C. After completion of the reaction, distilled water (50 mL) was slowly added dropwise to terminate the reaction, and 2N-sodium hydroxide aqueous solution was added at 0°C to neutralize it. MC (50 mL) was added to the mixture to extract the organic layer three times. The obtained organic layer was dried over anhydrous magnesium sulfate, filtered, and then concentrated under reduced pressure. The resulting residue was subjected to column chromatography to obtain Core C-4 in the form of an ivory solid (32.8 mg, 55 %).

¹H-NMR (400 MHz, DMSO-d₆) δ 10.01 (s, 1H), 8.23 (s, 1H), 8.12 (d, *J =* 8 Hz, 1H), 7.02 (d, *J* = 8 Hz, 1H), 3.90 (s, 3H).

### Preparation Example 12: Preparation of Core C-5

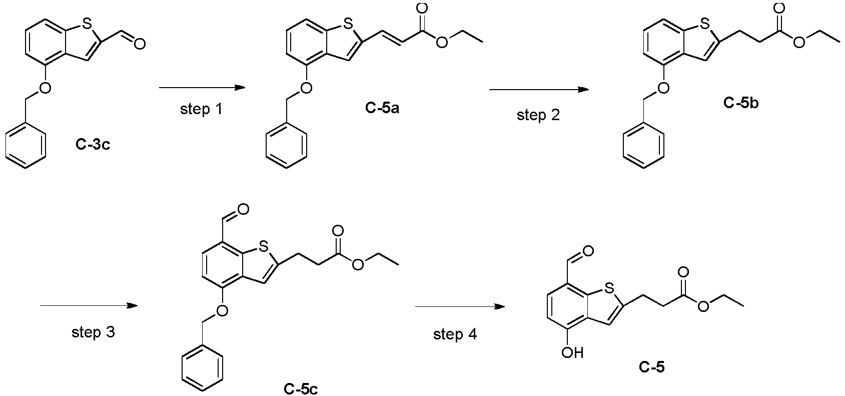

### Step 1: Preparation of Compound C-5a

Compound C-3c (2.1 g, 7.82 mmol) prepared in Step 2 of Preparation Example 10 was dissolved in MC (40 mL) under a nitrogen atmosphere at room temperature, and then ethyl (triphenylphosphoranylidene)acetate (5.45 g, 15.6 mmol) was added, and the mixture was stirred at room temperature for 16 hours. After completion of the reaction, the organic layer was extracted twice with MC (100 mL) and distilled water (100 mL). The obtained organic layer was dried over anhydrous magnesium sulfate, filtered, and then concentrated under reduced pressure. The residue was subjected to column chromatography to obtain Compound C-5a in the form of a white solid (2.5 g, 95 %).

¹H-NMR (400 MHz, CDCl₃) δ 7.85 (d, *J* = 15.6 Hz, 1H), 7.69 (s, 1H), 7.48 - 7.46 (m, 2H), 7.43 - 7.35 (m, 4H), 7.31 - 7.27 (m, 1H), 6.79 (d, *J* = 7.6 Hz, 1H), 6.25 (d, *J* = 15.6 Hz, 1H), 5.21 (s, 2H), 4.26 (q, *J* = 7.2 Hz, 2H), 1.33 (t, *J* = 7.2 Hz, 3H).

### Step 2: Preparation of Compound C-5b

Compound C-5a (4.7 g 13.3 mmol) was dissolved in THF (150 mL) and methanol (500 mL) under a nitrogen atmosphere at room temperature, and 5% palladium charcoal (5% Pd/C, 5.67 g) was added, and then the mixture was reacted under hydrogen atmosphere for 1 hour. After completion of the reaction, the solution was filtered using Celite and concentrated under reduced pressure. The resulting residue was subjected to column chromatography to obtain Compound C-5b in the form of a white solid (4.3 g, 91 %).

¹H-NMR (400 MHz, CDCl₃) δ 7.48 - 7.41 (m, 1H), 7.42 - 7.34 (m, 4H), 7.26 - 7.25 (m, 1H), 7.18 (t, *J* = 8 Hz, 1H), 6.77 (d, *J* = 7.6 Hz, 1H), 5.19 (s, 2H), 4.15 (q, *J* = 7.2 Hz, 2H), 3.22 (t, *J* = 8.0 Hz, 2H), 2.74 (t, *J* = 8.0 Hz, 2H), 1.25 (t, *J* = 7.2 Hz, 3H).

### Step 3: Preparation of Compound C-5c

Compound C-5b (485 mg, 1.42 mmol) was dissolved in MC (30 mL) under a nitrogen atmosphere at -78°C, and then dichloromethyl methyl ether (Merck, CAS NO. 4885-02-3, 400 µL, 4.27 mmol) and titanium tetrachloride solution (1M-TiCl₄ in MC, 4.3 mL, 4.27 mmol) were sequentially and slowly added, and the mixture was stirred for 2 hours while maintaining the temperature. After completion of the reaction, distilled water (250 mL) was slowly added dropwise to terminate the reaction, and MC (250 mL) was added to extract the organic layer twice. The obtained organic layer was dried over anhydrous magnesium sulfate, filtered, and then concentrated under reduced pressure. The resulting residue was subjected to column chromatography to obtain Compound C-5c (290 mg, 55 %).

¹H-NMR (400 MHz, CDCl₃) δ 10.03 (s, 1H), 7.74 (d, *J* = 8.4 Hz, 1H), 7.49 - 7.35 (m, 6H), 6.94 (d, *J* = 8.4 Hz, 1H), 5.30 (s, 2H), 4.15 (q, *J* = 7.2 Hz, 2H), 3.27 (t, *J* = 7.6 Hz, 2H), 2.78 (t, *J* = 7.6 Hz, 2H), 1.24 (t, *J* = 7.2 Hz, 3H).

### Step 4: Preparation of Core C-5

Compound C-5c (279 mg, 0.81 mmol) was dissolved in MC (16 mL) under a nitrogen atmosphere at -78°C, and then boron trichloride solution (1M-BCl₃ in MC, 2.44 mL, 2.44 mmol) was slowly added, and the mixture was stirred for 1.5 hours. After completion of the reaction, the temperature was raised to -50°C, and then distilled water (15 mL) was added dropwise to terminate the reaction. The temperature was raised to 0°C, and 2N-sodium hydroxide aqueous solution (5 mL) was added dropwise. The organic layer was extracted twice from the mixture using MC (30 mL). The obtained organic layer was dried over anhydrous magnesium sulfate, filtered, and then concentrated under reduced pressure. The resulting residue was subjected to column chromatography to obtain Core C-5 in the form of a white solid (150 mg, 66 %).

¹H-NMR (400 MHz, CDCl₃) δ 10.02 (s, 1H), 7.67 (d, J = 7.6 Hz, 1H), 6.84 (d, J = 8 Hz, 1H), 5.94 (s, 1H), 4.16 (q, J = 6.8 Hz, 2H), 3.29 (t, J = 7.6 Hz, 2H), 2.79 (t, J = 7.6 Hz, 2H), 1.26 (t, J = 7.2 Hz, 3H).

### Preparation Example 13: Preparation of Core C-6

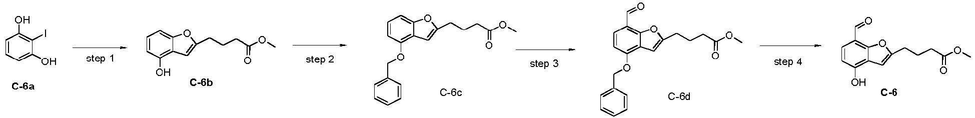

### Step 1: Preparation of Compound C-6b

Compound C-6a (2-iodoresorcinol, CAS NO. 41046-67-7, TCI, 1 g, 4.23 mmol) was dissolved in ACN (150 mL) under a nitrogen atmosphere at 0°C, and then methyl 5-hexynoate (CAS NO. 77758-51-1, Thermoscientific, 600 µL), PdCl₂(TPP)₂ (100 mg), CuI (40 mg) and TEA (5.9 mL, 42.3 mmol) were sequentially added, and the mixture was stirred at room temperature for 1 hour. The mixture was stirred at 60°C for additional 16 hours, and then EA (100 mL) and 2N-hydrochloric acid aqueous solution (100 mL) were added to extract the organic layer, and the obtained organic layer was dried over anhydrous magnesium sulfate, filtered, and then concentrated under reduced pressure. The resulting residue was subjected to column chromatography to obtain Compound C-6b (340 mg, 34.2 %).

¹H-NMR (400 MHz, CDCl₃) δ 7.08-7.01 (m, 2H), 6.59 (m, 1H), 6.47 (s, 1H), 5.15 (s, 1H), 3.67 (s, 3H), 2.82 (t, *J* = 7.2 Hz, 2H), 2.41 (t, *J* = 7.2 Hz, 2H), 2.08 (m, 2H).

### Step 2: Preparation of Compound C-6c

Compound C-6b (690 mg, 2.94 mmol) was dissolved in ACN (20 mL) under a nitrogen atmosphere at 0°C, and then potassium carbonate (1.01 g, 7.36 mmol) and benzyl bromide (420 µL, 3.53 mmol) were slowly added, and the mixture was stirred at 0°C for 30 minutes, and then the mixture was stirred at room temperature for 16 hours. After completion of the reaction, EA (300 mL) and distilled water (300 mL) were added to extract the organic layer. The obtained organic layer was dried over anhydrous magnesium sulfate, filtered, and then concentrated under reduced pressure. The residue was subjected to column chromatography to obtain Compound C-6c in the form of a yellow oil (894 mg, 93 %).

¹H-NMR (400 MHz, CDCl₃) δ 7.48 - 7.46 (m, 2H), 7.41 - 7.32 (m, 3H), 7.13 - 7.04 (m, 2H), 6.69 (d, *J* = 8 Hz, 1H), 6.55 (s, 1H), 5.18 (s, 2H), 3.66 (s, 3H), 2.81 (t, *J* = 7.2 Hz, 2H), 2.41 (t, *J* = 7.2 Hz, 2H), 2.07 (p, *J* = 7.2 Hz, 2H).

### Step 3: Preparation of Compound C-6d

Compound C-6c (894 mg, 2.75 mmol) was dissolved in MC (50 mL) under a nitrogen atmosphere at -78°C, and then dichloromethyl methyl ether (748 µL, 8.27 mmol) and titanium tetrachloride solution (1M-TiCl₄ in MC, 8.27 mL, 8.27 mmol) were sequentially and slowly added, and the mixture was stirred for 1.5 hours while maintaining the temperature. After completion of the reaction, distilled water (250 mL) was slowly added dropwise to terminate the reaction, and MC (250 mL) was added to extract the organic layer twice. The obtained organic layer was dried over anhydrous magnesium sulfate, filtered, and then concentrated under reduced pressure. The resulting residue was subjected to column chromatography to obtain Compound C-6d in the form of a yellow oil (511 mg, 53 %).

¹H-NMR (400 MHz, CDCl₃) δ 10.25 (s, 1H), 7.68 (d, *J* = 8.4 Hz, 1H), 7.47 - 2.27 (m, 5H), 6.81 (d, *J* = 8.4 Hz, 1H), 6.61 (s, 1H), 5.27 (s, 2H), 3.67 (s, 3H), 2.89 (t, *J* = 7.2 Hz, 2H), 2.43 (t, *J* = 7.2 Hz, 2H), 2.11 (p, *J* = 7.2 Hz, 2H).

### Step 4: Preparation of Core C-6

Compound C-6d (511 mg, 1.45 mmol) was dissolved in MC (30 mL) under a nitrogen atmosphere at -78°C, and then boron trichloride solution (1M-BCl₃ in MC, 4.35 mL, 4.35 mmol) was slowly added, and the mixture was stirred for 1.5 hours. After completion of the reaction, distilled water (150 mL) was slowly added dropwise to terminate the reaction, and 2N-sodium hydroxide aqueous solution was added at 0°C to neutralize it. MC (150 mL) was added to the mixture to extract the organic layer twice. The obtained organic layer was dried over anhydrous magnesium sulfate, filtered, and then concentrated under reduced pressure. The resulting residue was subjected to column chromatography to obtain Core C-6 in the form of a white solid (200.2 mg, 52%).

¹H-NMR (400 MHz, DMSO-d₆) δ 10.04 (s, 1H), 7.61 (d, J = 8.4 Hz, 1H), 6.74 (d, J = 8.4 Hz, 1H), 6.71 (s, 1H), 3.58 (s, 3H), 2.82 (t, J = 7.2 Hz, 2H), 2.43 (t, J = 7.2 Hz, 2H), 1.95 (p, J = 7.2 Hz, 2H).

### Preparation Example 14: Preparation of Core C-7

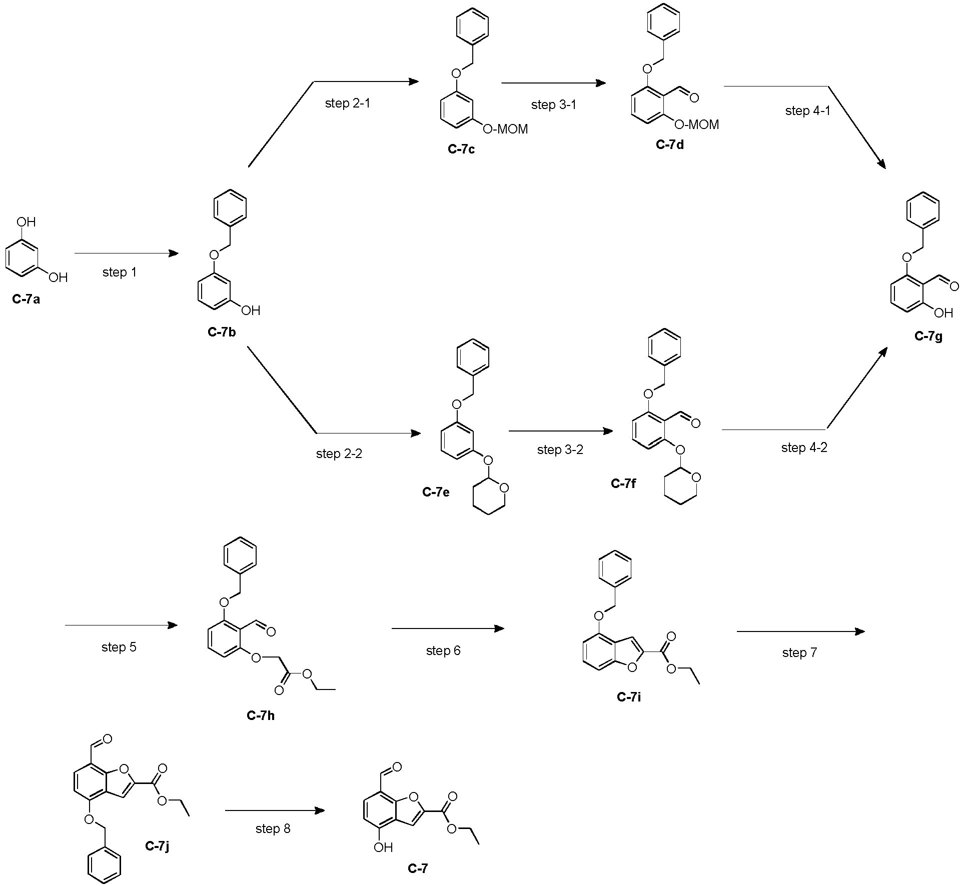

### Step 1: Preparation of Compound C-7b

Compound C-7a (10 g, 90.8 mmol) was dissolved in ACN (200 mL) under a nitrogen atmosphere at room temperature, and then benzyl bromide (7.18 mL, 60.5 mmol) was added, and then the mixture was cooled to 0°C. Potassium carbonate (20.9 g, 151.2 mmol) was added, and the mixture was stirred at room temperature for 20 hours. After completion of the reaction, the organic layer was extracted twice with EA (400 mL) and distilled water (500 mL). The obtained organic layer was dried over anhydrous magnesium sulfate, filtered, and then concentrated under reduced pressure. The residue was subjected to column chromatography to obtain Compound C-7b in the form of a yellow solid (6.6 g, 54 %).

¹H-NMR (400 MHz, CDCl₃) δ 7.43 - 7.36 (m, 4H), 7.34 - 7.32 (m, 1H), 7.13 (t, *J* = 8.4 Hz, 1H), 6.56 (dd, *J* = 2.4, 0.8 Hz, 1H), 6.48 (t, *J* = 2.4 Hz, 1H), 6.43 (dd, *J* = 2.4, 0.8 Hz, 1H), 5.03 (s, 2H), 4.97 (s, 1H).

### Step 2-1: Preparation of Compound C-7c

Compound C-7b (1.0 g, 4.99 mmol) was dissolved in ACN (25 mL) under a nitrogen atmosphere at room temperature, and then chloromethyl methyl ether (7.18 mL, 5.99 mmol) and potassium carbonate (1.37 g, 9.98 mmol) were added, and the mixture was stirred at room temperature for 16 hours. After completion of the reaction, the organic layer was extracted twice with EA (100 mL) and distilled water (100 mL). The obtained organic layer was dried over anhydrous magnesium sulfate, filtered, and then concentrated under reduced pressure. The residue was subjected to column chromatography to obtain Compound C-7c in the form of a white solid (908 mg, 74 %).

¹H-NMR (400 MHz, CDCl₃) δ 7.44 - 7.42 (m, 2H), 7.39 - 7.36 (m, 2H), 7.33 - 7.31 (m, 1H), 7.18 (t, *J* = 8.4 Hz, 1H), 6.70 - 6.69 (m, 1H), 6.66 - 6.62 (m, 2H), 5.15 (s, 2H), 5.04 (s, 2H), 3.47 (s, 3H).

### Step 3-1: Preparation of Compound C-7d

Compound C-7c (900 mg, 3.68 mmol) was added to THF (30 mL) under a nitrogen atmosphere at room temperature, and the mixture was cooled to -40°C, and then n-butyl lithium solution (2.5M n-BuLi in hexane, 1.76 mL, 4.42 mmol) was slowly added dropwise. The temperature of the mixture was raised to -20°C, and then DMF (0.85 mL) was slowly added dropwise, and the temperature was slowly raised to room temperature, and then the mixture was stirred for 1 hour. After completion of the reaction, distilled water (30 mL) was added dropwise to terminate the reaction. The organic layer was extracted twice from the reaction solution using EA (30 mL), and the obtained organic layer was dried over anhydrous magnesium sulfate, filtered, and then concentrated under reduced pressure. The residue was subjected to column chromatography to obtain Compound C-7d in the form of a white solid (300 mg, 30 %).

¹H-NMR (400 MHz, CDCl₃) δ 10.60 (s, 1H), 7.46 - 7.45 (m, 2H), 7.41 - 7.34 (m, 3H), 7.32 - 7.30 (m, 1H), 6.80 (d, *J* = 8.4 Hz, 1H), 6.67 (d, *J* = 8.4 Hz, 1H), 5.26 (s, 2H), 5.18 (s, 2H), 3.50 (s, 3H).

### Step 4-1: Preparation of Compound C-7g (Method 1)

Compound C-7d (300 mg, 1.10 mmol) was dissolved in ethanol (10 mL) under a nitrogen atmosphere at room temperature, and then 2N-hydrochloric acid aqueous solution (1.1 mL) was added, and the mixture was stirred for 16 hours. After completion of the reaction, distilled water (50 mL) was added dropwise to terminate the reaction, and the organic layer was extracted twice with EA (50 mL). The obtained organic layer was dried over anhydrous magnesium sulfate, filtered, and then concentrated under reduced pressure. The residue was subjected to column chromatography to obtain Compound C-7g in the form of a white solid (150 mg, 60 %).

¹H-NMR (400 MHz, CDCl₃) δ 11.95 (s, 1H), 10.41 (s, 1H), 7.41 - 7.35 (m, 6H), 6.54 (d, *J* = 8.4 Hz, 1H), 6.45 (d, *J* = 8.4 Hz, 1H), 5.14 (s, 2H), 3.88 (td, *J* = 10.8, 3.2 Hz, 1H), 3.63 - 3.60 (m, 1H), 2.05 - 1.98 (m, 1H), 1.97 - 1.86 (m, 2H), 1.73 - 1.60 (m, 3H).

### Step 2-2: Preparation of Compound C-7e

Compound C-7b (22 g, 109.8 mmol) was dissolved in MC (200 mL) under a nitrogen atmosphere at room temperature, and then 3,4-dihydro-2H-pyran (12.0 mL, 131.8 mmol) and PPTS (2.76 g, 10.9 mmol) were added, and the mixture was stirred at room temperature for 16 hours. After completion of the reaction, the organic layer was extracted twice with MC (500 mL) and distilled water (500 mL). The obtained organic layer was dried over anhydrous magnesium sulfate, filtered, and then concentrated under reduced pressure. The residue was subjected to column chromatography to obtain Compound C-7e in the form of a white solid (23.9 g, 76 %).

¹H-NMR (400 MHz, CDCl₃) δ 7.44 - 7.42 (m, 2H), 7.40 - 7.36 (m, 2H), 7.33 - 7.29 (m, 1H), 7.17 (t, *J* = 8.4 Hz, 1H), 6.72 (t, *J* = 2.4 Hz, 1H), 6.66 (dd, *J* = 2.4, 0.8 Hz, 1H), 6.61 (dd, *J* = 2.4, 0.8 Hz, 1H), 5.39 (t, *J* = 3.6 Hz, 1H), 5.04 (s, 2H), 3.94 - 3.88 (m, 1H), 3.62 - 3.57 (m, 1H), 2.04 - 1.95 (m, 1H), 1.86 - 1.82 (m, 2H), 1.69 - 1.53 (m, 3H).

### Step 3-2: Preparation of Compound C-7f

Compound C-7e (7.7 g, 27.0 mmol) was dissolved in THF (200 mL) under a nitrogen atmosphere at room temperature, and then the mixture was cooled to 0°C, and then n-butyl lithium solution (2.5M n-BuLi in hexane, 13.0 mL, 32.4 mmol) was slowly added dropwise. The reaction solution was stirred for 30 minutes, and then DMF (6.3 mL) was slowly added dropwise, and the temperature was slowly raised to room temperature, and then the mixture was stirred for an additional 1 hour. After completion of the reaction, distilled water (500 mL) was added dropwise to terminate the reaction. The organic layer was extracted twice from the reaction solution using EA (500 mL), and the obtained organic layer was dried over anhydrous magnesium sulfate, filtered, and then concentrated under reduced pressure. The residue was subjected to column chromatography to obtain Compound C-7f in the form of a white solid (2.5 g, 27 %).

¹H-NMR (400 MHz, CDCl₃) δ 10.62 (s, 1H), 7.47 - 7.46 (m, 2H), 7.40 - 7.36 (m, 3H), 7.35 - 7.31 (m, 1H), 6.82 (d, *J* = 8.4 Hz, 1H), 6.64 (d, *J* = 8.4 Hz, 1H), 3.88 (td, *J* = 10.8, 3.2 Hz, 1H), 3.63 - 3.60 (m, 1H), 2.05 - 1.98 (m, 1H), 1.97 - 1.86 (m, 2H), 1.73 - 1.60 (m, 3H).

### Step 4-2: Preparation of Compound C-7g (Method 2)

Compound C-7f (3.98 g, 12.7 mmol) was dissolved in ethanol (80 mL) under a nitrogen atmosphere at room temperature, and then PPTS (2.76 g, 10.9 mmol) was added. The mixture was stirred at 60°C for 2 hours. After completion of the reaction, distilled water (300 mL) was added dropwise to terminate the reaction. EA (300 mL) was added to the reaction solution to extract the organic layer twice, and the obtained organic layer was dried over anhydrous magnesium sulfate, filtered, and then concentrated under reduced pressure. The residue was subjected to column chromatography to obtain Compound C-7g in the form of a white solid (2.6 g, 89 %).

¹H-NMR (400 MHz, CDCl₃) δ 11.97 (s, 1H), 10.41 (s, 1H), 7.42 - 7.38 (m, 6H), 6.54 (d, *J* = 8.4 Hz, 1H), 6.45 (d, *J* = 8.4 Hz, 1H), 5.14 (s, 2H), 3.88 (td, *J* = 10.8, 3.2 Hz, 1H), 3.63 - 3.60 (m, 1H), 2.05 - 1.98 (m, 1H), 1.97 - 1.86 (m, 2H), 1.73 - 1.60 (m, 3H).

### Step 5: Preparation of Compound C-7h

Compound C-7g (2.5 g, 10.9 mmol) was dissolved in DMF (50 mL) under a nitrogen atmosphere at room temperature, and then ethyl chloroacetate (1.40 mL, 13.1 mmol) and potassium carbonate (3.02 g, 21.9 mmol) were added, and the mixture was stirred at 80°C for 2 hours. After completion of the reaction, distilled water (300 mL) was added dropwise to terminate the reaction. EA (300 mL) was added to the reaction solution to extract the organic layer twice, and an aqueous sodium chloride solution was added to the obtained organic layer to extract the organic layer again. The extracted organic layer was dried over anhydrous magnesium sulfate, filtered, and then concentrated under reduced pressure. The residue was subjected to column chromatography to obtain Compound C-7h in the form of a white solid (2.7g mg, 77 %).

¹H-NMR (400 MHz, CDCl₃) δ 10.63 (s, 1H), 7.46 - 7.45 (m, 2H), 7.40 - 7.36 (m, 3H), 7.33 - 7.32 (m, 1H), 6.68 (d, *J* = 8.4 Hz, 1H), 6.45 (d, *J* = 8.4 Hz, 1H), 5.18 (s, 2H), 4.72 (s, 2H), 4.26 (q, *J* = 7.2 Hz, 2H), 1.28 (d, *J* = 7.2 Hz, 3H).

### Step 6: Preparation of Compound C-7i

Compound C-7h (2.7 g, 8.59 mmol) was dissolved in DMF (50 mL) under a nitrogen atmosphere at room temperature, and then potassium carbonate (1.78 g, 12.9 mmol) was added, and the mixture was stirred at 100°C for 2 hours. After completion of the reaction, distilled water (200 mL) was added dropwise to terminate the reaction. The organic layer was extracted twice from the reaction solution using EA (300 mL), and the obtained organic layer was washed by adding an aqueous sodium chloride solution. The washed organic layer was dried over anhydrous magnesium sulfate, filtered, and then concentrated under reduced pressure. The residue was subjected to column chromatography to obtain Compound C-7i in the form of a white solid (2.1g mg, 82 %).

¹H-NMR (400 MHz, CDCl₃) δ 7.67 (s, 1H), 7.48 - 7.46 (m, 2H), 7.43 - 7.39 (m, 2H), 7.37 - 7.32 (m, 2H), 7.20 (d, *J* = 8.4 Hz, 1H), 6.75 (d, *J* = 8.4 Hz, 1H), 5.21 (s, 2H), 4.42 (q, *J* = 7.2 Hz, 2H), 1.42 (t, *J* = 7.2 Hz, 3H).

### Step 7: Preparation of Compound C-7j

Compound C-7i (910 mg, 3.07 mmol) was dissolved in MC (60 mL) under a nitrogen atmosphere at -78°C, and then dichloromethyl methyl ether (833 µL, 9.21 mmol) and titanium tetrachloride solution (1M-TiCl₄ in MC, 9.2 mL, 9.21 mmol) were sequentially and slowly added, and the mixture was stirred for 2 hours while maintaining the temperature. After completion of the reaction, distilled water (100 mL) was slowly added dropwise to terminate the reaction, and EA (100 mL) was added to extract the organic layer. The obtained organic layer was dried over anhydrous magnesium sulfate, filtered, and then concentrated under reduced pressure. The resulting residue was subjected to column chromatography to obtain Compound C-7j (270 mg, 26.7%).

¹H-NMR (400 MHz, CDCl₃) δ 10.43 (s, 1H), 7.95 (d, *J* = 8.4 Hz, 1H), 7.71 (s, 1H), 7.47-7.38 (m, 5H), 6.88 (d, *J* = 8.4 Hz, 1H), 5.30 (s, 2H), 4.44 (q, *J* = 7.2 Hz, 2H), 1.42 (t, *J* = 7.2 Hz, 3H).

### Step 8: Preparation of Core C-7

Compound C-7j (250 mg, 0.77 mmol) was dissolved in MC (15 mL) under a nitrogen atmosphere at -78°C, and then boron trichloride solution (1M-BCl₃ in MC, 4.3 mL, 4.3 mmol) was slowly added, and the mixture was stirred for 1 hour. After completion of the reaction, distilled water (50 mL) was slowly added dropwise at -50°C to terminate the reaction, and EA (100 mL) and distilled water (100 mL) were further added at room temperature to extract the organic layer. The obtained organic layer was dried over anhydrous magnesium sulfate, filtered, and then concentrated under reduced pressure. The resulting residue was subjected to column chromatography to obtain Core C-7 in the form of a white solid (150 mg, 83 %).

¹H-NMR (400 MHz, CDCl₃) δ 10.37 (s, 1H), 7.85 (d, J = 8.4 Hz, 1H), 7.76 (s, 1H), 6.81 (d, J = 8.4 Hz, 1H), 4.44 (q, J = 7.2 Hz, 2H), 1.43 (t, J = 7.2 Hz, 3H).

### Preparation Example 15: Preparation of Core C-8

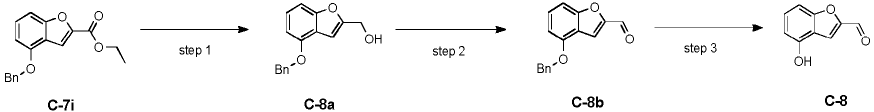

### Step 1: Preparation of Compound C-8a

LAH (2.65 g, 69.77 mmol) was added to THF (350 mL) under a nitrogen atmosphere at 0°C, and then Compound C-7i (8.27 g, 27.91 mmol) was dissolved in THF (50 mL) and slowly added, and the mixture was stirred at 0°C for 1 hour. After completion of the reaction, distilled water (300 mL) was added to terminate the reaction, and the reaction solution was diluted with EA (300 mL) and filtered using Celite. The filtrate was concentrated under reduced pressure at low temperature to reduce the amount of the THF-containing solvent by half, and then EA (500 mL) was added to extract the organic layer twice. The obtained organic layer was dried over anhydrous magnesium sulfate, filtered, and then concentrated under reduced pressure. The resulting residue was subjected to column chromatography to obtain C-8a in the form of a white solid (6.99 g, 98 %).

¹H-NMR (400 MHz, CDCl₃) δ 7.47 - 7.45 (m, 2H), 7.41 - 7.37 (m, 2H), 7.35 (m, 1H), 7.18 (t, *J* = 8 Hz, 1H), 7.10 (d, *J* = 8 Hz, 1H), 6.80 (s, 1H), 6.71 (d, *J* = 8 Hz, 1H), 5.19 (s, 2H), 4.73 (d, *J* = 6 Hz, 2H), 1.88 (t, *J* = 6 Hz, 1H).

### Step 2: Preparation of Compound C-8b

Dess-Martin Periodinane (7.02 g, 16.56 mmol) was added to MC (25 mL) under a nitrogen atmosphere at 0°C, and then Compound C-8a (3.5 g, 13.80 mmol) dissolved in MC (10 mL) was slowly added, and the mixture was stirred at 0°C for 30 minutes. The mixture was stirred for additional 3 hours while raising the temperature to room temperature. After completion of the reaction, the reaction solution was diluted with MC (200 mL) and filtered using Celite, and then distilled water (200 mL) was added to extract the organic layer twice. The obtained organic layer was dried over anhydrous magnesium sulfate, filtered, and then concentrated under reduced pressure. The resulting residue was subjected to column chromatography to obtain C-8b in the form of a pale yellow solid (3.31 g, 95 %).

¹H-NMR (400 MHz, CDCl₃) δ 9.80 (s, 1H), 7.71 (s, 1H), 7.48 - 7.36 (m, 6H), 7.20 (d, *J* = 8 Hz, 1H), 6.77 (d, *J* = 8 Hz, 1H), 5.22 (s, 2H).

### Step 3: Preparation of Core C-8

Compound C-8b (1 g, 3.96 mmol) was dissolved in MC (80 mL) under a nitrogen atmosphere at -78°C, and then boron trichloride solution (1M-BCl₃ in MC, 11.89 mL, 11.89 mmol) was slowly added, and the mixture was stirred at -78°C for 2 hours. After completion of the reaction, distilled water (250 mL) was slowly added dropwise to terminate the reaction, and 2N-sodium hydroxide aqueous solution was added at 0°C to neutralize it. MC (250 mL) was added to the mixture to extract the organic layer three times. The obtained organic layer was dried over anhydrous magnesium sulfate, filtered, and then concentrated under reduced pressure. The resulting residue was subjected to column chromatography to obtain Core C-8 in the form of a yellow solid (580 mg, 90 %).

¹H-NMR (400 MHz, DMSO-d₆) δ 10.61 (brs, 1H), 9.78 (s, 1H), 7.97 (s, 1H), 7.38 (t, *J* = 8.4 Hz, 1H), 7.12 (d, *J* = 8.4 Hz, 1H), 6.71 (d, *J* = 8.4 Hz, 1H).

### Preparation Example 16: Preparation of Core C-9

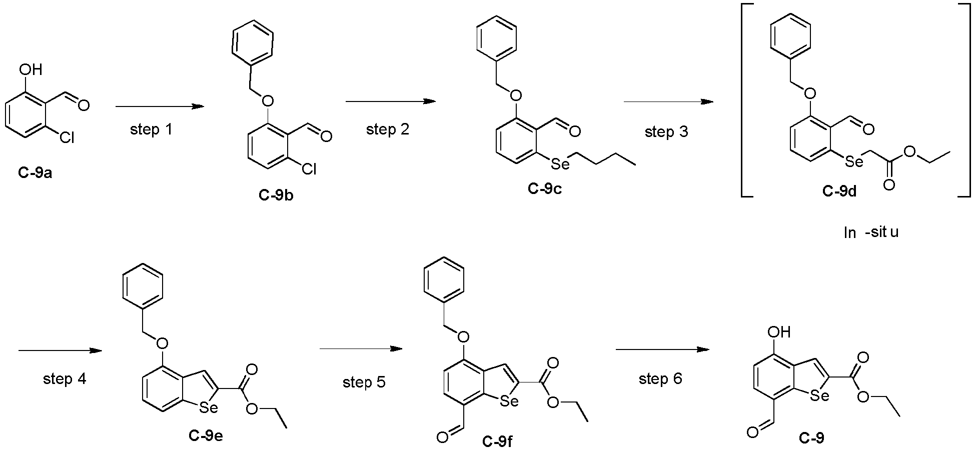

### Step 1: Preparation of Compound C-9b

Compound C-9a (2-chloro-6-hydroxybenzaldehdye, Merck, CAS NO. 18362-30-6, 500 mg, 3.19 mmol) was dissolved in ACN (10 mL) under a nitrogen atmosphere at 0°C, and then benzyl bromide (400 µL, 3.35 mmol) and potassium carbonate (1.1 g, 7.98 mmol) were added, and the mixture was stirred at room temperature for 16 hours. After completion of the reaction, EA (100 mL) and distilled water (100 mL) were added to extract the organic layer. The obtained organic layer was dried over anhydrous magnesium sulfate, filtered, and then concentrated under reduced pressure. The residue was subjected to column chromatography to obtain Compound C-9b in the form of a white solid (750 mg, 95 %).

¹H-NMR (400 MHz, CDCl₃) δ 10.57 (s, 1H), 7.45 - 7.34 (m, 6H), 7.04 (d, *J* = 8 Hz, 1H), 6.96 (d, *J* = 8.8 Hz, 1H), 5.19 (s, 2H).

### Step 2: Preparation of Compound C-9c

Selenium powder (256 mg, 3.24 mmol) was added to THF (10 mL) under a nitrogen atmosphere at room temperature, and the mixture was cooled to 0°C, and then n-butyl lithium solution (2.5M n-BuLi in hexane, 1.42 mL, 3.56 mmol) was slowly added dropwise. The mixture was stirred at 0°C for 40 minutes, and then Compound C-9b (800 mg, 3.24 mmol) dissolved in DMF (2 mL) was added, and the mixture was stirred for 12 hours at room temperature. After completion of the reaction, the mixture was cooled to 0°C, and distilled water (100 mL) was slowly added dropwise to terminate the reaction. EA (100 mL) was added to the reaction solution, and then the reaction solution was extracted, and the obtained organic layer was dried over anhydrous magnesium sulfate, filtered, and then concentrated under reduced pressure. The residue was subjected to column chromatography to obtain Compound C-9c in the form of a light yellow solid (900 mg, 80 %).

¹H-NMR (400 MHz, CDCl₃) δ 10.70 (s, 1H), 7.42-7.33 (m, 6H), 7.04 (d, *J* = 8 Hz, 1H), 6.81 (d, *J* = 8.8 Hz, 1H), 5.18 (s, 2H), 2.85 (t, *J* = 7.6 Hz, 2H), 1.75 (qui, *J* = 7.6 Hz, 2H), 1.51 (m, 2H), 0.95 (t, *J* = 7.6 Hz, 3H).

### Step 3: Preparation of Compound C-9d

Compound C-9c (900 mg, 2.59 mmol) was dissolved in DMF (10 mL) under a nitrogen atmosphere at room temperature, and then ethyl bromoacetate (574 µL, 6.48 mmol) was added, and the mixture was stirred at 120°C for 12 hours. After completion of the reaction, the temperature of the reaction solution was cooled to room temperature, which was used in the next reaction without an additional purification process.

### Step 4: Preparation of Compound C-9e

Potassium carbonate (716 mg, 6.48 mmol) was added to the reaction solution under a nitrogen atmosphere, and the mixture was stirred at 120°C for 2.5 hours. After completion of the reaction, EA (100 mL) and distilled water (200 mL) were added to extract the organic layer. The organic layer was further washed by adding distilled water (200 mL) to the obtained organic layer again, and the organic layer was dried over anhydrous magnesium sulfate, filtered, and then concentrated under reduced pressure. The residue was subjected to column chromatography to obtain Compound C-9e in the form of a white solid (840 mg, 90 %).

¹H-NMR (400 MHz, CDCl₃) δ 8.55 (s, 1H), 7.49-7.47 (m, 3H), 7.43-7.29 (m, 4H), 6.84 (d, *J* = *8* Hz, 1H), 5.21 (s, 2H), 4.37 (q, *J* = 7.2 Hz, 2H), 1.39 (t, *J* = 7.2 Hz, 3H); EI-MS m/z: 361 [M+H]⁺.

### Step 5: Preparation of Compound C-9f

Compound C-9e (400 mg, 1.11 mmol) was dissolved in MC (30 mL) under a nitrogen atmosphere at -78°C, and then dichloromethyl methyl ether (300 µL, 3.33 mmol) and titanium tetrachloride solution (1M-TiCl₄ in MC, 3.33 mL, 3.33 mmol) were sequentially and slowly added, and the mixture was stirred for 2 hours while maintaining the temperature. After completion of the reaction, distilled water (100 mL) was slowly added dropwise to terminate the reaction, and EA (100 mL) was added to extract the organic layer. The obtained organic layer was dried over anhydrous magnesium sulfate, filtered, and then concentrated under reduced pressure. The resulting residue was subjected to column chromatography to obtain Compound C-9f (310 mg, 72 %).

¹H-NMR (400 MHz, CDCl₃) δ 10.09 (s,1H), 8.62 (s, 1H), 7.89 (d, *J* = 8 Hz, 1H), 7.49-7.38 (m, 5H), 7.03 (d, *J* = 8 Hz, 1H), 5.34 (s, 2H), 4.39 (q, *J* = 7.2 Hz, 2H), 1.41 (t, *J* = 7.2 Hz, 3H); EI-MS m/z: 388 [M+H]⁺.

### Step 6: Preparation of Core C-9

Compound C-9f (300 mg, 0.77 mmol) was dissolved in MC (15 mL) under a nitrogen atmosphere at -78°C, and then boron trichloride solution (1M-BCl₃ in MC, 4.62 mL, 4.62 mmol) was slowly added, and the mixture was stirred for 1.5 hours. After completion of the reaction, distilled water (100 mL) was slowly added dropwise to terminate the reaction, and EA (100 mL) was added to extract the organic layer. The obtained organic layer was dried over anhydrous magnesium sulfate, filtered, and then concentrated under reduced pressure. The resulting residue was subjected to column chromatography to obtain Core C-9 in the form of a white solid (185 mg, 80 %).

¹H-NMR (400 MHz, DMSO-d₆) δ 10.05 (s, 1H), 8.47 (s, 1H), 8.09 (d, *J* = 8 Hz, 1H), 7.05 (d, *J* = 8 Hz, 1H), 4.33 (q, *J* = 7.2 Hz, 2H), 1.34 (t, *J* = 7.2 Hz, 3H).

### Preparation Example 17: Preparation of Core C-10

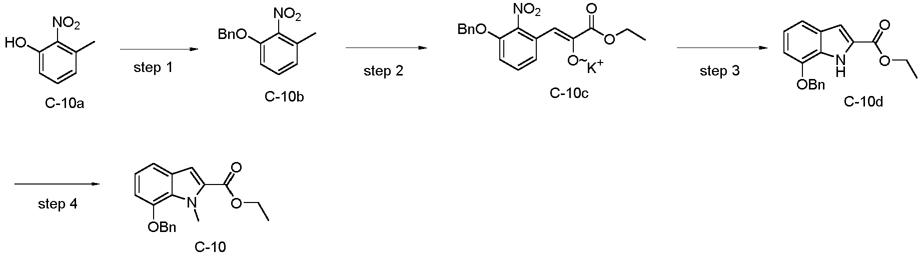

### Step 1: Preparation of Compound C-10b

Compound C-10a (2-nitro-*m*-cresol, TCI, CAS NO. 4920-77-8, 20 g, 130.59 mmol) was dissolved in ACN (250 mL) under a nitrogen atmosphere at 0°C, and then benzyl chloride (15.78 mL, 137.12 mmol) and potassium carbonate (K₂CO₃, 22.56 g, 163.24 mmol) were added, and the mixture was stirred for 16 hours at 80°C. After completion of the reaction, EA (200 mL) and distilled water (300 mL) were added to extract the organic layer. The obtained organic layer was dried over anhydrous magnesium sulfate, filtered, and then concentrated under reduced pressure. The residue was subjected to column chromatography to obtain Compound C-10b in the form of a light yellow oil (31.76 g, 99 %).

¹H-NMR (400 MHz, CDCl₃) δ 7.37 - 7.31 (m, 5H), 7.24 (m, 1H), 6.89 - 6.83 (m, 2H), 5.15 (s, 2H), 2.31 (s, 3H).

### Step 2: Preparation of Compound C-10c

KO^{t}Bu (15.35 g, 136.82 mmol) was dissolved in THF (100 mL) and diethyl ether (300 mL) under a nitrogen atmosphere at room temperature, and diethyl oxalate (Merck, CAS NO. 95-92-1, 19.46 mL, 143.34 mmol) was added, and then the mixture was stirred for 15 minutes. Compound C-10b (31.7 g, 31.7 mmol) dissolved in THF (50 mL) was slowly added to the reaction solution, and then the mixture was stirred at room temperature for 19 hours and stirred at 80°C for additional 3 hours. After completion of the reaction, the reaction solution was cooled to room temperature, diethyl ether (200 mL) was added dropwise, and the resulting precipitate was filtered, further washed with diethyl ether (100 mL), and then dried to obtain Compound C-10c in the form of a light orange solid (41.5g, 83 %).

¹H-NMR (400 MHz, DMSO-d₆) δ 8.66 (d, *J* = 8 Hz, 1H), 7.40-7.30 (m, 5H), 7.04 (t, *J* = 8 Hz, 1H), 6.50 (d, *J* = 8 Hz, 1H), 5.15 (s, 1H), 5.10 (s, 2H), 3.99 (q, *J* = 7.2 Hz, 2H), 1.17 (t, *J* = 7.2 Hz, 3H).

### Step 3: Preparation of Compound C-10d

Iron powder (57.1 g, 1.02 mol) was slowly added to acetic acid (200 mL) under a nitrogen atmosphere at room temperature, and while stirring, Compound C-10c (39 g, 102.24 mmol) dissolved in acetic acid (150 mL) was added, and the mixture was stirred at 80°C for 6 hours. After completion of the reaction, the temperature of the reaction solution was lowered to 45°C, and EA (500 mL) was added dropwise, and then the mixture was stirred for additional 30 minutes, and the filtrate obtained through Celite filtration was concentrated under reduced pressure. EA (300 mL) was added to the concentrate thus obtained, and the resulting precipitate was filtered once again. The filtrate was concentrated under reduced pressure. The resulting residue was subjected to column chromatography to obtain Compound C-10d in the form of a light yellow solid (22.18 g, 73 %).

¹H-NMR (400 MHz, CD₃OD-d₄) 7.57-7.55 (m, 2H), 7.43-7.39 (m, 2H), 7.36-7.32 (m, 1H), 7.23 (d, *J* = *8* Hz, 1H), 7.15 (s, 1H), 6.98 (t, *J* = 8 Hz, 1H), 6.83 (d, *J* = 7.6 Hz, 1H), 5.29 (s, 2H), 4.39 (q, *J* = 7.2 Hz, 2H), 1.42 (t, *J* = 7.2 Hz, 3H).

### Step 4: Preparation of Core C-10

Compound C-10d (6 g, 20.31 mmol) was dissolved in DMF (30 mL) under a nitrogen atmosphere at 0°C, and then potassium carbonate (4.21 g, 30.46 mmol) and methyl iodide (1.64 mL, 26.40 mmol) were sequentially added, and the mixture was stirred at 0°C for 1 hour. The reaction temperature was raised to room temperature, and the mixture was stirred for 12 hours. Then, the mixture was stirred for additional 3 hours at 60°C to complete the reaction. The temperature of the reaction solution was cooled to room temperature, and EA (200 mL) and distilled water (400 mL) were added to extract the organic layer. Distilled water (300 mL) was added to extract the obtained organic layer again. The extracted organic layer was dried over anhydrous magnesium sulfate, filtered, and then concentrated under reduced pressure. The obtained Core C-10 was used in the next reaction without an additional purification process (6.28 g, 99 %).

¹H-NMR (400 MHz, CDCl₃) δ 7.49-7.47 (m, 2H), 7.42-7.39 (m, 2H), 7.37-7.33 (m, 1H), 7.25-7.23 (m, 2H), 6.99 (t, *J* = 8 Hz, 1H), 6.77 (d, *J* = 7.6 Hz, 1H), 5.18 (s, 2H), 4.37 (s, 3H), 4.35 (q, *J* = 7.2 Hz, 2H), 1.40 (t, *J* = 7.2 Hz, 3H).

### Preparation Example 18: Preparation of Linker Q-1

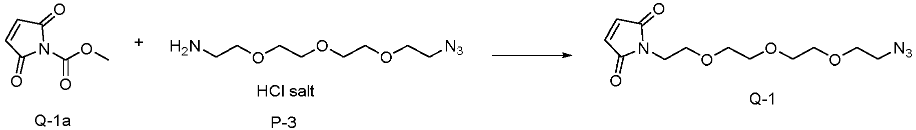

Linker P-3 (164 mg, 0.64 mmol) was dissolved in a saturated sodium bicarbonate aqueous solution (3.5 mL) under a nitrogen atmosphere at 0°C, and then the mixture was stirred at 0°C for 15 minutes. Compound Q-1a (N-methoxycarbonylmaleimide, TCI, CAS No. 55750-48-6, 100 mg, 0.64 mmol) was slowly added to the mixture, and the mixture was stirred at the same temperature for 1.5 hours. After completion of the reaction, EA (50 mL) and distilled water (50 mL) were added to extract the organic layer twice. The obtained organic layer was dried over anhydrous magnesium sulfate, filtered, and then concentrated under reduced pressure at a temperature of 15°C or lower. The residue thus obtained was subjected to column chromatography to obtain Linker Q-1 in the form of a colorless oil (85.3 mg, 44 %).

¹H-NMR (400 MHz, CDCl₃) δ 6.70 (s, 2H), 3.74 - 3.61 (m, 14H), 3.39 (t, *J* = 5.2 Hz, 2H); EI-MS m/z: 321[M+Na]⁺.

### Preparation Example 19: Preparation of Linker Q-2

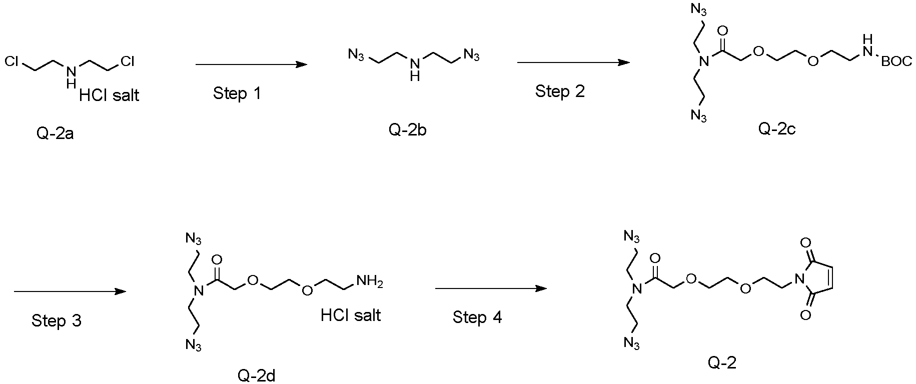

### Step 1: Preparation of Compound Q-2b

Compound Q-2a (bis(2-chloroethyl)amine hydrochloride, CAS NO. 821-48-7, 2 g, 11.20 mmol) was dissolved in distilled water (50 mL) under a nitrogen atmosphere at room temperature, and then sodium azide (1.7 g, 25.77 mmol) was added, and the mixture was stirred at 60°C for 48 hours. After completion of the reaction, the mixture was cooled to room temperature, concentrated under reduced pressure, and filtered by adding EA (50 mL) to the residue. The filtrate was concentrated under reduced pressure to obtain Compound Q-2b in the form of a colorless liquid (1.37 g, 78 %). The obtained Compound Q-2b was used in the next reaction without an additional purification process.

¹H-NMR (400 MHz, CD₃OD) δ 3.44 (t, *J* = 6 Hz, 4H), 2.78 (t, *J* = 6 Hz, 4H).

### Step 2: Preparation of Compound Q-2c

Compound Q-2b (287 mg, 1.85 mmol) and Linker P-2 (487 mg, 1.85 mmol) were dissolved in DMF (10 mL) under a nitrogen atmosphere at 0°C, and then EDCI·HCl (532 mg, 2.77 mmol), HOBt (250 mg, 1.85 mmol), DMAP (22 mg, 0.18 mmol) and DIPEA (1 mL, 5.55 mmol) were sequentially added, and the mixture was stirred for 16 hours while slowly raising the temperature from 0°C to room termperature. After completion of the reaction, the organic layer was extracted twice with EA (150 mL), distilled water (100 mL) and 2N-hydrochloric acid solution (50 mL). The organic layer was washed twice by adding a saturated aqueous sodium chloride solution (200 mL). The obtained organic layer was dried over anhydrous magnesium sulfate, filtered, and then concentrated under reduced pressure. The resulting residue was subjected to column chromatography to obtain Q-2c in the form of a colorless oil (432 mg, 58 %).

¹H-NMR (400 MHz, CDCl₃) δ 5.03 (brs, 1H), 4.28 (s, 2H), 3.72 - 3.68 (m, 2H), 3.67 - 3.63 (m, 2H), 3.60 - 3.48 (m, 10H), 3.35 - 3.30 (m, 2H), 1.45 (s, 9H); EI-MS m/z: 401[M+H]⁺.

### Step 3: Preparation of Compound Q-2d

Compound Q-2c (432 mg, 1.08 mmol) was dissolved in MC (2 mL) under a nitrogen atmosphere at 0°C, and then 4N-hydrochloric acid solution (4M-HCl in dioxane, 4 mL) was added, and the mixture was stirred for 1 hour at 0°C, followed by stirring for 2 hours at room temperature. After completion of the reaction, the mixture was concentrated under reduced pressure to obtain Compound Q-2d in the form of a light yellow solid (369 mg, 99 %). The obtained Compound Q-2d was used in the next reaction without an additional purification process.

¹H-NMR (400 MHz, CDCl₃) δ 8.39 (brs, 2H), 4.37 (s, 2H), 3.92 - 3.87 (m, 2H), 3.77 - 3.71 (m, 4H), 3.63 - 3.56 (m, 4H), 3.54 - 3.48 (m, 2H), 3.48 - 3.44 (m, 2H), 3.29 - 3.21 (m, 2H); EI-MS m/z: 301[M+H]⁺.

### Step 4: Preparation of Linker Q-2

Compound Q-2d (48 mg, 0.14 mmol) was dissolved in a saturated sodium bicarbonate aqueous solution (1mL) under a nitrogen atmosphere at 0°C, and then the mixture was stirred for 15 minutes at 0°C. Compound Q-1a (TCI, 22 mg, 0.14 mmol) was slowly added to the mixture, and the mixture was stirred at the same temperature for 1.5 hours. After completion of the reaction, EA (50 mL) and distilled water (50 mL) were added to extract the organic layer twice. The obtained organic layer was dried over anhydrous magnesium sulfate, filtered, and then concentrated under reduced pressure at a temperature of 10°C or lower. The residue thus obtained was subjected to column chromatography to obtain Linker Q-2 in the form of a colorless oil (17.2 mg, 32%).

¹H-NMR (400 MHz, CDCl₃) δ 6.71 (s, 2H), 4.26 (s, 2H), 3.76 - 3.72 (m, 2H), 3.67 - 3.61 (m, 6H), 3.59 - 3.54 (m, 6H), 3.52 - 3.48 (m, 2H).

### Preparation Example 20: Preparation of Linker Q-3

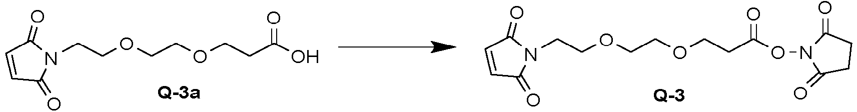

Compound Q-3a (Mal-PEG2-acid, CAS NO. 1374666-32-6, TCI, 100 mg, 0.388 mmol) was dissolved in MC (5 mL) under a nitrogen atmosphere at 0°C, and then NHS (49.2 mg, 0.427 mmol) and DCC (88.2 mg, 0.427 mmol) were added, and the mixture was stirred for 15 hours. After completion of the reaction, EA/n-hexane (1:1 volume ratio, 20 mL) was added, and the resulting precipitate was removed by filtration. The filtrate was concentrated, and then EA/n-hexane (1:1 volume ratio, 20 mL) was added once again, and the resulting precipitate was removed, and the filtrate was concentrated under reduced pressure to obtain Linker Q-3 (139 mg, 99 %).

¹H-NMR (400 MHz, CDCl₃) δ 6.70 (s, 2H), 3.81 (t, *J* = 6.4 Hz, 2H), 3.72 (m, 2H), 3.65-3.58 (m, 6H), 2.87 (t, *J* = 6.4 Hz, 2H), 2.84 (s 4H); EI-MS m/z: 355 [M+H]⁺.

### Preparation Example 21: Preparation of Linker Q-4

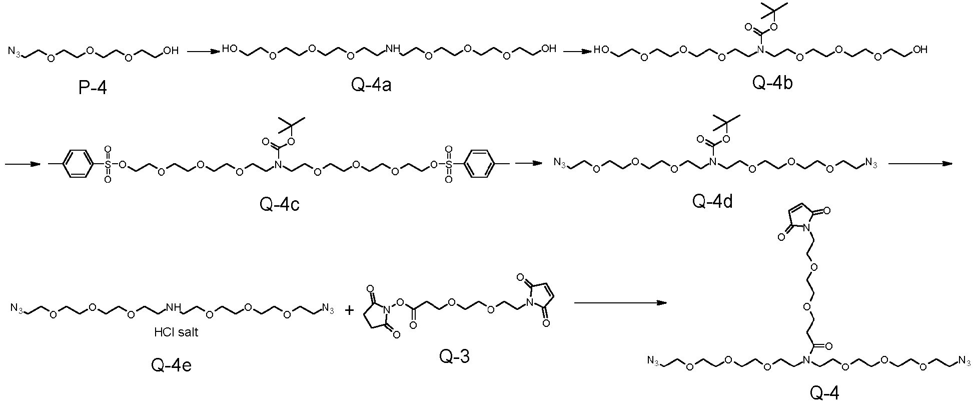

### Step 1: Preparation of Compound Q-4a

Compound P-4 (4.05 g, 18.47 mmol) was dissolved in ethanol (30 mL) under a nitrogen atmosphere at room temperature, and then 5% palladium charcoal (5% Pd/C, 236 mg, 0.11 mmol) was added, and the mixture was stirred for 16 hours under a hydrogen environment at room temperature. After completion of the reaction, the reaction solution was diluted with EA (250 mL) and filtered using a Celite filter to obtain a yellow liquid mixture containing Compound Q-4a (3.48 g). The obtained mixture containing Q-4a was used in the next reaction without an additional purification process.

¹H-NMR (400 MHz, CDCl₃) δ 3.74 - 3.69 (m, 4H), 3.69 - 3.59 (m, 24H), 2.80 (t, *J* = 5.2 Hz, 4H); EI-MS m/z: 370[M+H]⁺.

### Step 2: Preparation of Compound Q-4b

Compound Q-4a (3.48 g, 9.42 mmol), and TEA (4 mL, 28.26 mmol) and di-tert-butyl dicarbonate (Boc anhydride, 3.08 g, 14.13 mmol), which were dissolved in MC (60 mL), were sequentially added under a nitrogen atmosphere at 0°C, and the mixture was stirred at room temperature for 16 hours. After completion of the reaction, the organic layer was extracted three times with MC (150 mL), distilled water (100 mL) and a saturated aqueous sodium chloride solution (50 mL). The obtained organic layer was dried over anhydrous magnesium sulfate, filtered, and then concentrated under reduced pressure. The resulting residue was subjected to column chromatography to obtain Q-4b in the form of a colorless oil (1.32 g, 32% 2 step-yield).

¹H-NMR (400 MHz, CDCl₃) δ 3.76 - 3.69 (m, 4H), 3.69 - 3.54 (m, 24H), 3.50 - 3.40 (m, 4H), 2.77 (brs, 2H), 1.45 (s, 9H); EI-MS m/z: 470[M+H]⁺.

### Step 3: Preparation of Compound Q-4c

Compound Q-4b (595 mg, 1.27 mmol) was dissolved in MC (16 mL) under a nitrogen atmosphere at 0°C, and then TEA (1.8 mL, 5.07 mmol) and DMAP (31 mg, 0.25 mmol) were added, and the mixture was stirred for 10 minutes at the same temperature. 4-methylbenzenesulfonyl chloride (966 mg, 5.07 mmol) was added to the reaction solution, and the mixture was stirred for 10 minutes at 0°C and then for 3.5 hours at room temperature. After completion of the reaction, the organic layer was washed three times by adding MC (100 mL) and a saturated sodium bicarbonate aqueous solution (100 mL). The obtained organic layer was dried over anhydrous magnesium sulfate, filtered, and then concentrated under reduced pressure. The residue was subjected to column chromatography to obtain Compound Q-4c in the form of a light yellow oil (866 mg, 88%).

¹H-NMR (400 MHz, CDCl₃) δ 7.79 (d, *J* = 8 Hz, 4H), 7.34 (d, *J* = 8 Hz, 4H), 4.16 (t, *J* = 4.8 Hz, 4H), 3.68 (t, *J* = 4.8 Hz, 4H), 3.61 - 3.52 (m, 20H), 3.46 - 3.38 (m, 4H), 2.44 (s, 6H), 1.44 (s, 9H); EI-MS m/z: 778[M+H]⁺.

### Step 4: Preparation of Compound Q-4d

Compound Q-4c (866 mg, 1.11 mmol) was dissolved in DMF (10 mL) under a nitrogen atmosphere at room temperature, and then sodium azide (289 mg, 4.46 mmol) was added, and the mixture was stirred for 16 hours at 60°C. After completion of the reaction, the organic layer was washed seven times by adding MC (150 mL) and a saturated aqueous sodium chloride solution (100 mL). The obtained organic layer was dried over anhydrous magnesium sulfate, filtered, and then concentrated under reduced pressure. The residue was subjected to column chromatography to obtain Compound Q-4d in the form of a colorless oil (519 mg, 90%).

¹H-NMR (400 MHz, CDCl₃) δ 3.71 - 3.53 (m, 24H), 3.47 - 3.41 (m, 4H), 3.39 (t, *J* = 4.8 Hz, 4H), 1.45 (s, 9H).

### Step 5: Preparation of Linker Q-4e

Compound Q-4d (99 mg, 0.19 mmol) was dissolved in MC (1.2 mL) under a nitrogen atmosphere at 0°C, and then 4M-hydrochloric acid solution (4M-HCl in 1.4-dioxane, 0.7 mL) was added, and the mixture was stirred at room temperature for 16 hours. After completion of the reaction, the process of adding MC (200 mL), diluting, and then concentrating under reduced pressure was repeated five times to obtain Compound Q-4e in the form of an orange liquid (86 mg, 99%).

¹H-NMR (400 MHz, CDCl₃) δ 9.33 (brs, 1H), 3.93 (t, *J* = 4.8 Hz, 4H), 3.73 - 3.65 (m, 20H), 3.41 (t, *J* = 4.8 Hz, 4H), 3.28 - 3.22 (m, 4H); EI-MS m/z: 420[M+H]⁺.

### Step 6: Preparation of Linker Q-4

Compound Q-4e (47 mg, 0.103 mmol) and Compound Q-3 (47.5 mg, 0.134 mmol) were dissolved in THF (5 mL) under a nitrogen atmosphere at 0°C, and then DIPEA (53.9 µL, 0.309 mmol) was added, and the mixture was stirred for 1.5 hours at 0°C. Pyridine (24.9 µL, 0.309 mmol) was added to the reaction solution, and the mixture was warmed to room temperature and stirred for 16 hours. After completion of the reaction, the organic layer was extracted three times with EA (50 mL) and 2N-hydrochloric acid aqueous solution (50 mL). The obtained organic layer was dried over anhydrous magnesium sulfate, filtered, and then concentrated under reduced pressure at a temperature of 10°C or lower to obtain Compound Q-4 in the form of a colorless oil (67 mg, 99%). The obtained Compound Q-4 was used in the next reaction without an additional purification process; EI-MS m/z: 659 [M+H]⁺.

### Example I-1: Preparation of Compound A-1

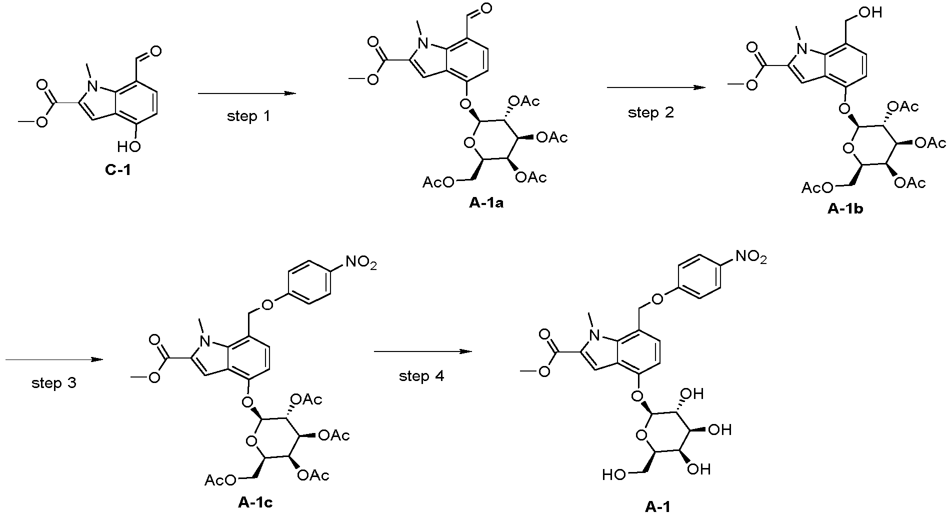

### Step 1: Preparation of Compound A-1a

Core C-1 (99.1 mg, 0.42 mmol) was dissolved in MC (15 mL) under a nitrogen atmosphere at 0°C, and then acetobromo-alpha-D-galactose (192 mg, 0.46 mmol) and benzyltributylammonium chloride (132 mg, 0.42 mmol) were added. 5N-sodium hydroxide aqueous solution (255 µL, 1.27 mmol) was slowly added to this reaction solution, and the mixture was stirred at room temperature for 16 hours. After completion of the reaction, MC (50 mL) and distilled water (50 mL) were added to extract the organic layer three times. The obtained organic layer was dried over anhydrous magnesium sulfate, filtered, and then concentrated under reduced pressure. The resulting residue was subjected to column chromatography to obtain Compound A-1a in the form of an ivory solid (131.3 mg, 55 %).

¹H-NMR (400 MHz, CDCl₃) δ 10.19 (s, 1H), 7.82 (d, *J* = 8 Hz, 1H), 7.38 (s, 1H), 6.80 (d, *J* = 8 Hz, 1H), 5.64 (dd, *J* =10.4*,* 8 Hz, 1H), 5.50 (d, *J* =3.2 Hz, 1H), 5.29 (d, *J* = 8 Hz, 1H), 5.16 (dd, *J* =10.4, 3.2 Hz, 1H), 4.34 (s, 3H), 4.26 - 4.09 (m, 3H), 3.93 (s, 3H), 2.21 (s, 3H), 2.08 (s, 3H), 2.06 (s, 3H), 2.05 (s, 3H).

### Step 2: Preparation of Compound A-1b

Compound A-1a (131.3 mg, 0.23 mmol) was dissolved in THF (3 mL) under a nitrogen atmosphere at 0°C, and then sodium borohydride (26.5 mg, 0.70 mmol) was added, and the mixture was stirred at room temperature for 2 hours. After completion of the reaction, distilled water (50 mL) was added to terminate the reaction, and EA (50 mL) was added to extract the organic layer twice. The obtained organic layer was dried over anhydrous magnesium sulfate, filtered, and then concentrated under reduced pressure. The resulting residue was subjected to column chromatography to obtain Compound A-1b in the form of a yellow solid (101.5 mg, 77 %).

¹H-NMR (400 MHz, CDCl₃) δ 7.32 (s, 1H), 7.12 (d, *J* = 8 Hz, 1H), 6.63 (d, *J* = 8 Hz, 1H), 5.61 (dd, *J* = 10.4, 8 Hz, 1H), 5.48 (d, *J* = 2.8 Hz, 1H), 5.16 - 5.12 (m, 2H), 4.99 - 4.97 (m, 2H), 4.41 (s, 3H), 4.28 - 4.08 (m, 3H), 3.91 (s, 3H), 2.20 (s, 3H), 2.07 (s, 3H), 2.06 (s, 3H), 2.04 (s, 3H).

### Step 3: Preparation of Compound A-1c

Compound A-1b (101.5 mg, 0.18 mmol) was dissolved in MC (5 mL) under a nitrogen atmosphere at 0°C, and then 4-nitrophenyl chloroformate (108.5 mg, 0.54 mmol) and DIPEA (156 µL, 0.89 mmol) were sequentially added, and the mixture was stirred for 20 hours. After completion of the reaction, EA (30 mL) and distilled water (30 mL) were added to extract the organic layer. The obtained organic layer was dried over anhydrous magnesium sulfate, filtered, and then concentrated under reduced pressure. The resulting residue was subjected to column chromatography to obtain Compound A-1c (44.1 mg, 36 %).

¹H-NMR (400 MHz, CDCl₃) δ 8.25 (d, *J* = 9.2 Hz, 2H), 7.35 (s, 1H), 7.23 (d, *J* = 8 Hz, 1H), 7.08 (d, *J* = 9.2 Hz, 2H), 6.69 (d, *J* = 8 Hz, 1H), 5.62 (dd, *J* =10.4, 8 Hz, 1H), 5.49 (d, *J* = 2.8 Hz, 1H), 5.44 (m, 2H), 5.18 (d, *J* = 8 Hz, 1H), 5.15 (dd, *J* = 10.4, 3.6 Hz, 1H), 4.27 (m, 1H), 4.24 (s, 3H), 4.20 - 4.09 (m, 2H), 3.91 (s, 3H), 2.21 (s, 3H), 2.06 (m, 6H), 2.04 (s, 3H).

### Step 4: Preparation of Compound A-1

Compound A-1c (37.2 mg, 0.054 mmol) was dissolved in methanol (1.5 mL) and ACN (1.5 mL) under a nitrogen atmosphere at 0°C, and then potassium carbonate (52.4 mg, 0.38 mmol) was added, and the mixture was stirred for 1.5 hours while raising the temperature from 0°C to room temperature. After completion of the reaction, 2N-hydrochloric acid aqueous solution (1 mL) was added to terminate the reaction, and the reaction solution was diluted with ACN (1 mL) and distilled water (1 mL), then purified using Preparative-HPLC, and freeze-dried to obtain Compound A-1 in the form of a white solid (5 mg, 18 %).

¹H-NMR (400 MHz, DMSO-d₆) δ 8.25 (d, *J* = 9.2 Hz, 2H), 7.47 (s, 1H), 7.38 (d, *J* = 8 Hz, 1H), 7.32 (d, *J* = 9.2 Hz, 2H), 6.76 (d, *J* = 8 Hz, 1H), 5.59 (s, 2H), 5.33 (m, 1H), 4.94 - 4.92 (m, 2H), 4.68 (m, 1H), 4.57 (m, 1H), 4.18 (s, 3H), 3.85 (s, 3H), 3.73 (m, 1H), 3.64 (m, 1H).

### Example 1-2: Preparation of Compound A-2

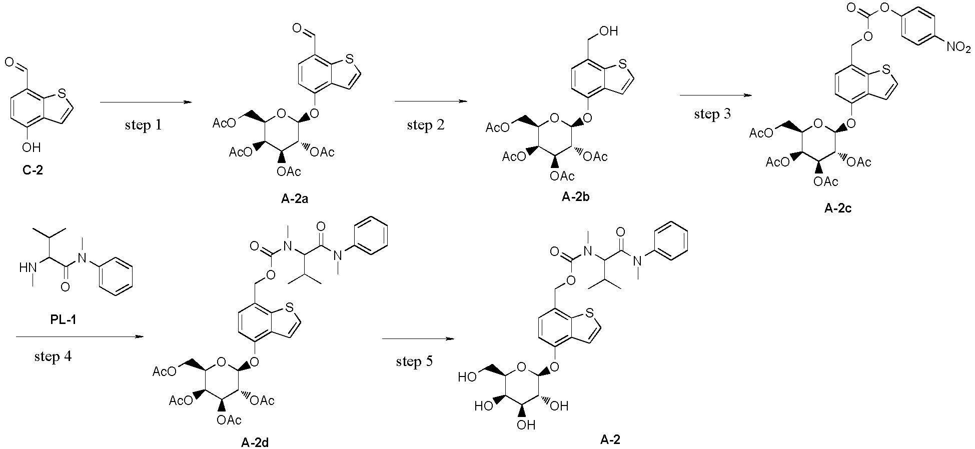

### Step 1: Preparation of Compound A-2a

Core C-2 (160 mg, 0.89 mmol) was dissolved in ACN (12 mL) under a nitrogen atmosphere at 0°C, and then acetobromo-alpha-D-galactose (406 mg, 0.98 mmol), silver oxide (I) (520 mg, 2.22 mmol) and a molecular sieve (70 mg) were sequentially added, and the mixture was stirred at room temperature for 30 minutes. After completion of the reaction, the reaction solution was diluted with EA (100 mL), filtered using a Celite filter, and extracted by adding distilled water (100 mL) to the solution. The obtained organic layer was dried over anhydrous magnesium sulfate, filtered, and then concentrated under reduced pressure. The resulting residue was subjected to column chromatography to obtain Compound A-2a in the form of a solid (400 mg, 87.7 %).

¹H-NMR (400 MHz, CDCl₃) δ 10.13 (s, 1H), 7.84 (d, *J* = 8 Hz, 1H), 7.59 (d, *J* = 5.6 Hz, 1H), 7.49 (d, *J* = 5.6 Hz, 1H), 7.09 (d, *J* = 8 Hz, 1H), 5.67 (dd, *J* = 10.4, 8 Hz, 1H), 5.52 (d, *J* = 3.2 Hz, 1H), 5.30 (d, *J* = 8 Hz, 1H), 5.19 (dd, *J* =10.4, 3.6 Hz, 1H), 4.29 (m, 1H), 4.21-4.17 (m, 2H), 2.21 (s, 3H), 2.10 (s, 3H), 2.04 (s, 6H).

### Step 2: Preparation of Compound A-2b

Compound A-2a (183 mg, 0.36 mmol) was dissolved in THF (10 mL) under a nitrogen atmosphere at 0°C, and then sodium borohydride (34 mg, 0.90 mmol) was added, and the mixture was stirred at 0°C for 1 hour, and then the mixture was stirred at room temperature for 2.5 hours. After completion of the reaction, distilled water (50 mL) was added to terminate the reaction, and EA (50 mL) was added to extract the organic layer twice. The obtained organic layer was dried over anhydrous magnesium sulfate, filtered, and then concentrated under reduced pressure. The resulting residue was subjected to column chromatography to obtain Compound A-2b in the form of a white solid (137.2 mg, 75 %).

¹H-NMR (400 MHz, CDCl₃) δ 7.45 (d, *J* = 5.6 Hz, 1H), 7.40 (d, *J* = 5.6 Hz, 1H), 7.28 (d, *J* = 8 Hz, 1H), 6.95 (d, *J* = *8* Hz, 1H), 5.63 (dd, *J* = 10.4, 8 Hz, 1H), 5.50 (d, *J* = 2.8 Hz, 1H), 5.17 - 5.14 (m, 2H), 4.92 (d, *J* = 5.6 Hz, 2H), 4.29 - 4.11 (m, 3H), 2.20 (s, 3H), 2.08 (s, 3H), 2.05 (s, 3H), 2.03 (s, 3H), 1.79 (t, *J* = 6 Hz, 1H).

### Step 3: Preparation of Compound A-2c

Compound A-12b (137.2 mg, 0.18 mmol) was dissolved in MC (10 mL) under a nitrogen atmosphere at 0°C, and then 4-nitrophenyl chloroformate (81.2 mg, 0.40 mmol) and DIPEA (140 µL, 0.81 mmol) were sequentially added, and the mixture was stirred for 16 hours while slowly raising the temperature from 0°C to room temperature. After completion of the reaction, EA (50 mL) and distilled water (50 mL) were added to extract the organic layer. The obtained organic layer was dried over anhydrous magnesium sulfate, filtered, and then concentrated under reduced pressure. The residue thus obtained was subjected to column chromatography to obtain Compound A-2c in the form of a white solid (114.7 mg, 63 %).

¹H-NMR (400 MHz, CDCl₃) δ 8.27 (d, *J* = 9.2 Hz, 2H), 7.48 (d, *J =* 5.6 Hz, 1H), 7.44 (d, *J* = *5.6* Hz, 1H), 7.39 - 7.36 (m, 3H), 6.97 (d, *J* = 8 Hz, 1H), 5.64 (dd, *J* = 10.4, 8 Hz, 1H), 5.24 (s, 2H), 5.51 (d, *J* = *3.2* Hz, 1H), 5.19 - 5.14 (m, 2H), 4.29 - 4.13 (m, 3H), 2.21 (s, 3H), 2.08 (s, 3H), 2.05 (s, 3H), 2.04 (s, 3H).

### Step 4: Preparation of Compound A-2d

Compound A-2c (64 mg, 0.094 mmol) was dissolved in DMF (3 mL) under a nitrogen atmosphere at 0°C, and then Compound PL-1 (25 mg, 0.113 mmol), HOBt (20 mg, 0.141 mmol), pyridine (500 µL) and DIPEA (41 µL, 0.235 mmol) were sequentially added, and the mixture was stirred at room temperature for 12 hours. After completion of the reaction, extraction was performed using EA (50 mL) and 1N-hydrochloric acid aqueous solution (50 mL). The obtained organic layer was dried over anhydrous magnesium sulfate, filtered, and then concentrated under reduced pressure. The resulting residue was subjected to column chromatography to obtain Compound A-2d (49 mg, 68.3 %).

¹H-NMR (400 MHz, CDCl₃) δ 7.46 - 7.39 (m, 2H), 7.34 (m, 1H), 7.28 - 7.22 (m, 2H), 7.16 - 7.03 (m, 3H), 7.01 - 6.65 (m, 2H), 5.63 (m, 1H), 5.50 (m, 1H), 5.17 - 4.93 (m, 4H), 4.63 - 4.09 (m, 4H), 3.26 - 3.22 (m, 3H), 2.88 - 3.87 (m, 3H), 2.32 (m, 1H), 2.20 (s, 3H), 2.08 - 2.07 (m, 3H), 2.05 - 2.03 (m, 6H), 0.90 - 0.87 (m, 3H), 0.72 - 0.55 (m, 3H); MS m/z: 757 [M+H]⁺.

### Step 5: Preparation of Compound A-2

Compound A-2d (49 mg, 0.065 mmol) was dissolved in methanol (2 mL) at 0°C, and then lithium hydroxide monohydrate (13.6 mg, 0.324 mmol) dissolved in distilled water (400 µL) was slowly added dropwise, and the mixture was stirred at 0°C for 10 minutes. After completion of the reaction, 2N-hydrochloric acid aqueous solution (1 mL) was added to terminate the reaction, and the reaction solution was diluted with ACN (1 mL) and distilled water (1 mL), then purified using Preparative-HPLC, and freeze-dried to obtain Compound A-2 in the form of a white solid (28.5 mg, 74 %).

¹H-NMR (400 MHz, DMSO-d₆) δ 7.72 (m, 0.5H), 7.68 - 7.65 (m, 1H), 7.58 (m, 0.5H), 7.31 - 7.21 (m, 3H), 7.15 - 7.02 (m, 4H), 5.31 (m, 1H), 5.10 - 4.91 (m, 3H), 4.68 (m, 1H), 4.55 (m, 1H), 4.24 (m, 1H), 3.73 - 3.44 (m, 3H), 3.14 (s, 3H), 2.72 (m, 3H), 2.16 (m, 1H), 0.83 - 0.77 (m, 3H), 0.63 - 0.43 (m, 3H); EI-MS m/z: 589 [M+H]⁺.

### Example I-3: Preparation of Compound A-3

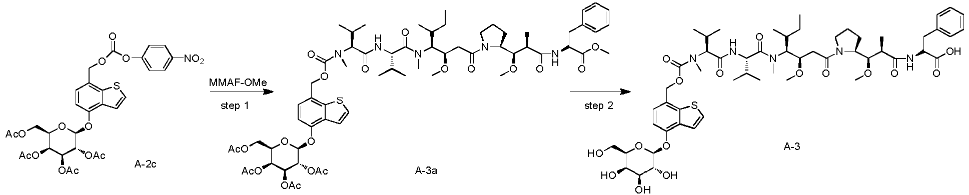

Compound A-3 was obtained in the form of a white solid in the same manner as in Example I-2, except that MMAF-OMe was used instead of Compound PL-1 in Step 4 of Example I-2 (10.4 mg, 65 %); EI-MS m/z: 1101 [M+H]⁺.

### Example I-4: Preparation of Compound A-4

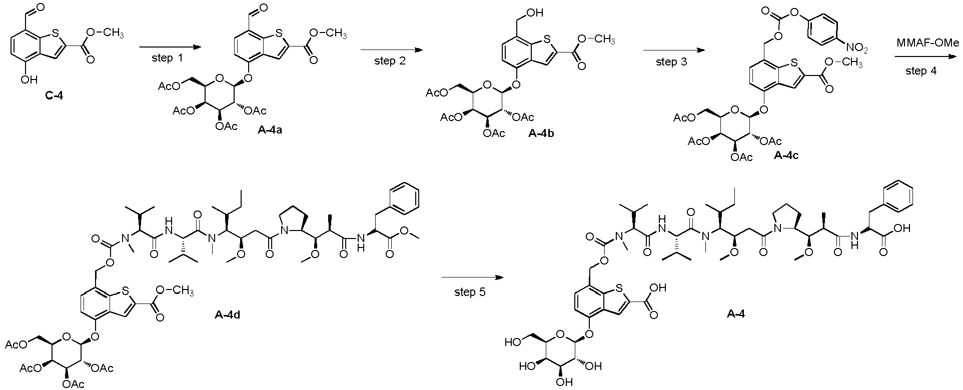

Compound A-4 was obtained in the form of a white solid using Core C-4 as a starting material in the same manner as in Example I-2, except that MMAF-OMe was used instead of Compound PL-1 in Step 4 of Example I-2 (11.7 mg, 64%); EI-MS m/z: 1145 [M+H]⁺.

### Example I-5: Preparation of Compound A-5

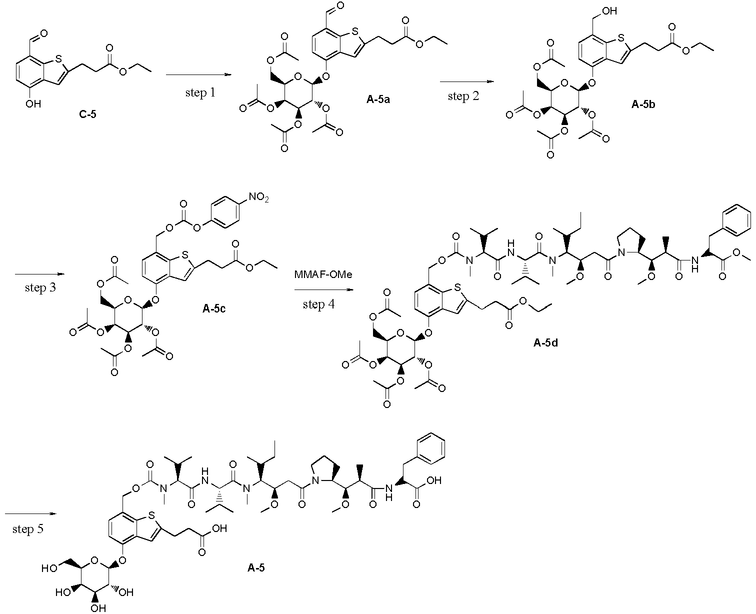

Compound A-5 was obtained in the form of a white solid using Core C-5 as a starting material in the same manner as in Example I-2, except that MMAF-OMe was used instead of Compound PL-1 in Step 4 of Example I-2 (7.7 mg, 60 %); EI-MS m/z: 1172 [M+H]⁺.

### Example I-6: Preparation of Compound A-6

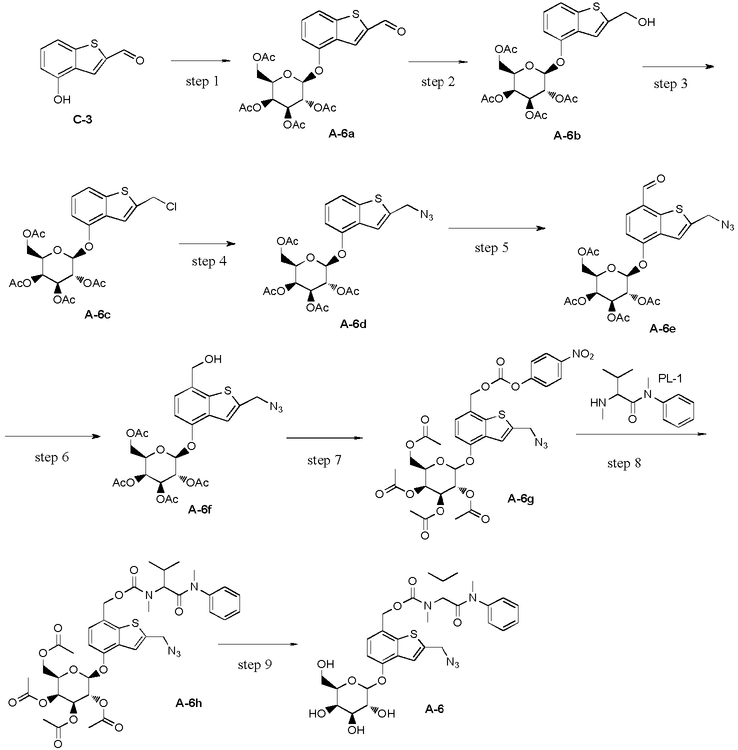

### Step 1: Preparation of Compound A-6a

Core C-3 (174 mg, 0.976 mmol) was dissolved in MC (30 mL) under a nitrogen atmosphere at room temperature, and then acetobromo-alpha-D-galactose (440 mg, 1.07 mmol) and benzyltributylammonium chloride (Sigma-Aldrich, CAS NO. 23616-79-7, 304 mg, 0.976 mmol) were sequentially added. 5N-sodium hydroxide aqueous solution (586 µL, 2.93 mmol) was added to this reaction solution, and the mixture was stirred for 5 hours. After completion of the reaction, MC (100 mL) and distilled water (100 mL) were added to extract the organic layer. The obtained organic layer was dried over anhydrous magnesium sulfate, filtered, and then concentrated under reduced pressure. The residue was subjected to column chromatography to obtain Compound A-6a (250 mg, 50.4 %).

¹H-NMR (400 MHz, CDCl₃) δ 10.09 (s, 1H), 8.09 (s, 1H), 7.59 (d, *J* = 8 Hz, 1H), 7.44 (t, *J* = 8 Hz, 1H), 6.95 (d, *J* = 8 Hz, 1H), 5.64 (dd, *J* =10.4, 8 Hz, 1H), 5.50 (d, *J* = 3.2 Hz, 1H), 5.23 *(d, J* = 8 Hz, 1H), 5.18 (dd, *J* =10.4, 8 Hz, 1H), 4.27 (m, 1H), 4.20-4.11 (m, 2H), 2.21 (s, 3H), 2.06 (s, 6H), 2.05 (s, 3H).

### Step 2: Preparation of Compound A-6b

Compound A-6a (250 mg, 0.49 mmol) was dissolved in THF (10 mL) under a nitrogen atmosphere at 0°C, and then sodium borohydride (46.5 mg, 1.225 mmol) was added, and the mixture was stirred for 1 hour. After completion of the reaction, distilled water (50 mL) was added to terminate the reaction, and EA (50 mL) was added to extract the organic layer. The obtained organic layer was dried over anhydrous magnesium sulfate, filtered, and then concentrated under reduced pressure. The resulting residue was subjected to column chromatography to obtain Compound A-6b (230 mg, 92 %).

¹H-NMR (400 MHz, CDCl₃) δ 7.53 (d, *J* = 8 Hz, 1H), 7.28-7.22 (m, 2H), 6.93 (d, *J* = 8 Hz, 1H), 5.60 (dd, *J* =10.4, 8 Hz, 1H), 5.48 (d, *J* = 2.4 Hz, 1H), 5.16-5.12 (m, 2H), 4.91 (d, = 5.2 Hz, 2H), 4.31 (dd, *J* =11.2, 7.2 Hz, 1H), 4.16-4.10 (m, 2H), 2.21 (s, 3H), 2.07 (s, 3H), 2.04 (s, 3H), 2.03 (s, 3H).

### Step 3: Preparation of Compound A-6c

Compound A-6b (300 mg, 0.58 mmol) was dissolved in concentrated hydrochloric acid (8 mL) under a nitrogen atmosphere at 0°C, and then the mixture was stirred for 1 hour at 0°C. After completion of the reaction, EA (50 mL) and distilled water (50 mL) were added to extract the organic layer, and the obtained organic layer was dried over anhydrous magnesium sulfate, filtered, and then concentrated under reduced pressure to obtain Compound A-6c in the form of a white solid (300 mg, 96.7 %); MS m/z: 529 [M+H]⁺.

### Step 4: Preparation of Compound A-6d

Compound A-6c (300 mg, 0.56 mmol) was dissolved in DMF (15 mL) under a nitrogen atmosphere at room temperature, and then sodium azide (55 mg, 0.84 mmol) was added, and the mixture was stirred at 60°C for 2 hours. After completion of the reaction, EA (100 mL) and distilled water (200 mL) were added to extract the organic layer, and the obtained organic layer was dried over anhydrous magnesium sulfate, filtered, and then concentrated under reduced pressure. The resulting residue was subjected to column chromatography to obtain Compound A-6d (230 mg, 75.6 %).

¹H-NMR (400 MHz, CDCl₃) δ 7.52 (d, *J* = 8 Hz, 1H), 7.32 (s, 1H), 7.28 (t, *J* = 8 Hz, 1H), 6.94 (d, *J* = 8 Hz, 1H), 5.61 (dd, *J* = 10.4, 8 Hz, 1H), 5.50 (d, *J* = 2.4 Hz, 1H), 5.17-5.14 (m, 2H), 4.60 (d, *J* = 14.4 Hz, 1H), 4.52 (d, *J* = 14.4 Hz, 1H), 4.26 (dd, *J* =11.2, 6.8 Hz, 1H), 4.20-4.11 (m, 2H), 2.20 (s, 3H), 2.07 (s, 3H), 2.05(s, 3H), 2.04 (s, 3H); MS m/z: 558 [M+Na]⁺.

### Step 5: Preparation of Compound A-6e

A-6d (230 mg, 0.42 mmol) was dissolved in MC (15 mL) under a nitrogen atmosphere at -78°C, and then dichloromethyl methyl ether (116 µL, 1.26 mmol) and titanium tetrachloride solution (1M-TiCl₄ in MC, 1.28 mL, 1.26 mmol) were sequentially and slowly added, and the mixture was stirred for 1 hour while maintaining the temperature. After completion of the reaction, cooled distilled water (100 mL) was slowly added dropwise to terminate the reaction, and EA (100 mL) was added to extract the organic layer. The obtained organic layer was dried over anhydrous magnesium sulfate, filtered, and then concentrated under reduced pressure. The resulting residue was subjected to column chromatography to obtain Compound A-6e in the form of a white solid (130 mg, 53.7 %).

¹H-NMR (400 MHz, CDCl₃) δ 10.11 (s, 1H), 7.84 (d, *J* = 8 Hz, 1H), 7.40 (s, 1H), 7.08 (d, *J* = 8 Hz, 1H), 5.66 (dd, *J* =10.4, 8 Hz, 1H), 5.52 (d, *J* = 3.2 Hz, 1H), 5.29 (d, *J* = 8 Hz, 1H), 5.19 (dd, *J* =10.4, 3.2 Hz, 1H), 4.65 (d, *J* = 14.4 Hz, 1H), 4.52 (d, *J* = 14.4 Hz, 1H), 4.31-4.17 (m, 3H), 2.21 (s, 3H), 2.09 (s, 3H), 2.05 (s, 6H); MS m/z: 586 [M+Na]⁺.

### Step 6: Preparation of Compound A-6f

Compound A-6e (130 mg, 0.23 mmol) was dissolved in THF (10 mL) under a nitrogen atmosphere at 0°C, and then sodium borohydride (22 mg, 0.575 mmol) was added, and the mixture was stirred for 1 hour. After completion of the reaction, distilled water (100 mL) was added to terminate the reaction, and EA (100 mL) was added to extract the organic layer. The obtained organic layer was dried over anhydrous magnesium sulfate, filtered, and then concentrated under reduced pressure. The resulting residue was subjected to column chromatography to obtain Compound A-6f (120 mg, 92.3 %).

¹H-NMR (400 MHz, CDCl₃) δ 7.36 (s, 1H), 7.28 (d, *J* = 8 Hz, 1H), 6.94 (d, *J* = 8 Hz, 1H), 5.61 (dd, *J* =10.4, 8 Hz, 1H), 5.49 (d, *J* = 2.8 Hz, 1H), 5.17-5.14 (m, 2H), 4.89 (d, *J* = 5.6 Hz, 2H), 4.61 (d, *J* = 14.4 Hz, 1H), 4.54 (d, *J* = 14.4 Hz, 1H), 4.26 (dd, *J* = 11.2, 6.8 Hz, 1H), 4.20-4.11 (m, 2H), 2.20 (s, 3H), 2.07 (s, 3H), 2.05 (s, 3H), 2.04 (s, 3H), 1.79 (t, *J* = 5.6 Hz, 1H).

### Step 7: Preparation of Compound A-6g

Compound A-6f (120 mg, 0.21 mmol) was dissolved in MC (3 mL) under a nitrogen atmosphere at 0°C, and then 4-nitrophenyl chloroformate (85 mg, 0.42 mmol), pyridine (51 µL, 0.63 mmol) and DIPEA (55 µL, 0.32 mmol) were sequentially added, and the mixture was stirred at 0°C for 30 minutes and at room temperature for 2 hours. After completion of the reaction, the reaction solution was diluted with MC (50 mL), and 2N-hydrochloric acid aqueous solution (50 mL) was added to extract the organic layer twice. The obtained organic layer was dried over anhydrous magnesium sulfate, filtered, and then concentrated under reduced pressure. The residue thus obtained was subjected to column chromatography to obtain Compound A-6g (116.3 mg, 75 %).

¹H-NMR (400 MHz, CDCl₃) δ 8.27 (d, *J* = 9.2 Hz, 2H), 7.39 - 7.37 (m, 4H), 6.96 (d, *J* = 8 Hz, 1H), 5.62 (dd, *J* = 10.4, 8 Hz, 1H), 5.50 (d, *J* = 3.2 Hz, 1H), 5.48 (s, 2H), 5.19 - 5.15 (m, 2H), 4.63 (d, *J* = 14.4 Hz, 1H), 4.57 (d, *J* = 14.4 Hz, 1H), 4.26 (m, 1H), 4.20 - 4.11 (m, 2H), 2.21 (s, 3H), 2.07 (s, 3H), 2.05 (s, 3H), 2.04 (s, 3H).

### Step 8: Preparation of Compound A-6h

Compound A-6g (18.7 mg, 0.025 mmol) and Compound PL-1 (6.7 mg, 0.031 mmol) were dissolved in DMF (500 µL) under a nitrogen atmosphere at 0°C, and then HOBt (5.2 mg, 0.038 mmol), pyridine (500 µL) and DIPEA (11.1 µL, 0.064 mmol) were sequentially added, and the mixture was stirred at room temperature for 16 hours. After completion of the reaction, the organic layer was extracted twice with EA (50 mL) and 2N-hydrochloric acid aqueous solution (50 mL). The obtained organic layer was dried over anhydrous magnesium sulfate, filtered, and then concentrated under reduced pressure. The resulting residue was subjected to column chromatography to obtain A-6h in the form of a white solid (17.9 mg, 86 %).

¹H-NMR (400 MHz, CDCl₃) δ 7.37 - 7.32 (m, 1H), 7.28 - 7.22 (m, 2H), 7.19 - 6.85 (m, 5H), 5.61 (m, 1H), 5.50 (m, 1H), 5.18 - 5.09 (m, 4H), 4.64 - 4.48 (m, 3H), 4.39 - 4.13 (m, 3H), 3.25 -3.23 (m, 3H), 2.90 - 2.88 (m, 3H), 2.32 (m, 1H), 2.20 (s ,3H), 2.07 - 2.04 (m, 9H), 0.98 - 0.83 (m, 3H), 0.72 - 0.55 (m, 3H); EI-MS m/z: 812 [M+H]⁺.

### Step 9: Preparation of Compound A-6

Compound A-6h (17.9 mg, 0.022 mmol) was dissolved in methanol (1 mL) and THF (0.5 mL) under a nitrogen atmosphere at 0°C, and then potassium carbonate (21.3 mg, 0.154 mmol) was added, and the mixture was stirred at 0°C for 40 minutes. After completion of the reaction, 2N-hydrochloric acid aqueous solution (1 mL) was added to terminate the reaction, and the reaction solution was diluted with ACN (1 mL) and distilled water (1 mL), then purified using Preparative-HPLC, and freeze-dried to obtain Compound A-6 in the form of a white solid (7.5 mg, 53%); EI-MS m/z: 644 [M+H]⁺.

### Example I-7: Preparation of Compound A-7

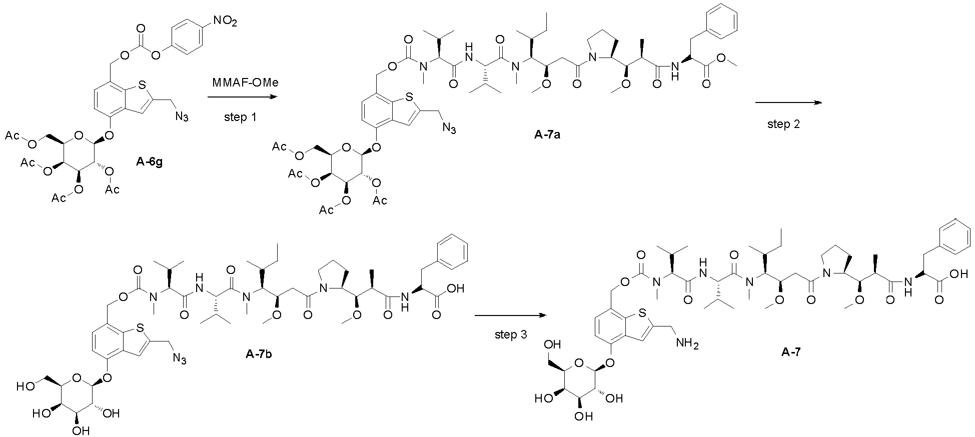

### Steps 1 and 2: Preparation of Compound A-7b

Compound A-7b was obtained as a white solid in the same manner as in Example I-6, except that MMAF-OMe was used instead of Compound PL-1 in Step 8 of Example I-6 and lithium hydroxide monohydrate was used instead of potassium carbonate in Step 9 (22 mg, 71 %); EI-MS m/z: 1156 [M+H]⁺.

### Step 3: Preparation of Compound A-7

Compound A-7b (17.8 mg, 0.0154 mmol) was dissolved in THF (1 mL) under a nitrogen atmosphere at room temperature, and then triphenylphosphine (4.4 mg, 0.017 mmol) and distilled water (0.3 mL) were sequentially added, and the mixture was stirred for 12 hours. 2N-sodium hydroxide aqueous solution (10 µL) was added to the above reaction solution at room temperature, and the mixture was stirred for 30 minutes, and then 2N-hydrochloric acid aqueous solution was slowly added dropwise to adjust the pH of the reaction solution to 3, and the reaction solution was purified using Preparative-HPLC, and then freeze-dried to obtain Compound A-7 (11.3 mg, 65 %); MS m/z: 1130 [M+H]⁺.

### Example I-8: Preparation of Compound A-8

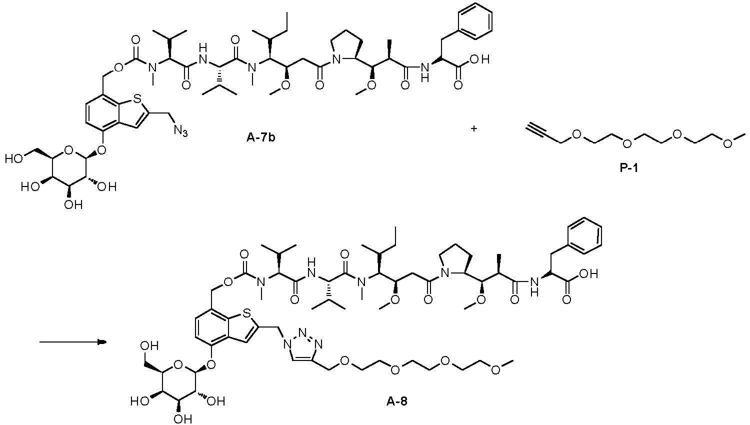

Compound A-7b (4.3 mg, 0.0037 mmol) was dissolved in DMSO (200 µL) under a nitrogen atmosphere at 0°C, and then 70 µL of a solution containing Linker P-1 (16.7 mg) dissolved in DMSO (1 mL) was taken and added to the reaction. Additionally, distilled water (2 mL), DMSO (0.4 mL), copper (II) sulfate pentahydrate (CuSO₄·5H₂O, 1 mg) and sodium ascorbate (1.47 mg) were sequentially added to the reaction solution, and then the mixture was stirred at room temperature for 30 minutes. After completion of the reaction, the reaction solution was diluted with distilled water (1 mL), purified using Preparative-HPLC, and then freeze-dried to obtain Compound A-8 (3.6 mg, 72 %); MS m/z: 1357.79 [M+H]⁺.

### Example I-9: Preparation of Compound A-9

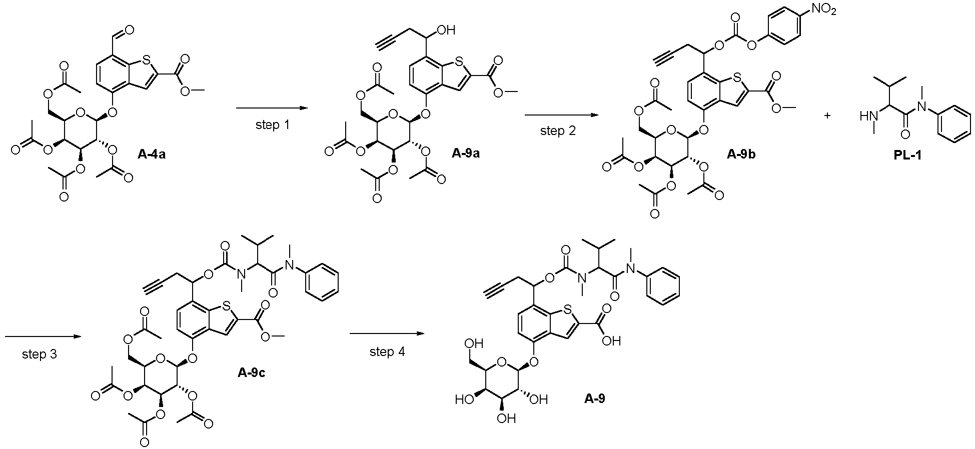

### Step 1: Preparation of Compound A-9a

Compound A-4a (114 mg, 0.2 mmol) was dissolved in THF (2 mL) under a nitrogen atmosphere at room temperature, and then propargyl bromide (35.8 µL, 0.4 mmol), 1,2-diiodoethane (85 mg, 0.3 mmol), and Zinc (Zn powder, 32.9 mg, 0.5 mmol) were sequentially added, followed by ultrasonication for 30 minutes. The mixture was cooled to 0°C, and then stirred at 0°C for 30 minutes. The mixture was warmed to room temperature and stirred for 5 hours. After completion of the reaction, the reaction solution was diluted with EA (100 mL) and filtered using a Celite filter. The obtained filtrate was concentrated under reduced pressure. The resulting residue was subjected to column chromatography to obtain Compound A-9a in the form of a white solid (76.9 mg, 63 %).

¹H-NMR (400 MHz, CDCl₃) δ 8.12 (s, 1H), 7.45 (m, 1H), 7.01 (m, 1H), 5.62 (dd, *J* = 10.4, 8 Hz, 1H), 5.48 (d, *J* = *3.2* Hz, 1H), 5.16 - 5.13 (m, 3H), 4.27 - 3.95 (m, 3H), 3.95 (s, 3H), 2.82 - 2.80 (m, 2H), 2.61 (m, 1H), 2.21 (s, 3H), 2.11 (t, *J* = 2.8 Hz, 1H), 2.09 (s, 3H), 2.07 (s, 3H), 2.04 (s, 3H).

### Step 2: Preparation of Compound A-9b

Compound A-9a (76.9 mg, 0.127 mmol) was dissolved in MC (2 mL) under a nitrogen atmosphere at 0°C, and then 4-nitrophenyl chloroformate (51.1 mg, 0.253 mmol) and DIPEA (66.2 µL, 0.38 mmol) were sequentially added, and the mixture was stirred for 16 hours while slowly raising the temperature from 0°C to room temperature. After completion of the reaction, the organic layer was extracted with EA (100 mL) and distilled water (100 mL). The obtained organic layer was dried over anhydrous magnesium sulfate, filtered, and then concentrated under reduced pressure. The residue thus obtained was subjected to column chromatography to obtain Compound A-9b (53.4 mg, 54 %).

¹H-NMR (400 MHz, CDCl₃) δ 8.25 (d, *J* = 9.2 Hz, 2H), 8.13 (s, 1H), 7.50 (d, *J* = 8.4 Hz, 1H), 7.36 (d, *J* = 9.2 Hz, 2H), 7.02 (d, *J* = 8.4 Hz, 1H), 6.05 (t, *J* = 6.4 Hz, 1H), 5.62 (dd, *J* = 10.4, 8 Hz, 1H), 5.50 (d, *J* = 3.2 Hz, 1H), 5.20 - 5.13 (m, 2H), 4.27 - 4.09 (m, 3H), 3.95 (s, 3H), 3.12 - 2.99 (m, 2H), 2.21 (s, 3H), 2.09 (m, 4H), 2.07 (s, 3H), 2.04 (s, 3H); EI-MS m/z: 794[M+Na]⁺.

### Step 3: Preparation of Compound A-9c

Compound A-9b (23.8 mg, 0.031 mmol) and Compound PL-1 (8.1 mg, 0.037 mmol) were dissolved in DMF (250 µL) under a nitrogen atmosphere at 0°C, and then HOBt (6.2 mg, 0.046 mmol), pyridine (250 µL) and DIPEA (13.4 µL, 0.077 mmol) were sequentially added, and the mixture was stirred for 5 hours at room temperature. After completion of the reaction, the organic layer was extracted twice with EA (50 mL) and 2N-hydrochloric acid aqueous solution (50 mL). The obtained organic layer was dried over anhydrous magnesium sulfate, filtered, and then concentrated under reduced pressure. The resulting residue was subjected to column chromatography to obtain Compound A-9c in the form of a white solid (23.9 mg, 90 %); EI-MS m/z: 853[M+H]⁺.

### Step 4: Preparation of Compound A-9

Compound A-9c (23.9 mg, 0.028 mmol) was dissolved in methanol (2 mL) and THF (1 mL) at 0°C, and then lithium hydroxide hydrate (11.7 mg, 0.28 mmol) dissolved in distilled water (0.4 mL) was slowly added dropwise, and the mixture was stirred for 4.5 hours while slowly rasing the temperature from 0°C to room termperature. After completion of the reaction, 2N-hydrochloric acid aqueous solution (1 mL) was added to terminate the reaction, and the reaction solution was diluted with ACN (1 mL) and distilled water (1 mL), then purified using Preparative-HPLC, and freeze-dried to obtain Compound A-9 in the form of a white solid (8 mg, 42 %); EI-MS m/z: 671[M+H]⁺.

### Example I-10: Preparation of Compound A-10

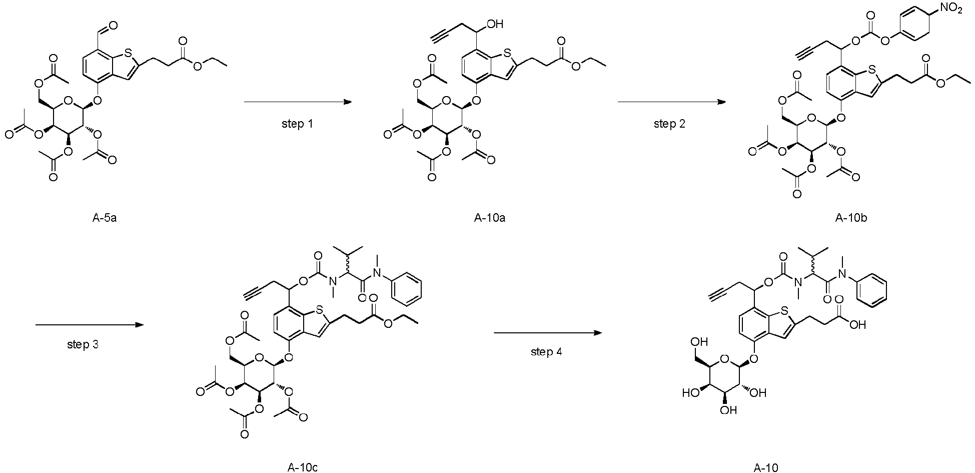

Compound A-10 was obtained in the form of a white solid in the same manner as in Example I-9, except that Compound A-5a was used as a core material in Example I-9 (9.8 mg, 64 %); EI-MS m/z: 699 [M+H]⁺.

### Example I-11: Preparation of Compound A-11

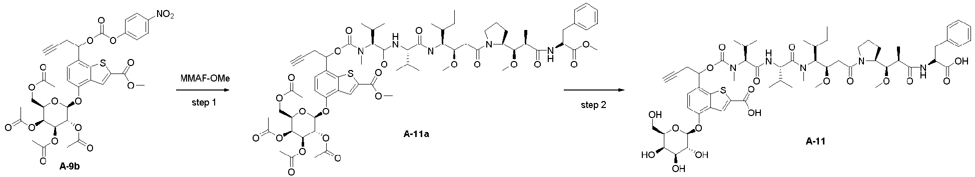

### Step 1: Preparation of Compound A-11a

Compound A-9b (31.6 mg, 0.041 mmol) was dissolved in DMF (700 µL) under a nitrogen atmosphere at 0°C, and then MMAF-OMe (monomethyl auristatin F methyl ester, CAS NO. 863971-12-4, 27.8 mg, 0.037 mmol), HOBt (7.5 mg, 0.056 mmol), pyridine (700 µL) and DIPEA (16.2 µL, 0.093 mmol) were sequentially added, and the mixture was stirred for 16 hours at room temperature. After completion of the reaction, the organic layer was extracted twice with EA (50 mL) and 2N-hydrochloric acid aqueous solution (50 mL). The obtained organic layer was dried over anhydrous magnesium sulfate, filtered, and then concentrated under reduced pressure. The resulting residue was subjected to column chromatography to obtain Compound A-11a in the form of a white solid (37.2 mg, 72 %); EI-MS m/z: 1379[M+H]⁺.

### Step 2: Preparation of Compound A-11

Compound A-11a (37.2 mg, 0.027 mmol) was dissolved in methanol (1 mL) and THF (0.5 mL) at 0°C, and then lithium hydroxide hydrate (17 mg, 0.405 mmol) dissolved in distilled water (0.2 mL) was slowly added dropwise, and the mixture was stirred for 2.5 hours while rasing the temperature from 0°C to room termperature. After completion of the reaction, 2N-hydrochloric acid aqueous solution (1 mL) was added to terminate the reaction, and the reaction solution was diluted with ACN (1 mL) and distilled water (1 mL), then purified using Preparative-HPLC, and freeze-dried to obtain Compound A-11 in the form of a white solid (20.4 mg, 64 %); EI-MS m/z: 1183[M+H]⁺.

### Example I-12: Preparation of Compound A-12

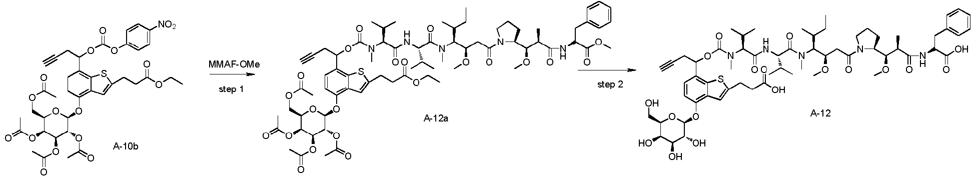

### Step 1: Preparation of Compound A-12a

Compound A-10b (40 mg, 0.049 mmol) was dissolved in DMF (1 mL) under a nitrogen atmosphere at room temperature, and MMAF-OMe (36.7 mg, 0.049 mmol) was added, and then the mixture was cooled to 0°C. HOBt (10.0 mg, 0.073 mmol), pyridine (1 mL) and DIPEA (21.0 µL, 0.012 mmol) were sequentially added, and the mixture was warmed to room temperature, and then stirred for 16 hours. After completion of the reaction, the reaction solution was diluted with EA (20 mL) and the organic layer was extracted twice with distilled water (20 mL) and 2N-hydrochloric acid aqueous solution (5 mL). The extracted organic layer was dried over anhydrous magnesium sulfate, filtered, and then concentrated under reduced pressure. The resulting residue was subjected to column chromatography to obtain A-12a (19 mg, 27 %); EI-MS m/z: 1421[M+H]⁺.

### Step 2: Preparation of Compound A-12

Compound A-12a (19.7 mg, 0.014 mmol) was dissolved in methanol (1 mL) and THF (0.5 mL) at 0°C, and then lithium hydroxide hydrate (8.7 mg, 0.208 mmol) dissolved in distilled water (0.2 mL) was added dropwise, and the mixture was stirred for 2.5 hours while slowly rasing the temperature from 0°C to room termperature. After completion of the reaction, 2N-hydrochloric acid aqueous solution (1 mL) was added to terminate the reaction, and the reaction solution was diluted with ACN (1 mL) and distilled water (1 mL), then purified using Preparative-HPLC, and freeze-dried to obtain Compound A-12 in the form of a white solid (11.7 mg, 70 %); EI-MS m/z: 1211[M+H]⁺.

### Example I-13: Preparation of Compound A-13

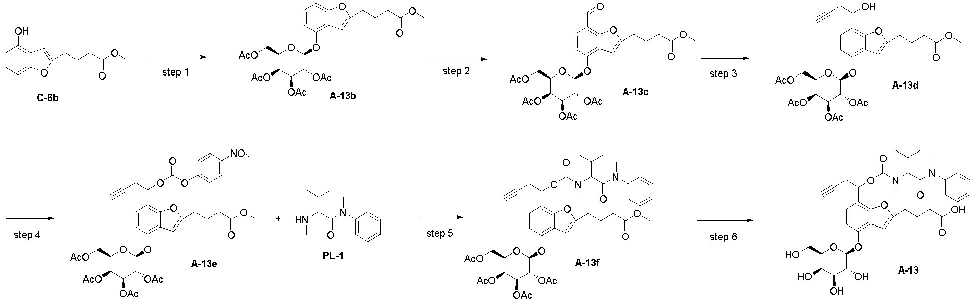

### Step 1: Preparation of Compound A-13b

Core C-6b (572.6 mg, 2.44 mmol) was dissolved in MC (80 mL) under a nitrogen atmosphere at 0°C, and then acetobromo-alpha-D-galactose (Merck, CAS No. 30668-32-4, 1.1 g, 2.69 mmol) and benzyltributylammonium chloride (762.5 mg, 2.44 mmol) were added. 5N-sodium hydroxide aqueous solution (1.46 mL, 7.33 mmol) was slowly added to the reaction solution, and the mixture was stirred for 16 hours while slowly raising the temperature to room temperature. After completion of the reaction, MC (200 mL) and distilled water (200 mL) were added to extract the organic layer three times. The obtained organic layer was dried over anhydrous magnesium sulfate, filtered, and then concentrated under reduced pressure. The resulting residue was subjected to column chromatography to obtain Compound A-13b in the form of a solid (1.017 g, 74 %).

¹H-NMR (400 MHz, CDCl₃) δ 7.17 - 7.10 (m, 2H), 6.80 (dd, *J* = 7.6, 0.8 Hz, 1H), 6.42 (s, 1H), 5.57 (dd, *J* = 10.4, 8 Hz, 1H), 5.48 (d, *J* = 3.2 Hz, 1H), 5.15 - 5.10 (m, 2H), 4.28 - 4.23 (m, 1H), 4.20 - 4.16 (m, *J* = 1H), 4.10 - 4.07 (m, 1H), 3.67 (s, 3H), 2.81 (t, *J* = 7.2 Hz, 2H), 2.39 (t, *J* = 7.2 Hz, 2H), 2.20 (s, 3H), 2.10 - 2.05 (m, 8H), 2.03 (s, 3H).

### Step 2: Preparation of Compound A-13c

Compound A-13b (700 mg, 1.23 mmol) was dissolved in MC (40 mL) under a nitrogen atmosphere at -30°C, and then dichloromethyl methyl ether (450 µL, 4.92 mmol) and titanium tetrachloride solution (1M-TiCl₄ in MC, 4.9 mL, 4.92 mmol) were sequentially and slowly added, and the mixture was stirred for 2 hours at -10°C while maintaining the temperature. After completion of the reaction, distilled water (100 mL) was slowly added dropwise to terminate the reaction, and EA (100 mL) was added to extract the organic layer. The obtained organic layer was dried over anhydrous magnesium sulfate, filtered, and then concentrated under reduced pressure. The resulting residue was subjected to column chromatography to obtain Compound A-13c (190 mg, 25.8 %).

¹H-NMR (400 MHz, CDCl₃) δ 10.33 (s, 1H), 7.69 (d, *J* = 8.4 Hz, 1H), 6.88 (d, *J* = 8.4 Hz, 1H), 6.49 (s, 1H), 5.59 (dd, *J* = 10.4, 8 Hz, 1H), 5.49 (d, *J* = 3.6 Hz, 1H), 5.23 (d, *J* = 8 Hz, 1H), 5.16 (dd, *J* =10.4, 3.6 Hz, 1H), 4.27-4.14 (m, 3H), 3.68 (s, 3H), 2.89 (t, *J* = 7.2 Hz, 2H), 2.42 (t, *J* = 7.2 Hz, 2H), 2.20 (s, 3H), 2.11 (m, 2H), 2.07 (s, 3H), 2.06 (s, 3H), 2.03 (s, 3H).

### Steps 3 to 6: Preparation of Compound A-13

Compound A-13 was obtained in the form of a white solid in the same manner as in Example I-9, except that Compound A-13c was used instead of Compound A-4a in Step 1 of Example I-9 (5.8 mg, 27 %); EI-MS m/z: 719 [M+Na]⁺.

### Example I-14: Preparation of Compound A-14

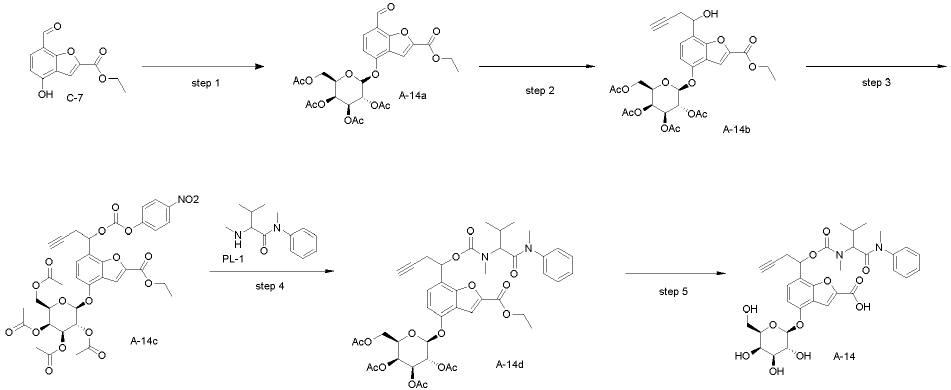

Compound A-14 was obtained in the form of a white solid using Core C-7 in a manner similar to Example I-13 (3.5 mg, 15 %); EI-MS m/z: 655 [M+Na]⁺.

### Example I-15: Preparation of Compound A-15

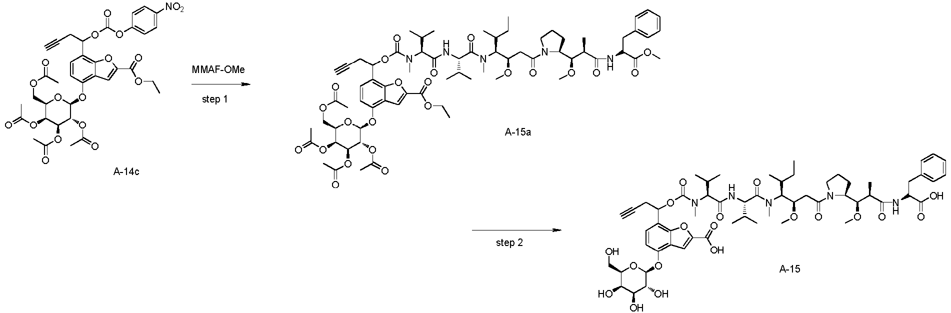

Compound A-15 was obtained in the form of a white solid in the same manner as in Example I-11, except that Compound A-14c was used instead of Compound A-9b in Step 1 of Example I-11 (20.4 mg, 46 %); EI-MS m/z: 1167 [M+Na]⁺.

### Example I-16: Preparation of Compound A-16

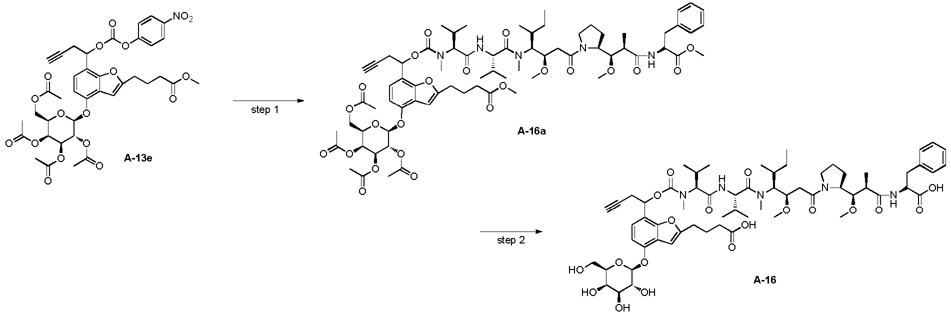

Compound A-16 was obtained in the form of a white solid in the same manner as in Example I-11, except that Compound A-13e was used instead of Compound A-9b in Step 1 of Example I-11 (9.3 mg, 61 %); EI-MS m/z: 1209 [M+H]⁺.

### Example I-17: Preparation of Compound A-17

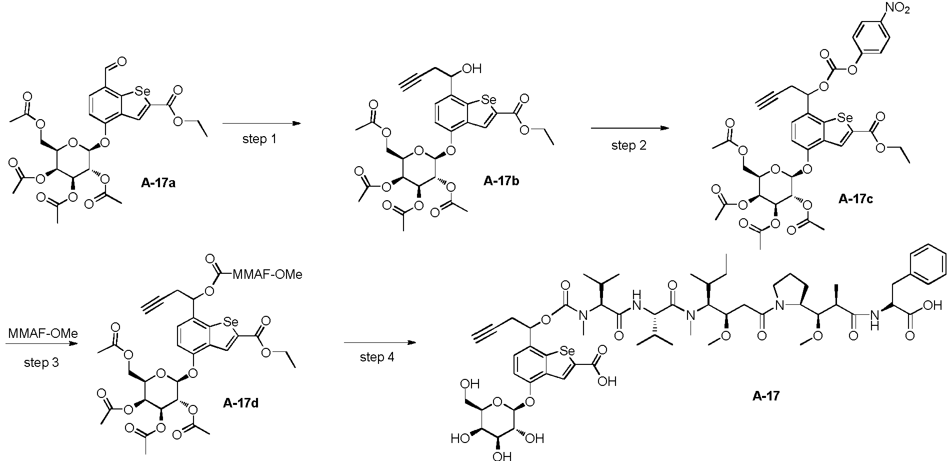

### Step 1: Preparation of Compound A-17b

Compound A-17a (120 mg, 0.19 mmol) was dissolved in anhydrous THF (3 mL) under a nitrogen atmosphere at room temperature, and then Zn powder (CAS NO. 7440-66-6, DAEJUNG, 62.5 mg, 0.95 mmol), 1,2-diiodoethane (CAS NO. 624-73-7, Alfa Aesar, 53.9 mg, 0.19 mmol), and propargyl bromide (80 % in toluene, 27.1 µL, 0.285 mmol) were sequentially added, and the mixture was reacted by ultrasound in an ultrasonic cleaner (powersonic 410) for 30 minutes. After completion of the reaction, the reaction solution was diluted with EA (50 mL), filtered using Celite, and extracted by adding distilled water (50 mL) and 2N-hydrochloric acid aqueous solution (0.7 mL) to the filtered solution. The obtained organic layer was dried over anhydrous magnesium sulfate, filtered, and then concentrated under reduced pressure. The resulting residue was subjected to column chromatography to obtain Compound A-17b in the form of a white solid (115 mg, 90 %).

¹H-NMR (400 MHz, CDCl₃) δ 8.38 (d, *J* = 2.4 Hz, 1H), 7.29 (m, 1H), 7.04 (dd, *J* = *8,* 1.2 Hz, 1H), 5.62 (dd, *J* = 9.2, 8 Hz, 1H), 5.47 (d, *J* = 3.6 Hz, 1H), 5.15-5.08 (m, 3H), 4.38 (q, *J* = 7.2 Hz, 2H), 4.29-4.23 (m, 1H), 4.20-4.11 (m, 2H), 2.80-2.71 (m, 2H), 2.63 (d, *J* = 2.8 Hz, 1H), 2.21 (s, 3H), 2.11 (m, 3H), 2.07 (m, 3H), 2.03 (s, 3H), 1.40 (t, *J* = 7.2 Hz, 3H); MS m/z: 690[M+H]⁺.

### Step 2: Preparation of Compound A-17c

Compound A-17b (115 mg, 0.17 mmol) was dissolved in MC (3 mL) under a nitrogen atmosphere at 0°C, and then 4-nitrophenyl chloroformate (69.5 mg, 0.34 mmol), pyridine (41.6 µL, 051 mmol) and DIPEA (30 µL, 0.17 mmol) were sequentially added, and the mixture was stirred for 16 hours at room temperature. After completion of the reaction, the reaction solution was diluted with EA (100 mL). The organic layer was extracted by adding distilled water (100 mL) and 2N-hydrochloric acid aqueous solution (10 mL), dried over anhydrous magnesium sulfate, filtered, and then concentrated under reduced pressure. The residue thus obtained was subjected to column chromatography to obtain A-17c (113 mg, 79 %).

¹H-NMR (400 MHz, CDCl₃) δ 8.39 (s, 1H), 8.25 (d, *J* = 9.2 Hz, 2H), 7.42 (d, *J* = 8 Hz, 1H), 7.36 (d, *J* = 9.2 Hz, 2H), 7.07 (d, *J* = 8 Hz, 1H), 5.96 (t, *J* = 6.4 Hz, 1H), 5.62 (dd, *J* = 10.4, 8 Hz, 1H), 5.48 (d, *J* = 3.2 Hz, 1H), 5.15-5.13 (m, 2H), 4.37 (m, 2H), 4.27-4.12 (m, 1H), 4.19-4.08 (m, 2H), 3.13-2.95 (m, 2H), 2.21 (s, 3H), 2.10 (s, 3H), 2.07 (s, 3H), 2.03 (s, 3H), 1.41 (t, *J* = 7.2 Hz, 3H); MS m/z: 855[M+Na]⁺.

### Step 3: Preparation of Compound A-17d

Compound A-17c (45 mg, 0.054 mmol) was dissolved in DMF (3 mL) under a nitrogen atmosphere at 0°C, and then MMAF-OMe (59.3 mg, 0.081 mmol), HOBt (73 mg, 0.054 mmol), pyridine (300 µL) and DIPEA (19 µL, 0.108 mmol) were sequentially added, and the mixture was stirred for 30 minutes. Additionally, the reaction mixture was stirred at room temperature for 20 hours, and then extracted with EA (50 mL) and 1N-hydrochloric acid aqueous solution (50 mL). The obtained organic layer was dried over anhydrous magnesium sulfate, filtered, and then concentrated under reduced pressure. The resulting residue was subjected to column chromatography to obtain A-17d (47 mg, 60.4 %); EI-MS m/z: 1440 [M+H]⁺.

### Step 4: Preparation of Compound A-17

Compound A-17d (47 mg, 0.032 mmol) was dissolved in methanol (2 mL) and THF (1 mL) at 0°C, and then lithium hydroxide hydrate (27.4 mg, 0.64 mmol) dissolved in distilled water (0.6 mL) was added dropwise, and the mixture was stirred for 5 hours. After completion of the reaction, 2N-hydrochloric acid aqueous solution (100 µL) was added to terminate the reaction, and the reaction solution was diluted with ACN (1 mL) containing 5% formic acid and distilled water (1 mL), then purified using Preparative-HPLC, and freeze-dried to obtain Compound A-17 in the form of a white solid (34 mg, 84 %); EI-MS m/z: 1230[M+H]⁺.

### Example I-18: Preparation of Compound A-18

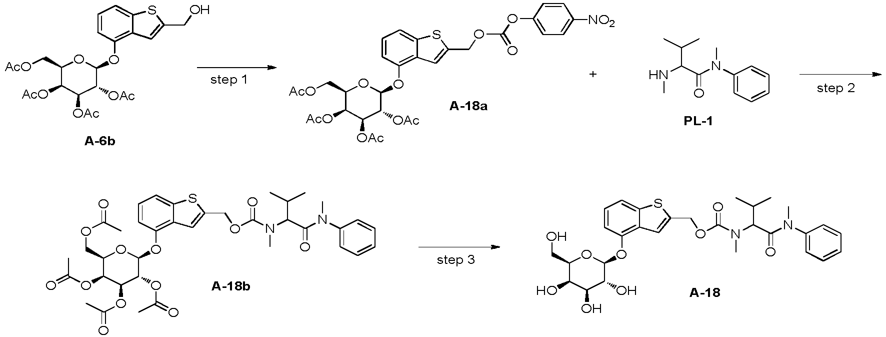

Compound A-18 was obtained in the form of a white solid in the same manner as in Steps 3 to 5 of Example I-2, except that Compound A-6b was used instead of Compound A-2b in Step 3 of Example I-2 (12.3 mg, 59 %); EI-MS m/z: 589 [M+H]⁺.

### Example I-19: Preparation of Compound A-19

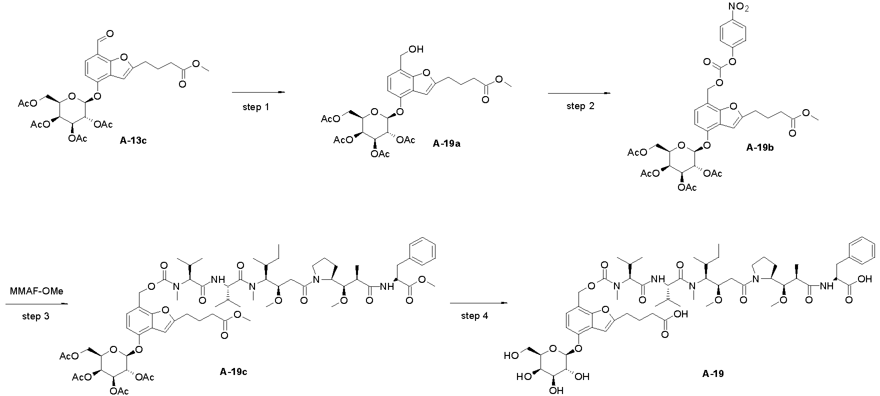

Compound A-19 was obtained in the form of a white solid using Compound A-13c as a starting material in the same manner as in Example I-2, except that MMAF-OMe was used instead of Compound PL-1 in Step 4 of Example I-2 (8 mg, 58%); EI-MS m/z: 1171 [M+H]⁺.

### Example I-20: Preparation of Compound A-20

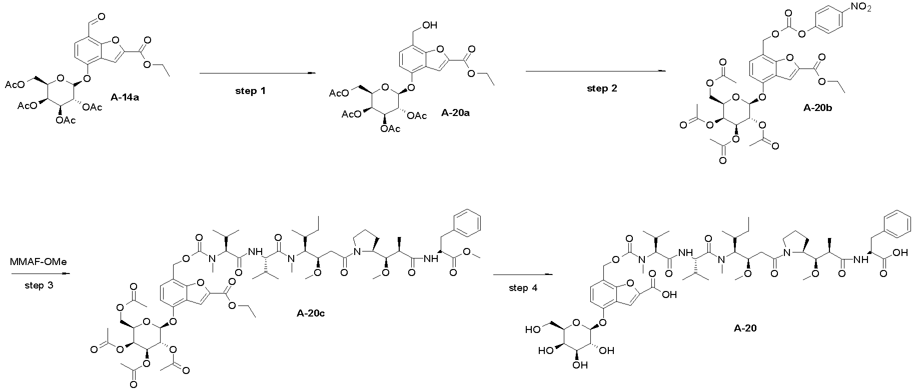

Compound A-20 was obtained in the form of a white solid using Compound A-14a as a starting material in the same manner as in Example I-2, except that MMAF-OMe was used instead of Compound PL-1 in Step 4 of Example I-2 (12.1 mg, 68%); EI-MS m/z: 1129 [M+H]⁺.

### Example I-21: Preparation of Compound A-21

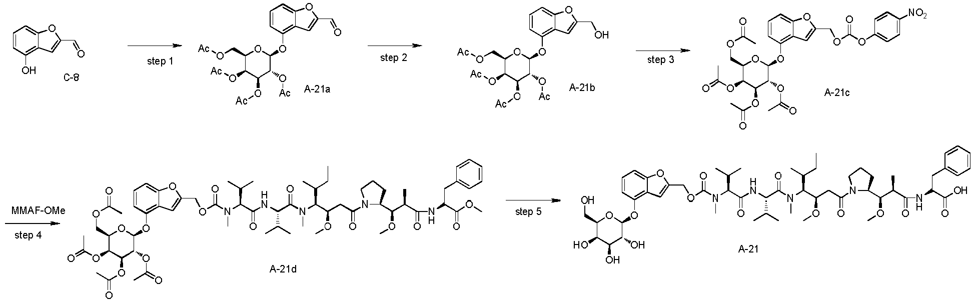

Compound A-21 was obtained in the form of a white solid using Core C-8 as a starting material in the same manner as in Example I-2, except that MMAF-OMe was used instead of Compound PL-1 in Step 4 of Example I-2 (17.2 mg, 74%); EI-MS m/z: 1085 [M+H]⁺.

### Example I-22: Preparation of Compound A-22

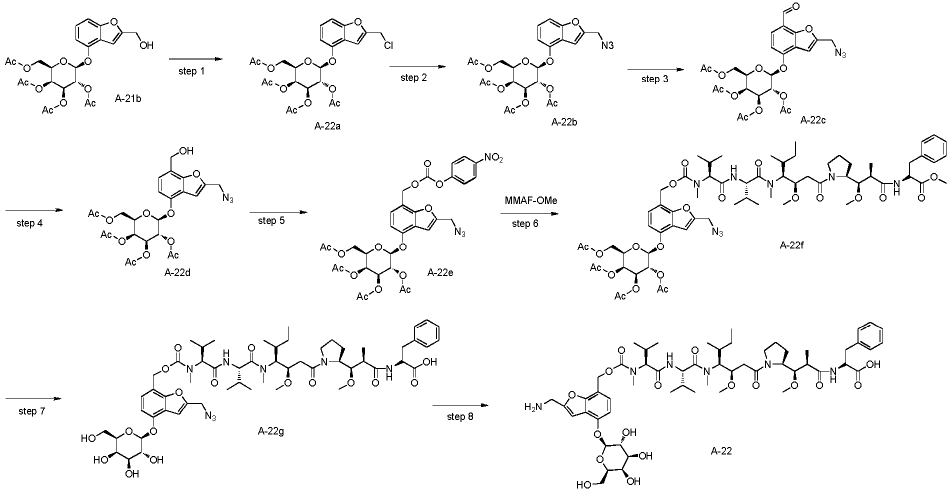

### Steps 1 to 7: Preparation of Compound A-22g

Compound A-22g was obtained in the form of a white solid in the same manner as in Steps 3 to 8 of Example I-6, except that Compound A-21b was used instead of Compound A-6b in Step 3 of Example I-6 and MMAF-OMe was used instead of Compound PL-1 in Step 8 of Example I-6 (21.3 mg, 61 %); EI-MS m/z: 1140 [M+H]⁺.

### Step 8: Preparation of Compound A-22

Compound A-22g (15.7 mg, 0.013 mmol) was dissolved in THF (1 mL) under a nitrogen atmosphere at room temperature, and then triphenylphosphine (5.4 mg, 0.0195 mmol) and distilled water (0.3 mL) were sequentially added, and the mixture was stirred for 16 hours. 2N-sodium hydroxide aqueous solution (10 µL) was added to the above reaction solution at room temperature, and the mixture was stirred for an additional 1 hour, and then 2N-hydrochloric acid aqueous solution was slowly added dropwise to adjust the pH of the reaction solution to 3, and the reaction solution was purified using Preparative-HPLC, and then freeze-dried to obtain Compound A-22 in the form of a white solid (10.5 mg, 65 %); MS m/z: 1113 [M+H]⁺.

### Example I-23: Preparation of Compound A-23

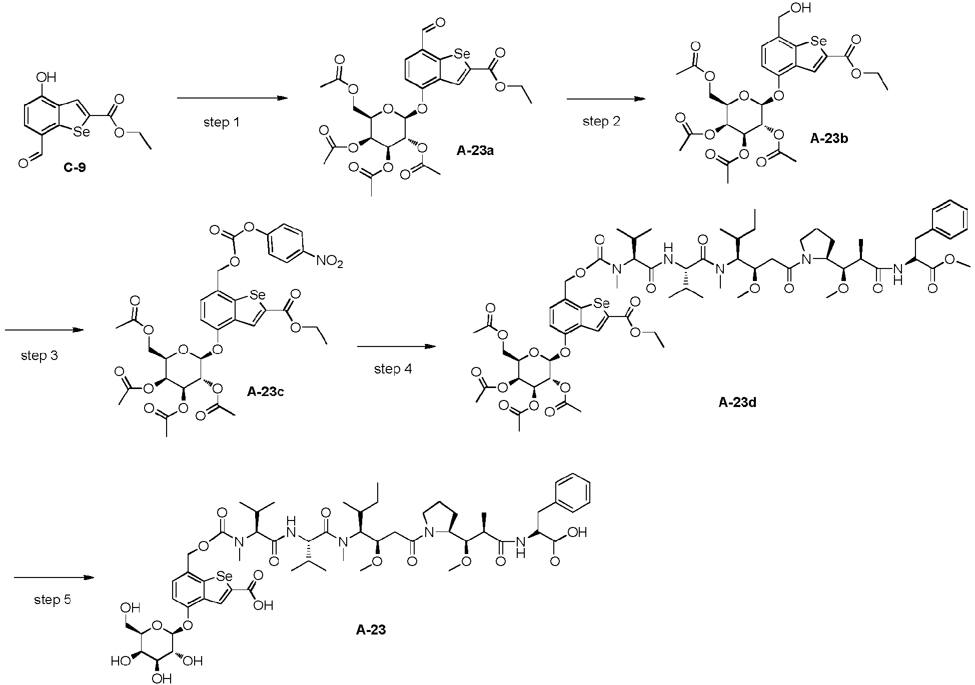

Compound A-23 was obtained in the form of a white solid using Core C-9 as a starting material in the same manner as in Example I-2, except that IPA and chloroform were used instead of THF as a solvent in Step 2 of Example I-2, silica gel was added with sodium borohydride, and MMAF-OMe was used instead of Compound PL-1 in Step 4 (15.9 mg, 64 %); EI-MS m/z: 1192 [M+H]⁺.

### Example I-24: Preparation of Compound A-24

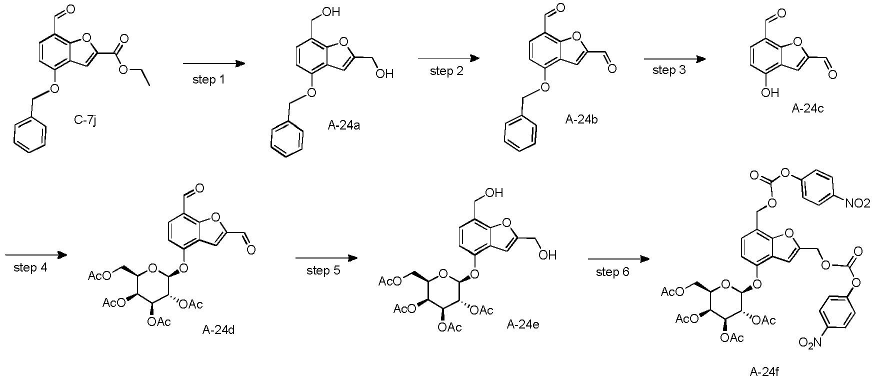

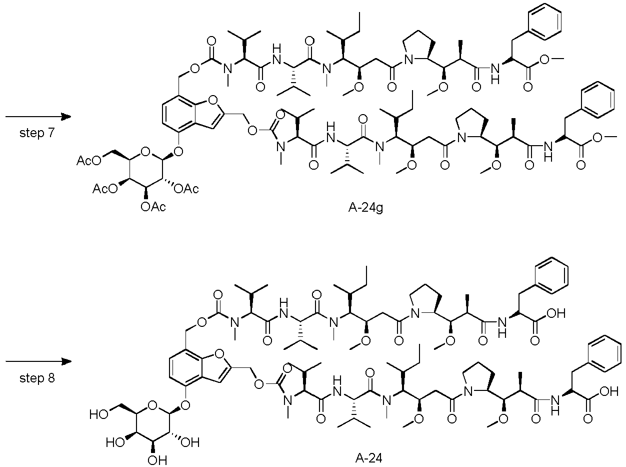

### Step 1: Preparation of Compound A-24a

Lithium aluminum hydride (144 mg, 3.8 mmol) was added to THF (15 mL) under a nitrogen atmosphere at 0°C, and then Compound C-7j (352 mg, 1.08 mmol) dissolved in THF (5 mL) was slowly added, and the mixture was stirred at 0°C for 3 hours. After completion of the reaction, distilled water (100 mL) was added to terminate the reaction, and the reaction solution was diluted with EA (100 mL) and filtered using Celite. The obtained solution was concentrated under reduced pressure to remove the organic layer, and then EA (100 mL) was added to extract the organic layer. The obtained organic layer was dried over anhydrous magnesium sulfate, filtered, and then concentrated under reduced pressure. The resulting residue was subjected to column chromatography to obtain Compound A-24a in the form of an ivory solid (241 mg, 78 %).

¹H-NMR (400 MHz, CDCl₃) δ 7.47 - 7.33 (m, 5H), 7.17 (d, *J* = 8 Hz, 1H), 6.84 (s, 1H), 6.70 (d, *J* = 8 Hz, 1H), 5.20 (s, 2H), 4.92 (d, *J* = 6 Hz, 2H), 4.78 (d, *J* = 6.4 Hz, 2H), 1.87 (t, *J* = 6.4 Hz, 1H), 1.79 (t, *J* = 6 Hz, 1H).

### Step 2: Preparation of Compound A-24b

DMP (Dess-Martin periodinane, 1.3 g, 3.08 mmol) was added to MC (10 mL) under a nitrogen atmosphere at 0°C, and then Compound A-24a (350 mg, 1.23 mmol) dissolved in MC (10 mL) and ACN (20 mL) was slowly added, and the mixture was stirred at 0°C for 30 minutes. The mixture was stirred for additional 3 hours while raising the temperature to room temperature. After completion of the reaction, the reaction solution was diluted with MC (50 mL) and filtered using Celite, and then distilled water (50 mL) was added to extract the organic layer. The obtained organic layer was dried over anhydrous magnesium sulfate, filtered, and then concentrated under reduced pressure. The resulting residue was subjected to column chromatography to obtain A-24b in the form of an ivory solid (292 mg, 85 %).

¹H-NMR (400 MHz, CDCl₃) δ 10.40 (s, 1H), 9.92 (s, 1H), 8.01 (d, *J* = 8.4 Hz, 1H), 7.75 (s, 1H), 7.48 - 7.39 (m, 5H), 6.91 (d, *J* = 8.4 Hz, 1H), 5.32 (s, 2H); EI-MS m/z: 281[M+H]⁺.

### Step 3: Preparation of Compound A-24c

Compound A-24b (190 mg, 0.68 mmol) was dissolved in MC (15 mL) under a nitrogen atmosphere at -78°C, and then boron trichloride solution (1M-BCl₃ in MC, 2.03 mL, 2.03 mmol) was slowly added, and the mixture was stirred for 1.5 hours at -78°C. After completion of the reaction, distilled water (50 mL) was slowly added dropwise to terminate the reaction, and EA (50 mL) was added to extract the organic layer three times. The obtained organic layer was dried over anhydrous magnesium sulfate, filtered, and then concentrated under reduced pressure at a temperature of 15°C or lower. The resulting residue was subjected to column chromatography to obtain Compound A-24c in the form of a yellow solid (56 mg, 43 %).

¹H-NMR (400 MHz, DMSO-d₆) δ 10.09 (s, 1H), 9.84 (s, 1H), 8.07 (s, 1H), 7.95 (d, *J* = 8.4 Hz, 1H), 6.88 (d, *J* = 8.4 Hz, 1H).

### Step 4: Preparation of Compound A-24d

Compound A-24c (71.4 mg, 0.37 mmol) was dissolved in MC (13 mL) under a nitrogen atmosphere at 0°C, and then acetobromo-alpha-D-galactose (185.3 mg, 0.45 mmol) and benzyltributylammonium chloride (117.1 g, 0.37 mmol) were added. 5N-sodium hydroxide aqueous solution (0.3 mL, 1.5 mmol) was slowly added to the reaction solution, and the mixture was stirred at 0°C for 30 minutes and at room temperature for additional 8 hours. After completion of the reaction, MC (100 mL) and distilled water (100 mL) were added to extract the organic layer. The obtained organic layer was dried over anhydrous magnesium sulfate, filtered, and then concentrated under reduced pressure. The resulting residue was subjected to column chromatography to obtain Compound A-24d in the form of a white solid (71 mg, 36 %).

¹H-NMR (400 MHz, CDCl₃) δ 10.46 (s, 1H), 9.37 (s, 1H), 8.03 (d, *J* = 8.4 Hz, 1H), 7.63 (s, 1H), 6.99 (d, *J* = 8.4 Hz, 1H), 5.62 (dd, *J* = 10.4, 8 Hz, 1H), 5.52 (m, 1H), 5.31 (d, *J* = 8.0 Hz, 1H), 5.19 (dd, *J* = 10.4, 3.4 Hz, 1H), 4.17 (m, 3H), 2.21 (s, 3H), 2.08 (s, 3H), 2.07 (s, 3H), 2.04 (s, 3H); EI-MS m/z: 543[M+Na]⁺.

### Step 5: Preparation of Compound A-24e

Compound A-24d (71 mg, 0.13 mmol) was dissolved in THF (7 mL) under a nitrogen atmosphere at 0°C, and then sodium borohydride (23 mg, 0.61 mmol) was added, and the mixture was stirred for 2.5 hours at 0°C. After completion of the reaction, distilled water (100 mL) was added to terminate the reaction, and then EA (100 mL) was added to extract the organic layer. The obtained organic layer was dried over anhydrous magnesium sulfate, filtered, and then concentrated under reduced pressure. The resulting residue was subjected to column chromatography to obtain Compound A-24e in the form of a white solid (35.5 mg, 50 %).

¹H-NMR (400 MHz, CDCl₃) δ 7.21 (d, *J* = 8 Hz, 1H), 6.82 (d, *J* = 8 Hz, 1H), 6.71 (s, 1H), 5.56 (dd, *J* = 10.4, 8 Hz, 1H), 5.48 (d, *J* = 2.8 Hz, 1H), 5.13 (dd, *J* = 10.4, 3.6 Hz, 1H), 5.10 (d, *J* = 7.6 Hz, 1H), 4.94 (d, *J* = 6 Hz, 2H), 4.77 (d, *J* = 6.4 Hz, 2H), 4.26 (dd, *J* = 11.2, 6.8 Hz, 1H), 4.18 - 4.08 (m, 2H), 2.20 (s, 3H), 2.06 (s, 3H), 2.05 (s, 3H), 2.03 (s, 3H), 2.01 (t, *J* = 6.4 Hz, 1H), 1.86 (t, *J* = 6 Hz, 1H); EI-MS m/z: 547[M+Na]⁺.

### Step 6: Preparation of Compound A-24f

Compound A-24e (35.5 mg, 0.067 mmol) was dissolved in MC (4 mL) under a nitrogen atmosphere at 0°C, and then 4-nitrophenyl chloroformate (54.6 mg, 0.271 mmol), pyridine (32.7 µL, 0.406 mmol) and DIPEA (35 µL, 0.203 mmol) were sequentially added, and the mixture was stirred at 0°C for 30 minutes and at room temperature for 2 hours. After completion of the reaction, the reaction solutiom was diluted with MC (50 mL), and distilled water (50 mL) and 2N-hydrochloric acid aqueous solution (10 mL) were added to extract the organic layer twice. The obtained organic layer was dried over anhydrous magnesium sulfate, filtered, and then concentrated under reduced pressure. The residue thus obtained was subjected to column chromatography to obtain A-24f in the form of a white sticky gum (39.7 mg, 69 %).

¹H-NMR (400 MHz, CDCl₃) δ 8.29 - 8.25 (m, 4H), 7.41 - 7.37 (m, 5H), 6.97 (s, 1H), 6.87 (d, *J* = 8.4 Hz, 1H), 5.62 - 5.55 (m, 3H), 5.50 (d, *J* = 2.8 Hz, 1H), 5.41 (m, 2H), 5.17 (d, *J* = 7.6 Hz, 1H), 5.15 (dd, *J* = 10.4, 3.2 Hz, 1H), 4.26 (dd, *J* = 11.2, 6.8 Hz, 1H), 4.19 - 4.09 (m, 2H), 2.20 (s, 3H), 2.07 (s, 3H), 2.05 (s, 3H), 2.03 (s, 3H).

### Step 7: Preparation of Compound A-24g

Compound A-24f (12.4 mg, 0.014 mmol) was dissolved in DMF (500 µL) under a nitrogen atmosphere at 0°C, and then MMAF-OMe (25 mg, 0.033 mmol), HOBt (6 mg, 0.043 mmol), pyridine (500 µL) and DIPEA (12.6 µL, 0.072 mmol) were sequentially added, and the mixture was stirred at room temperature for 16 hours. After completion of the reaction, the organic layer was extracted twice with EA (50 mL) and 2N-hydrochloric acid aqueous solution (50 mL). The obtained organic layer was dried over anhydrous magnesium sulfate, filtered, and then concentrated under reduced pressure. The resulting residue was subjected to column chromatography to obtain A-24g in the form of a white solid (20.7 mg, 70 %); EI-MS m/z: 2068 [M+H]⁺.

### Step 8: Preparation of Compound A-24

Compound A-24g (20.7 mg, 0.01 mmol) was dissolved in methanol (1 mL) and THF (0.5 mL) at 0°C, and then lithium hydroxide hydrate (6.3 mg, 0.15 mmol) dissolved in distilled water (0.2 mL) was added dropwise, and the mixture was stirred for 2.5 hours while slowly raising the temperature from 0°C to room temperature. After completion of the reaction, 2N-hydrochloric acid aqueous solution (1 mL) was added to terminate the reaction, and the reaction solution was diluted with ACN (1 mL) and distilled water (1 mL), then purified using Preparative-HPLC, and freeze-dried to obtain Compound A-24 in the form of a white solid (12 mg, 64 %); EI-MS m/z: 1873[M+H]⁺.

### Example I-25: Preparation of Compound A-25

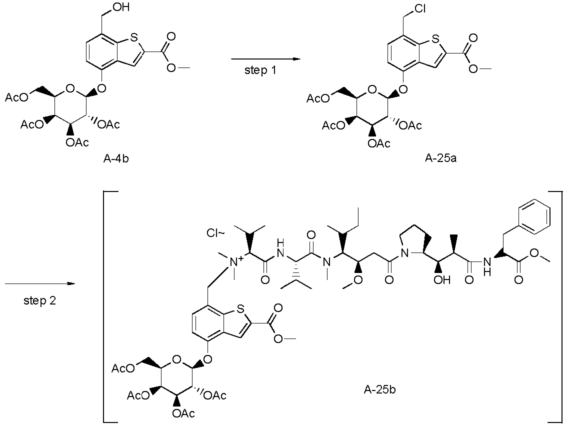

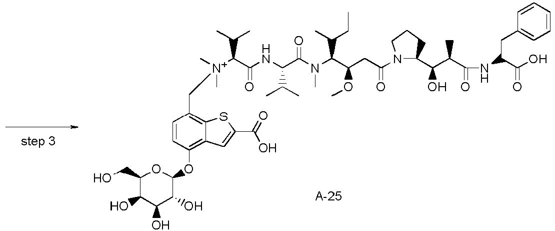

### Step 1: Preparation of Compound A-25a

Compound A-4b (180 mg, 0.31 mmol) was dissolved in MC (20 mL) under a nitrogen atmosphere at 0°C, and then thionyl chloride (48.2 µL, 0.65 mmol) was slowly added, and the mixture was stirred at 0°C for 2 hours. After completion of the reaction, the mixture was diluted by adding MC (50 mL) and then concentrated under reduced pressure. The residue was solidified by adding n-hexane (50 mL) and then concentrated under reduced pressure to obtain Compound A-25a in the form of a white solid. The obtained compound was used in the next reaction without an additional purification process (185 mg, 99 %).

¹H-NMR (400 MHz, CDCl₃) δ 8.12 (s, 1H), 7.41 (d, *J* = 8 Hz, 1H), 6.98 (d, *J* = 8 Hz, 1H), 5.62 (dd, *J* = 10.8, 8 Hz, 1H), 5.48 (d, *J* = 2.8 Hz, 1H), 5.17 - 5.13 (m, 2H), 4.80 (s, 2H), 4.22 (m, 1H), 4.18 (m, 1H), 4.12 (m, 1H), 3.96 (s ,3H), 2.21 (s , 3H), 2.08 (s, 3H), 2.07 (s, 3H), 2.04 (s, 3H); EI-MS m/z: 587 [M+H]⁺.

### Steps 2 and 3: Preparation of Compound A-25

Compound A-25a (30 mg, 0.051 mmol) was dissolved in DMF (3 mL) under a nitrogen atmosphere at 0°C, and then the compound auristatin F-OMe (42.7 mg, 0.056 mmol), DIPEA (26.7 µL, 0.153 mmol) and TBAI (2 mg, 0.005 mmol) were sequentially added to the reaction, and the mixture was stirred at 40°C for 16 hours. After completion of the reaction, the reaction solution containing the produced Compound A-25b (EI-MS m/z: 1311 [M+H]⁺) was cooled to room temperature, and lithium hydroxide hydrate (21.4 mg, 0.51 mmol) was dissolved in distilled water (1 mL) and slowly added at 0°C, and the mixture was stirred for 2 hours. 2N-hydrochloric acid aqueous solution (0.3 mL) was slowly added dropwise to the reaction solution to terminate the reaction, and the reaction solution was diluted with ACN (1 mL) and distilled water (1 mL), then purified using Preparative-HPLC, and then freeze-dried to obtain Compound A-25 (1.1 mg, 2 %); EI-MS m/z: 1115 [M+H]⁺.

### Example I-26: Preparation of Compound A-26

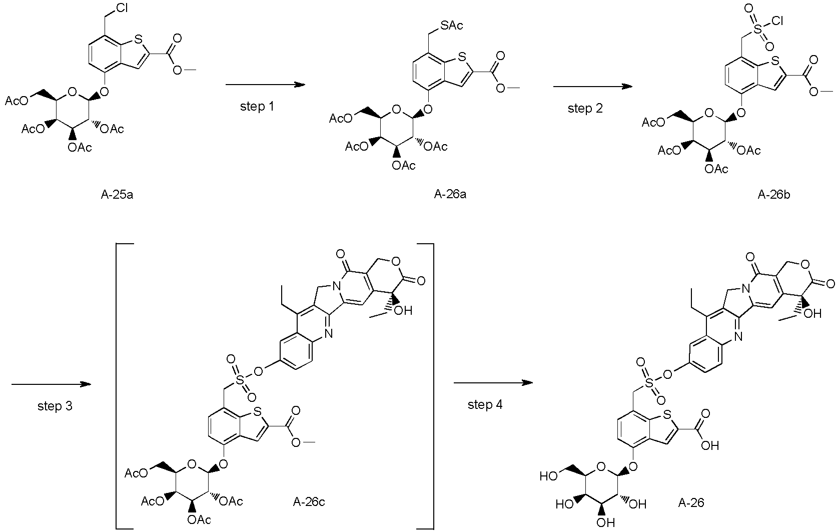

### Step 1: Preparation of Compound A-26a

Compound A-25a (135 mg, 0.229 mmol) was dissolved in ACN (5 mL) under a nitrogen atmosphere at room temperature, and then potassium thioacetate (31.5 mg, 0.275 mmol) was added, and the mixture was stirred for 3 hours. After completion of the reaction, EA (50 mL) and distilled water (50 mL) were added to extract the organic layer. The obtained organic layer was dried over anhydrous magnesium sulfate, filtered, and then concentrated under reduced pressure. The resulting residue was subjected to column chromatography to obtain Compound A-26a (140 mg, 97 %).

¹H-NMR (400 MHz, CDCl₃) δ 8.11 (s, 1H), 7.40 (d, *J* = 8 Hz, 1H), 6.95 (d, *J* = 8 Hz, 1H), 5.60 (dd, *J* = 10.4, 8 Hz, 1H), 5.47 (d, *J* = 2.8 Hz, 1H), 5.15 - 5.12 (m, 2H), 4.31 (d, *J* = 5.2 Hz, 1H), 4.25 - 4.08 (m, 3H), 3.95 (s, 3H), 2.36 (s, 3H), 2.20 (s ,3H), 2.08 (s ,2H), 2.06 (s, 3H), 2.03 (s ,3H); EI-MS m/z: 627 [M+H]⁺.

### Step 2: Preparation of Compound A-26b

N-chlorosuccinimide (NCS, 120 mg, 0.88 mmol) was added to a mixed solution of 2N-hydrochloric acid aqueous solution (60 µL) and ACN (300 µL) under a nitrogen atmosphere at 0°C, and then Compound A-26a (140 mg, 0.22 mmol) was dissolved in ACN (100 µL) and added. The reaction solution was stirred at 0°C for 3 hours, and after completion of the reaction, diethyl ether (100 mL) and distilled water (100 mL) were added to extract the organic layer. The obtained organic layer was dried over anhydrous magnesium sulfate, filtered, and then concentrated under reduced pressure to obtain Compound A-26b in the form of a white solid, which was used in the next reaction without an additional purification process (35 mg, 24 %).

### Steps 3 and 4: Preparation of Compound A-26

Compound A-26b (35 mg, 0.053 mmol) was dissolved in DMF (3 mL) under a nitrogen atmosphere at 0°C, and then SN-38 (CAS NO. 86639-52-3, 21 mg, 0.053 mmol) and TEA (18.7 µL, 0.132 mmol) were sequentially added, and the mixture was stirred at 0°C for 1 hour. After completion of the reaction, EA (50 mL) and 2N-hydrochloric acid aqueous solution (10 mL), distilled water (100 mL) were added to extract the organic layer. The obtained organic layer was dried over anhydrous magnesium sulfate, filtered, and then concentrated under reduced pressure to obtain Compound A-26c. The obtained Compound A-26c was dissolved by adding methanol (2 mL) and THF (1 mL), and then lithium hydroxide hydrate (22.5 mg, 0.53 mmol) was dissolved in distilled water (0.5 mL) and slowly added at 0°C, and the mixture was stirred for 30 minutes. The mixture was stirred at room temperature for additional 30 minutes, and then 2N-hydrochloric acid aqueous solution (0.3 mL) was slowly added dropwise to terminate the reaction. The reaction solution was diluted with ACN (1 mL) and distilled water (1 mL), then purified using Preparative-HPLC, and freeze-dried to obtain Compound A-26 (0.4 mg, 1%); EI-MS m/z: 825 [M+H]⁺.

### Example I-27: Preparation of Compound A-35

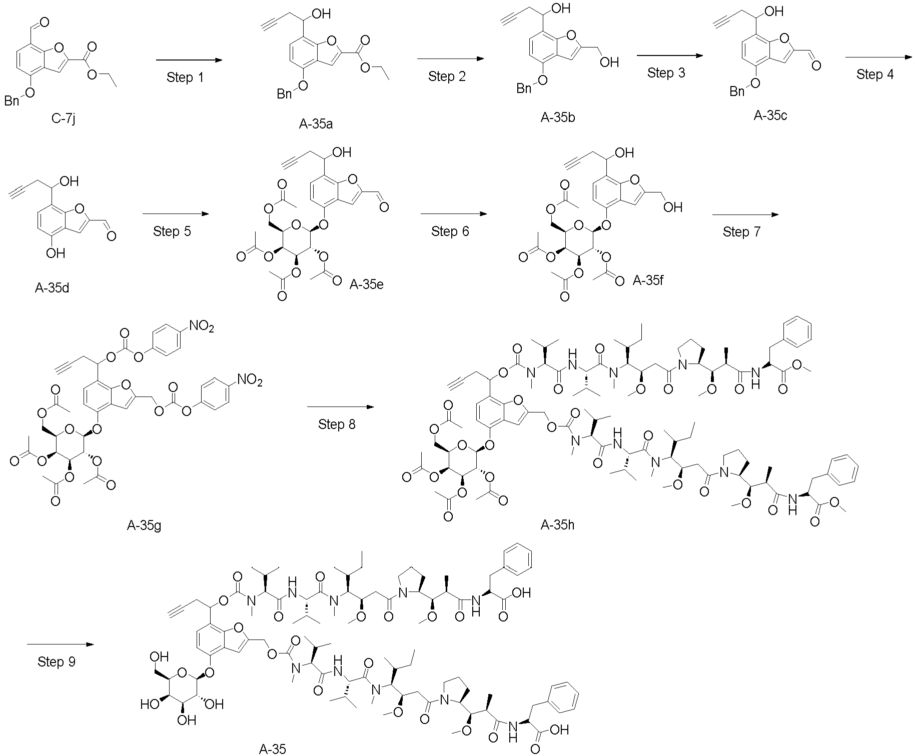

### Step 1: Preparation of Compound A-35a

Compound C-7j (904 mg, 2.79 mmol) was dissolved in THF (40 mL) under a nitrogen atmosphere at room temperature, and then Zinc (Zn powder, 1.3 g, 19.88 mmol), 1,2-diiodoethane (1.3 g, 4.61 mmol) and propargyl bromide (760 µL, 6.82 mmol) were sequentially added, followed by ultrasonication for 4.5 hours. After completion of the reaction, the reaction solution was diluted with EA (200 mL) and filtered using Celite. Distilled water (150 mL) and 2N-hydrochloric acid aqueous solution (50 mL) were added to the filtered residue to extract the organic layer. The obtained organic layer was dried over anhydrous magnesium sulfate, filtered, and then concentrated under reduced pressure. The resulting residue was subjected to column chromatography to obtain A-35a in the form of an ivory solid (770 mg, 76 %).

¹H-NMR (400 MHz, CDCl₃) δ 7.67 (s, 1H), 7.49 - 7.45 (m, 3H), 7.43 - 7.38 (m, 2H), 7.38 - 7.33 (m, 1H), 6.76 (d, *J* = 8 Hz, 1H), 5.39 (m, 1H), 5.20 (s, 2H), 4.41 (q, *J* = 7.2 Hz, 2H), 2.91 (m, 1H), 2.83 (m, 1H), 2.58 (d, *J* = 4.8 Hz, 1H), 2.07 (t, *J* = 2.8 Hz, 1H), 1.41 (t, *J* = 7.2 Hz, 3H); EI-MS m/z: 364[M+H]⁺, 387[M+Na]⁺, 347[M-OH]⁺.

### Step 2: Preparation of Compound A-35b

Compound A-35a (770 mg, 2.11 mmol) was dissolved in MC (9 mL) and toluene (27 mL) under a nitrogen atmosphere at -78°C, and then DIBAL-H (Diisobutylaluminum hydride 1M in toluene, 6.3 mL, 6.33 mmol) was slowly added, and the mixture was stirred for 4 hours while maintaining the temperature. After completion of the reaction, methanol (20 mL) was added at the same temperature, and the mixture was stirred for 15 minutes, then distilled water (10 mL) and 2N sodium hydroxide aqueous solution (10 mL) were added to terminate the reaction. The reaction solution was diluted by adding EA (250 mL) and filtered using Celite. A saturated potassium sodium tartrate (200 mL) was added to the filtered residue to extract the organic layer. The obtained organic layer was dried over anhydrous magnesium sulfate, filtered, and then concentrated under reduced pressure. The resulting residue was subjected to column chromatography to obtain A-35b in the form of a yellow solid (507 mg, 74 %).

¹H-NMR (400 MHz, CDCl₃) δ 7.48 - 7.43 (m, 2H), 7.42 - 7.36 (m, 2H), 7.36 - 7.31 (m, 1H), 7.27 - 7.23 (m, 1H), 6.81 (s, 1H), 6.69 (d, *J* = 8.4 Hz, 1H), 5.27 (m, 1H), 5.18 (s, 2H), 4.73 (d, *J* = 6 Hz, 2H), 2.91 - 2.79 (m, 2H), 2.68 (d, *J* = 4.4 Hz, 1H), 2.22 (t, *J* = 6 Hz, 1H), 2.06 (t, *J* = 2.8 Hz, 1H); EI-MS m/z: 323[M+H]⁺, 345[M+Na]⁺, 305[M-OH]⁺.

### Step 3: Preparation of Compound A-35c

Compound A-35b (507 mg, 1.57 mmol) was dissolved in MC (20 mL) under a nitrogen atmosphere at room temperature, and then TBAI (58 mg, 0.16 mmol) and TEMPO (25 mg, 0.16 mmol), an aqueous solution of sodium bicarbonate (840 mg, 10 mmol) and potassium carbonate (138 mg, 1 mmol) dissolved in distilled water (20 mL), N-chlorosuccinimide (NCS, 231 mg, 1.73 mmol) were sequentially added, and the mixture was stirred for 3 hours. After completion of the reaction, the organic layer was extracted twice with MC (100 mL) and distilled water (100 mL) and washed twice by adding a saturated aqueous sodium chloride solution (100 mL) to the obtained organic layer. The washed organic layer was dried over anhydrous magnesium sulfate, filtered, and then concentrated under reduced pressure. The residue was subjected to column chromatography to obtain Compound A-35c in the form of an ivory solid (401 mg, 80 %).

¹H-NMR (400 MHz, CDCl₃) δ 9.81 (s, 1H), 7.72 (s, 1H), 7.56 (d, *J* = 8.4 Hz, 1H), 7.49 - 7.34 (m, 5H), 6.79 (d, *J* = 8.4 Hz, 1H), 5.39 (m, 1H), 5.23 (s, 2H), 2.93 (m, 1H), 2.81 (m, 1H), 2.57 (d, *J* = 4.4 Hz, 1H), 2.07 (t, *J* = 2.8 Hz, 1H); EI-MS m/z: 321[M+H]⁺, 343[M+Na]⁺, 303[M-OH]⁺.

### Step 4: Preparation of Core A-35d

Compound A-35c (502 mg, 1.57 mmol) was dissolved in MC (65 mL) under a nitrogen atmosphere at -78°C, and then boron trichloride solution (1M-BCl₃ in MC, 4.7 mL, 4.7 mmol) was slowly added, and then the mixture was stirred for 2.5 hours while maintaining the temperature. After completion of the reaction, distilled water (50 mL) was slowly added dropwise at -50°C to terminate the reaction, and 2N-sodium hydroxide aqueous solution was added at 0°C for neutralization. MC (100 mL) and distilled water (100 mL) were further added to the mixture to extract the organic layer twice, and then EA (100 mL) was added to the distilled water layer to extract the organic layer once again. The obtained organic layer was dried over anhydrous magnesium sulfate, filtered, and then concentrated under reduced pressure. The resulting residue was subjected to column chromatography to obtain Compound A-35d in the form of a yellow solid (131 mg, 36 %).

¹H-NMR (400 MHz, DMSO-d₆) δ 10.52 (brs, 1H), 9.78 (s, 1H), 7.97 (s, 1H), 7.43 (d, *J* = 8 Hz, 1H), 6.70 (d, *J* = 8 Hz, 1H), 5.59 (d, *J* = 4.4 Hz, 1H), 5.08 (m, 1H), 2.75 - 2.59 (m, 3H); EI-MS m/z: 231[M+H]⁺, 253[M+Na]⁺, 213[M-OH]⁺.

### Step 5: Preparation of Core A-35e

Compound A-35d (131 mg, 0.57 mmol) was dissolved in MC (20 mL) under a nitrogen atmosphere at 0°C, and then acetobromo-alpha-D-galactose (281 mg, 0.68 mmol) and benzyltributylammonium chloride (178 mg, 0.54 mmol) were sequentially added. 5N-sodium hydroxide aqueous solution (0.34 mL, 1.71 mmol) was slowly added to the reaction solution, and the mixture was stirred at 0°C for 30 minutes and at room temperature for 4.5 hours. After completion of the reaction, 2N-hydrochloric acid aqueous solution was added for neutralization, and MC (100 mL) and distilled water (100 mL) were further added to extract the organic layer twice. The obtained organic layer was dried over anhydrous magnesium sulfate, filtered, and then concentrated under reduced pressure. The resulting residue was subjected to column chromatography to obtain Compound A-35e in the form of a yellow gum (168 mg, 52 %).

¹H-NMR (400 MHz, CDCl₃) δ 9.84 (s, 1H), 7.63 - 7.56 (m, 2H), 6.90 (m, 1H), 5.59 (dd, *J* = 10.4, 8 Hz, 1H), 5.50 (m, 1H), 5.40 (m, 1H), 5.21 - 5.13 (m, 2H), 4.29 - 4.07 (m, 3H), 2.91 (m, 1H), 2.81 (m, 1H), 2.60 (m, 1H), 2.21 (s, 3H), 2.09 - 2.01 (m, 10H); EI-MS m/z: 583[M+Na]⁺.

### Step 6: Preparation of Compound A-35f

Compound A-35e (168 mg, 0.3 mmol) was dissolved in THF (5 mL) under a nitrogen atmosphere at 0°C, and then sodium borohydride (28 mg, 0.75 mmol) was added, and the mixture was stirred at 0°C for 2.5 hours. After completion of the reaction, distilled water (50 mL) was added at the same temperature to terminate the reaction, and EA (50 mL) was added to extract the organic layer twice. The obtained organic layer was dried over anhydrous magnesium sulfate, filtered, and then concentrated under reduced pressure. The resulting residue was subjected to column chromatography to obtain Compound A-35f in the form of a white solid (119 mg, 70 %).

¹H-NMR (400 MHz, CDCl₃) δ 7. 31 (m, 1H), 6.83 (m, 1H), 6.71 (s, 1H), 5. 56 (dd, *J* = 10.4, 8 Hz, 1H), 5.48 (d, *J* = 3.2 Hz, 1H), 5.31 (m, 1H), 5.17 - 5.09 (m, 2H), 4.76 (d, *J* = 6 Hz, 2H), 4.31 - 4.24 (m, 1H), 4.19 - 4.06 (m, 2H), 2.92 - 2.76 (m, 2H), 2.60 (m, 1H), 2.20 (s, 3H), 2.11 - 2.01 (m, 11H); EI-MS m/z: 585[M+Na]⁺.

### Step 7: Preparation of Compound A-35g

Compound A-35f (50 mg, 0.089 mmol) was dissolved in MC (2 mL) under a nitrogen atmosphere at 0°C, and then 4-nitrophenyl chloroformate (72 mg, 0.356 mmol) and pyridine (43 µL, 0.533 mmol), DIPEA (46.4 µL, 0.267 mmol) were sequentially added, and the mixture was stirred for 16 hours while slowly raising the temperature from 0°C to room temperature. After completion of the reaction, the organic layer was extracted with EA (50 mL) and 2N-hydrochloric acid aqueous solution (50 mL). The extracted organic layer was dried over anhydrous magnesium sulfate, filtered, and then concentrated under reduced pressure. The residue thus obtained was subjected to column chromatography to obtain A-35g in the form of a solid (27 mg, 34 %).

¹H-NMR (400 MHz, CDCl₃) δ 8.33 - 8.23 (m, 4H), 8.12 - 8.08 (m, 1H), 7.45 - 7.34 (m, 4H), 7.01 - 6.95 (m, 2H), 6.35 - 6.26 (m, 1H), 5.61 - 5.53 (m, 1H), 5.51 - 5.46 (m, 1H), 5.43 - 5.38 (m, 2H), 5.19 - 5.11 (m, 2H), 4.29 - 4.10 (m, 3H), 3.19 - 2.91 (m, 2H), 2.04 (s, 3H), 2.08 - 2.02 (m, 10H); EI-MS m/z: 915[M+Na]⁺.

### Step 8: Preparation of Compound A-35h

Compound A-35g (27 mg, 0.03 mmol) was dissolved in DMF (1 mL) under a nitrogen atmosphere at 0°C, and then MMAF-OMe (CAS NO. 863971-12-4, 34 mg, 0.045 mmol), HOBt (12 mg, 0.091 mmol), DIPEA (26.3 µL, 0.151 mmol) and pyridine (1 mL) were sequentially added, and the mixture was stirred at room temperature for 16 hours. After completion of the reaction, 2N-hydrochloric acid aqueous solution (1 mL) was added to terminate the reaction. The reaction solution was diluted with ACN (1 mL) and distilled water (1 mL), then purified using Preparative-HPLC, and freeze-dried to obtain Compound A-35h in the form of a solid (16.8 mg, 26 %); EI-MS m/z: 2107 [M+H]⁺, 1054 1/2[M+H]+.

### Step 9: Preparation of Compound A-35

Compound A-35h (16.8 mg, 0.008 mmol) was dissolved in methanol (1 mL) and THF (0.5 mL) at 0°C, and then lithium hydroxide hydrate (5 mg, 0.119 mmol) dissolved in distilled water (0.2 mL) was slowly added dropwise, and the mixture was stirred at 0°C for 1 hour and at room temperature for an additional 1 hour. After completion of the reaction, 2N-hydrochloric acid aqueous solution (1 mL) was added to terminate the reaction, and the reaction solution was diluted with ACN (1 mL) and distilled water (1 mL), then purified using Preparative-HPLC, and freeze-dried to obtain Compound A-35 in the form of a white solid (12.5 mg, 82%); EI-MS m/z: 1911 [M+H]⁺, 956 1/2[M+H]⁺.

### Example I-28: Preparation of Compound A-36

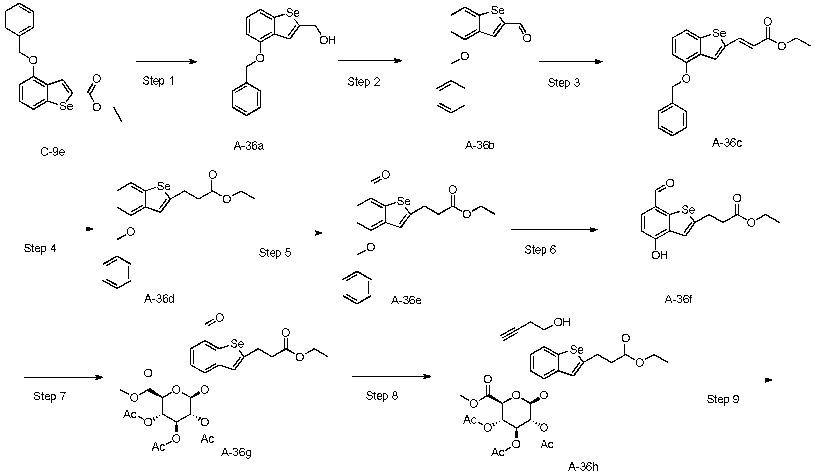

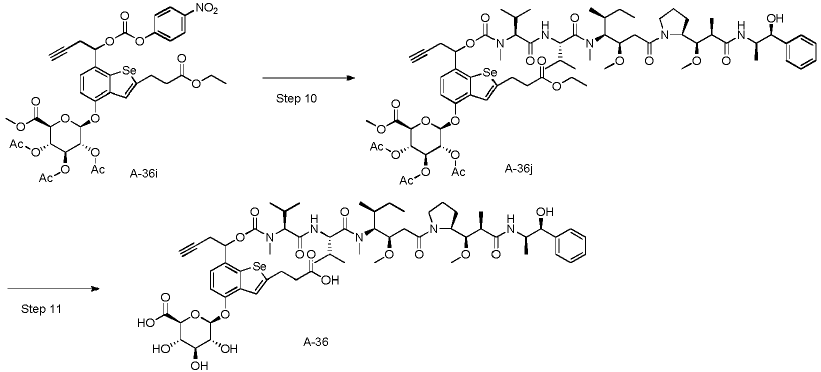

### Step 1: Preparation of Compound A-36a

Compound C-9e (5.42 mg, 15.09 mmol) was dissolved in MC (60 mL) and toluene (180 mL) under a nitrogen atmosphere at -78°C, and then DIBAL-H (Diisobutylaluminum hydride, 1M in toluene, 37.7 mL, 37.7 mmol) was slowly added, and the mixture was stirred for 2.5 hours while maintaining the temperature. After completion of the reaction, methanol (50 mL) was added at the same temperature and stirred for 15 minutes, and then distilled water (50 mL) and 2N sodium hydroxide aqueous solution (50 mL) were added at 0°C to terminate the reaction. The reaction solution was diluted with EA (800 mL) and filtered using Celite. A saturated potassium sodium tartrate (300 mL) was added to the filtered residue to extract the organic layer. The obtained organic layer was dried over anhydrous magnesium sulfate, filtered, and then concentrated under reduced pressure. The resulting residue was subjected to column chromatography to obtain A-36a in the form of an ivory solid (4.52 mg, 94 %).

¹H-NMR (400 MHz, CDCl₃) δ 7.65 (s, 1H), 7.50 - 7.44 (m, 3H), 7.44 - 7.37 (m, 2H), 7.37 - 7.32 (m, 1H), 7.18 (t, *J* = 8 Hz, 1H), 6.82 (d, *J* = 8 Hz, 1H), 5.18 (s, 2H), 4.92 (d, *J* = 5.2 Hz, 2H), 1.96 (t, *J* = 5.2 Hz, 1H); EI-MS m/z: 317[M+H]⁺, 299[M-OH]⁺.

### Step 2: Preparation of Compound A-36b

Compound A-36a (4.52 g, 14.25 mmol) was dissolved in MC (92 mL) under a nitrogen atmosphere at room temperature, and then TBAI (526 mg, 1.42 mmol) and TEMPO (223 mg, 1.42 mmol), an aqueous solution of sodium bicarbonate (3.86 mg, 46 mmol) and potassium carbonate (636 mg, 4.6 mmol) dissolved in distilled water (92 mL), N-chlorosuccinimide (NCS, 2.09 g, 15.67 mmol) were sequentially added, and the mixture was stirred for 3 hours. After completion of the reaction, the organic layer was extracted twice with MC (500 mL) and distilled water (500 mL) and washed twice by adding a saturated aqueous sodium chloride solution (400 mL) to the obtained organic layer. The washed organic layer was dried over anhydrous magnesium sulfate, filtered, and then concentrated under reduced pressure. The residue was subjected to column chromatography to obtain Compound A-36b in the form of a light orange solid (4.13 g, 92 %).

¹H-NMR (400 MHz, CDCl₃) δ 9.94 (s, 1H), 8.53 (s, 1H), 7.53 - 7.46 (m, 3H), 7.46 - 7.40 (m, 2H), 7.40 - 7.34 (m, 2H), 6.87 (d, *J* = 8 Hz, 1H), 5.23 (s, 2H); EI-MS m/z: 315[M+H]⁺.

### Step 3: Preparation of Compound A-36c

Compound A-36b (4.13 g, 13.12 mmol) was dissolved in MC (65 mL) under a nitrogen atmosphere at room temperature, and then (carbethoxymethylene)triphenylphosphorane (combi-blocks, CAS No. 1099-45-2, 9.14 g, 26.23 mmol) was added, and the mixture was stirred at room temperature for 3 hours. After completion of the reaction, the reaction solution was concentrated under reduced pressure. The resulting residue was subjected to column chromatography to obtain Compound A-36c in the form of a pink solid (4.81 g, 95 %).

¹H-NMR (400 MHz, CDCl₃) δ 7.93 (s, 1H), 7.85 (d, *J* = 15.2 Hz, 1H), 7.49 - 7.44 (m, 2H), 7.44 - 7.38 (m, 3H), 7.38 - 7.33 (m, 1H), 7.27 - 7.21 (m, 1H), 6.81 (d, *J* = 8 Hz, 1H), 6.09 (d, *J* = 15.2 Hz, 1H), 5.19 (s, 2H), 4.25 (q, *J* = 7.2 Hz, 2H), 1.33 (t, *J* = 7.2 Hz, 3H); EI-MS m/z: 385[M+H]⁺.

### Step 4: Preparation of Compound A-36d

Compound A-36c (4.81 g, 12.48 mmol) was dissolved in EA (140 mL) and methanol (420 mL) under a nitrogen atmosphere at room temperature, and then 5% palladium charcoal (5% Pd/C, 5.1 g) was added, and the mixture was stirred under a hydrogen environment for 2 days. After completion of the reaction, the reaction solution was filtered using Celite and concentrated under reduced pressure. The resulting residue was subjected to column chromatography to obtain Compound A-36d in the form of a light yellow oil (3.87 g, 80 %).

¹H-NMR (400 MHz, CDCl₃) δ 7.51 - 7.44 (m, 3H), 7.44 - 7.37 (m, 3H), 7.37 - 7.32 (m, 1H), 7.14 (t, *J* = 8 Hz, 1H), 6.80 (d, *J* = 8 Hz, 1H), 5.18 (s, 2H), 4.16 (q, *J* = 7.2 Hz, 2H), 3.27 (t, *J* = 7.6 Hz, 2H), 2.72 (t, *J* = 7.6 Hz, 2H), 1.25 (t, *J* = 7.2 Hz, 3H); EI-MS m/z: 387[M+H]⁺.

### Step 5: Preparation of Compound A-36e

Compound A-36d (3.87 g, 9.99 mmol) was dissolved in MC (285 mL) under a nitrogen atmosphere at -78°C, and then dichloromethyl methyl ether (2.74 mL, 29.97 mmol) and titanium tetrachloride solution (1M-TiCl₄ in MC, 29.9 mL, 29.97 mmol) were sequentially and slowly added, and the mixture was stirred for 2 hours while maintaining the temperature. After completion of the reaction, distilled water (700 mL) was slowly added dropwise to terminate the reaction, and MC (600 mL) was added to extract the organic layer twice. The obtained organic layer was dried over anhydrous magnesium sulfate, filtered, and then concentrated under reduced pressure. The resulting residue was subjected to column chromatography to obtain Compound A-36e (2.64 g, 64%).

¹H-NMR (400 MHz, CDCl₃) δ 10.04 (s, 1H), 7.72 (d, *J* = 8.4 Hz, 1H), 7.58 (s, 1H), 7.51 - 7.34 (m, 5H), 6.98 (d, *J* = 8.4 Hz, 1H), 5.30 (s, 2H), 4.16 (q, *J* = 7.2 Hz, 2H), 3.32 (t, *J* = 7.6 Hz, 2H), 2.74 (t, *J* = 7.6 Hz, 2H), 1.25 (t, *J* = 7.2 Hz, 3H); EI-MS m/z: 415[M+H]⁺.

### Step 6: Preparation of Compound A-36f

Compound A-36e (2.64 g, 6.35 mmol) was dissolved in MC (250 mL) under a nitrogen atmosphere at -78°C, and then boron trichloride solution (1M-BCl₃ in MC, 19 mL, 19.07 mmol) was slowly added, and the mixture was stirred for 2.5 hours while maintaining the temperature. After completion of the reaction, distilled water (150 mL) was slowly added dropwise at -50°C to terminate the reaction, and 2N-sodium hydroxide aqueous solution was added at 0°C to neutralize the reaction solution. MC (250 mL) and distilled water (150 mL) were further added to the mixture to extract the organic layer twice. The obtained organic layer was dried over anhydrous magnesium sulfate, filtered, and then concentrated under reduced pressure. The resulting residue was subjected to column chromatography to obtain Compound A-36f in the form of a light yellow solid (1.15 g, 55 %).

¹H-NMR (400 MHz, CDCl₃) δ 10.00 (s, 1H), 7.62 (d, *J* = 8 Hz, 1H), 7.51 (s, 1H), 6.96 (s, 1H), 6.87 (d, *J* = 8 Hz, 1H), 4.19 (q, *J* = 7.2 Hz, 2H), 3.33 (t, *J* = 7.2 Hz, 2H), 2.79 (t, *J =* 7.2 Hz, 2H), 1.27 (t, *J* = 7.2 Hz, 3H); EI-MS m/z: 325[M+H]⁺.

### Step 7: Preparation of Compound A-36g

Compound A-36f (481 mg, 1.48 mmol) was dissolved in MC (50 mL) under a nitrogen atmosphere at 0°C, and then acetobromo-α-D-glucuronic acid methyl ester (TCI, CAS No. 21085-72-3, 705 mg, 1.77 mmol), benzyltributylammonium chloride (461 mg, 1.48 mmol) and 5N-sodium hydroxide aqueous solution (0.89 mL, 4.43 mmol) were slowly added, and the mixture was stirred at 0°C for 30 minutes and at room temperature for additional 16 hours. After completion of the reaction, 2N-hydrochloric acid aqueous solution was added to neutralize the reaction solution, and MC (200 mL) and distilled water (200 mL) were further added to extract the organic layer twice. The obtained organic layer was dried over anhydrous magnesium sulfate, filtered, and then concentrated under reduced pressure. The resulting residue was subjected to column chromatography to obtain Compound A-36g in the form of an ivory solid (238 mg, 25 %).

¹H-NMR (400 MHz, CDCl₃) δ 10.10 (s, 1H), 7.74 (d, *J* = 8 Hz, 1H), 7.39 (s, 1H), 7.09 (d, *J* = 8 Hz, 1H), 5.48 - 5.37 (m, 4H), 4.32 - 4.27 (m, 1H), 4.18 (q, *J* = 7.2 Hz, 2H), 3.71 (s, 3H), 3.31 (t, *J* = 7.6 Hz, 2H), 2.74 (t, *J* = 7.6 Hz, 2H), 2.09 (s, 3H), 2.07 (s, 3H), 2.06 (s, 3H), 1.27 (t, *J* = 7.2 Hz, 3H); EI-MS m/z: 663[M+Na]⁺.

### Step 8: Preparation of Compound A-36h

Compound A-36g (67.6 mg, 0.105 mmol) was dissolved in THF (3 mL) under a nitrogen atmosphere at room temperature, and then Zinc (Zn powder, 51 mg, 0.79 mmol), 1,2-diiodoethane (59.6 mg, 0.21 mmol) and propargyl bromide (37.4 µL, 0.42 mmol) were sequentially added, followed by ultrasonication for 2.5 hours. After completion of the reaction, the reaction solution was diluted with EA (50 mL) and filtered using Celite. Distilled water (30 mL) and a saturated aqueous sodium chloride solution (20 mL) was added to the filtered residue to extract the organic layer. The obtained organic layer was dried over anhydrous magnesium sulfate, filtered, and then concentrated under reduced pressure. The resulting residue was subjected to column chromatography to obtain A-36h in the form of a white gum (61.8 mg, 86 %).

¹H-NMR (400 MHz, CDCl₃) δ 7.34 (s, 1H), 7.20 - 7.15 (m, 1H), 6.93 (d, *J* = 8 Hz, 1H), 5.43 - 5.33 (m, 3H), 5.22 - 5.18 (m, 1H), 5.01 (m, 1H), 4.22 - 4.09 (m, 3H), 3.73 (s, 3H), 3.25 (t, *J* = 7.6 Hz, 2H), 2.82 - 2.68 (m, 4H), 2.52 (m, 1H), 2.11 (t, *J* = 2.8 Hz, 1H), 2.10 - 2.02 (m, 9H), 1.27 (t, *J* = 7.2 Hz, 3H); EI-MS m/z: 681[M+H]⁺, 704[M+Na]⁺, 663[M-OH]⁺.

### Step 9: Preparation of Compound A-36i

Compound A-36h (65.1 mg, 0.095 mmol) was dissolved in MC (3 mL) under a nitrogen atmosphere at 0°C, and then 4-nitrophenyl chloroformate (57.7 mg, 0.286 mmol), DIPEA (75 µL, 0.43 mmol), pyridine (23.1 µL, 0.286 mmol), DMAP (2.3 mg, 0.019 mmol) and DMF (20 µL) were sequentially added, and the mixture was stirred for 24 hours while slowly raising the temperature from 0°C to room temperature . After completion of the reaction, the organic layer was extracted twice with EA (50 mL) and 2N-hydrochloric acid aqueous solution (50 mL). The obtained organic layer was dried over anhydrous magnesium sulfate, filtered, and then concentrated under reduced pressure. The residue thus obtained was subjected to column chromatography to obtain A-36i in the form of a white solid (62.6 mg, 77%).

¹H-NMR (400 MHz, CDCl₃) δ 8.25 (d, *J* = 9.2 Hz, 2H), 7.39 - 7.33 (m, 3H), 7.27 (m, 1H), 6.95 (d, *J* = 8.4 Hz, 1H), 5.91 (m, 1H), 5.42 - 5.34 (m, 3H), 5.26 - 5.21 (m, 1H), 4.25 - 4.05 (m, 3H), 3.73 (s, 3H), 3.26 (t, *J* = 7.2 Hz, 2H), 3.04 (m, 1H), 2.95 (m, 1H), 2.72 (t, *J* = 7.2 Hz, 2H), 2.09 - 2.03 (m, 10H), 1.27 (t, *J* = 7.2 Hz, 3H); EI-MS m/z: 869[M+Na]⁺.

### Step 10: Preparation of Compound A-36j

Compound A-36i (43.5 mg, 0.051 mmol) was dissolved in DMF (1 mL) under a nitrogen atmosphere at 0°C, and then MMAE (monomethyl auristatin E, CAS NO. 474645-27-7, 36.9 mg, 0.051 mmol), HOBt (10.4 mg, 0.077 mmol), DIPEA (22.4 µL, 0.128 mmol) and pyridine (1 mL) were sequentially added, and the mixture was stirred at room temperature for 16 hours. After completion of the reaction, the organic layer was extracted twice with EA (50 mL) and 2N-hydrochloric acid aqueous solution (50 mL). The obtained organic layer was dried over anhydrous magnesium sulfate, filtered, and then concentrated under reduced pressure. The resulting residue was subjected to column chromatography to obtain A-36j in the form of a white gum (48.5 mg, 66%); EI-MS m/z: 1426[M+H]⁺.

### Step 11: Preparation of Compound A-36

Compound A-36j (48.5 mg, 0.034 mmol) was dissolved in THF (0.34 mL), methanol (0.85 mL) and distilled water (0.22 mL) at -20°C, and then lithium hydroxide hydrate (10.7 mg, 0.255 mmol) dissolved in distilled water (0.63 mL) was slowly added dropwise, and the mixture was stirred for 4.5 hours while slowly raising the temperature from -20°C to -5°C. The reaction solution was stirred at 0°C for additional 3 hours. After completion of the reaction, 2N-hydrochloric acid aqueous solution (1 mL) was added to terminate the reaction, and the reaction solution was diluted with ACN (2 mL) and distilled water (2 mL), then purified using Preparative-HPLC, and freeze-dried to obtain Compound A-36 in the form of a white solid (29.8 mg, 70%); EI-MS m/z: 1258[M+H]⁺.

### Example I-29: Preparation of Compound A-37

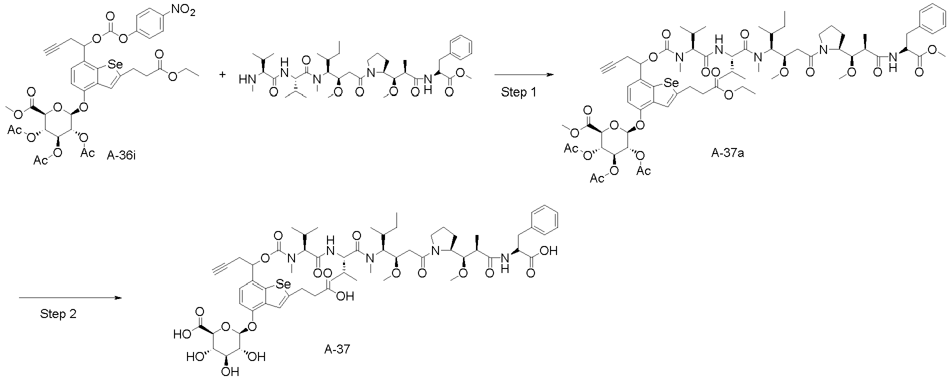

### Step 1: Preparation of Compound A-37a

Compound A-36i (32.5 mg, 0.038 mmol) was dissolved in DMF (1 mL) under a nitrogen atmosphere at 0°C, and then MMAF-OMe (28.6 mg, 0.038 mmol), HOBt (7.8 mg, 0.057 mmol), DIPEA (16.7 µL, 0.096 mmol) and pyridine (1 mL) were sequentially added, and the mixture was stirred at room temperature for 16 hours. After completion of the reaction, the organic layer was extracted with EA (50 mL) and 2N-hydrochloric acid aqueous solution (50 mL). The obtained organic layer was dried over anhydrous magnesium sulfate, filtered, and then concentrated under reduced pressure. The resulting residue was subjected to column chromatography to obtain Compound A-37a in the form of a solid (41.3 mg, 74 %); EI-MS m/z: 1454[M+H]⁺.

### Step 2: Preparation of Compound A-37

Compound A-37a (41.3 mg, 0.028 mmol) was dissolved in THF (0.35 mL), methanol (0.71 mL) and distilled water (0.2 mL) at -20°C, and then lithium hydroxide hydrate (8.9 mg, 0.213 mmol) dissolved in distilled water (0.51 mL) was slowly added dropwise, and the mixture was stirred for 2.5 hours while slowly raising the temperature from -20°C to -5°C. The reaction solution was stirred at 0°C for additional 3 hours. After completion of the reaction, 2N-hydrochloric acid aqueous solution (1 mL) was added to terminate the reaction, and the reaction solution was diluted with ACN (2 mL) and distilled water (2 mL), then purified using Preparative-HPLC, and freeze-dried to obtain Compound A-37 in the form of a white solid (24.9 mg, 69 %); EI-MS m/z: 1272 [M+H]⁺.

### Example I-30: Preparation of Compound A-38

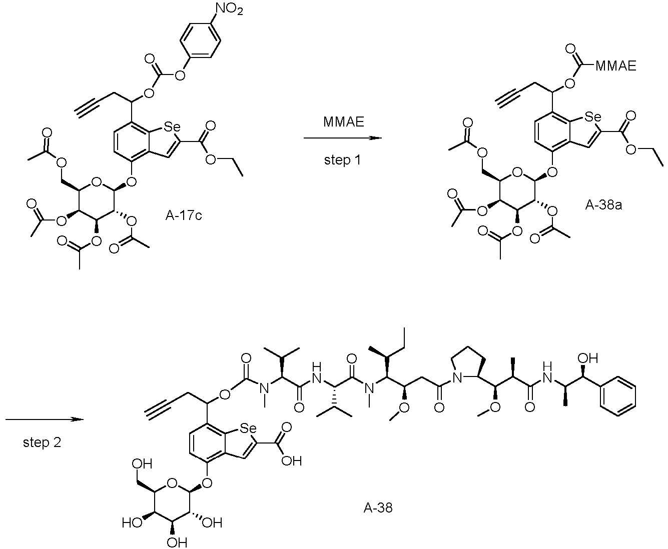

### Step 1: Preparation of Compound A-38a

Compound A-17c (70 mg, 0.084 mmol) was dissolved in DMF (3 mL) under a nitrogen atmosphere at 0°C, and then MMAE (112.4 mg, 0.10 mmol), HOBt (11.3 mg, 0.084 mmol), pyridine (300 µL) and DIPEA (29.3 µL, 0.168 mmol) were sequentially added, and the mixture was stirred for 30 minutes. The reaction mixture was stirred at room temperature for additional 15 hours, and then extracted with EA (50 mL) and 1N-hydrochloric acid aqueous solution (50 mL). The obtained organic layer was dried over anhydrous magnesium sulfate, filtered, and then concentrated under reduced pressure. The resulting residue was subjected to column chromatography to obtain A-38a (87 mg, 73.3 %); EI-MS m/z: 1412 [M+H]⁺, 1434 [M+Na]⁺.

### Step 2: Preparation of Compound A-38

Compound A-38a (87 mg, 0.061 mmol) was dissolved in methanol (3 mL) and THF (1.5 mL) at 0°C, and then lithium hydroxide hydrate (51.7 mg, 1.22 mmol) dissolved in distilled water (0.6 mL) was added dropwise, and the mixture was stirred for 30 minutes. The reaction mixture was stirred at room temperature for additional 5 hours, and then 2N-hydrochloric acid aqueous solution was slowly added to adjust the pH of the reaction solution to 3. The reaction solution was diluted with ACN (5 mL) containing 0.1% formic acid and distilled water (5 mL), then purified using Preparative-HPLC, and freeze-dried to obtain Compound A-38 in the form of a white solid (59.1 mg, 79 %); EI-MS m/z: 1216[M+H]⁺.

### Example I-31: Preparation of Compound A-39

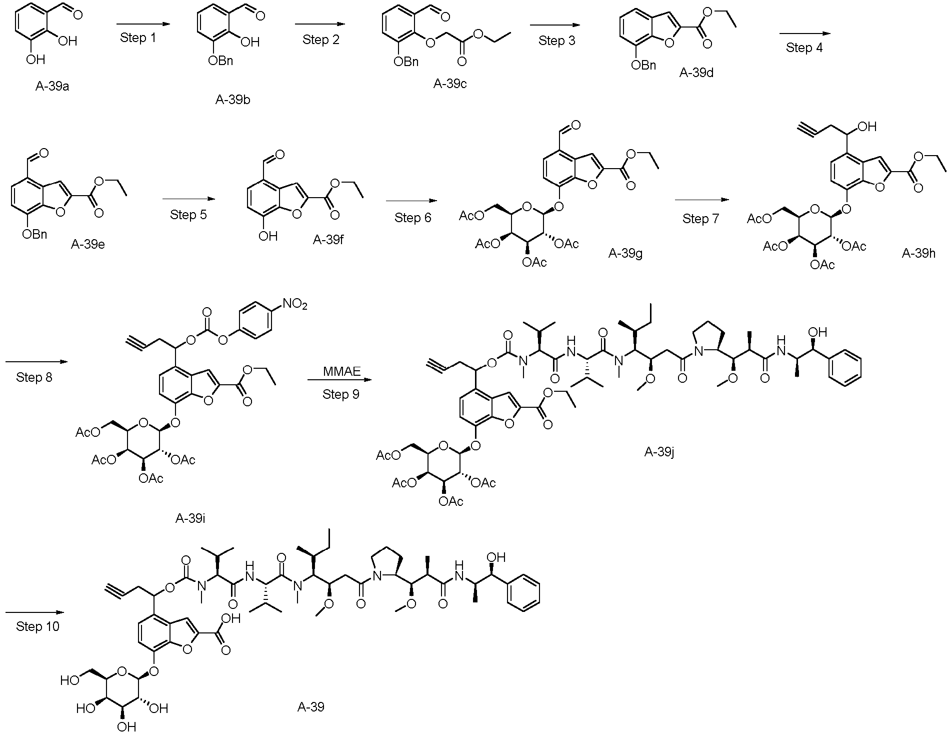

### Step 1: Preparation of Compound A-39b

THF (12.0 mL) was added dropwise to sodium hydride (NaH, 60 % dispersion in mineral oil, 724.0 mg, 18.1 mmol) under a nitrogen atmosphere at 0°C, and Compound A-39a (Alfa Aesar, CAS No. 24677-78-9, 1.0 g, 7.24 mmol) dissolved in THF (8.0 mL) was slowly added to the reaction solution, and then the mixture was stirred at room temperature for 1 hour. Benzyl bromide (0.86 mL, 7.24 mmol) was slowly added to the reaction solution, and the mixture was stirred for additional 16 hours. After completion of the reaction, distilled water (50 mL), 2N-hydrochloric acid aqueous solution (50 mL) and EA (100 mL) were added to extract the organic layer. The obtained organic layer was dried over anhydrous magnesium sulfate, filtered, and then concentrated under reduced pressure. Ethanol (30.0 mL) was added to the residue, and then the resulting solid was filtered to obtain Compound A-39b in the form of a yellow solid (680.0 mg, 41 %).

¹H-NMR (400 MHz, CDCl₃) δ 11.07 (s, 1H), 9.92 (s, 1H), 7.45 - 7.31 (m, 5H), 7.19 (dd, *J* = 7.6 Hz, *J* = 1.2 Hz, 1H), 7.13 (d, *J* = 7.6 Hz, 1H), 6.89 (t, *J* = 7.6 Hz, 1H), 5.19 (s, 3H).

### Step 2: Preparation of Compound A-39c

Compound A-39b (680.0 mg, 2.97 mmol) was dissolved in DMF (5.0 mL) under a nitrogen atmosphere, and then ethyl bromoacetate (0.38 mL, 3.57 mmol) and potassium carbonate (1.03 g, 7.44 mmol) were sequentially added, and then the mixture was stirred at 80°C for 2 hours. After completion of the reaction, distilled water (50 mL) and EA (50 mL) were added to extract the organic layer. The obtained organic layer was washed by adding a saturated aqueous sodium chloride solution (50 mL). The organic layer was dried over anhydrous magnesium sulfate, filtered, and then concentrated under reduced pressure. The residue was subjected to column chromatography to obtain Compound A-39c in the form of a white solid (870.0 mg, 93 %).

¹H-NMR (400 MHz, CDCl₃) δ 10.63 (s, 1H), 7.47 (d, *J* = 8.0 Hz, 1H), 7.44 - 7.34 (m, 5H), 7.19 (d, *J* = 8.0 Hz, 1H), 7.11 (t, *J* = 8.0 Hz, 1H), 5.13 (s, 2H), 4.85 (s, 2H) 4.15 (q, *J* = 7.2 Hz, 2H), 1.22 (t, *J* = 7.2 Hz, 3H).

### Step 3: Preparation of Compound A-39d

Compound A-39c (870.0 mg, 2.76 mmol) was dissolved in DMF (10.0 mL) under a nitrogen atmosphere, and then potassium carbonate (1.03 g, 7.44 mmol) was added, and then the mixture was stirred at 100°C for 3 hours. After completion of the reaction, distilled water (100 mL) and EA (100 mL) were added to extract the organic layer. The obtained organic layer was washed by adding a saturated aqueous sodium chloride solution (100 mL). The organic layer was dried over anhydrous magnesium sulfate, filtered, and then concentrated under reduced pressure. The residue was subjected to column chromatography to obtain Compound A-39d in the form of a white solid (640.0 mg, 78 %).

¹H-NMR (400 MHz, CDCl₃) δ 7.51 - 7.49 (m, 3H), 7.40 - 7.30 (m, 3H), 7.25 - 7.23 (m, 1H), 7.15 (t, *J* = 8.0 Hz, 1H), 6.94 (d, *J* = 8.0 Hz, 1H), 5.33 (s, 2H), 4.44 (q, *J* = 7.2 Hz, 2H), 1.42 (t, *J* = 7.2 Hz, 3H); EI-MS m/z: 297 [M+H]⁺.

### Step 4: Preparation of Compound A-39e

Compound A-39d (640.0 mg, 2.16 mmol) was dissolved in MC (20.0 mL) under a nitrogen atmosphere at -78°C, and then dichloromethyl methyl ether (0.57 mL, 6.48 mmol) and titanium tetrachloride solution (1M-TiCl₄ in MC, 6.48 mL, 6.48 mmol) were sequentially and slowly added, and the mixture was stirred for 2 hours while maintaining the temperature. After completion of the reaction, cooled distilled water (100 mL) was slowly added dropwise to terminate the reaction, and EA (100 mL) was added to extract the organic layer. The obtained organic layer was dried over anhydrous magnesium sulfate, filtered, and then concentrated under reduced pressure. The resulting residue was subjected to column chromatography to obtain Compound A-39e in the form of a white solid (420.0 mg, 60 %).

¹H-NMR (400 MHz, CDCl₃) δ 10.03 (s, 1H), 8.22 (s, 1H), 7.69 (d, *J* = 8.0 Hz, 1H), 7.50 - 7.48 (m, 2H), 7.42 - 7.34 (m, 3H), 7.04 (d, *J* = 8.0 Hz, 1H), 5.42 (s, 2H), 4.45 (q, *J* = 7.2 Hz, 2H), 1.43 (t, *J* = 7.2 Hz, 3H); EI-MS m/z: 325 [M+H]⁺.

### Step 5: Preparation of Compound A-39f

Compound A-39e (420.0 mg, 1.29 mmol) was dissolved in MC (13.0 mL) under a nitrogen atmosphere at -78°C, and then boron trichloride solution (1M-BCl₃ in MC, 3.88 mL, 3.88 mmol) was slowly added, and the mixture was stirred for 2 hours while maintaining the temperature. After completion of the reaction, distilled water (50.0 mL) was slowly added dropwise at -50°C to terminate the reaction, and EA (100 mL) and distilled water (100 mL) were further added at room temperature to extract the organic layer. The obtained organic layer was dried over anhydrous magnesium sulfate, filtered, and then concentrated under reduced pressure. The resulting residue was subjected to column chromatography to obtain Compound A-39f in the form of an ivory solid (145.0 mg, 47 %).

¹H-NMR (400 MHz, CDCl₃) δ 10.05 (s, 1H), 8.23 (s, 1H), 7.73 (d, *J =* 8.0 Hz, 1H), 7.10 (d, *J= 8.0* Hz, 1H), 6.51 (s, 1H), 4.46 (q, *J =* 7.2 Hz, 2H), 1.44 (t, *J =* 7.2 Hz, 3H).

### Step 6: Preparation of Compound A-39g

Compound A-39f (140.0 mg 0.59 mmol) was dissolved in ACN (20.0 mL) under a nitrogen atmosphere at 0°C, and then acetobromo-alpha-D-galactose (Merck, 295.0 mg, 0.71 mmol) and silver oxide (I) (346.0 mg, 1.49 mmol) were sequentially added, and the mixture was stirred at room temperature for 2 hours. After completion of the reaction, the reaction solution was diluted with EA (100 mL), filtered using Celite, and washed by adding a saturated aqueous sodium chloride solution (100 mL) to the solution. The obtained organic layer was dried over anhydrous magnesium sulfate, filtered, and then concentrated under reduced pressure. The resulting residue was subjected to column chromatography to obtain Compound A-39g in the form of a white solid (303.0 mg, 89 %).

¹H-NMR (400 MHz, CDCl₃) δ 10.10 (s, 1H), 8.23 (s, 1H), 7.73 (d, *J =* 8.0 Hz, 1H), 7.27 (d, *J* = 8.0 Hz, 1H), 5.60 - 5.59 (m, 2H), 5.50 (d, *J =* 3.2 Hz, 1H), 5.21 - 5.17 (m, 1H), 4.45 (q, *J* = 7.2 Hz, 2H), 4.23 - 4.17 (m, 3H), 2.19 (s, 3H), 2.14 (s, 3H), 2.03 (s, 3H), 1.98 (s, 3H), 1.43 (t, *J* = 7.2 Hz, 3H); EI-MS m/z: 586 [M+Na]⁺.

### Step 7: Preparation of Compound A-39h

Compound A-39g (100.0 mg, 0.2 mmol) was dissolved in THF (2.0 mL) under a nitrogen atmosphere at room temperature, and then propargyl bromide (23.6 µL, 0.265 mmol), 1,2-diiodoethane (57.8 mg, 0.177 mmol) and Zinc (Zn powder, 57.8 mg, 0.885 mmol) were sequentially added, followed by ultrasonication for 2 hours. After completion of the reaction, the reaction solution was diluted with EA (100 mL) and filtered using Celite. The obtained filtrate was concentrated under reduced pressure. The resulting residue was subjected to column chromatography to obtain Compound A-39h in the form of a white solid (85.0 mg, 79 %).

¹H-NMR (400 MHz, CDCl₃) δ 7.75 (d, *J =* 4.0 Hz, 1H), 7.25 (d, *J =* 4.0 Hz, 1H), 7.20 (s, 1H), 5.57 (dd, *J =* 10.4 Hz, *J =* 8.0 Hz, 1H), 5.47 (d, *J =* 3.2 Hz, 1H), 5.27 (dd, *J= 8.0* Hz, *J = 2.0* Hz, 1H), 5.15 (dd, *J =* 10.4 Hz, *J =* 3.2 Hz, 2H), 4.43 (q, *J =* 7.2 Hz, 2H), 4.21 - 4.18 (m, 1H) 4.11 - 4.06 (m, 1H), 2.75 - 2.71 (m, 2H), 2.51 (d, *J =* 3.2 Hz, 1H), 2.19 (s, 6H), 2.10 (t, *J =* 3.2 Hz, 1H), 2.02 (s, 3H), 2.01 (s, 3H), 1.42 (t, *J =* 7.2 Hz, 3H), 1.25 (t, *J =* 7.2 Hz, 1H).

### Step 8: Preparation of Compound A-39i

Compound A-39h (85.0 mg, 0.14 mmol) was dissolved in MC (3.0 mL) under a nitrogen atmosphere at 0°C, and then 4-nitrophenyl chloroformate (42.5 mg, 0.21 mmol) and DIPEA (25.0 µL, 0.14 mmol) and pyridine (34.0 µL, 0.42 mmol) were sequentially added, and the mixture was stirred for 16 hours while slowly raising the temperature from 0°C to room temperature. After completion of the reaction, EA (50 mL) and distilled water (50 mL) were added to extract the organic layer. The obtained organic layer was dried over anhydrous magnesium sulfate, filtered, and then concentrated under reduced pressure. The resulting residue was subjected to column chromatography to obtain Compound A-39i in the form of a white solid (56.0 mg, 51 %).

¹H-NMR (400 MHz, CDCl₃) δ 8.25 (d, *J =* 9.2 Hz, 2H), 7.72 (d, *J =* 0.8 Hz, 1H), 7.34 (d, *J =* 9.2 Hz, 2H), 7.31 - 7.21 (m, 2H), 6.07 (t, *J =* 7.2 Hz, 1H), 5.59 (dd, *J =* 10.4 Hz, *J =* 8.0 Hz, 1H), 5.48 (d, *J* = 3.2 Hz, 1H), 5.33 (dd, *J* = 8.0 Hz, *J =* 2.0 Hz, 1H), 5.15 (dd, *J =* 10.4 Hz, *J* = 3.2 Hz, 1H), 4.44 (t, *J =* 7.2 Hz, 2H), 4.22 - 4.18 (m, 2H), 4.15 - 4.09 (m, 2H), 3.08 - 3.02 (m, 1H), 2.93 - 2.87 (m, 1H), 2.19 (s, 3H), 2.17 (s, 3H), 2.03 (s, 3H), 2.01 (s, 3H), 1.43 (t, *J =* 7.2 Hz, 3H).

### Step 9: Preparation of Compound A-39j

Compound A-39i (56.0 mg, 0.072 mmol) was dissolved in DMF (2.0 mL) under a nitrogen atmosphere at 0°C, and then MMAE (52.2 mg, 0.072 mmol), HOBt (14.7 mg, 0.109 mmol), DIPEA (31.7 µL, 0.181 mmol) and pyridine (1.0 mL) were sequentially added, and the mixture was stirred at room temperature for 6 hours. After completion of the reaction, extraction was performed using distilled water (50 mL), 2N-hydrochloric acid aqueous solution (50 mL) and EA (100 mL). The obtained organic layer was dried over anhydrous magnesium sulfate, filtered, and then concentrated under reduced pressure. The resulting residue was subjected to column chromatography to obtain Compound A-39j in the form of a white solid (56.0 mg, 57 %); EI-MS m/z: 1349 [M+H]⁺.

### Step 10: Preparation of Compound A-39

Compound A-39j (29.0 mg, 0.021 mmol) was dissolved in methanol (1.0 mL) under a nitrogen atmosphere at 0°C, and then lithium hydroxide (6.8 mg, 0.161 mmol) dissolved in distilled water (1.0 mL) was slowly added dropwise, and the mixture was stirred at 0°C for 2 hours. After completion of the reaction, 2N-hydrochloric acid aqueous solution (1.0 mL) was added to terminate the reaction, and the reaction solution was diluted with ACN (1.0 mL) and distilled water (1.0 mL), then purified using Preparative-HPLC, and freeze-dried to obtain Compound A-39 in the form of a white solid (18.0 mg, 72 %); EI-MS m/z: 1153 [M+H]⁺.

### Example 1-32: Preparation of Compound A-40

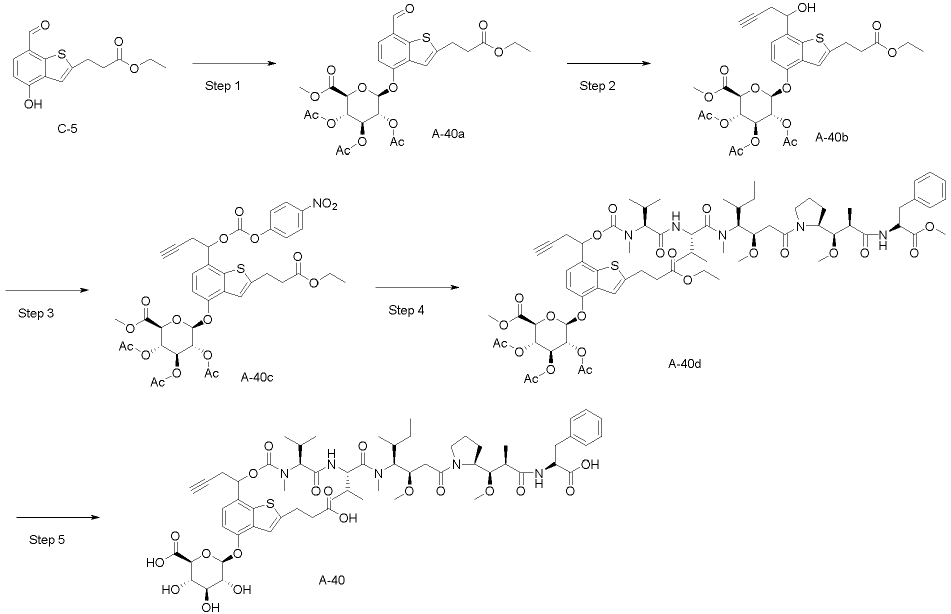

### Step 1: Preparation of Compound A-40a

Core C-5 (500 mg, 1.79 mmol) was dissolved in MC (60 mL) under a nitrogen atmosphere at 0°C, and then acetobromo-α-D-glucuronic acid methyl ester (TCI, 856 mg, 2.15 mmol), benzyltributylammonium chloride (560 mg, 1.79 mmol) and 5N-sodium hydroxide aqueous solution (1.08 mL, 5.39 mmol) were slowly added, and the mixture was stirred for 16 hours while slowly raising the temperature from 0°C to room temperature. After completion of the reaction, 2N-hydrochloric acid aqueous solution was added to neutralize the reaction solution, and MC (100 mL) and distilled water (100 mL) were further added to extract the organic layer three times. The obtained organic layer was dried over anhydrous magnesium sulfate, filtered, and then concentrated under reduced pressure. The resulting residue was subjected to column chromatography to obtain Compound A-40a in the form of an ivory solid (88.7 mg, 8 %).

¹H-NMR (400 MHz, CDCl₃) δ 10.08 (s, 1H), 7.76 (d, *J =* 8 Hz, 1H), 7.16 (s, 1H), 7.05 (d, *J* = 8 Hz, 1H), 5.46 - 5.37 (m, 4H), 4.32 - 4.28 (m, 1H), 4.16 (q, *J =* 7.2 Hz, 2H), 3.71 (s, 3H), 3.27 (t, *J =* 7.6 Hz, 2H), 2.76 (t, *J =* 7.6 Hz, 2H), 2.09 (s, 3H), 2.07 (s, 3H), 2.06 (s, 3H), 1.27 (t, *J* = 7.2 Hz, 3H); EI-MS m/z: 594[M+H]⁺.

### Step 2: Preparation of Compound A-40b

Compound A-40a (110 mg, 0.185 mmol) was dissolved in THF (3 mL) under a nitrogen atmosphere at room temperature, and then Zinc (Zn powder, 60.5 mg, 0.925 mmol), 1,2-diiodoethane (104.2 mg, 0.37 mmol) and propargyl bromide (46.3 µL, 0.416 mmol) were sequentially added, followed by ultrasonication for 3.5 hours. After completion of the reaction, the reaction solution was diluted with EA (50 mL) and filtered using Celite. The organic layer was extracted by adding distilled water (30 mL) and a saturated aqueous sodium chloride solution (20 mL). The obtained organic layer was dried over anhydrous magnesium sulfate, filtered, and then concentrated under reduced pressure. The resulting residue was subjected to column chromatography to obtain Compound A-40b in the form of a white solid (93.1 mg, 79 %).

¹H-NMR (400 MHz, CDCl₃) δ 7.29 (m, 1H), 7.12 (s, 1H), 6.91 (d, *J =* 8.4 Hz, 1H), 5.43 - 5.33 (m, 3H), 5.24 - 5.19 (m, 1H), 5.10 (m, 1H), 4.24 - 4.11 (m, 3H), 3.73 (s, 3H), 3.22 (t, *J =* 7.6 Hz, 2H), 2.81 - 2.77 (m, 2H), 2.73 (t, *J=* 7.6 Hz, 2H), 2.52 (t, *J =* 3.2 Hz, 1H), 2.12 - 2.02 (m, 10H), 1.27 (t, *J =* 7.2 Hz, 3H); EI-MS m/z: 634[M+H]⁺, 656[M+Na]⁺, 616[M-OH]⁺.

### Step 3: Preparation of Compound A-40c

Compound A-40b (93.1 mg, 0.147 mmol) was dissolved in MC (3 mL) under a nitrogen atmosphere at 0°C, and then 4-nitrophenyl chloroformate (59.1 mg, 0.293 mmol) and DIPEA (38.3 µL, 0.22 mmol) and pyridine (35.4 µL, 0.44 mmol) were sequentially added, and the mixture was stirred at 0°C for 30 minutes. After completion of the reaction, 2N-hydrochloric acid aqueous solution (10 mL) was added at 0°C to acidify the reaction solution, and then the organic layer was further extracted once with MC (50 mL) and 2N-hydrochloric acid aqueous solution (40 mL). The obtained organic layer was dried over anhydrous magnesium sulfate, filtered, and then concentrated under reduced pressure. The residue thus obtained was subjected to column chromatography to obtain A-40c in the form of a white solid (84.9 mg, 72 %).

¹H-NMR (400 MHz, CDCl₃) δ 8.25 (d, *J =* 9.2 Hz, 2H), 7.36 (d, *J =* 9.2 Hz, 2H), 7.32 (d, *J =* 8.4 Hz, 1H), 7.14 (s, 1H), 6.93 (d, *J =* 8.4 Hz, 1H), 6.01 (t, *J =* 6.4 Hz, 1H), 5.44 - 5.34 (m, 3H), 5.28 - 5.23 (m, 1H), 4.26 - 4.09 (m, 3H), 3.73 (s, 1.5H), 3.72 (s, 1.5H), 3.23 (t, *J =* 7.6 Hz, 2H), 3.05 (m, 1H), 2.98 (m, 1H), 2.74 (t, *J =* 7.6 Hz, 2H), 2.09 - 2.03 (m, 10H), 1.26 (t, *J =* 7.2 Hz, 3H); EI-MS m/z: 821[M+Na]⁺.

### Step 4: Preparation of Compound A-40d

Compound A-40c (47.5 mg, 0.059 mmol) was dissolved in DMF (1 mL) under a nitrogen atmosphere at 0°C, and then MMAF-OMe (44.3 mg, 0.059 mmol), HOBt (12 mg, 0.089 mmol), DIPEA (25.8 µL, 0.148 mmol) and pyridine (1 mL) were sequentially added, and the mixture was stirred at room temperature for 16 hours. After completion of the reaction, the organic layer was extracted twice with EA (50 mL) and 2N-hydrochloric acid aqueous solution (50 mL) and washed by adding a saturated aqueous sodium chloride solution (50 mL). The obtained organic layer was dried over anhydrous magnesium sulfate, filtered, and then concentrated under reduced pressure. The resulting residue was subjected to column chromatography to obtain A-40d in the form of a white gum (69.2 mg, 83 %); EI-MS m/z: 1407[M+H]⁺.

### Step 5: Preparation of Compound A-40

Compound A-40d (69.2 mg, 0.049 mmol) was dissolved in THF (0.6 mL) and methanol (1.2 mL) and distilled water (0.2 mL) at -20°C, and then lithium hydroxide hydrate (20.6 mg, 0.492 mmol) dissolved in distilled water (1 mL) was slowly added dropwise, and the mixture was stirred for 2.5 hours while slowly raising the temperature from -20°C to -5°C. The reaction solution was stirred at 0°C for additional 4 hours. After completion of the reaction, 2N-hydrochloric acid aqueous solution (1 mL) was added to terminate the reaction, and the reaction solution was diluted with ACN (3 mL) and distilled water (3 mL), then purified using Preparative-HPLC, and freeze-dried to obtain Compound A-40 in the form of a white solid (45.5 mg, 75%); EI-MS m/z: 1224[M+H]⁺.

### Example I-33: Preparation of Compound A-41

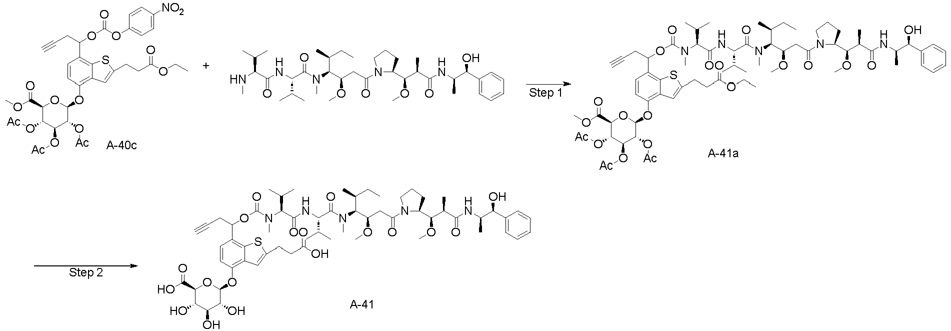

### Step 1: Preparation of Compound A-41a

Compound A-40c (37.7 mg, 0.047 mmol) was dissolved in DMF (1 mL) under a nitrogen atmosphere at 0°C, and then MMAE (33.8 mg, 0.047 mmol), HOBt (9.6 mg, 0.071 mmol), DIPEA (20.5 µL, 0.118 mmol) and pyridine (1 mL) were sequentially added, and the mixture was stirred at room temperature for 16 hours. After completion of the reaction, the organic layer was extracted twice with EA (50 mL) and 2N-hydrochloric acid aqueous solution (50 mL) and washed by adding a saturated aqueous sodium chloride solution (50 mL). The obtained organic layer was dried over anhydrous magnesium sulfate, filtered, and then concentrated under reduced pressure. The resulting residue was subjected to column chromatography to obtain Compound A-41a in the form of a white gum (48 mg, 74 %); EI-MS m/z: 1379[M+H]⁺.

### Step 2: Preparation of Compound A-41

THF (0.35 mL) and methanol (0.87 mL) and distilled water (0.2 mL) were added to Compound A-41a (48 mg, 0.035 mmol) at -20°C to dissolve the compound, and then lithium hydroxide hydrate (11 mg, 0.261 mmol) dissolved in distilled water (0.63 mL) was slowly added dropwise, and the mixture was stirred for 2.5 hours while slowly raising the temperature from - 20°C to -5°C. The reaction solution was stirred at 0°C for additional 4.5 hours. After completion of the reaction, 2N-hydrochloric acid aqueous solution (1 mL) was added to terminate the reaction, and the reaction solution was diluted with ACN (1 mL) and distilled water (1 mL), then purified using Preparative-HPLC, and freeze-dried to obtain Compound A-41 in the form of a white solid (33.2 mg, 79%); EI-MS m/z: 1210[M+H]⁺.

### Example 1-34: Preparation of Compound A-42

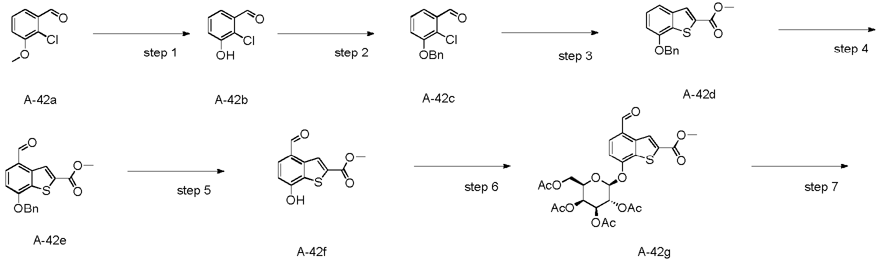

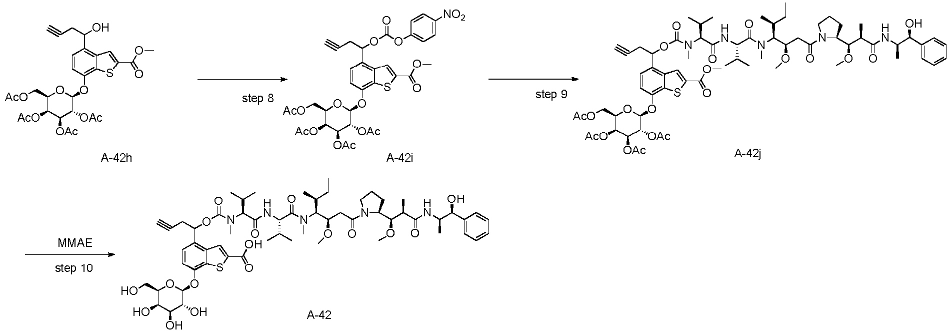

### Step 1: Preparation of Compound A-42b

Compound A-42a (Sigma Aldrich, CAS NO. 54881-49-1, 3.0 g, 17.6 mmol) was dissolved in MC (60.0 mL) under a nitrogen atmosphere at -78°C, and then boron tribromide solution (1M-BBr₃ in MC, 53.0 mL, 53.0 mmol) was slowly added, and the mixture was stirred at room temperature for 16 hours. After completion of the reaction, distilled water (100 mL) was slowly added dropwise to terminate the reaction, and EA (500 mL) and distilled water (500 mL) were added to extract the organic layer. The obtained organic layer was dried over anhydrous magnesium sulfate, filtered, and then concentrated under reduced pressure. The resulting residue was subjected to column chromatography to obtain Compound A-42b in the form of a pink solid (1.8 g, 65 %).

¹H-NMR (400 MHz, CDCl₃) δ 10.39 (s, 1H), 7.52 (dd, *J =* 7.2 Hz, 2.0 Hz, 1H), 7.35 - 7.27 (m, 2H), 5.81 (s, 1H).

### Step 2: Preparation of Compound A-42c

Compound A-42b (1.8 g, 11.5 mmol) was dissolved in ACN (60.0 mL) under a nitrogen atmosphere at 0°C, and then potassium carbonate (3.98 g, 28.7 mmol) and benzyl bromide (2.05 mL, 1.72 mmol) were slowly added, and the mixture was stirred at 0°C for 10 minutes. The reaction temperature was raised to room temperature, and the mixture was stirred for additional 16 hours. After completion of the reaction, EA (200 mL) and distilled water (200 mL) were added to extract the organic layer. The obtained organic layer was dried over anhydrous magnesium sulfate, filtered, and then concentrated under reduced pressure. The resulting residue was subjected to column chromatography to obtain Compound A-42c in the form of a white solid (2.0 g, 71 %).

¹H-NMR (400 MHz, CDCl₃) δ 10.54 (s, 1H), 7.54 (dd, *J =* 7.6 Hz, 1.2 Hz, 1H), 7.48 - 7.27 (m, 5H), 7.19 (dd, *J =* 7.6 Hz, *J =* 1.2 Hz, 1H), 5.21 (s, 2H).

### Step 3: Preparation of Compound A-42d

Compound A-42c (650.0 mg, 2.63 mmol) was dissolved in DMF (25.0 mL) under a nitrogen atmosphere, and then methyl thioglycolate (Merck, CAS NO. 2365-48-2, 0.47 mL, 5.27 mmol) and potassium carbonate (728.0 mg, 5.27 mmol) were added, and the mixture was stirred at room temperature for 30 minutes. The reaction temperature was raised to 80°C, and the mixture was stirred for additional 3 hours. After completion of the reaction, EA (250 mL) and distilled water (250 mL) were added to extract the organic layer. The obtained organic layer was washed by adding a saturated aqueous sodium chloride solution (250 mL). The organic layer was dried over anhydrous magnesium sulfate, filtered, and then concentrated under reduced pressure to obtain Compound A-42d in the form of a yellow solid, which was used in the next reaction without an additional purification process (230.0 mg, 29 %).

### Step 4: Preparation of Compound A-42e

Compound A-42d (400.0 mg, 1.34 mmol) was dissolved in MC (30.0 mL) under a nitrogen atmosphere at -78°C, and then dichloromethyl methyl ether (0.71 mL, 8.04 mmol) and titanium tetrachloride solution (1M-TiCl₄ in MC, 8.04 mL, 8.04 mmol) were sequentially and slowly added, and the mixture was stirred for 2 hours while maintaining the temperature. After completion of the reaction, cooled distilled water (100 mL) was slowly added dropwise to terminate the reaction, and EA (150 mL) was added to extract the organic layer. The obtained organic layer was dried over anhydrous magnesium sulfate, filtered, and then concentrated under reduced pressure. The resulting residue was subjected to column chromatography to obtain Compound A-42e in the form of a white solid (125.0 mg, 28 %).

¹H-NMR (400 MHz, CDCl₃) δ 10.08 (s, 1H), 9.01 (s, 1H), 7.84 (d, *J =* 8.0 Hz, 1H), 7.53 - 7.35 (m, 5H), 7.04 (d, *J =* 8.0 Hz, 1H), 5.38 (s, 2H), 3.97 (s, 3H).

### Step 5: Preparation of Compound A-42f

Compound A-42e (400.0 mg, 1.34 mmol) was dissolved in MC (10.0 mL) under a nitrogen atmosphere at -78°C, and then boron trichloride solution (1M-BCl₃ in MC, 1.14 mL, 1.14 mmol) was slowly added, and the mixture was stirred for 1 hour while maintaining the temperature. After completion of the reaction, distilled water (50.0 mL) was slowly added dropwise at -50°C to terminate the reaction, and EA (100 mL) and distilled water (50 mL) were further added at room temperature to extract the organic layer. The obtained organic layer was dried over anhydrous magnesium sulfate, filtered, and then concentrated under reduced pressure. The resulting residue was subjected to column chromatography to obtain Compound A-42f in the form of a white solid (40.0 mg, 44 %).

¹H-NMR (400 MHz, CDCl₃) δ 10.09 (s, 1H), 9.03 (s, 1H), 7.81 (d, *J =* 8.0 Hz, 1H), 6.96 (d, *J= 8.0* Hz, 1H), 5.93 (s, 1H), 3.98 (s, 3H).

### Step 6: Preparation of Compound A-42g

Compound A-42f (40.0 mg 0.169 mmol) was dissolved in ACN (10.0 mL) under a nitrogen atmosphere at 0°C, and then acetobromo-alpha-D-galactose (Merck, 83.0 mg, 0.203 mmol) and silver oxide (I) (98.0 mg, 0.423 mmol) were sequentially added, and the mixture was stirred at room temperature for 2 hours. After completion of the reaction, the reaction solution was diluted with EA (50 mL), filtered using Celite, and washed by adding a saturated aqueous sodium chloride solution (50 mL) to the solution. The reaction solution was dried over anhydrous magnesium sulfate, filtered, and then concentrated under reduced pressure. The resulting residue was subjected to column chromatography to obtain Compound A-42g in the form of a white solid (60.0 mg, 62 %).

¹H-NMR (400 MHz, CDCl₃) δ 10.14 (s, 1H), 9.00 (s, 1H), 7.86 (d, *J =* 8.0 Hz, 1H), 7.21 (d, *J =* 8.0 Hz, 1H), 5.66 (dd, *J =* 10.4 Hz, *J =* 8.0 Hz, 1H), 5.51 (d, 3.2 Hz, 1H), 5.29 (d, 8.0 Hz, 1H), 5.17 (dd, *J =* 10.4 Hz, *J =* 3.2 Hz, 1H), 4.29 - 4.19 (m, 3H), 2.22 (s, 3H), 2.108 (s, 3H), 2.100 (s, 3H), 2.04 (s, 3H).

### Step 7: Preparation of Compound A-42h

Compound A-42g (59 mg, 0.104 mmol) was dissolved in THF (3.0 mL) under a nitrogen atmosphere at room temperature, and then propargyl bromide (14.0 µL, 0.156 mmol), 1,2-diiodoethane (29.3 mg, 0.104 mmol) and Zinc (Zn powder, 34.0 mg, 0.52 mmol) were sequentially added, followed by ultrasonication for 2 hours. After completion of the reaction, the reaction solution was diluted with EA (100 mL) and filtered using Celite. The obtained filtrate was concentrated under reduced pressure. The resulting residue was subjected to column chromatography to obtain Compound A-42h in the form of a white solid (55.0 mg, 87 %).

¹H-NMR (400 MHz, CDCl₃) δ 8.30 (d, *J =* 9.6 Hz, 1H), 7.44 (t, *J =* 9.6 Hz, 1H), 7.11 (dd, *J =* 8.0 Hz, *J =* 4.0 Hz, 1H), 5.61 (dd, *J =* 10.4 Hz, *J =* 8.0 Hz, 1H), 5.49 (d, *J =* 3.2 Hz, 1H), 5.34 - 5.29 (m, 1H), 5.16 - 5.11 (m, 2H), 4.29 - 4.25 (m, 1H), 4.21 - 4.17 (m, 1H), 4.14 - 4.09 (m, 1H), 3.95 (s, 3H), 2.77 - 2.75 (m, 2H), 2.51 (t, *J =* 4.0 Hz, 1H), 2.21 (s, 3H), 2.13 (s, 3H), 2.08 (s, 3H), 2.03 (s, 3H).

### Step 8: Preparation of Compound A-42i

Compound A-42h (55.0 mg, 0.090 mmol) was dissolved in MC (3.0 mL) under a nitrogen atmosphere at 0°C, and then 4-nitrophenyl chloroformate (27.5 mg, 0.136 mmol) and DIPEA (15.7 µL, 0.090 mmol) and pyridine (22.0 µL, 0.27 mmol) were sequentially added, and the mixture was stirred at room temperature for 16 hours. After completion of the reaction, EA (50 mL) and distilled water (50 mL) were added to extract the organic layer. The obtained organic layer was dried over anhydrous magnesium sulfate, filtered, and then concentrated under reduced pressure. The resulting residue was subjected to column chromatography to obtain Compound A-42i in the form of a white solid (32.0 mg, 45 %).

¹H-NMR (400 MHz, CDCl₃) δ 8.29 (s, 1H), 8.25 (d, *J* = 9.2 Hz, 2H), 7.47 (d, *J =* 8.0 Hz, 1H), 7.34 (d, *J =* 9.2 Hz, 2H), 7.13 (d, *J =* 8.0 Hz, 1H), 5.63 (dd, *J =* 10.4 Hz, *J =* 8.0 Hz, 1H), 5.50 (d, *J =* 3.2 Hz, 1H), 5.20 - 5.12 (m, 2H), 4.28 - 4.25 (m, 1H), 4.21 - 4.16 (m, 1H), 4.14 - 4.11 (m, 1H), 3.96 (s, 3H), 3.10 - 3.04 (m, 1H), 2.96 - 2.91 (m, 1H), 2.21 (s, 3H), 2.12 (s, 3H), 2.08 (s, 3H), 2.06 (t, *J =* 4.0 Hz, 1H), 2.04 (s, 3H).

### Step 9: Preparation of Compound A-42j

Compound A-42i (32.0 mg, 0.041 mmol) was dissolved in DMF (1.0 mL) under a nitrogen atmosphere at 0°C, and then MMAE (29.7 mg, 0.041 mmol), HOBt (8.3 mg, 0.061 mmol), DIPEA (18.0 µL, 0.102 mmol) and pyridine (1.0 mL) were sequentially added, and the mixture was stirred at room temperature for 16 hours. After completion of the reaction, extraction was performed using distilled water (10 mL), 2N-hydrochloric acid aqueous solution (5 mL) and EA (15 mL). The obtained organic layer was dried over anhydrous magnesium sulfate, filtered, and then concentrated under reduced pressure. The resulting residue was subjected to column chromatography to obtain Compound A-42j in the form of a white solid (31.0 mg, 55 %); EI-MS m/z: 1351 [M+H]⁺.

### Step 10: Preparation of Compound A-42

Compound A-42j (30.0 mg, 0.022 mmol) was dissolved in methanol (1.0 mL) under a nitrogen atmosphere at 0°C, and then lithium hydroxide (7.0 mg, 0.166 mmol) dissolved in distilled water (1.0 mL) was slowly added dropwise, and the mixture was stirred at room temperature for 2 hours. After completion of the reaction, 2N-hydrochloric acid aqueous solution (1.0 mL) was added to terminate the reaction, and the reaction solution was diluted with ACN (1.0 mL) and distilled water (1.0 mL), then purified using Preparative-HPLC, and freeze-dried to obtain Compound A-42 in the form of a white solid (14.8 mg, 68 %); EI-MS m/z: 1169 [M+H]⁺.

### Example I-35: Preparation of Compound A-43

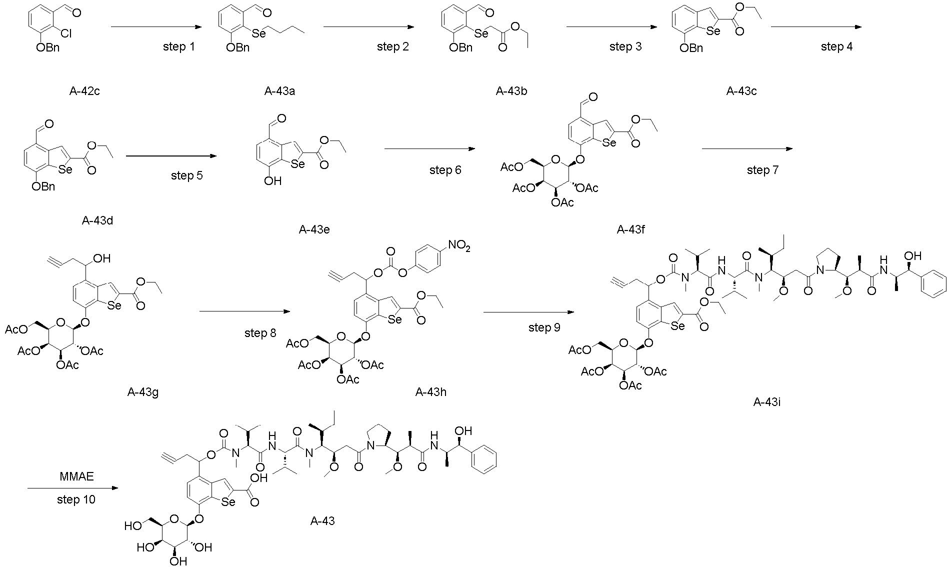

### Step 1: Preparation of Compound A-43a

Selenium powder (640.0 mg, 8.10 mmol) was added to THF (27.0 mL) under a nitrogen atmosphere at room temperature, and the mixture was cooled to 0°C, and then n-butyl lithium solution (2.5M n-BuLi in hexane, 3.56 mL, 8.91 mmol) was slowly added dropwise. The mixture was stirred at 0°C for 40 minutes, and then Compound A-42c (2.0 g, 8.10 mmol) was dissolved in DMF (5.4 mL) and added, and the mixture was stirred at room temperature for 3 hours. After completion of the reaction, distilled water (100 mL) was slowly added dropwise to terminate the reaction. EA (100 mL) was added to the reaction solution, and then the organic layer was extracted. The obtained organic layer was dried over anhydrous magnesium sulfate, filtered, and then concentrated under reduced pressure. The resulting residue was subjected to column chromatography to obtain Compound A-43a in the form of a yellow liquid (2.03 g, 72 %).

¹H-NMR (400 MHz, CDCl₃) δ 10.71 (s, 1H), 7.54 (d, *J =* 8.0 Hz, 1H), 7.50 - 7.48 (m, 2H), 7.42 - 7.32 (m, 4H), 7.14 (d, *J =* 8.0 Hz, 1H), 5.20 (s, 2H), 2.89 (t, *J =* 7.6 Hz, 2H), 1.71 - 1.50 (m, 2H), 1.39 - 1.31 (m, 2H), 0.81 (t, *J =* 7.6 Hz, 3H).

### Step 2: Preparation of Compound A-43b

Compound A-43a (2.0 g, 5.75 mmol) was dissolved in DMF (25.0 mL) under a nitrogen atmosphere, and then ethyl bromoacetate (1.6 mL, 13.4 mmol) and potassium carbonate (1.85 g, 13.4 mmol) were sequentially added, and then the mixture was stirred at 120°C for 16 hours. After completion of the reaction, distilled water (250 mL) and EA (250 mL) were added to extract the organic layer. The obtained organic layer was washed by adding a saturated aqueous sodium chloride solution (250 mL). The organic layer was dried over anhydrous magnesium sulfate, filtered, and then concentrated under reduced pressure. The residue was subjected to column chromatography to obtain Compound A-43b in the form of a white solid (1.5 g, 69 %).

¹H-NMR (400 MHz, CDCl₃) δ 10.63 (s, 1H), 7.56 (d, *J =* 8.0 Hz, 1H), 7.50 - 7.49 (m, 2H), 7.44 - 7.39 (m, 3H), 7.37 - 7.33 (m, 1H), 7.18 (d, *J =* 8.0 Hz, 1H), 5.22 (s, 1H), 3.95 (q, *J=* 7.2 Hz, 2H), 3.53 (s, 2H), 1.05 (t, *J =* 7.2 Hz, 3H).

### Step 3: Preparation of Compound A-43c

Compound A-43b (1.5 g, 3.97 mmol) was dissolved in DMF (20.0 mL) under a nitrogen atmosphere at 0°C, and then potassium carbonate (824.0 mg, 5.96 mmol) was added, and the mixture was stirred at 120°C for 2 hours. After completion of the reaction, EA (200 mL) and distilled water (200 mL) were added to extract the organic layer. The obtained organic layer was washed by adding a saturated aqueous sodium chloride solution (200 mL). The organic layer was dried over anhydrous magnesium sulfate, filtered, and then concentrated under reduced pressure. The resulting residue was subjected to column chromatography to obtain Compound A-43c in the form of a white solid (1.2 g, 84 %).

¹H-NMR (400 MHz, CDCl₃) δ 8.30 (s, 1H), 7.51 (d, *J =* 8.0 Hz, 1H), 7.48 - 7.46 (m, 2H), 7.42 - 7.38 (m, 2H), 7.35 - 7.32 (m, 2H), 6.87 (d, *J =* 8.0 Hz, 1H), 5.27 (s, 2H), 4.38 (q, *J =* 7.2 Hz, 2H), 4.38 (t, *J =* 7.2 Hz, 3H); EI-MS m/z: 360 [M+H]⁺.

### Step 4: Preparation of Compound A-43d

Compound A-43c (1.2 g, 3.34 mmol) was dissolved in MC (30.0 mL) under a nitrogen atmosphere at 0°C, and then dichloromethyl methyl ether (1.19 mL, 13.36 mmol) and titanium tetrachloride solution (1M-TiCl₄ in MC, 13.36 mL, 13.36 mmol) were sequentially and slowly added, and the mixture was stirred for 3 hours while maintaining the temperature. After completion of the reaction, cooled distilled water (150 mL) was slowly added dropwise to terminate the reaction, and EA (150 mL) was added to extract the organic layer. The obtained organic layer was dried over anhydrous magnesium sulfate, filtered, and then concentrated under reduced pressure. The resulting residue was subjected to column chromatography to obtain Compound A-43d in the form of a white solid (1.09 g, 89 %).

¹H-NMR (400 MHz, CDCl₃) δ 10.04 (s, 1H), 9.32 (s, 1H), 7.84 (d, *J =* 8.0 Hz, 1H), 7.48 - 7.35 (m, 5H), 7.00 (d, *J =* 8.0 Hz, 1H), 5.37 (s, 2H), 4.41 (q, *J =* 7.2 Hz, 2H), 1.42 (t, *J =* 7.2 Hz, 3H).

### Step 5: Preparation of Compound A-43e

Compound A-43d (420.0 mg, 1.11 mmol) was dissolved in MC (20.0 mL) under a nitrogen atmosphere at -78°C, and then boron trichloride solution (1M-BCl₃ in MC, 3.33 mL, 3.33 mmol) was slowly added, and the mixture was stirred for 2 hours while maintaining the temperature. After completion of the reaction, distilled water (50 mL) was slowly added dropwise at -50°C to terminate the reaction, and EA (150 mL) and distilled water (100 mL) were further added at room temperature to extract the organic layer. The obtained organic layer was dried over anhydrous magnesium sulfate, filtered, and then concentrated under reduced pressure. The resulting residue was subjected to column chromatography to obtain Compound A-43e in the form of a white solid (300.0 mg, 90 %).

¹H-NMR (400 MHz, CDCl₃) δ 10.05 (s, 1H), 9.33 (s, 1H), 7.81 (d, *J =* 8.0 Hz, 1H), 6.92 (d, *J =* 8.0 Hz, 1H), 5.98 (s, 1H), 4.41 (q, *J =* 7.2 Hz, 2H), 1.43 (t, *J =* 7.2 Hz, 3H).

### Step 6: Preparation of Compound A-43f

Compound A-43e (150.0 mg 0.50 mmol) was dissolved in ACN (30.0 mL) under a nitrogen atmosphere at 0°C, and then acetobromo-alpha-D-galactose (Merck, 250.0 mg, 0.60 mmol) and silver oxide (I) (290.0 mg, 1.25 mmol) were sequentially added, and the mixture was stirred at room temperature for 2 hours. After completion of the reaction, the reaction solution was diluted with EA (50 mL), filtered using Celite, and washed by adding a saturated aqueous sodium chloride solution (80 mL) to the solution. The reaction solution was dried over anhydrous magnesium sulfate, filtered, and then concentrated under reduced pressure. The resulting residue was subjected to column chromatography to obtain Compound A-43f in the form of a white solid (290.0 mg, 91 %).

¹H-NMR (400 MHz, CDCl₃) δ 10.11 (s, 1H), 9.30 (s, 1H), 7.87 (d, *J =* 8.0 Hz, 1H), 7.15 (d, *J= 8.0* Hz, 1H), 5.63 (dd, *J =* 10.4 Hz, 8.0 Hz, 1H), 7.52 (d, *J =* 3.2 Hz, 1H), 5.28 (d, *J =* 8.0 Hz, 1H), 5.17 (dd, *J =* 10.4 Hz, 3.2 Hz, 1H), 4.42 (d, *J* = 7.2 Hz, 1H), 4.31 - 4.26 (m, 1H), 4.22 - 4.14 (m, 2H), 2.22 (s, 3H), 2.10 (s, 6H), 2.04 (s, 3H), 1.43 (t, *J =* 7.2 Hz, 3H).

### Step 7: Preparation of Compound A-43g

Compound A-43f (52.0 mg, 0.082 mmol) was dissolved in THF (3.0 mL) under a nitrogen atmosphere at room temperature, and then propargyl bromide (11.1 µL, 0.124 mmol), 1,2-diiodoethane (23.0 mg, 0.082 mmol) and Zinc (Zn powder, 27.0 mg, 0.414 mmol) were sequentially added, followed by ultrasonication for 3 hours. After completion of the reaction, the reaction solution was diluted with EA (100 mL) and filtered using Celite. The obtained filtrate was concentrated under reduced pressure. The resulting residue was subjected to column chromatography to obtain Compound A-43g in the form of a white solid (24.0 mg, 44 %).

¹H-NMR (400 MHz, CDCl₃) δ 8.55 (d, *J =* 9.2 Hz, 1H), 7.51 - 7.46 (m, 1H), 7.07 - 7.03 (m, 1H), 5.60 (dd, *J =* 10.4 Hz, 8.0 Hz, 1H), 5.49 (d, *J =* 3.2 Hz, 1H), 5.34 - 5.29 (m, 1H), 5.17 - 5.12 (m, 2H), 4.40 (q, *J =* 7.2 Hz, 2H), 4.29 - 4.25 (m, 1H), 4.21 - 4.17 (m, 1H), 4.14 - 4.09 (m, 1H), 2.77 - 2.75 (m, 2H), 2.50 - 2.48 (m, 1H), 2.21 (s, 3H), 2.12 (s, 3H), 2.09 (s, 3H), 2.03 (s, 3H), 1.42 (t, *J =* 7.2 Hz, 3H); EI-MS m/z: 690[M+Na]⁺.

### Step 8: Preparation of Compound A-43h

Compound A-43g (22.0 mg, 0.033 mmol) was dissolved in MC (3.0 mL) under a nitrogen atmosphere at 0°C, and then 4-nitrophenyl chloroformate (10.0 mg, 0.049 mmol) and DIPEA (15.0 µL, 0.085 mmol) and pyridine (8.0 µL, 0.099 mmol) were sequentially added, and the mixture was stirred at room temperature for 2 hours. After completion of the reaction, EA (50 mL) and distilled water (50 mL) were added to extract the organic layer. The obtained organic layer was dried over anhydrous magnesium sulfate, filtered, and then concentrated under reduced pressure. The resulting residue was subjected to column chromatography to obtain Compound A-43h in the form of a white solid (10.0 mg, 37 %).

¹H-NMR (400 MHz, CDCl₃) δ 8.54 (s, 1H), 8.25 (d, *J=* 9.2 Hz, 2H), 7.51 - 7.49 (m, 1H), 7.35 (d, *J* = 9.2 Hz, 2H), 7.08 - 7.06 (m, 1H), 6.25 (t, *J =* 7.2 Hz, 2H), 5.60 (dd, *J =* 10.4 Hz, *J =* 8.0 Hz, 1H), 5.50 (d, *J =* 3.2 Hz, 1H), 5.20 - 5.12 (m, 2H), 4.41 (t, *J =* 7.2 Hz, 2H), 4.30 - 4.25 (m, 1H), 4.21 - 4.09 (m, 2H), 3.08 - 3.02 (m, 1H), 2.96 - 2.90 (m, 1H), 2.21 (s, 3H), 2.11 (s, 3H), 2.09 (s, 3H), 2.07 (t, *J =* 4.0 Hz, 1H), 2.04 (s, 3H) 1.42 (t, *J =* 7.2 Hz, 3H); EI-MS m/z: 855[M+Na]⁺.

### Step 9: Preparation of Compound A-43i

Compound A-43h (10.0 mg, 0.012 mmol) was dissolved in DMF (1.0 mL) under a nitrogen atmosphere at 0°C, and then MMAE (8.6 mg, 0.012 mmol), HOBt (2.43 mg, 0.018 mmol), DIPEA (5.23 µL, 0.03 mmol) and pyridine (1.0 mL) were sequentially added, and the mixture was stirred at room temperature for 16 hours. After completion of the reaction, extraction was performed using distilled water (20 mL), 2N-hydrochloric acid aqueous solution (5 mL) and EA (25 mL). The obtained organic layer was dried over anhydrous magnesium sulfate, filtered, and then concentrated under reduced pressure. The resulting residue was subjected to column chromatography to obtain Compound A-43i in the form of a white solid (10.0 mg, 58 %); EI-MS m/z: 1412 [M+H]⁺.

### Step 10: Preparation of Compound A-43

Compound A-43i (10.0 mg, 0.007 mmol) was dissolved in methanol (1.0 mL) under a nitrogen atmosphere at 0°C, and then lithium hydroxide (2.23 mg, 0.053 mmol) dissolved in distilled water (1.0 mL) was slowly added dropwise, and the mixture was stirred at room temperature for 5 hours. After completion of the reaction, 2N-hydrochloric acid aqueous solution (1.0 mL) was added to terminate the reaction, and the reaction solution was diluted with ACN (1.0 mL) and distilled water (1.0 mL), then purified using Preparative-HPLC, and freeze-dried to obtain Compound A-43 in the form of a white solid (5.2 mg, 60 %); EI-MS m/z: 1216 [M+H]⁺.

### Example 1-36: Preparation of Compound A-44

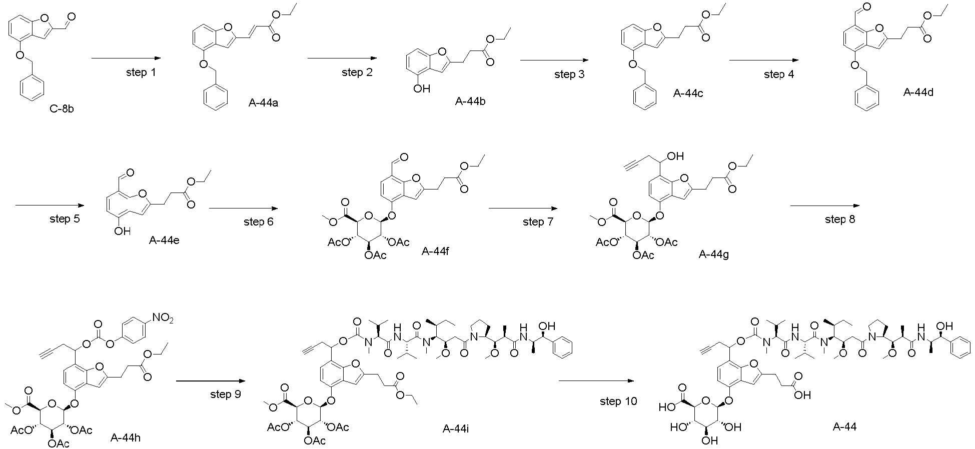

### Step 1: Preparation of Compound A-44a

Compound C-8b (8.5 g, 33.69 mmol) was dissolved in MC (168 mL) under a nitrogen atmosphere at room temperature, and then (carbethoxymethylene)triphenylphosphorane (combi-blocks, CAS No. 1099-45-2, 23.5 g, 67.46 mmol) was added, and the mixture was stirred at room temperature for 3 hours. After completion of the reaction, the reaction solution was concentrated under reduced pressure. The resulting residue was subjected to column chromatography to obtain Compound A-44a in the form of a white solid (9.96 g, 92 %).

¹H-NMR (400 MHz, CDCl₃) δ 7.53 - 7.33 (m, 6H), 7.28 - 7.24 (m, 1H), 7.11 - 7.07 (m, 2H), 6.71 (d, *J =* 8 Hz, 1H), 6.53 (d, *J =* 15.6 Hz, 1H), 5.20 (s, 2H), 4.27 (q, *J =* 7.2 Hz, 2H), 1.34 (t, *J=* 7.2 Hz, 3H); EI-MS m/z: 323[M+H]⁺.

### Step 2: Preparation of Compound A-44b

Compound A-44a (9.96 g, 30.9 mmol) was dissolved in THF (250 mL) and methanol (750 mL) under a nitrogen atmosphere at room temperature, and then 5% palladium charcoal (5% Pd/C, 5 g) was added, and the mixture was stirred under a hydrogen environment for 2 hours. After completion of the reaction, the reaction solution was filtered using Celite. The filtered solution was concentrated under reduced pressure. The resulting residue was subjected to column chromatography to obtain Compound A-44b in the form of a white solid (7 g, 97 %).

¹H-NMR (400 MHz, CDCl₃) δ 7.10 - 7.01 (m, 2H), 6.59 (d, *J =* 7.6 Hz, 1H), 6.49 (s, 1H), 5.05 (s, 1H), 4.16 (q, *J =* 7.2 Hz, 2H), 3.10 (t, *J =* 7.6 Hz, 2H), 2.75 (t, *J =* 7.6 Hz, 2H), 1.26 (t, *J* = 7.2 Hz, 3H); EI-MS m/z: 235[M+H]⁺.

### Step 3: Preparation of Compound A-44c

Compound A-44b (7 g, 29.88 mmol) was dissolved in ACN (230 mL) under a nitrogen atmosphere at room temperature, and then potassium carbonate (10.3 g, 74.7 mmol) and benzyl bromide (4.6 mL, 38.85 mmol) were added, and the mixture was stirred at 80°C for 2.5 hours. After completion of the reaction, EA (1 L), distilled water (500 mL) and 2N-hydrochloric acid aqueous solution (200 mL) were added to extract the organic layer. The obtained organic layer was dried over anhydrous magnesium sulfate, filtered, and then concentrated under reduced pressure. The residue was subjected to column chromatography to obtain Compound A-44c in the form of a colorless oil (9.63 g, 99 %).

¹H-NMR (400 MHz, CDCl₃) δ 7.49 - 7.44 (m, 2H), 7.42 - 7.36 (m, 2H), 7.36 - 7.30 (m, 1H), 7.12 (t, *J =* 8 Hz, 1H), 7.05 (d, *J =* 8 Hz, 1H), 6.68 (d, *J =* 8 Hz, 1H), 6.57 (s, 1H), 5.18 (s, 2H), 4.16 (q, *J =* 7.2 Hz, 2H), 3.10 (t, *J =* 7.6 Hz, 2H), 2.74 (t, *J =* 7.6 Hz, 2H), 1.25 (t, *J =* 7.2 Hz, 3H); EI-MS m/z: 325[M+H]⁺.

### Step 4: Preparation of Compound A-44d

Compound A-44c (9.63 g, 29.69 mmol) was dissolved in MC (600 mL) under a nitrogen atmosphere at -78°C, and then dichloromethyl methyl ether (8 mL, 89.06 mmol) and titanium tetrachloride solution (1M-TiCl₄ in MC, 89 mL, 89.06 mmol) were sequentially and slowly added, and the mixture was stirred for 2 hours while maintaining the temperature. After completion of the reaction, distilled water (800 mL) was slowly added dropwise to terminate the reaction, and MC (400 mL) was added to extract the organic layer twice. The obtained organic layer was dried over anhydrous magnesium sulfate, filtered, and then concentrated under reduced pressure. The resulting residue was subjected to column chromatography to obtain Compound A-44d in the form of a yellow solid (5.1 g, 49 %).

¹H-NMR (400 MHz, CDCl₃) δ 10.25 (s, 1H), 7.68 (d, *J =* 8.4 Hz, 1H), 7.48 - 7.33 (m, 5H), 6.80 (d, *J =* 8.4 Hz, 1H), 6.63 (s, 1H), 5.26 (s, 2H), 4.17 (q, *J =* 7.2 Hz, 2H), 3.18 (t, *J =* 7.6 Hz, 2H), 2.78 (t, *J =* 7.6 Hz, 2H), 1.25 (t, *J =* 7.2 Hz, 3H).

### Step 5: Preparation of Compound A-44e

Compound A-44d (1.5 g, 4.26 mmol) was dissolved in MC (85 mL) under a nitrogen atmosphere at -78°C, and then pentamethylbenzene (Alfa aesar CAS NO. 700-12-9, 1.89 g, 12.74 mmol) and boron trichloride solution (1M-BCl₃ in MC, 12.8 mL, 12.8 mmol) was slowly added, and then the mixture was stirred for 2 hours at -78°C. After completion of the reaction, distilled water (160 mL) was slowly added dropwise to terminate the reaction, and 2N-sodium hydroxide aqueous solution was added at 0°C to neutralize the reaction solution. EA (200 mL) and distilled water (100 mL) were added to the mixture to extract the organic layer. The obtained organic layer was washed by adding a saturated aqueous sodium chloride solution (100 mL), then dried over anhydrous magnesium sulfate, filtered, and then concentrated under reduced pressure. The resulting residue was subjected to column chromatography to obtain Compound A-44e in the form of a beige solid (954 g, 85 %).

¹H-NMR (400 MHz, DMSO-d₆) δ 11.13 (brs, 1H), 10.05 (s, 1H), 7.61 (d, *J =* 8.4 Hz, 1H), 6.75 - 6.71 (m, 2H), 4.09 (q, *J =* 7.2 Hz, 2H), 3.07 (t, *J =* 7.2 Hz, 2H), 2.76 (t, *J =* 7.2 Hz, 2H), 1.18 (t, *J =* 7.2 Hz, 3H); MS m/z: 263[M+H]⁺.

### Step 6: Preparation of Compound A-44f

Compound A-44e (433 mg, 1.65 mmol) was dissolved in ACN (24 mL) and THF (6 mL) under a nitrogen atmosphere at 0°C, and then acetobromo-α-D-glucuronic acid methyl ester (TCI, 787 mg, 1.98 mmol) was added, and the mixture was stirred for 20 minutes while maintaining the temperature. Additionally, silver oxide (I) (957 mg, 4.13 mmol) and molecular sieve (200 mg) were added and stirred for 10 minutes, and then the mixture was stirred at room temperature for 2 hours. After completion of the reaction, the reaction resolution was diluted with EA (200 mL) and filtered using a Celite filter, and then the solution was concentrated under reduced pressure. The resulting residue was subjected to column chromatography to obtain Compound A-44f in the form of a brown solid (90 mg, 9 %).

¹H-NMR (400 MHz, CDCl₃) δ 10.31 (s, 1H), 7.71 (d, *J =* 8.4 Hz, 1H), 6.88 (d, *J=* 8.4 Hz, 1H), 6.51 (s, 1H), 5.40 - 5.35 (m, 4H), 4.28 - 4.26 (m, 1H), 4.18 (q, *J =* 7.2 Hz, 2H), 3.72 (s, 1H), 3.17 (t, *J =* 7.6 Hz, 2H), 2.78 (t, *J =* 7.6 Hz, 2H), 2.08 - 2.05 (m, 9H), 1.29 - 1.24 (m, 3H); MS m/z: 579[M+H]⁺.

### Step 7: Preparation of Compound A-44g

Compound A-44f (88 mg, 0.15 mmol) was dissolved in THF (5 mL) under a nitrogen atmosphere at room temperature, and then propargyl bromide (25.4 µL, 0.29 mmol), 1,2-diiodoethane (43 mg, 0.15 mmol) and Zinc (Zn powder, 50 mg, 0.76 mmol) were sequentially added, followed by ultrasonication for 1.5 hours. After completion of the reaction, the reaction solution was diluted with EA (100 mL) and filtered using Celite. Distilled water (100 mL) was added to the filtered solution to extract the organic layer. The obtained organic layer was washed by adding a saturated aqueous sodium chloride solution (100 mL), then dried over anhydrous magnesium sulfate, filtered, and then concentrated under reduced pressure. The resulting residue was subjected to column chromatography to obtain Compound A-44g in the form of a beige solid (70 mg, 74 %).

¹H-NMR (400 MHz, CDCl₃) δ 7.24 - 7.23 (m, 1H), 6.80 (d, *J =* 8.4 Hz, 1H), 6.46 (s, 1H), 5.36 - 5.35 (m, 3H), 5.28 - 5.25 (m, 1H), 5.19 - 5.18 (m, 1H), 4.20 - 4.15 (m, 3H), 3.74 (s, 3H), 3.11 (t, *J =* 7.6 Hz, 2H), 2.88 - 2.77 (m, 2H), 2.73 (t, *J =* 7.6 Hz, 2H), 2.52 - 2.49 (m, 1H), 2.06 (s, 6H), 2.05 (s, 3H), 1.29 - 1.25 (m, 3H); MS m/z: 640[M+Na]⁺.

### Step 8: Preparation of Compound A-44h

Compound A-44g (67 mg, 0.11 mmol) was dissolved in MC (2 mL) under a nitrogen atmosphere at 0°C, and then 4-nitrophenyl chloroformate (44 mg, 0.22 mmol), pyridine (26.2 µL, 0.33 mmol) and DIPEA (28.3 µL, 0.16 mmol) were sequentially added, and the mixture was stirred at 0°C for 30 minutes. After completion of the reaction, the reaction solution was diluted with EA (15 mL) and 2N-hydrochloric acid aqueous solution (15 mL) was added to extract the organic layer. The obtained organic layer was washed by adding a saturated aqueous sodium chloride solution (15 mL), then dried over anhydrous magnesium sulfate, filtered, and then concentrated under reduced pressure. The residue thus obtained was subjected to column chromatography to obtain Compound A-44h in the form of a white solid (74.4 mg, 88 %).

¹H-NMR (400 MHz, CDCl₃) δ 8.26 (d, *J =* 8.8 Hz, 2H), 7.37 (d, *J =* 8 Hz, 2H), 7.27 (m, 1H), 6.81 (d, *J =* 8.4 Hz, 1H), 6.49 (s, 1H), 6.25 - 6.22 (m, 1H), 5.37 - 5.36 (m, 3H), 5.24 - 5.23 (m, 1H), 4.21 - 4.15 (m, 3H), 3.74 (s, 3H), 3.15 - 3.03 (m, 4H), 2.75 (t, *J =* 7.6 Hz, 2H), 2.07 - 2.05 (m, 10H), 1.29 - 1.25 (m, 3H); MS m/z: 805[M+Na]⁺.

### Step 9: Preparation of Compound A-44i

Compound A-44h (40 mg, 0.051 mmol) was dissolved in DMF (1 mL) under a nitrogen atmosphere at 0°C, and then MMAE (37 mg, 0.052 mmol), HOBt (10 mg, 0.074 mmol), DIPEA (22.2 µL, 0.128 mmol) and pyridine (1 mL) were sequentially added, and the mixture was stirred at room temperature for 16 hours. After completion of the reaction, the organic layer was extracted twice with EA (20 mL) and 2N-hydrochloric acid aqueous solution (20 mL). The obtained organic layer was washed by adding a saturated aqueous sodium chloride solution (20 mL), then dried over anhydrous magnesium sulfate, filtered, and then concentrated under reduced pressure. The resulting residue was subjected to column chromatography to obtain Compound A-44i in the form of a white solid (36 mg, 52 %); EI-MS m/z: 1363[M+H]⁺.

### Step 10: Preparation of Compound A-44

Compound A-44i (36 mg, 0.026 mmol) was dissolved in a mixture of methanol (0.8 mL), distilled water (0.2 mL) and THF (0.15 mL) at -20°C, and then lithium hydroxide hydrate (11.1 mg, 0.264 mmol) dissolved in distilled water (0.8 mL) was added dropwise, and the mixture was stirred at -20°C for 1.5 hours and at room temperature for 5.5 hours. After completion of the reaction, 2N-hydrochloric acid aqueous solution (1 mL) was added to terminate the reaction, and the reaction solution was diluted with ACN (1 mL) and distilled water (1 mL), then purified using Preparative-HPLC, and freeze-dried to obtain Compound A-44 in the form of a white solid (17.8 mg, 56 %); EI-MS m/z: 1195 [M+H]⁺.

### Example I-37: Preparation of Compound A-45

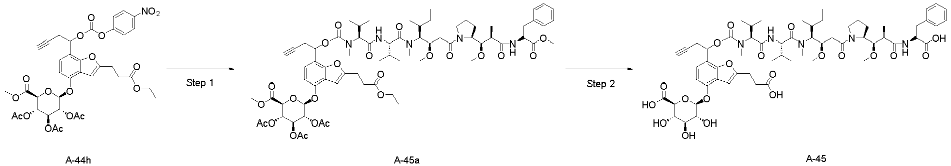

### Step 1: Preparation of Compound A-45a

Compound A-44h (45 mg, 0.057 mmol) was dissolved in DMF (1 mL) under a nitrogen atmosphere at 0°C, and then MMAF-OMe (CAS NO. 863971-12-4, 43 mg, 0.058 mmol), HOBt (12 mg, 0.089 mmol), DIPEA (25 µL, 0.144 mmol) and pyridine (1 mL) were sequentially added, and the mixture was stirred at room temperature for 16 hours. After completion of the reaction, the organic layer was extracted twice with EA (20 mL) and 2N-hydrochloric acid aqueous solution (20 mL). The obtained organic layer was washed by adding a saturated aqueous sodium chloride solution (20 mL), then dried over anhydrous magnesium sulfate, filtered, and then concentrated under reduced pressure. The resulting residue was subjected to column chromatography to obtain A-45a in the form of a white solid (47.7 mg, 60 %); EI-MS m/z: 1391[M+H]⁺.

### Step 2: Preparation of Compound A-45

Compound A-45a (47 mg, 0.034 mmol) was dissolved in a mixture of methanol (0.9 mL), distilled water (0.1 mL) and THF (0.17 mL) at -20°C, and then lithium hydroxide (14.2 mg, 0.338 mmol) dissolved in distilled water (0.8 mL) was added dropwise, and the mixture was stirred at - 20°C for 2 hours, and then for 5.5 hours while slowly raising the temperature to 0°C. After completion of the reaction, 2N-hydrochloric acid aqueous solution (1 mL) was added to terminate the reaction, and the reaction solution was diluted with ACN (1 mL) and distilled water (1 mL), then purified using Preparative-HPLC, and freeze-dried to obtain Compound A-45 in the form of a white solid (25.4 mg, 62 %); EI-MS m/z: 1209 [M+H]⁺.

### Example 1-38: Preparation of Compounds A-46, A-47 and A-48

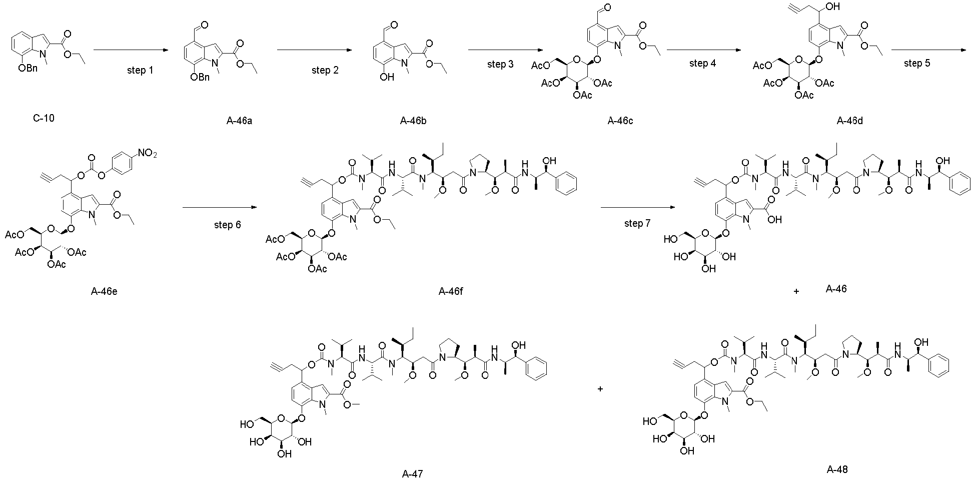

### Step 1: Preparation of Compound A-46a

Core C-10 (2.0 g, 6.46 mmol) was dissolved in MC (100.0 mL) under a nitrogen atmosphere at -78°C, and then dichloromethyl methyl ether (1.73 mL,19.4 mmol) and titanium tetrachloride solution (1M-TiCl₄ in MC, 19.4 mL, 19.4 mmol) were sequentially and slowly added, and the mixture was stirred for 2 hours while maintaining the temperature. After completion of the reaction, cooled distilled water (100 mL) was slowly added dropwise to terminate the reaction, and EA (300 mL) was added to extract the organic layer. The obtained organic layer was dried over anhydrous magnesium sulfate, filtered, and then concentrated under reduced pressure. The resulting residue was subjected to column chromatography to obtain Compound A-46a in the form of a white solid (1.32 g, 60 %).

¹H-NMR (400 MHz, CDCl₃) δ 10.04 (s, 1H), 8.04 (s, 1H), 7.56 (d, *J =* 8.0 Hz, 1H), 7.48 - 7.36 (m, 5H), 6.87 (d, *J =* 8.0 Hz, 1H), 5.29 (s, 2H), 4.39 - 4.34 (m, 5H), 1.41 (t, *J =* 7.2 Hz, 3H).

### Step 2: Preparation of Compound A-46b

Compound A-46a (1.32g, 3.91 mmol) was dissolved in MC (80.0 mL) under a nitrogen atmosphere at -78°C, and then boron trichloride solution (1M-BCl₃ in MC, 11.7 mL, 11.7 mmol) was slowly added, and then the mixture was stirred for 3 hours while maintaining the temperature. After completion of the reaction, distilled water (100.0 mL) was slowly added dropwise at -50°C to terminate the reaction, and EA (300 mL) and distilled water (200 mL) were further added at room temperature to extract the organic layer. The obtained organic layer was dried over anhydrous magnesium sulfate, filtered, and then concentrated under reduced pressure. The resulting residue was subjected to column chromatography to obtain Compound A-46b in the form of an ivory solid (550.0 g, 56 %).

¹H-NMR (400 MHz, CDCl₃) δ 10.03 (s, 1H), 8.03 (s, 1H), 7.49 (d, *J =* 8.0 Hz, 1H), 6.68 (d, *J= 8.0* Hz, 1H), 5.62 (s, 1H), 4.43 (s, 1H), 4.38 (q, *J =* 7.2 Hz, 2H), 1.42 (t, *J =* 7.2 Hz, 3H).

### Step 3: Preparation of Compound A-46c

Compound A-46b (550.0 mg, 2.23 mmol) was dissolved in MC (50.0 mL) under a nitrogen atmosphere at 0°C, and then acetobromo-alpha-D-galactose (Merck, 1.0 g, 2.45 mmol) and benzyltributylammonium chloride (694.0 mg, 2.23 mmol) were added. 5N-sodium hydroxide aqueous solution (1.33 mL, 6.69 mmol) was slowly added to the reaction solution, and the mixture was stirred at room temperature for 6 hours. After completion of the reaction, MC (150 mL) and distilled water (150 mL) were added to extract the organic layer. The obtained organic layer was dried over anhydrous magnesium sulfate, filtered, and then concentrated under reduced pressure. The resulting residue was subjected to column chromatography to obtain Compound A-46c in the form of an ivory solid (910.0 mg, 70 %).

¹H-NMR (400 MHz, CDCl₃) δ 10.10 (s, 1H), 8.03 (s, 1H), 7.55 (d, *J =* 8.0 Hz, 1H), 6.96 (d, *J=* 8.0 Hz, 1H), 5.66 (dd, *J =* 10.4 Hz, *J =* 8.0 Hz, 1H), 5.51 (d, *J =* 3.2 Hz, 1H), 5.39 (d *J =* 9.2 Hz, 1H) 5.18 (dd, *J =* 10.4 Hz, *J =* 3.2 Hz, 1H), 4.38 (t, *J =* 7.2 Hz, 2H), 4.30 (s, 3H), 4.27 - 4.20 (m, 1H), 4.19 - 4.16 (m, 2H), 2.20 (s, 3H), 2.08 (s, 3H), 2.07 (s, 3H), 2.03 (s, 3H), 1.42 (t, *J* = 7.2 Hz, 3H).

### Step 4: Preparation of Compound A-46d

Compound A-46c (900.0 mg, 1.56 mmol) was dissolved in THF (40.0 mL) under a nitrogen atmosphere at room temperature, and then propargyl bromide (0.2 mL, 2.33 mmol), 1,2-diiodoethane (440.0 mg, 1.56 mmol) and Zinc (Zn powder, 508.0 mg, 7.79 mmol) were sequentially added, followed by ultrasonication for 6 hours, and the mixture was stirred at room temperature for 16 hours. After completion of the reaction, the reaction solution was diluted with EA (100 mL) and filtered using Celite. The obtained filtrate was concentrated under reduced pressure. The resulting residue was subjected to column chromatography to obtain Compound A-46d in the form of an ivory solid (825.0 mg, 85 %).

¹H-NMR (400 MHz, CDCl₃) δ 7.38 (d, *J =* 9.2 Hz, 1H), 7.08 (t, *J =* 9.2 Hz, 1H), 6.88 - 6.85 (m, 1H), 5.62 (dd, *J =* 10.4 Hz, *J =* 8.0 Hz, 1H), 5.48 (d, *J =* 3.2 Hz, 1H), 5.26 - 5.20 (m, 2H), 5.15 (dd, *J =* 10.4 Hz, *J =* 3.2 Hz, 2H), 4.36 (q, *J =* 7.2 Hz, 2H), 4.26 (s, 3H) 4.24 - 4.09 (m, 3H), 2.78 - 2.77 (m, 2H), 2.41 (t, *J =* 3.2 Hz, 1H), 2.21 (s, 3H), 2.06 (s, 6H), 2.02 (s, 3H), 1.41 (t, *J =* 7.2 Hz, 3H), 1.25 (t, *J =* 7.2 Hz, 1H).

### Step 5: Preparation of Compound A-46e

Compound A-46d (100.0 mg, 0.162 mmol) was dissolved in MC (10.0 mL) under a nitrogen atmosphere at 0°C, and then 4-nitrophenyl chloroformate (49.0 mg, 0.243 mmol), DIPEA (70.0 µL, 0.405 mmol) and pyridine (40.0 µL, 0.486 mmol) were sequentially added, and the mixture was stirred for 3 hours while slowly raising the temperature from 0°C to room temperature. After completion of the reaction, EA (50 mL) and distilled water (50 mL) were added to extract the organic layer. The obtained organic layer was dried over anhydrous magnesium sulfate, filtered, and then concentrated under reduced pressure. The resulting residue was subjected to column chromatography to obtain Compound A-46e in the form of a white solid (56.0 mg, 44 %).

¹H-NMR (400 MHz, CDCl₃) δ 8.24 (d, *J =* 9.2 Hz, 2H), 7.38 (s, 1H), 7.34 (d, *J =* 9.2 Hz, 2H), 7.11 (d, *J =* 8.0 Hz, 2H), 6.88 (d, *J =* 8.0 Hz, 1H), 6.14 (t, *J =* 7.2 Hz, 1H), 5.63 (dd, *J =* 10.4 Hz, *J =* 8.0 Hz, 1H), 5.49 (d, *J =* 3.2 Hz, 1H), 5.29 - 5.23 (m, 1H), 5.15 (dd, *J =* 10.4 Hz, *J* = 3.2 Hz, 1H), 4.44 (t, *J =* 7.2 Hz, 2H), 4.27 (s, 3H), 4.24 - 4.09 (m, 3H), 3.09 - 3.04 (m, 1H), 2.97 - 2.90 (m, 1H), 2.21 (s, 3H), 2.06 (s, 6H), 2.01 (s, 3H), 1.42 (t, *J =* 7.2 Hz, 3H).

### Step 6: Preparation of Compound A-46f

Compound A-46e (56.0 mg, 0.071 mmol) was dissolved in DMF (1.0 mL) under a nitrogen atmosphere at 0°C, and then MMAE (51.3 mg, 0.071 mmol), HOBt (14.5 mg, 0.107 mmol), DIPEA (31 µL, 0.178 mmol) and pyridine (1.0 mL) were sequentially added, and the mixture was stirred at room temperature for 3 hours. After completion of the reaction, 2N-hydrochloric acid aqueous solution (1.0 mL) was added to terminate the reaction, and the reaction solution was diluted with ACN (1.0 mL) and distilled water (1.0 mL), then purified using Preparative-HPLC, and freez-dried to obtain Compound A-46f in the form of a white solid (4.0 mg, 4.1 %); EI-MS m/z: 1361[M+H]⁺.

### Step 7: Preparation of Compounds A-46, A-47 and A-48

Compound A-46f (4.0 mg, 0.0014 mmol) was dissolved in methanol (0.5 mL) under a nitrogen atmosphere at 0°C, and then lithium hydroxide hydrate (0.5 mg, 0.011 mmol) dissolved in distilled water (0.5 mL) was slowly added dropwise, and the mixture was stirred at 0°C for 2 hours. After completion of the reaction, 2N-hydrochloric acid aqueous solution (1.0 mL) was added to terminate the reaction, and the reaction solution was diluted with ACN (1.0 mL) and distilled water (1.0 mL), then purified using Preparative-HPLC, and freeze-dried to obtain Compounds A-46 (Acid form), A-47 (Methyl ester form) and A-48 (Ethylester form) in the form of a white solid, respectively.

(A-46: 0.5 mg, 14 %; EI-MS m/z: 1166[M+H]⁺), (A-47: 0.4 mg, 11 %; MS m/z: 1179[M+H]⁺), (A-48: 1.0 mg, 28 %; MS m/z: 1193[M+H]⁺).

### Example 1-39: Preparation of Compound A-49

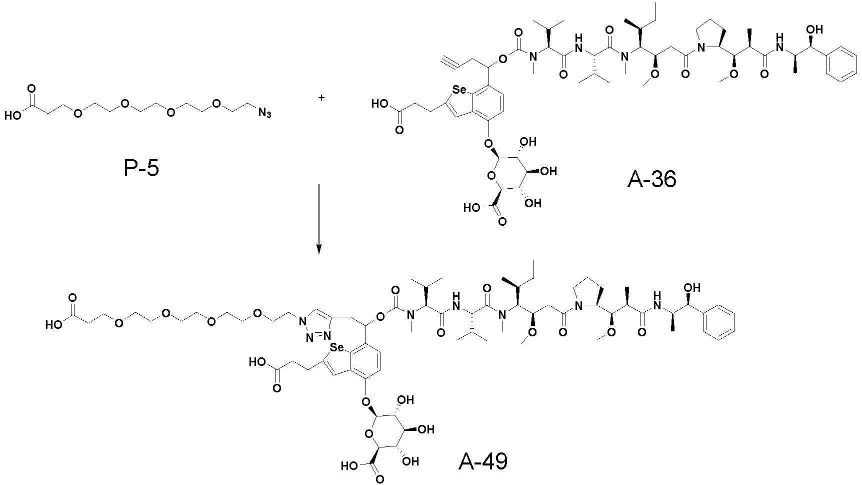

Compound A-36 (2.0 mg, 0.0015 mmol) was dissolved in DMSO (1.5 mL) under a nitrogen atmosphere at 0°C, and then Linker P-5 (1.0 mg) dissolved in DMSO (500 µL) was added to the reaction. Additionally, distilled water (2 mL), copper (II) sulfate pentahydrate (CuSO₄·5H₂O, 1 mg) and sodium ascorbate (1 mg) were sequentially added to the reaction solution, and then the mixture was stirred at room temperature for 1 hour. After completion of the reaction, the reaction solution was diluted with distilled water (3 mL), purified using Preparative-HPLC, and then freeze-dried to obtain Compound A-49 in the form of a white solid (1.7 mg, 69 %); MS m/z: 1549[M+H]⁺.

### Example I-40: Preparation of Compound A-50

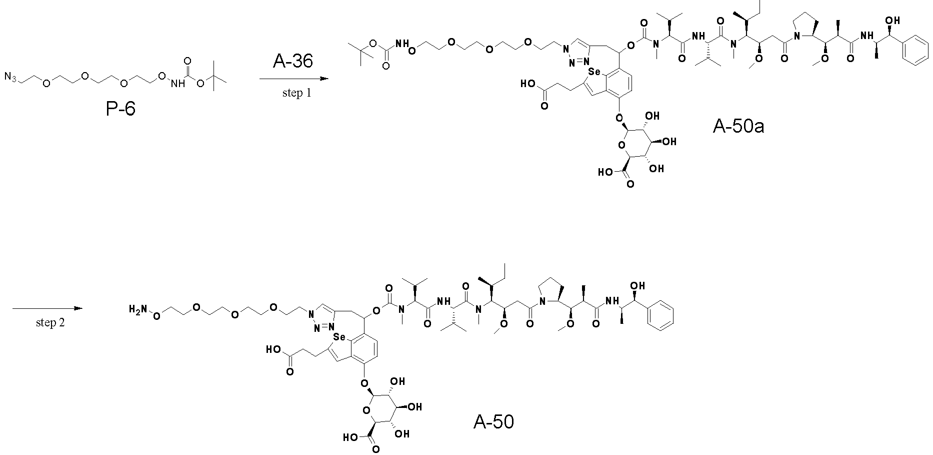

Compound A-50a was obtained in a manner similar to Example I-39, except that Linker P-6 was used instead of Linker P-5 in Example I-39. Then, Compound A-50a was subjected to Boc deprotection to obtain Compund A-50 (1.2 mg, 51%); MS m/z: 1492[M+H]⁺.

### Example I-41: Preparation of Compound A-51

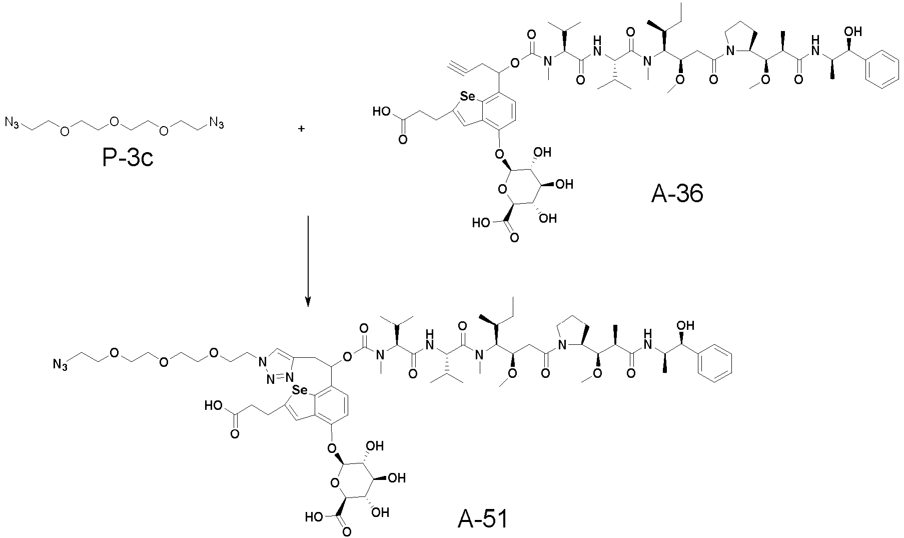

Compound A-51 was obtained in the form of a white solid in a manner similar to Example I-39, except that Compound P-3c was used instead of Linker P-5 in Example I-39 (1.5 mg, 63%); EI-MS m/z: 1502[M+H]+.

### Example I-42: Preparation of Compound A-52

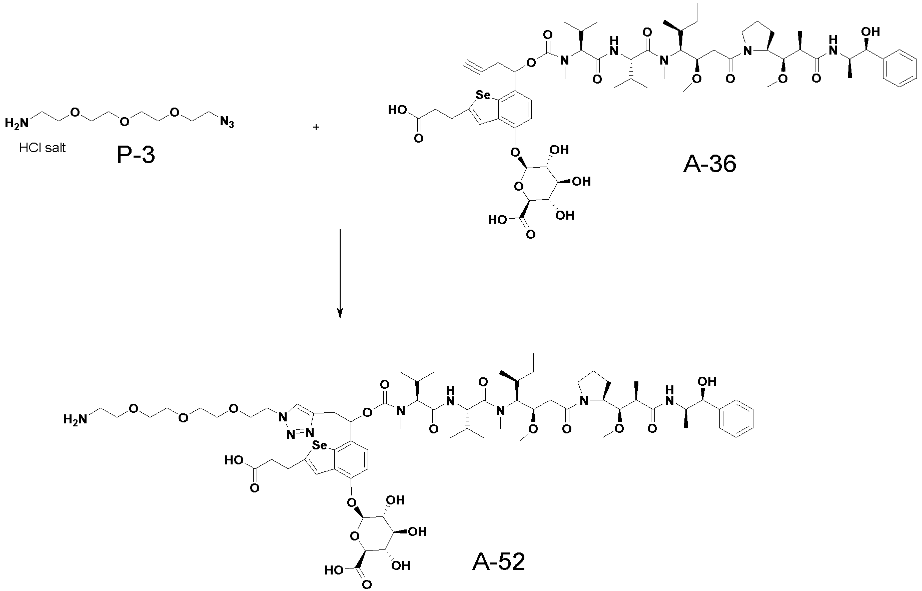

Compound A-52 was obtained in the form of a white solid in a manner similar to Example I-39, except that Compound P-3 was used instead of Linker P-5 in Example I-39 (1.2 mg, 51%); EI-MS m/z: 1476[M+H]+.

### Example II-1: Preparation of Compound B-1

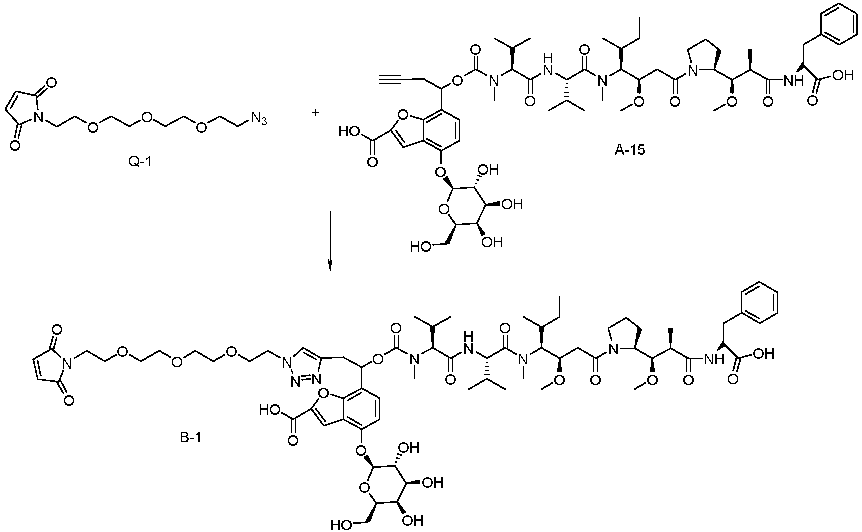

Compound A-15 (3.1 mg, 0.0026 mmol) was dissolved in DMSO (200 µL) under a nitrogen atmosphere at 0°C, and then 300 µL of a solution of Linker Q-1 (5 mg) dissolved in DMSO (1 mL) was taken and added to the reaction. Additionally, distilled water (2 mL), ethanol (20 µL), copper (II) sulfate pentahydrate (CuSO₄·5H₂O, 1 mg) and sodium ascorbate (1 mg) were sequentially added to the reaction solution, and then the mixture was stirred at room temperature for 20 minutes. After completion of the reaction, the reaction solution was diluted with distilled water (1 mL), purified using Preparative-HPLC, and then freeze-dried to obtain Compound B-1 in the form of a white solid (1.9 mg, 48.8 %); MS m/z: 1465[M+H]⁺.

### Example II-2: Preparation of Compound B-2

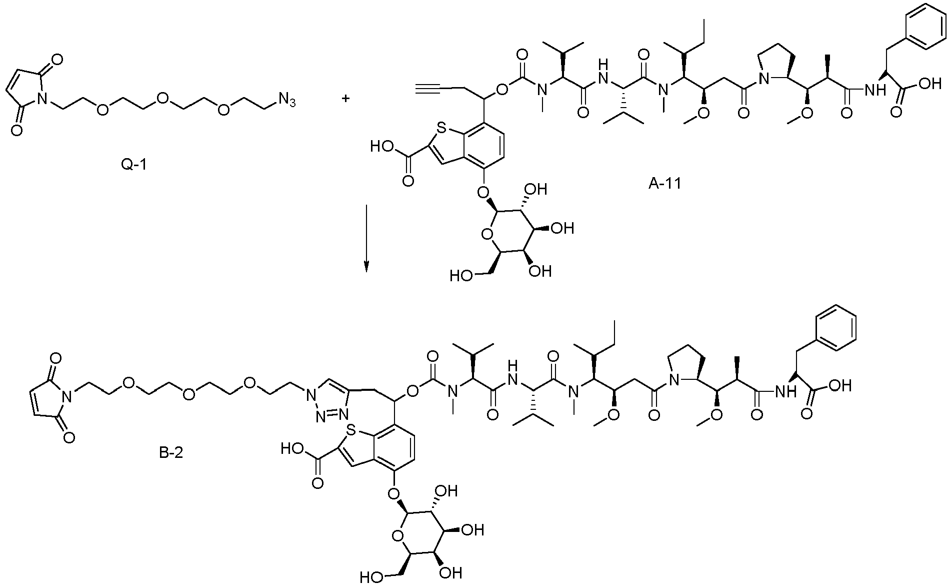

Compound A-11 (3.0 mg, 0.0025 mmol) was dissolved in DMSO (200 µL) under a nitrogen atmosphere at 0°C, and then 150 µL of a solution of Linker Q-1 (7.5 mg) dissolved in DMSO (1 mL) was taken and added to the reaction. Additionally, distilled water (2 mL), ethanol (20 µL), copper (II) sulfate pentahydrate (CuSO₄.5H₂O, 1 mg) and sodium ascorbate (1 mg) were sequentially added to the reaction solution, and then the mixture was stirred at room temperature for 30 minutes. After completion of the reaction, the reaction solution was diluted with distilled water (1 mL), purified using Preparative-HPLC, and then freeze-dried to obtain Compound B-2 in the form of a white solid (2.9 mg, 77.3 %); MS m/z: 1481[M+H]⁺.

### Example II-3: Preparation of Compound B-3

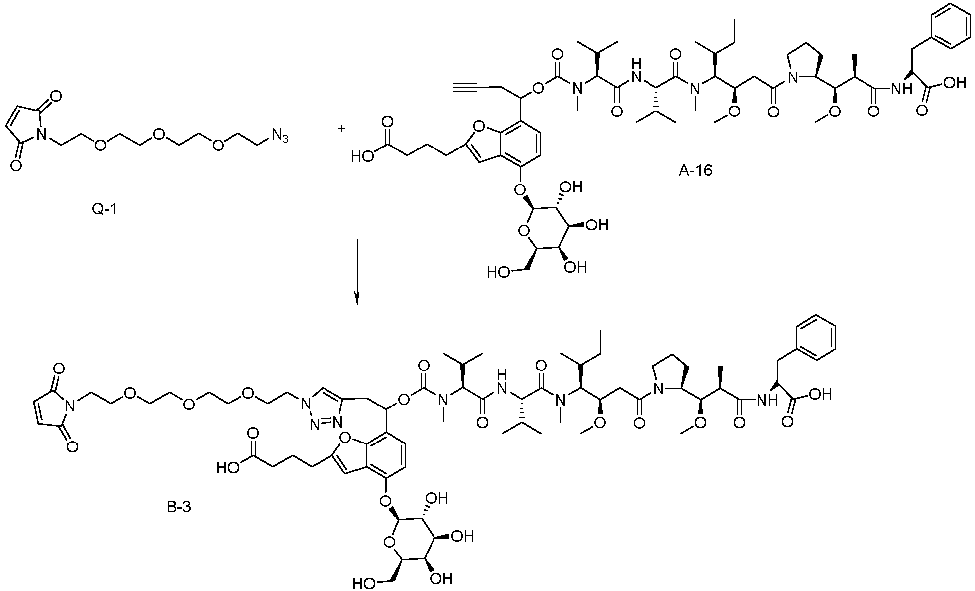

Compound A-16 (3.9 mg, 0.0032 mmol) was dissolved in DMSO (200 µL) under a nitrogen atmosphere at 0°C, and then 150 µL of a solution of Linker Q-1 (18 mg) dissolved in DMSO (1.8 mL) was taken and added to the reaction. Additionally, distilled water (2 mL), ethanol (20 µL), copper (II) sulfate pentahydrate (CuSO₄.5H₂O, 1 mg) and sodium ascorbate (1 mg) were sequentially added to the reaction solution, and then the mixture was stirred at room temperature for 30 minutes. After completion of the reaction, the reaction solution was diluted with distilled water (1 mL), purified using Preparative-HPLC, and then freeze-dried to obtain Compound B-3 in the form of a white solid (2.9 mg, 59.6 %); MS m/z: 1507[M+H]⁺.

### Example II-4: Preparation of Compound B-4

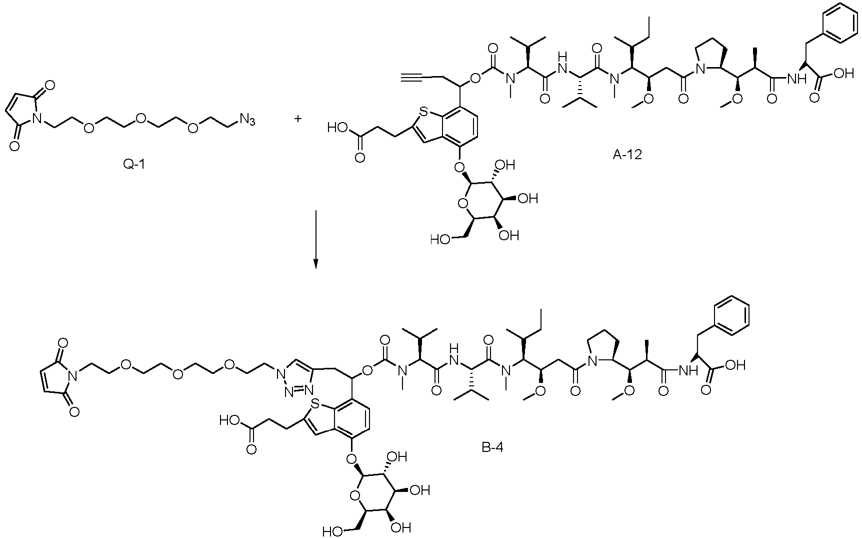

Compound A-12 (3.6 mg, 0.0029 mmol) was dissolved in DMSO (200 µL) under a nitrogen atmosphere at 0°C, and then 150 µL of a solution of Linker Q-1 (14 mg) dissolved in DMSO (1 mL) was taken and added to the reaction. Additionally, distilled water (2 mL), ethanol (20 µL), copper (II) sulfate pentahydrate (CuSO₄.5H₂O, 1 mg) and sodium ascorbate (1 mg) were sequentially added to the reaction solution, and then the mixture was stirred at room temperature for 30 minutes. After completion of the reaction, the reaction solution was diluted with distilled water (1 mL), purified using Preparative-HPLC, and then freeze-dried to obtain Compound B-4 in the form of a white solid (3.0 mg, 66.9 %); MS m/z: 1508[M+H]⁺.

### Example II-5: Preparation of Compound B-5

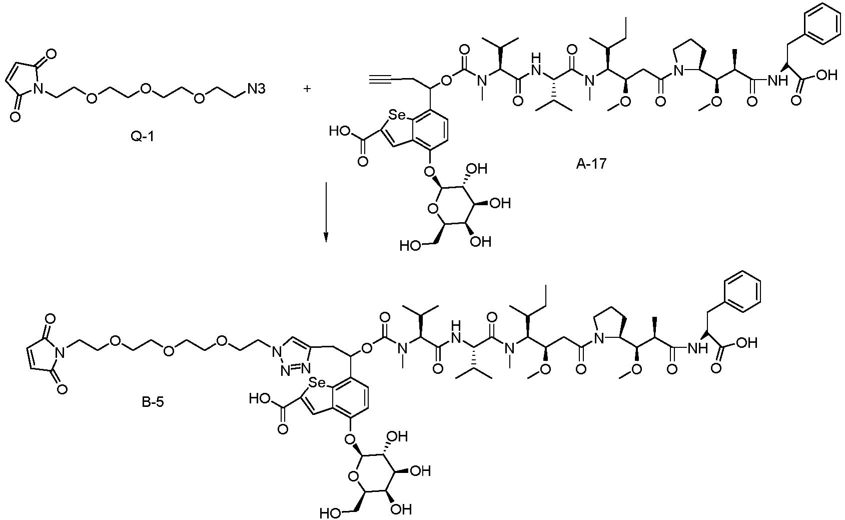

Compound A-17 (9 mg, 0.0073 mmol) was dissolved in DMSO (200 µL) under a nitrogen atmosphere at 0°C, and then 320 µL of a solution of Linker Q-1 (18 mg) dissolved in DMSO (1.8 mL) was taken and added to the reaction. Additionally, distilled water (2 mL), ethanol (20 µL), copper (II) sulfate pentahydrate (CuSO₄.5H₂O, 1 mg) and sodium ascorbate (2 mg) were sequentially added to the reaction solution, and then the mixture was stirred for 30 minutes at room temperature. After completion of the reaction, the reaction solution was diluted with distilled water (1 mL), purified using Preparative-HPLC, and then freeze-dried to obtain Compound B-5 in the form of a white solid (4.4 mg, 40 %); MS m/z: 1528[M+H]⁺.

### Example II-6: Preparation of Compound B-6

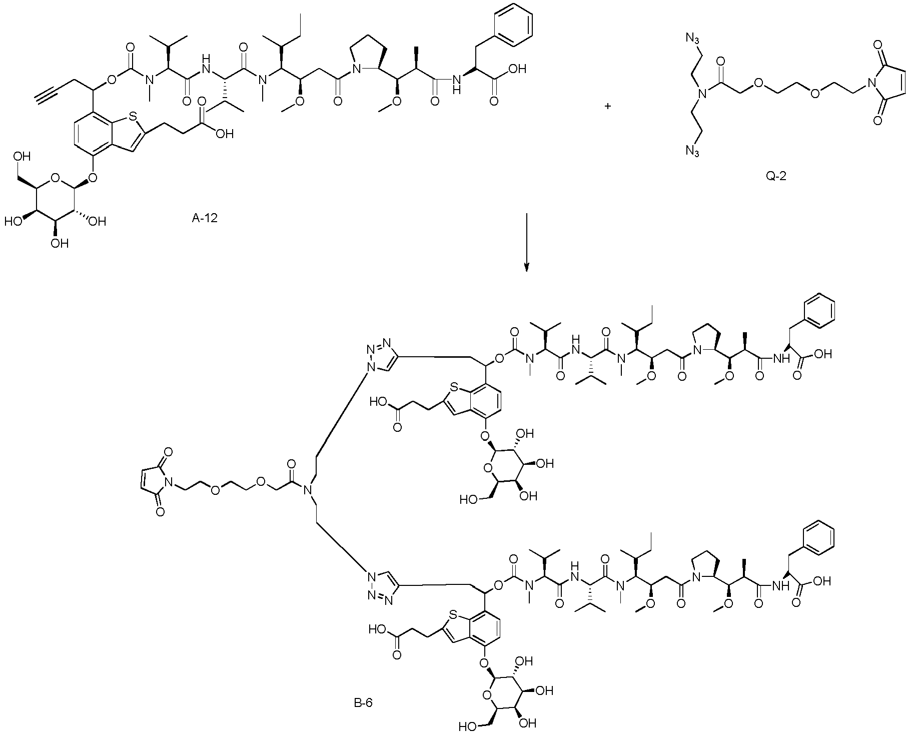

Compound A-12 (4.2 mg, 0.0034 mmol) was dissolved in DMSO (200 µL) under a nitrogen atmosphere at 0°C, and then 550 µL of a solution of Linker Q-2 (12 mg) dissolved in DMSO (1 mL) was taken and added to the reaction. Additionally, distilled water (2 mL), DMSO (400 Ml), copper (II) sulfate pentahydrate (CuSO₄.5H₂O, 1 mg) and sodium ascorbate (1.4 mg) were sequentially added to the reaction solution, and then the mixture was stirred at room temperature for 30 minutes. After completion of the reaction, the reaction solution was diluted with distilled water (1 mL) and ACN (1 mL), purified using Preparative-HPLC, and then freeze-dried to obtain Compound B-6 in the form of a white solid (3.1 mg, 66 %); EI-MS m/z: 1400 1/2[M+H]⁺, 934 1/3[M+H]⁺.

### Example II-7: Preparation of Compound B-7

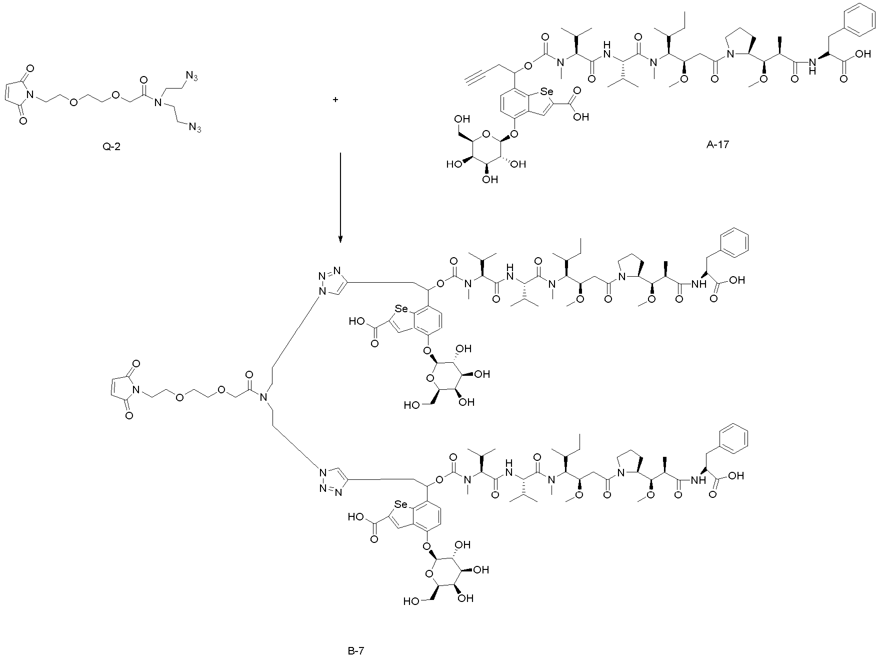

Compound A-17 (5 mg, 0.004 mmol) was dissolved in DMSO (1.5 mL) and ethanol (200 µL) under a nitrogen atmosphere at 0°C, and then 100 µL of a solution of Linker Q-2 (12 mg) dissolved in DMSO (1.2 mL) was taken and added to the reaction. Additionally, distilled water (2 mL), ethanol (20 µL), copper (II) sulfate pentahydrate (CuSO₄.5H₂O, 1 mg) and sodium ascorbate (1 mg) were sequentially added to the reaction solution, and then the mixture was stirred at room temperature for 30 minutes. After completion of the reaction, the reaction solution was diluted with distilled water (3 mL), purified using Preparative-HPLC, and then freeze-dried to obtain Compound B-7 in the form of a white solid (3.0 mg, 52 %); MS m/z: 2839[M+H]⁺, 1420 1/2[M+H]⁺, 947 1/3[M+H]⁺.

### Example II-8: Preparation of Compounds B-8 and B-13

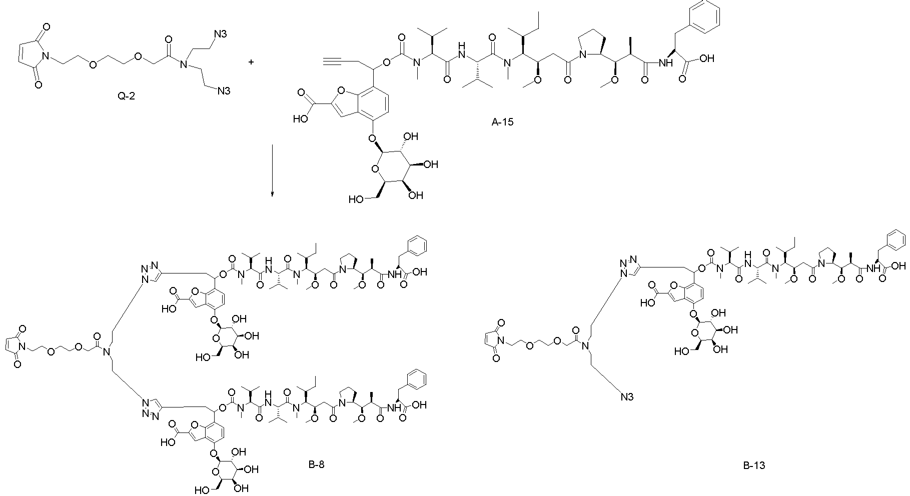

Compound A-15 (4.9 mg, 0.0042 mmol) was dissolved in DMSO (1.0 mL) under a nitrogen atmosphere at 0°C, and then 100 µL of a solution of Linker Q-2 (12 mg) dissolved in DMSO (1.2 mL) was taken and added to the reaction. Additionally, distilled water (2 mL), copper (II) sulfate pentahydrate (CuSO₄.5H₂O, 1 mg) and sodium ascorbate (1 mg) were sequentially added to the reaction solution, and then the mixture was stirred at room temperature for 1 hour. After completion of the reaction, the reaction solution was diluted with distilled water (3 mL), purified using Preparative-HPLC, and then freeze-dried to obtain Compounds B-8 and B-13 in the form of a white solid (B-8: 1.8 mg, 31.6 %; MS m/z: 2713[M+H]⁺, 1357 1/2[M+H]⁺, 905 1/3 [M+H]⁺), (B-13: 0.9 mg, 13.8 %; MS m/z: 1547[M+H]⁺, 774 1/2[M+H]⁺).

### Example II-9: Preparation of Compound B-9

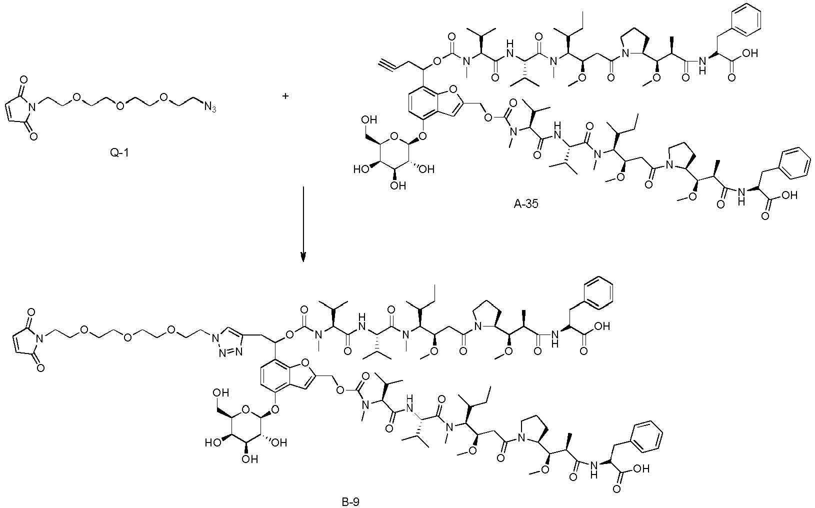

Compound A-35 (6.0 mg, 0.0031 mmol) was dissolved in DMSO (1.5 mL) under a nitrogen atmosphere at 0°C, and then 150 µL of a solution of Linker Q-1 (7.0 mg) dissolved in DMSO (700 µL) was taken and added to the reaction. Additionally, distilled water (2 mL), copper (II) sulfate pentahydrate (CuSO₄.5H₂O, 1 mg) and sodium ascorbate (1 mg) were sequentially added to the reaction solution, and then the mixture was stirred at room temperature for 1 hour. After completion of the reaction, the reaction solution was diluted with distilled water (3 mL), purified using Preparative-HPLC, and then freeze-dried to obtain Compound B-9 in the form of a white solid (5.1 mg, 73.9 %); MS m/z: 2209[M+H]⁺, 1105 1/2[M+H]⁺.

### Example II-10: Preparation of Compound B-10

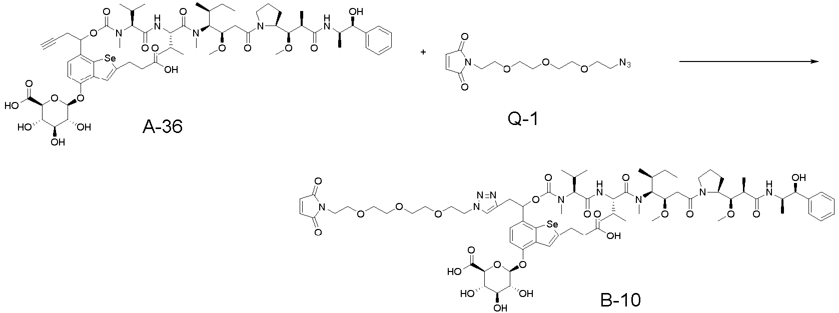

Compound A-36 (6.8 mg, 0.0054 mmol) was dissolved in DMSO (200 µL) under a nitrogen atmosphere at 0°C, and then 110 µL of a solution of Linker Q-1 (8.5 mg) dissolved in DMSO (0.5 mL) was taken and added to the reaction. Additionally, distilled water (2 mL), DMSO (300 µL), copper (II) sulfate pentahydrate (CuSO₄.5H₂O, 1.5 mg) and sodium ascorbate (2.1 mg) were sequentially added to the reaction solution, and then the mixture was stirred at room temperature for 1.5 hours. After completion of the reaction, the reaction solution was diluted with distilled water (1 mL) and ACN (1 mL), purified using Preparative-HPLC, and then freeze-dried to obtain Compound B-10 in the form of a white solid (6.2 mg, 74 %); EI-MS m/z: 1556[M+H]⁺, 778 1/2[M+H]⁺.

### Example II-11: Preparation of Compound B-11

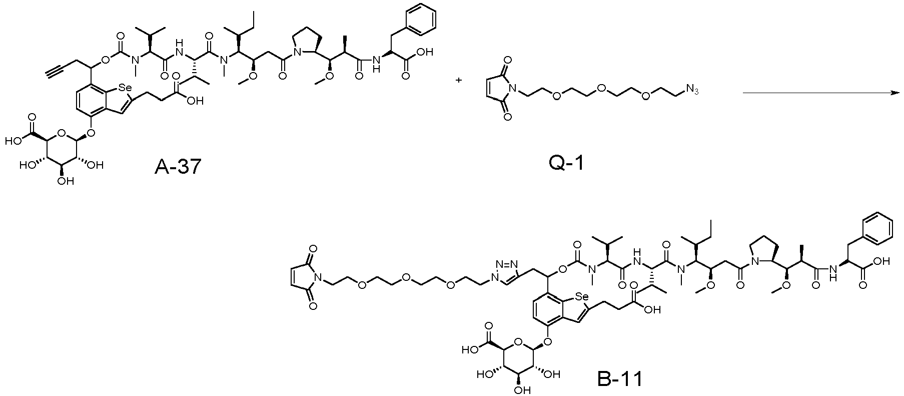

Compound A-37 (4.3 mg, 0.0034 mmol) was dissolved in DMSO (200 µL) under a nitrogen atmosphere at 0°C, and then 110 µL of a solution of Linker Q-1 (12 mg) dissolved in DMSO (1.2 mL) was taken and added to the reaction. Additionally, distilled water (2 mL), DMSO (300 µL), copper (II) sulfate pentahydrate (CuSO₄·5H₂O, 1 mg) and sodium ascorbate (1.3 mg) were sequentially added to the reaction solution, and then the mixture was stirred at room temperature for 2.5 hours. After completion of the reaction, the reaction solution was diluted with distilled water (1 mL) and ACN (1 mL), purified using Preparative-HPLC, and then freeze-dried to obtain Compound B-11 in the form of a white solid (2.4 mg, 45 %); EI-MS m/z: 1570[M+H]⁺, 785 1/2[M+H]⁺.

### Example II-12: Preparation of Compound B-12

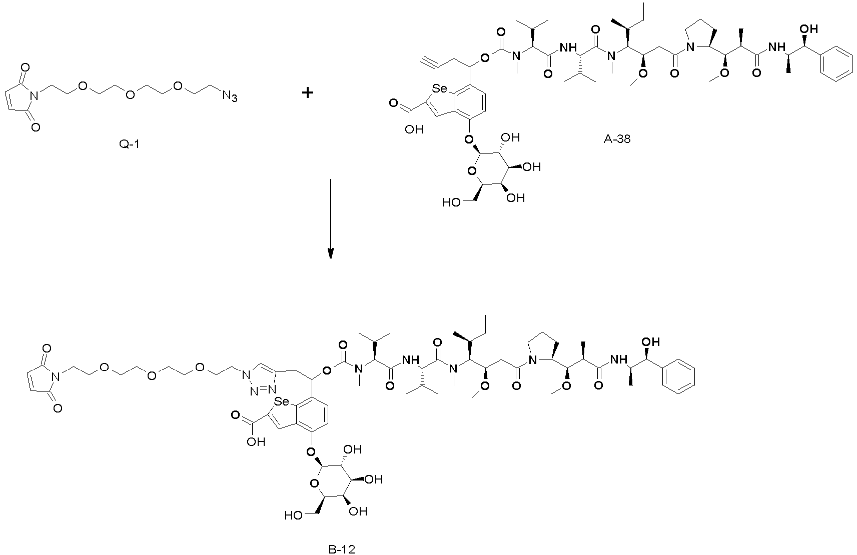

Compound A-38 (5.0 mg, 0.0041 mmol) was dissolved in DMSO (400 µL) under a nitrogen atmosphere at 0°C, and then 185 µL of a solution of Linker Q-1 (13.5 mg) dissolved in DMSO (1350 µL) was taken and added to the reaction. Additionally, distilled water (2 mL), copper (II) sulfate pentahydrate (CuSO₄·5H₂O, 1 mg) and sodium ascorbate (1 mg) were sequentially added to the reaction solution, and then the mixture was stirred at room temperature for 1 hour. After completion of the reaction, the reaction solution was diluted with distilled water (3 mL), purified using Preparative-HPLC, and then freeze-dried to obtain Compound B-12 in the form of a white solid (5.2 mg, 83.6 %); MS m/z: 1514[M+H]⁺, 757 1/2[M+H]⁺.

### Example II-13: Preparation of Compound B-14

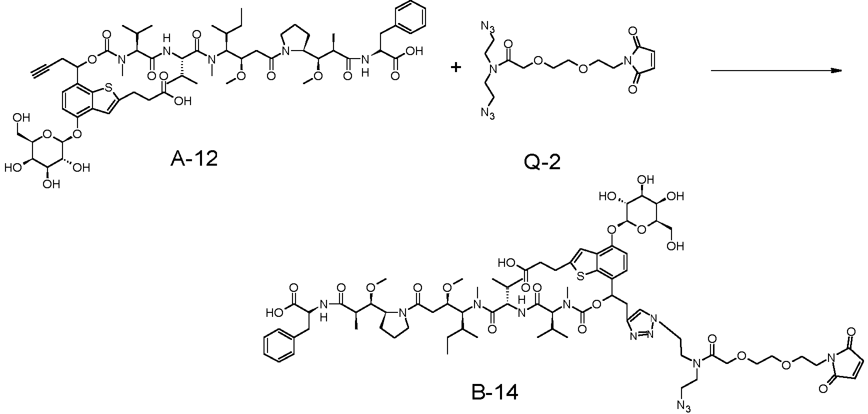

Compound A-12 (19 mg, 0.015 mmol) was dissolved in DMSO (3 mL) under a nitrogen atmosphere at 0°C, and then 3.78 mL of a solution of Linker Q-2 (39.8 mg) dissolved in DMSO (3.98 mL) was taken and added to the reaction. Additionally, distilled water (6 mL), copper (II) sulfate pentahydrate (CuSO₄·5H₂O, 4.3 mg) and sodium ascorbate (6.2 mg) were sequentially added to the reaction solution, and then the mixture was stirred at room temperature for 30 minutes. After completion of the reaction, the reaction solution was diluted with distilled water (5 mL) and ACN (5 mL), purified using Preparative-HPLC, and then freeze-dried to obtain Compound B-14 in the form of a white solid (6.6 mg, 26 %); EI-MS m/z: 1591 [M+H]⁺, 795 1/2 [M+H]⁺.

### Example II-14: Preparation of Compound B-15

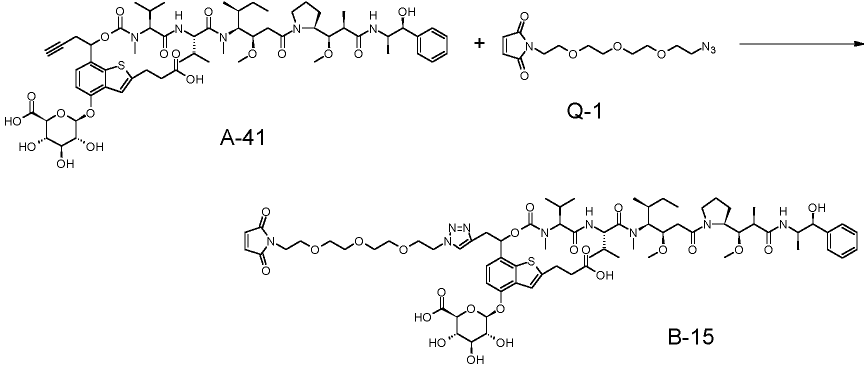

Compound A-41 (5.3 mg, 0.0044 mmol) was dissolved in DMSO (500 µL) under a nitrogen atmosphere at 0°C, and then 25 µL of a solution of Linker Q-1 (77 mg) dissolved in DMSO (0.77 mL) was taken and added to the reaction. Additionally, distilled water (2 mL), DMSO (500 µL), copper (II) sulfate pentahydrate (CuSO₄·5H₂O, 1.2 mg) and sodium ascorbate (1.7 mg) were sequentially added to the reaction solution, and then the mixture was stirred at room temperature for 2 hours. After completion of the reaction, the reaction solution was diluted with distilled water (1 mL) and ACN (1 mL), purified using Preparative-HPLC, and then freeze-dried to obtain Compound B-15 in the form of a white solid (3.3 mg, 50 %); EI-MS m/z: 1508 [M+H]⁺, 754 1/2 [M+H]⁺.

### Example II-15: Preparation of Compound B-16

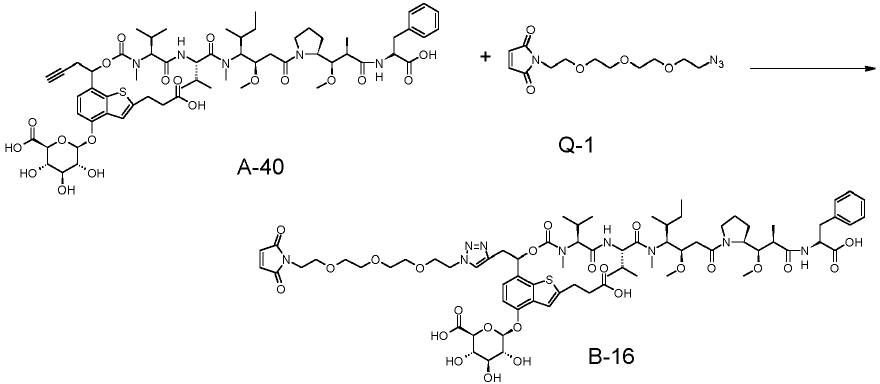

Compound A-40 (5.6 mg, 0.0046 mmol) was dissolved in DMSO (500 µL) under a nitrogen atmosphere at 0°C, and then 25.7 µL of a solution of Linker Q-1 (77 mg) dissolved in DMSO (0.77 mL) was taken and added to the reaction. Additionally, distilled water (2 mL), DMSO (500 µL), copper (II) sulfate pentahydrate (CuSO₄·5H₂O, 1.2 mg) and sodium ascorbate (1.8 mg) were sequentially added to the reaction solution, and then the mixture was stirred at room temperature for 2 hours. After completion of the reaction, the reaction solution was diluted with distilled water (1 mL) and ACN (1 mL), purified using Preparative-HPLC, and then freeze-dried to obtain Compound B-16 in the form of a white solid (4.8 mg, 69 %); EI-MS m/z: 1522[M+H]⁺, 761 1/2[M+H]⁺.

### Example II-16: Preparation of Compound B-17

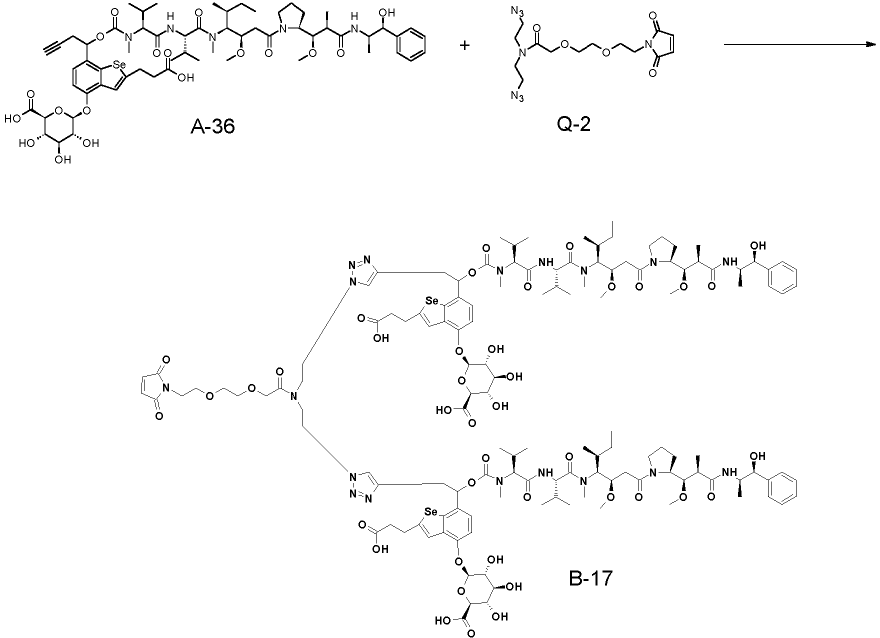

Compound A-36 (6.6 mg, 0.0052 mmol) was dissolved in DMSO (300 µL) under a nitrogen atmosphere at 0°C, and then 100 µL of a solution of Linker Q-2 (17.2 mg) dissolved in DMSO (1.72 mL) was taken and added to the reaction. Additionally, distilled water (2 mL), DMSO (200 µL), copper (II) sulfate pentahydrate (CuSO₄·5H₂O, 1.5 mg) and sodium ascorbate (2.1 mg) were sequentially added to the reaction solution, and then the mixture was stirred at room temperature for 2 hours. After completion of the reaction, the reaction solution was diluted with distilled water (1 mL) and ACN (1 mL), purified using Preparative-HPLC, and then freeze-dried to obtain Compound B-17 in the form of a white solid (5.8 mg, 76 %); EI-MS m/z: 2895[M+H]⁺, 1448 1/2[M+H]⁺, 965 1/3[M+H]⁺.

### Example II-17: Preparation of Compound B-18

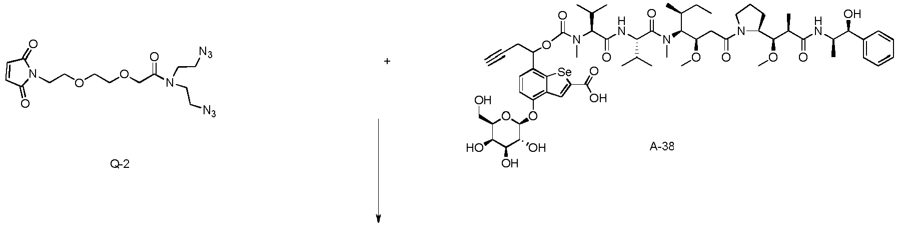

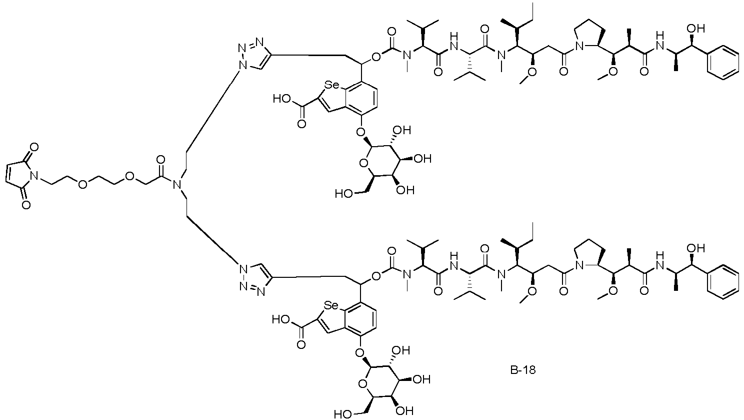

Compound A-38 (10 mg, 0.0082 mmol) was dissolved in DMSO (1000 µL) under a nitrogen atmosphere at 0°C, and then 156 µL of a solution of Linker Q-2 (37.5 mg) dissolved in DMSO (3750 µL) was taken and added to the reaction. Additionally, distilled water (1 mL), copper (II) sulfate pentahydrate (CuSO₄·5H₂O, 1 mg) and sodium ascorbate (1 mg) were sequentially added to the reaction solution, and then the mixture was stirred at room temperature for 1 hour. After completion of the reaction, the reaction solution was diluted with distilled water (3 mL), purified using Preparative-HPLC, and then freeze-dried to obtain Compound B-18 in the form of a white solid (6.8 mg, 58.8 %); MS m/z: 2811[M+H]⁺, 1406 1/2[M+H]⁺, 937 1/3[M+H]⁺.

### Example II-18: Preparation of Compound B-19

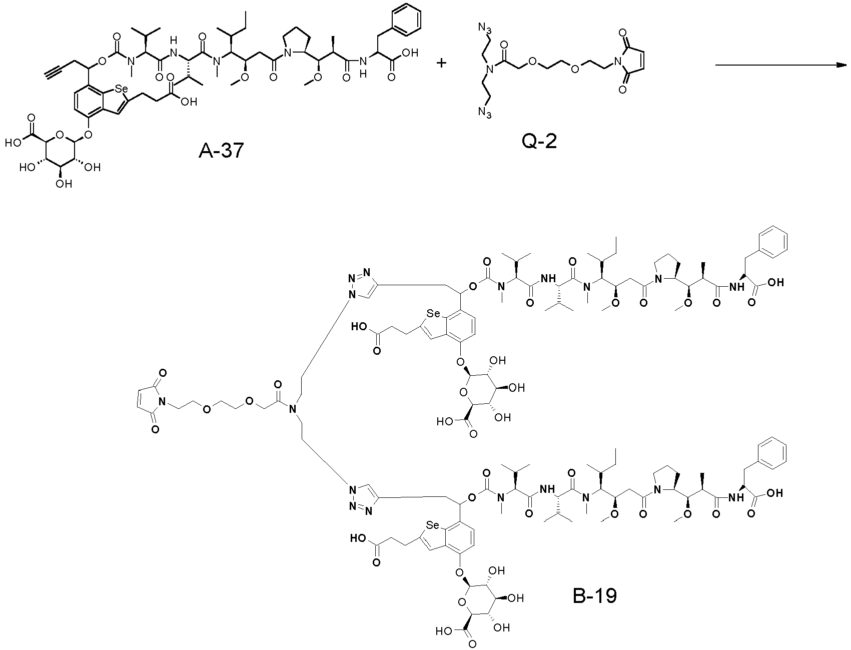

Compound A-37 (6 mg, 0.0047 mmol) was dissolved in DMSO (1 mL) under a nitrogen atmosphere at 0°C, and then 200 µL of a solution of Linker Q-2 (39.8 mg) dissolved in DMSO (3.98 mL) was taken and added to the reaction. Additionally, distilled water (2 mL), DMSO (1 mL), copper (II) sulfate pentahydrate (CuSO₄·5H₂O, 1.3 mg) and sodium ascorbate (1.9 mg) were sequentially added to the reaction solution, and then the mixture was stirred at room temperature for 2 hours. After completion of the reaction, the reaction solution was diluted with distilled water (2 mL) and ACN (2 mL), purified using Preparative-HPLC, and then freeze-dried to obtain Compound B-19 in the form of a white solid (3.6 mg, 52 %); EI-MS m/z: 2922[M+H]⁺, 1461 1/2[M+H]⁺, 975 1/3[M+H]⁺.

### Example II-19: Preparation of Compound B-20

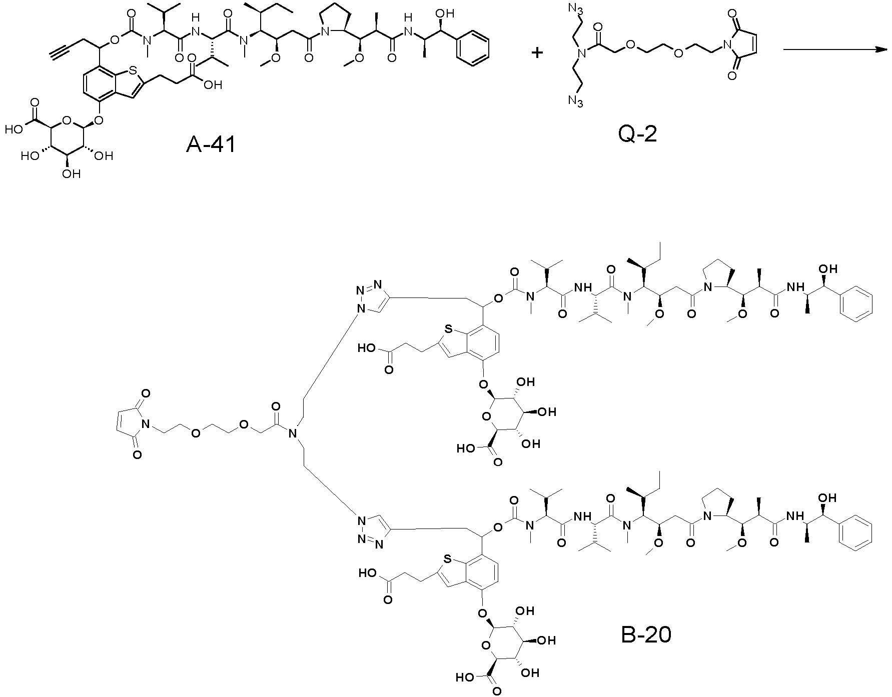

Compound A-41 (6.2 mg, 0.0051 mmol) was dissolved in DMSO (1 mL) under a nitrogen atmosphere at 0°C, and then 100 µL of a solution of Linker Q-2 (18.5 mg) dissolved in DMSO (1.85 mL) was taken and added to the reaction. Additionally, distilled water (2 mL), DMSO (1 mL), copper (II) sulfate pentahydrate (CuSO₄·5H₂O, 1.4 mg) and sodium ascorbate (2 mg) were sequentially added to the reaction solution, and then the mixture was stirred at room temperature for 2 hours. After completion of the reaction, the reaction solution was diluted with distilled water (2 mL) and ACN (2 mL), purified using Preparative-HPLC, and then freeze-dried to obtain Compound B-20 in the form of a white solid (3.3 mg, 46 %); EI-MS m/z: 2801[M+H]⁺, 1400 1/2[M+H]⁺, 933 1/3[M+H]⁺.

### Example II-20: Preparation of Compound B-21

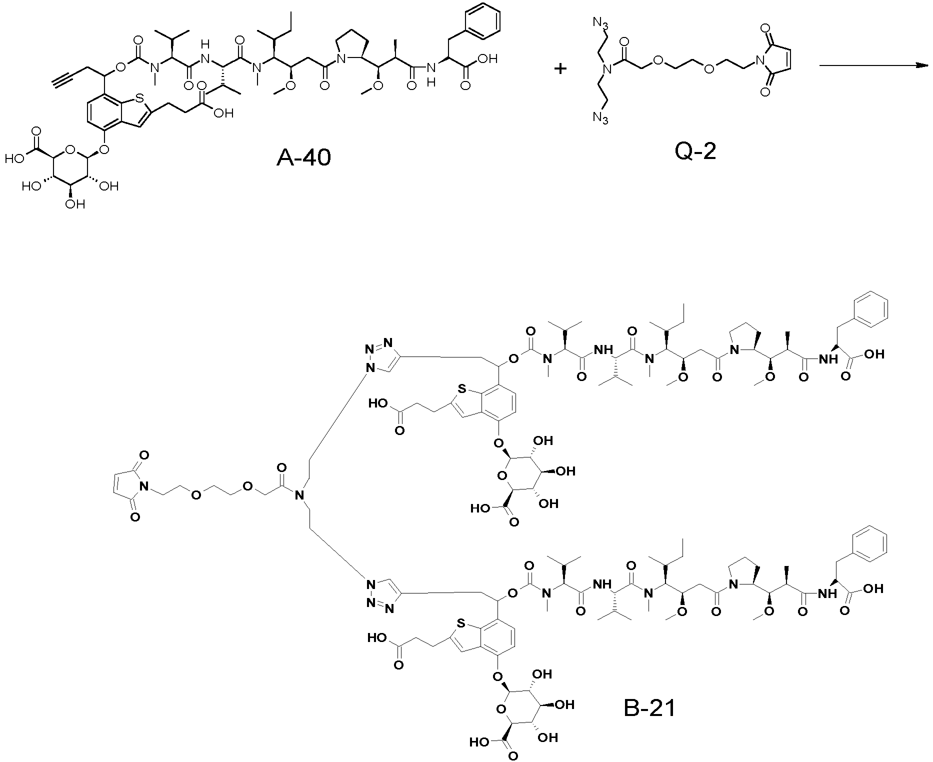

Compound A-40 (6.5 mg, 0.0053 mmol) was dissolved in DMSO (1 mL) under a nitrogen atmosphere at 0°C, and then 101 µL of a solution of Linker Q-2 (18.5 mg) dissolved in DMSO (1.85 mL) was taken and added to the reaction. Additionally, distilled water (2 mL), DMSO (1 mL), copper (II) sulfate pentahydrate (CuSO₄·5H₂O, 1.5 mg) and sodium ascorbate (2.1 mg) were sequentially added to the reaction solution, and then the mixture was stirred at room temperature for 2.5 hours. After completion of the reaction, the reaction solution was diluted with distilled water (2 mL) and ACN (2 mL), purified using Preparative-HPLC, and then freeze-dried to obtain Compound B-21 in the form of a white solid (4.1 mg, 54 %); EI-MS m/z: 2829[M+H]⁺, 1414 1/2[M+H]⁺, 943 1/3[M+H]⁺.

### Example II-21: Preparation of Compound B-22

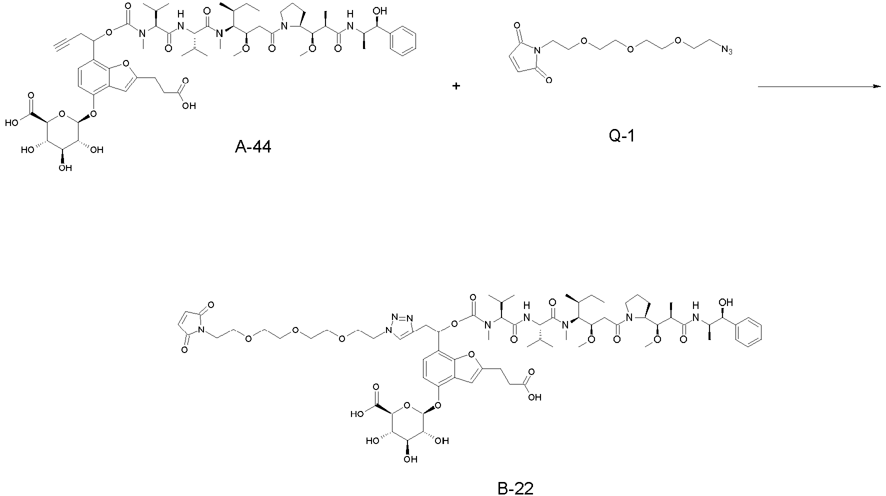

Compound A-44 (4.5 mg, 0.0038 mmol) was dissolved in DMSO (1 mL) under a nitrogen atmosphere at room temperature, and then 97 µL of a solution of Linker Q-1 (114 mg, 0.3822 mmol) dissolved in DMSO (5.7 mL) was taken and added to the reaction. Additionally, distilled water (2mL), copper (II) sulfate pentahydrate (CuSO₄·5H₂O, 0.66 mg, 0.0041 mmol) and sodium ascorbate (1.3 mg, 0.0075 mmol) were sequentially added to the reaction solution, and then the mixture was stirred for 40 minutes. After completion of the reaction, the reaction solution was diluted with distilled water (1 mL), purified using Preparative-HPLC, and then freeze-dried to obtain Compound B-22 in the form of a white solid (4.2 mg, 75 %); MS m/z: 1493[M+H]⁺, 746 1/2[M+H]⁺.

### Example II-22: Preparation of Compound B-23

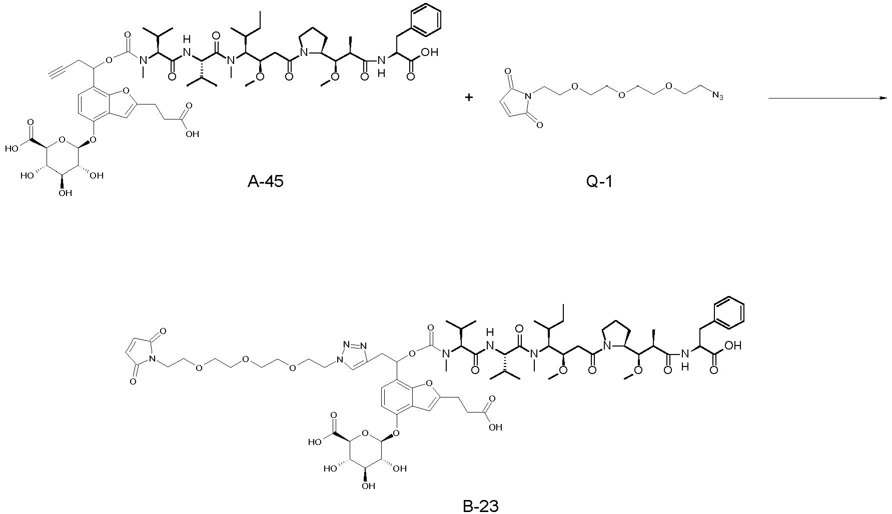

Compound A-45 (4.5 mg, 0.0037 mmol) was dissolved in DMSO (1 mL) under a nitrogen atmosphere at room temperature, and then 97 µL of a solution of Linker Q-1 (114 mg, 0.3822 mmol) dissolved in DMSO (5.7 mL) was taken and added to the reaction. Additionally, distilled water (2 mL), copper (II) sulfate pentahydrate (CuSO₄·5H₂O, 0.65 mg, 0.0041 mmol) and sodium ascorbate (1.3 mg, 0.0074 mmol) were sequentially added to the reaction solution, and then the mixture was stirred for 1 hour. After completion of the reaction, the reaction solution was diluted with distilled water (1 mL), purified using Preparative-HPLC, and then freeze-dried to obtain Compound B-23 in the form of a white solid (3.7 mg, 66 %); MS m/z: 1507[M+H]⁺, 753 1/2[M+H]⁺.

### Example II-23: Preparation of Compound B-24

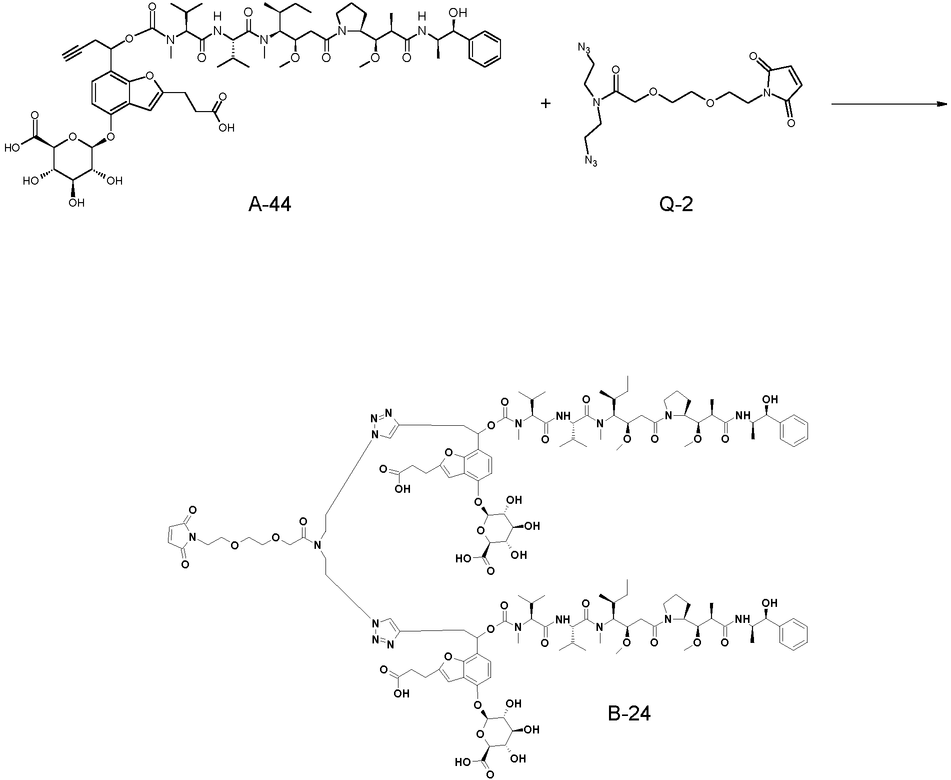

Compound A-44 (6.3 mg, 0.0053 mmol) was dissolved in DMSO (1 mL) under a nitrogen atmosphere at room temperature, and then 200 µL of a solution of Linker Q-2 (11.2 mg, 0.0294 mmol) dissolved in DMSO (1.12 mL) was taken and added to the reaction. Additionally, distilled water (2 mL), copper (II) sulfate pentahydrate (CuSO₄·5H₂O, 0.93 mg, 0.0058 mmol) and sodium ascorbate (1.9 mg, 0.0106 mmol) were sequentially added to the reaction solution, and then the mixture was stirred for 5 hours. After completion of the reaction, the reaction solution was diluted with distilled water (1 mL), purified using Preparative-HPLC, and then freeze-dried to obtain Compound B-24 in the form of a white solid (3.1 mg, 21 %); MS m/z: 2770[M+H]⁺, 1384 1/2[M+H]⁺, 923 1/3[M+H]⁺.

### Example II-24: Preparation of Compound B-25

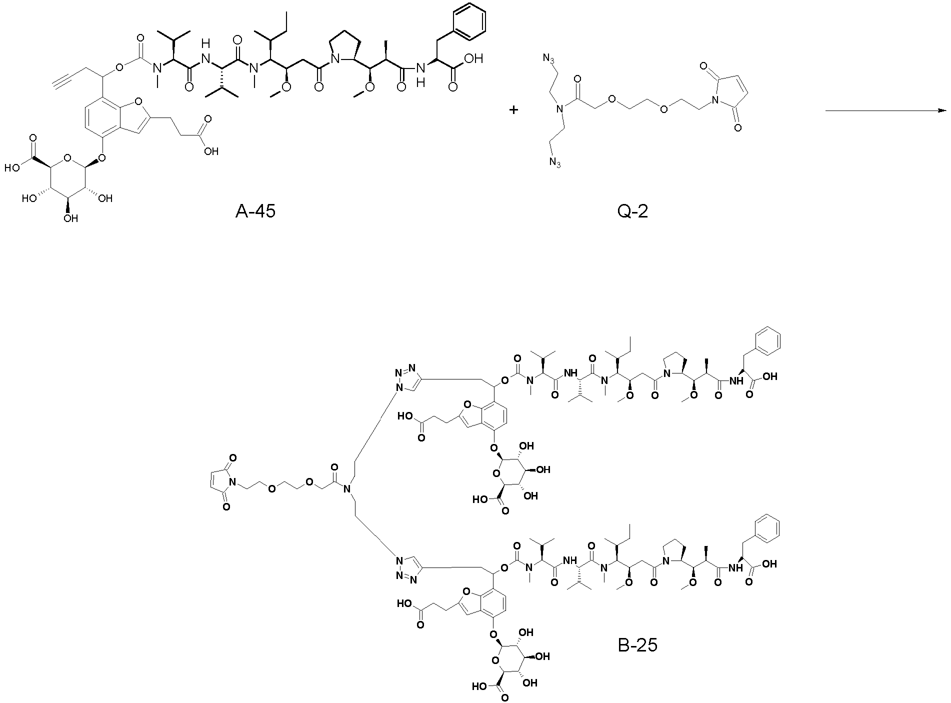

Compound A-45 (6.4 mg, 0.0053 mmol) was dissolved in DMSO (1 mL) under a nitrogen atmosphere at room temperature, and then 200 µL of a solution of Linker Q-2 (11.2 mg, 0.0294 mmol) dissolved in DMSO (1.12 mL) was taken and added to the reaction. Additionally, distilled water (2 mL), copper (II) sulfate pentahydrate (CuSO₄·5H₂O, 0.93 mg, 0.0058 mmol) and sodium ascorbate (1.9 mg, 0.0106 mmol) were sequentially added to the reaction solution, and then the mixture was stirred for 5 hours. After completion of the reaction, the reaction solution was diluted with distilled water (1 mL), purified using Preparative-HPLC, and then freeze-dried to obtain Compound B-25 in the form of a white solid (3.1 mg, 21 %); MS m/z: 2798[M+H]⁺, 1398 1/2[M+H]⁺, 932 1/3[M+H]⁺.

### Example II-25: Preparation of Compound B-26

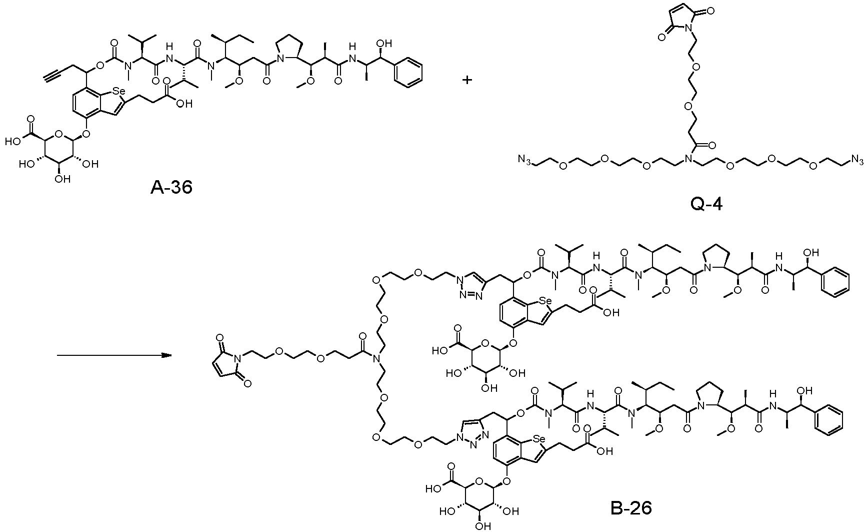

Compound A-36 (4.1 mg, 0.0032 mmol) was dissolved in DMSO (0.6 mL) under a nitrogen atmosphere at room temperature, and then 200 µL of a solution of Linker Q-4 (21.5 mg, mmol) dissolved in DMSO (2.15 mL) was taken and added to the reaction. Additionally, distilled water (2 mL), copper (II) sulfate pentahydrate (CuSO₄·5H₂O, 0.93 mg, 0.0058 mmol) and sodium ascorbate (1.9 mg, 0.0106 mmol) were sequentially added to the reaction solution, and then the mixture was stirred for 2 hours. After completion of the reaction, the reaction solution was diluted with distilled water (2 mL) , purified using Preparative-HPLC, and then freeze-dried to obtain Compound B-26 in the form of a white solid (2.4 mg, 46 %); EI-MS m/z: 1586 1/2[M+H]⁺, 1058 1/3[M+H]⁺.

### Example III: Preparation of antibody-drug conjugate (ADC)

Referring to the methods described in Nature Biotechnology, 2008, 26, 925-932; Bioconjugate Chem., 2013, 24, 1256-1263; Bioconjugate Chem., 2016, 27, 1324-1331; Bioconjugate Chem. 2014, 25, 460-469, etc., Compounds B-1 to B-9, B-11, B-10, B-19 and B-17 were each specifically bound to Herceptin substituted with a thiol group at a specific position to produce ADC-1 to ADC-13, respectively, as a thiomab drug conjugate (TDC). The structures of ADC-1 to ADC-13 are shown in Tables D and E. At this time, the ratio of drug bound to the antibody (DAR, drug-antibody ratio) was measured using HPLC. Herceptin (Trastuzumab) used in this example was purchased from Y Biologics Co., Ltd., and this was produced by substituting alanine at position 149 of the antibody light chain with cysteine through a genetic recombination method, transiently injecting DNA into HEK293 cells using transient transfection, and purifying the antibody secreted into the culture solution.

### Test Example 1: Kinetic study of enzymatic reaction rate using enzyme cleavage assay

### Method 1: E. coli β-galactosidase enzyme

The compounds of the present invention (A-5, A-8, A-9, A-11, A-12, A-14, A-15, A-16, A-19, A-21, A-23, A-24 and A-35) were each dissolved in DMSO (dimethylsulfoxide: Sigma, SA-276855-1L) to a concentration of 10 mM, and then mixed with a PBS (pH 7.4; Hyclone, SH30256.01) buffer solution to prepare a solution having a concentration of 500 µM (5% DMSO). MPS (Methylphenylsulfoxide: Alfa aesar, A15009-5G), used as a standard substance, was also prepared as a solution having a concentration of 500 µM in a PBS buffer solution. 415.8 µL of a PBS buffer solution and 140 µL of each of 500 µM compounds of the present invention and MPS were mixed, and then 4.2 µL of 3.36 mg/mL enzyme solution was added to prepare a total of 700 µL of enzymatic reaction solution. When comparing with human β-galactosidase, 21 µL of 1 mg/mL enzyme solution was added to achieve the same molar concentration, and 140 µL of each of the compound of the present invention and MPS were mixed with 399 µL of a PBS buffer solution. The reaction of all conditions was initiated in a constant temperature incubator at 37°C. *E. coli* β-galactosidase enzyme (Sigma, G4155) was used in the reaction mixture. The enzymatic reaction solution was aliquoted in an amount of 70 µL at 0 minutes before the reaction and at certain times after the reaction, and the remaining compounds of the present invention or MPS and substances liberated by the enzymatic reaction were quantitatively analyzed using the HPLC method.

### Method 2: Human β-galactosidase enzyme

Compound A-19 was dissolved in DMSO to a concentration of 10 mM and then mixed with a PBS buffer solution to prepare as a solution having a concentration of 500 µM (5% DMSO). MPS, used as a standard substance, was also prepared as a solution having a concentration of 500 µM in a PBS buffer solution. 200 µL of 50 mM sodium citrate buffer solution (pH 4.5) and 100 µL of each of 500 µM Compound A-19 of the present invention and MPS were mixed, and then 100 µL of 0.1 mg/mL enzyme solution was added to prepare a total of 500 µL of enzymatic reaction solution. The reaction of this solution was initiated in a constant temperature incubator at 37°C. Human β-galactosidase enzyme (R&D, 6464-GH-020) was used in the reaction mixture. The enzymatic reaction solution was aliquoted in an amount of 50 µL at 0 minutes before the reaction and at certain times after the reaction, and the remaining Compound A-19 or MPS and MMAF (monomethyl auristatin F) liberated by the enzymatic reaction were quantitatively analyzed using the HPLC method.

### Method 3: E. coli β-glucuronidase enzyme

The compound of the present invention (A-36) was dissolved in DMSO to a concentration of 10 mM and then mixed with a PBS buffer solution to prepare as a solution having a concentration of 500 µM (5% DMSO). MPS, used as a standard substance, was also prepared as a solution having a concentration of 500 µM in a PBS buffer solution. 406 µL of a PBS buffer solution (pH 7.4) and 140 µL of each of the 500 µM compound of the present invention and MPS were mixed, and then 14 µL of 1 mg/mL enzyme solution was added to prepare a total of 700 µL of enzymatic reaction solution. The reaction of this solution was initiated in a constant temperature incubator at 37°C. *E. coli* β-glucuronidase enzyme (Sigma G7396) was used in the reaction mixture. The enzymatic reaction solution was aliquoted in an amount of 70 µL at 0 minutes before the reaction and at certain times after the reaction, and the remaining compounds of the present invention or MPS and substances liberated by the enzymatic reaction were quantitatively analyzed using the HPLC method.

### Method 4: Human β-glucuronidase enzyme

The compound of the present invention (A-36) was dissolved in DMSO to a concentration of 10 mM and then mixed with a PBS buffer solution to prepare as a solution having a concentration of 500 µM (5% DMSO). MPS, used as a standard substance, was also prepared as a solution having a concentration of 500 µM in a PBS buffer solution. 200 µL of 50 mM sodium citrate buffer solution (pH 4.5) and 100 µL of each of the 500 µM compound of the present invention A-36 and MPS were mixed, and then 100 µL of 0.1 mg/mL enzyme solution was added to prepare a total of 500 µL of enzymatic reaction solution. The reaction of this solution was initiated in a constant temperature incubator at 37°C. Human β-glucuronidase enzyme (R&D Systems, 6144-GH-020) was used in the reaction mixture. The enzymatic reaction solution was aliquoted in an amount of 50 µL at 0 minutes before the reaction and at certain times after the reaction, and the remaining compound A-36 or MPS and MMAF (monomethyl auristatin F) liberated by the enzymatic reaction were quantitatively analyzed using the HPLC method.

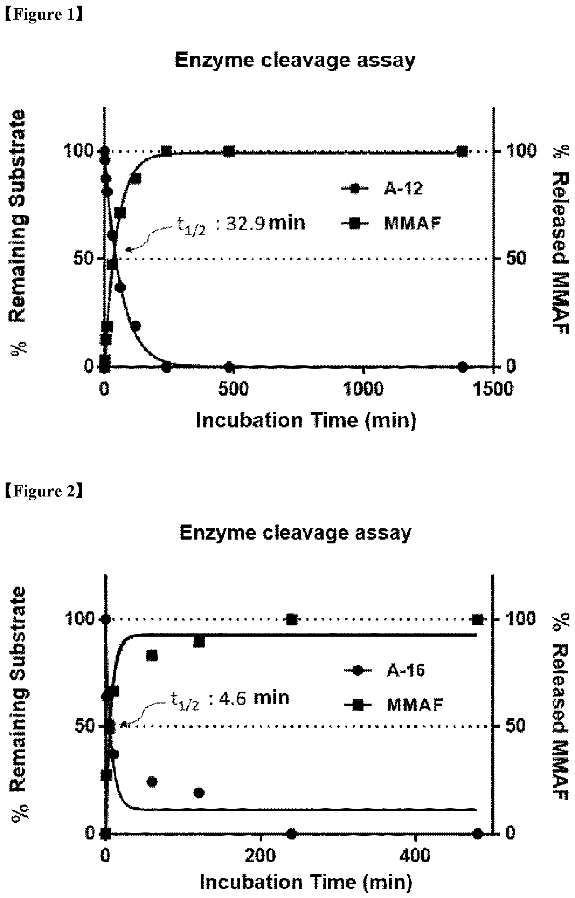

The results of measuring the payload release half-life of the compounds of the present invention using Methods 1, 2, 3 and 4 above are shown in Table 1. In addition, the results of measuring the enzyme cleavage rates of Compounds A-15, A-16 and A-19 are shown in Figures 1 to 3, respectively.

**[Table 1]**

| Compounds of the present invention | Payload release t_{1/2} (min) | Test method | pH condition |
|---|---|---|---|
| A-5 | 22.5 | Method 1 | 7.4 |
| A-8 | 4.8 | Method 1 | 7.4 |
| A-9 | 24.4 | Method 1 | 7.4 |
| A-11 | 43.2 | Method 1 | 7.4 |
| A-12 | 32.9 | Method 1 | 7.4 |
| A-14 | 15.7 | Method 1 | 7.4 |
| A-15 | 41.1 | Method 1 | 7.4 |
| A-16 | 4.6 | Method 1 | 7.4 |
| A-19 | 1.3 | Method 1 | 7.4 |
| | 6.4 | Method 2 | 4.5 |
| A-21 | 357.4 | Method 1 | 7.4 |
| A-23 | 27.5 | Method 1 | 7.4 |
| A-24 | 14.3 | Method 1 | 7.4 |
| A-35 | 31.5 | Method 1 | 7.4 |
| A-36 | 15.1 | Method 3 | 7.4 |
| | 152.3 | Method 4 | 4.5 |

From the results in Table 1 above and Figures 1 to 3, it was confirmed that the compounds of the present invention were rapidly degraded by β-galactosidase and efficiently released the payload.

### Test Example 2: Chemical stability

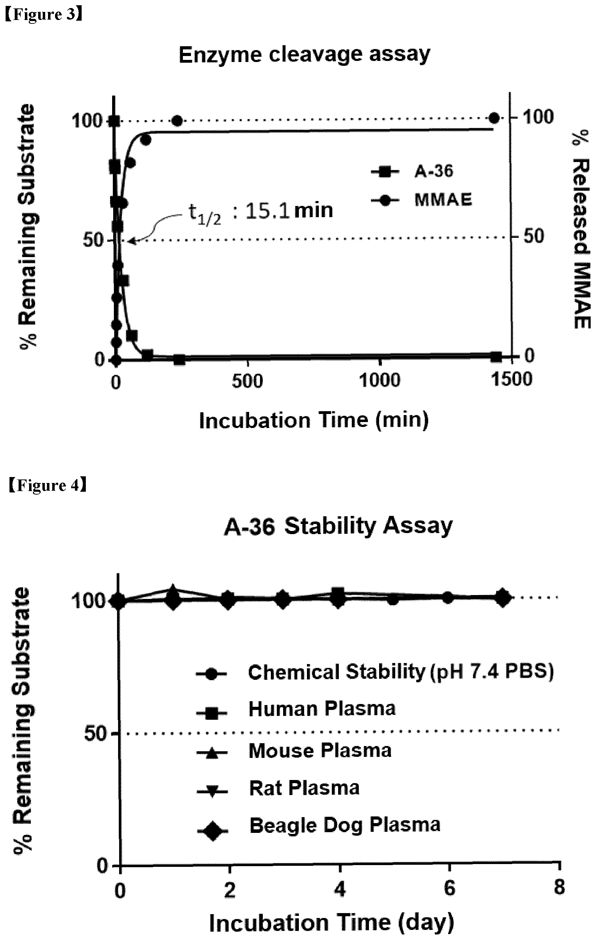

The compounds of the present invention (A-5, A-8, A-9, A-11, A-12, A-14, A-15, A-16, A-19, A-23, A-24, A-35 and A-36) were each dissolved in DMSO to a concentration of 10 mM and then mixed with a PBS buffer solution (pH 7.4) to prepare as a solution having a concentration of 500 µM (5% DMSO). MPS, used as a standard substance, was also prepared as a solution having a concentration of 500 µM in a PBS buffer solution. 420 µL of a PBS buffer solution and 140 µL of each of 500 µM compounds of the present invention and MPS were mixed to prepare a total of 700 µL of reaction solution. The reaction of the mixed solution was initiated in a constant temperature incubator at 37°C. The reaction solution was aliquoted in an amount of 70 µL at 0 minutes before the reaction and at certain times after the reaction, and the remaining compounds of the present invention or MPS or the liberated MMAF were quantitatively analyzed using the HPLC method. The results of measuring the half-life of the compounds of the present invention in a PBS buffer solution (pH 7.4) are shown in Table 2 below. In addition, the remaining amount of Compound A-19 in a PBS buffer solution (pH 7.4) according to the incubation time is shown in Figure 4.

**[Table 2]**

| Compounds of the present invention | Chemical stability t_{1/2} (day) | Test method |
|---|---|---|
| A-5 | > 7 | Method 1 |
| A-8 | > 7 | Method 1 |
| A-9 | > 7 | Method 1 |
| A-11 | > 7 | Method 1 |
| A-12 | > 7 | Method 1 |
| A-14 | > 7 | Method 1 |
| A-15 | > 7 | Method 1 |
| A-16 | > 7 | Method 1 |
| A-19 | > 7 | Method 1 |
| A-23 | > 7 | Method 1 |
| A-24 | > 7 | Method 1 |
| A-35 | > 7 | Method 1 |
| A-36 | > 7 | Method 1 |

Referring to Table 2 above and Figure 4, it was confirmed that the compounds of the present invention are chemically stable in a neutral buffer solution for a long time.

### Test Example 3: Plasma stability test

### Method 1: Mouse plasma stability

The compounds of the present invention (A-5, A-9, A-14, A-19, A-35, A-36) and MPS, used as a standard substance, were each dissolved in DMSO and a PBS buffer solution to a concentration of 30 mM, and then mixed in mouse plasma (Biochemed, 029-APSC-PMG) to a final concentration of 300 µM (final 1.0% DMSO) and 600 µM of the compounds and MPS, respectively. The reaction of the mixed solution in plasma was initiated in a constant temperature incubator at 37°C. Before the reaction and on certain days after the reaction, the above samples were aliquoted in an amount of 100 µL, and 200 µL of acetonitrile (ACN, Sigma, SA-271004-1L) was added to terminate the reaction, mixed, and then vortexed for about 1 minute, and then centrifuged (Beckman, B30150) at 4°C for 15 minutes at 14,000 rpm to precipitate plasma proteins. Each supernatant obtained by centrifugation was collected and quantitatively analyzed using the HPLC method.

### Method 2: Human plasma stability

The compounds of the present invention (A-8, A-9, A-14, A-19, A-35, A-36) and MPS, used as a standard substance, were each dissolved in DMSO and a PBS buffer solution to a concentration of 30 mM, and then mixed in human plasma (Biochemed, 752PR-SC-PMG) to a final concentration of 300 µM (final 1.0% DMSO) and 600 µM of the compounds and MPS, respectively. The reaction of the mixed solution in plasma was initiated in a constant temperature incubator at 37°C. Before the reaction and on certain days after the reaction, the above samples were aliquoted in an amount of 100 µL, and 200 µL of ACN was added to terminate the reaction, mixed, and then vortexed for about 1 minute, and then centrifuged at 4°C for 15 minutes at 14,000 rpm to precipitate plasma proteins. Each supernatant obtained by centrifugation was collected and quantitatively analyzed using the HPLC method.

### Method 3: Rat plasma stability

The compounds of the present invention (A-19, A-36) and MPS, used as a standard substance, were each dissolved in DMSO and a PBS buffer solution to a concentration of 30 mM, and then mixed in rat plasma (Biochemed, 031-APSC-PMG) to a final concentration of 300 µM (final 1.0% DMSO) and 600 µM of the compounds and MPS, respectively. The reaction of the mixed solution in plasma was initiated in a constant temperature incubator at 37°C. Before the reaction and on certain days after the reaction, the above samples were aliquoted in an amount of 100 µL, and 200 µL of ACN was added to terminate the reaction, mixed, and then vortexed for about 1 minute, and then centrifuged at 4°C for 15 minutes at 14,000 rpm to precipitate plasma proteins. Each supernatant obtained by centrifugation was collected and quantitatively analyzed using the HPLC method.

### Method 4: Beagle dog plasma stability

The compounds of the present invention (A-19, A-36) and MPS, used as a standard substance, were each dissolved in DMSO and a PBS buffer solution to a concentration of 30 mM, and then mixed in beagle dog plasma (Biochemed, 014-APSC-PMG) to a final concentration of 300 µM (final 1.0% DMSO) and 600 µM of the compounds and MPS, respectively. The reaction of the mixed solution in plasma was initiated in a constant temperature incubator at 37°C. Before the reaction and on certain days after the reaction, the above samples were aliquoted in an amount of 100 µL, and 200 µL of ACN was added to terminate the reaction, mixed, and then vortexed for about 1 minute, and then centrifuged at 4°C for 15 minutes at 14,000 rpm to precipitate plasma proteins. Each supernatant obtained by centrifugation was collected and quantitatively analyzed using the HPLC method.

The results of measuring the plasma half-life of the compounds of the present invention using Methods 1 to 4 above are shown in Table 3. In addition, the remaining amount of Compound A-19 in plasma according to the incubation time is shown in Figure 4.

**[Table 3]**

| Compounds of the present invention | Plasma stability t_{1/2} (day) | Test method |
|---|---|---|
| A-5 | > 7 | Method 1(mouse plasma) |
| A-8 | > 7 | Method 2(human plasma) |
| A-9 | > 7 | Method 1(mouse plasma) |
| | > 7 | Method 2(human plasma) |
| A-14 | > 7 | Method 1(mouse plasma) |
| | > 7 | Method 2(human plasma) |
| A-19 | > 7 | Method 1(mouse plasma) |
| | > 7 | Method 2(human plasma) |
| | > 7 | Method 3(rat plasma) |
| | > 7 | Method 4(beagle dog plasma) |
| A-35 | > 7 | Method 1(mouse plasma) |
| | > 7 | Method 2(human plasma) |
| A-36 | > 7 | Method 1(mouse plasma) |
| | > 7 | Method 2(human plasma) |
| | > 7 | Method 3(rat plasma) |
| | > 7 | Method 4(beagle dog plasma) |

Referring to Table 3 and Figure 4, it was confirmed that the compounds of the present invention are stable in plasma for a long time.

### Test Example 4: In vitro analysis of antibody-drug conjugate

### Method 1: Cell cytotoxicity (JIMT-1) experiment

JIMT-1 breast cancer cell line (DSMZ, ACC589) was prepared at a density of 40,000 cells per 1 mL of DMEM culture solution (Hyclone, SH30243.01) containing 10% fetal bovine serum (Hyclone, SH30919.03) and 1% antibiotic-antimycotic solution (Hyclone, SV30079.01), then dispensed into a 96-well plate (Sarstedt, 83.3924) at 100 mL (4,000 cells) per well, and cultured in a CO₂ constant temperature incubator (Forma, 51030303-TIF) for 24 hours. The cells were treated with 13 antibody-drug conjugates prepared in Example III, serially diluted in 1/3 from 41 nM to 0.0020 nM. In order to quantify living cells 96 hours later, 20 mL of MTS dye solution (Promega, G3581) was added to each well of the plate and left in a CO₂ constant temperature incubator for 4 hours. The absorbance of formazan formed by reduction of MTS (3-(4,5-dimethylthiazol-2-yl)-5-(3-carboxymethoxyphenyl)-2-(4-sulfophenyl)-2H-tetrazolium, inner salt) dye by mitochondrial oxidoreductase within the cell was measured at 450 nm using a spectrometer (Promega, GM3000) and then analyzed using the GraphPad Prism 7 program. The results of the cytotoxicity test of the antibody-drug conjugates are shown in Table 4 below.

### Method 2: Cell cytotoxicity (NCI-N87) experiment

NCI-N87 gastric cancer cell line (Korean Cell Line Bank, 60113) was prepared at a density of 100,000 cells per 1 mL of RPMI1640 culture solution (Hyclone, SH30027.01) containing 10% fetal bovine serum (Hyclone, SH30919.03) and 1% antibiotic-antimycotic solution (Hyclone, SV30079.01), then dispensed into a 96-well plate (Sarstedt, 83.3924) at 100 mL (10,000 cells) per well, and cultured in a CO₂ constant temperature incubator (Forma, 51030303-TIF) for 24 hours. The cells were treated with 8 antibody-drug conjugates prepared in Example III, serially diluted in 1/2 from 4.5 nM to 0.0088 nM. In order to quantify living cells 96 hours later, 20 mL of MTS dye solution (Promega, G3581) was added to each well of the plate and left in a CO₂ constant temperature incubator for 4 hours. The absorbance of formazan formed by reduction of MTS (3-(4,5-dimethylthiazol-2-yl)-5-(3-carboxymethoxyphenyl)-2-(4-sulfophenyl)-2H-tetrazolium, inner salt) dye by mitochondrial oxidoreductase within the cell was measured at 490 nm using a spectrometer (Promega, GM3000) and then analyzed using the GraphPad Prism 7 program. The results of the cytotoxicity test of the antibody-drug conjugates are shown in Table 4 below.

### Method 3: Cell cytotoxicity (SK-BR3) experiment

SK-BR3 breast cancer cell line (Korean Cell Line Bank, 30030) was prepared at a density of 80,000 cells per 1 mL of RPMI1640 culture solution (Hyclone, SH30027.01) containing 10% fetal bovine serum (Hyclone, SH30919.03) and 1% antibiotic-antimycotic solution (Hyclone, SV30079.01), then dispensed into a 96-well plate (Sarstedt, 83.3924) at 100 mL (8,000 cells) per well, and cultured in a CO₂ constant temperature incubator (Forma, 51030303-TIF) for 24 hours. The cells were treated with 4 antibody-drug conjugates prepared in Example III, serially diluted in 1/2 from 0.5 nM to 0.00098 nM. In order to quantify living cells 96 hours later, 20 mL of MTS dye solution (Promega, G3581) was added to each well of the plate and left in a CO₂ constant temperature incubator for 4 hours. The absorbance of formazan formed by reduction of MTS (3-(4,5-dimethylthiazol-2-yl)-5-(3-carboxymethoxyphenyl)-2-(4-sulfophenyl)-2H-tetrazolium, inner salt) dye by mitochondrial oxidoreductase within the cell was measured at 490 nm using a spectrometer (Promega, GM3000) and then analyzed using the GraphPad Prism 7 program. The results of the cytotoxicity test of the antibody-drug conjugates are shown in Table 4 below.

### Method 4: Cell cytotoxicity (BT-474) experiment

BT-474 breast cancer cell line (Korean Cell Line Bank, 60062) was prepared at a density of 100,000 cells per 1 mL of RPMI1640 culture solution (Hyclone, SH30027.01) containing 10% fetal bovine serum (Hyclone, SH30919.03) and 1% antibiotic-antimycotic solution (Hyclone, SV30079.01), then dispensed into a 96-well plate (Sarstedt, 83.3924) at 100 mL (10,000 cells) per well, and cultured in a CO₂ constant temperature incubator (Forma, 51030303-TIF) for 24 hours. The cells were treated with 4 antibody-drug conjugates prepared in Example III, serially diluted in 1/2 from 1.5 nM to 0.0029 nM. In order to quantify living cells 96 hours later, 20 mL of MTS dye solution (Promega, G3581) was added to each well of the plate and left in a CO₂ constant temperature incubator for 4 hours. The absorbance of formazan formed by reduction of MTS (3-(4,5-dimethylthiazol-2-yl)-5-(3-carboxymethoxyphenyl)-2-(4-sulfophenyl)-2H-tetrazolium, inner salt) dye by mitochondrial oxidoreductase within the cell was measured at 490 nm using a spectrometer (Promega, GM3000) and then analyzed using the GraphPad Prism 7 program. The results of the cytotoxicity test of the antibody-drug conjugates are shown in Table 4 below.

### Method 5: Cell cytotoxicity (MCF-7) experiment

MCF-7 breast cancer cell line (Korean Cell Line Bank, 30022) was prepared at a density of 40,000 cells per 1 mL of RPMI1640 culture solution (Hyclone, SH30027.01) containing 10% fetal bovine serum (Hyclone, SH30919.03) and 1% antibiotic-antimycotic solution (Hyclone, SV30079.01), then dispensed into a 96-well plate (Sarstedt, 83.3924) at 100 mL (4,000 cells) per well, and cultured in a CO₂ constant temperature incubator (Forma, 51030303-TIF) for 24 hours. The cells were treated with 4 antibody-drug conjugates prepared in Example III, diluted at a concentration of 50 nM and 3.125 nM. In order to quantify living cells 96 hours later, 20 mL of MTS dye solution (Promega, G3581) was added to each well of the plate and left in a CO₂ constant temperature incubator for 4 hours. The absorbance of formazan formed by reduction of MTS (3-(4,5-dimethylthiazol-2-yl)-5-(3-carboxymethoxyphenyl)-2-(4-sulfophenyl)-2H-tetrazolium, inner salt) dye by mitochondrial oxidoreductase within the cell was measured at 490 nm using a spectrometer (Promega, GM3000) and then analyzed using the GraphPad Prism 7 program. The results of the cytotoxicity test of the antibody-drug conjugates are shown in Table 4 below.

**[Table 4]**

| ADC / IC₅₀(nM) | DAR | JIMT-1 | NCI-N87 | SK-BR3 | BT-474 | MCF-7 |
|---|---|---|---|---|---|---|
| ADC-1 | 1.9 | 0.324 | | | | |
| ADC-2 | 1.9 | 0.267 | | | | |
| ADC-3 | 1.9 | 0.234 | | | | |
| ADC-4 | 1.8 | 0.249 | | | | |
| ADC-5 | 1.9 | 0.255 | | | | |
| ADC-6 | 3.6 | 0.153 | 0.128 | 0.023 | 0.052 | > 50 |
| ADC-7 | 3.7 | 0.160 | 0.156 | 0.024 | 0.065 | > 50 |
| ADC-8 | 3.7 | 0.172 | 0.148 | 0.030 | 0.062 | > 50 |
| ADC-9 | 3.4 | 0.260 | 0.202 | 0.033 | 0.074 | > 50 |
| ADC-10 | 1.9 | 0.262 | 0.262 | | | |
| ADC-11 | 1.9 | 0.475 | 0.278 | | | |
| ADC-12 | 3.8 | 0.112 | 0.129 | | | |
| ADC-13 | 3.8 | 0.121 | 0.137 | | | |

From the results in Table 4 above, the excellent anticancer activity of the antibody-drug conjugate of the present invention was confirmed through in vitro experiments.

### Test Example 5: In vivo analysis of antibody-drug conjugate

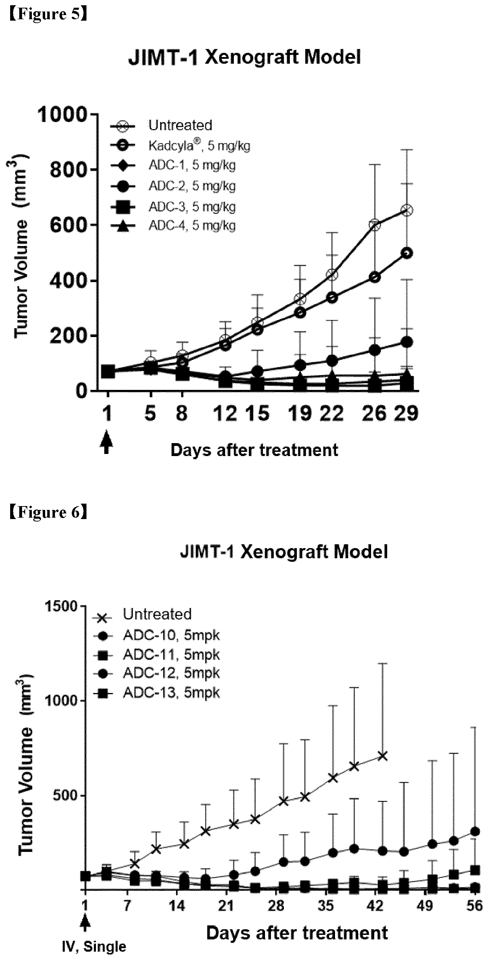

The *in vivo* activity of the antibody-drug conjugates (ADC-1 to ADC-4 and ADC-10 to ADC-13) prepared in Example III was measured in a tumor xenograft mouse model. JIMT-1 breast cancer cell line was cultured in DMEM culture solution containing 10% fetal bovine serum and 1% antibiotic-antimycotic solution (Gibco, 15240-062) at 37°C, 5% CO₂ conditions for 3 to 4 days. When the viability was 97% or higher, the cell line was harvested using trypsin-EDTA, then mixed with a DPBS (Hyclone, SH30028.02) buffer solution to obtain a concentration of 1.59 × 10⁸ cells/mL. Thereafter, this was diluted 1:1 with matrigel (Corning, 356235) to obtain a concentration of 7.14 × 10⁷ cells/mL. The cell suspension was injected subcutaneously into athymic nude mice (6-week, female) at a dose of 0.07 mL (5×10⁶ cells/mouse) using a disposable syringe (1 mL). When the tumor volume was 80 to 120 mm³, the mice were separated into groups such that the average tumor volume was distributed as evenly as possible and drug administration was then initiated. Kadcyla^{®} (T-DM1, Roche, CAS No. 1018448-65-1) and the antibody-drug conjugate of the present invention were each administered intravenously at a single dose of 5 mg/kg, and the tumor volume was measured twice a week at intervals of 3 to 4 days. The tumor volume was calculated by the following formula: volume = (a²b)/2, where a is the short diameter and b is the long diameter. All measurement results obtained in the experiments were statistically processed using SPSS (Version 27.0, IBM corporation, U.S.A.). The results of *in vivo* activity analysis of Kadcyla^{®}, used as a control drug, and the antibody-drug conjugate of the present invention are shown in Figures 5 and 6.

Referring to Figures 5 and 6, it was confirmed that the antibody-drug conjugates of the present invention, ADC-1 to ADC-4 and ADC-10 to ADC-13, exhibit significantly excellent anticancer activity compared to the control drug Kadcyla^{®} or the untreated control group.

### Test Example 6: In vivo pharmacokinetic (PK) test in rats

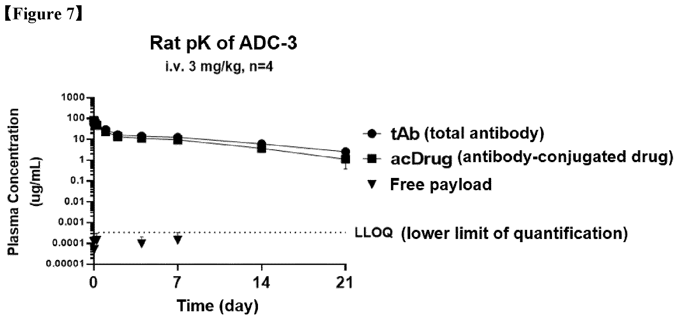

The following test was conducted to investigate the pharmacokinetic behavior of Herceptin and ADC-3 when a single intravenous administration of a test substance was given to rats. The test substances, Herceptin and ADC-3, were administered intravenously to female rats at a dose of 3.0 mg/kg, respectively. Blood samples were collected from the jugular vein at predetermined times (i.e., 3 minutes, 1 hour, 3 hours, 6 hours, 1 day, 2 days, 3 days, 4 days, 7 days, 9 days, 14 days, 17 days, and 21 days after administration) by using a 1 mL (25 gauge) syringe treated with heparin (85 IU/mL, 35 µL). 0.4 mL of blood was collected in a microtube, rolled in a mixer for several minutes, and then centrifuged at 14,000 rpm for 5 minutes to separate the plasma. The separated plasma was placed in microtubes and stored in a deep freezer for analysis. Measurement of the test substance in plasma was performed by LC-MS. The results are shown in Figure 7. The test results confirmed that ADC-3 is very stable in the blood of a living organism, with the antibody and the drug well bound.

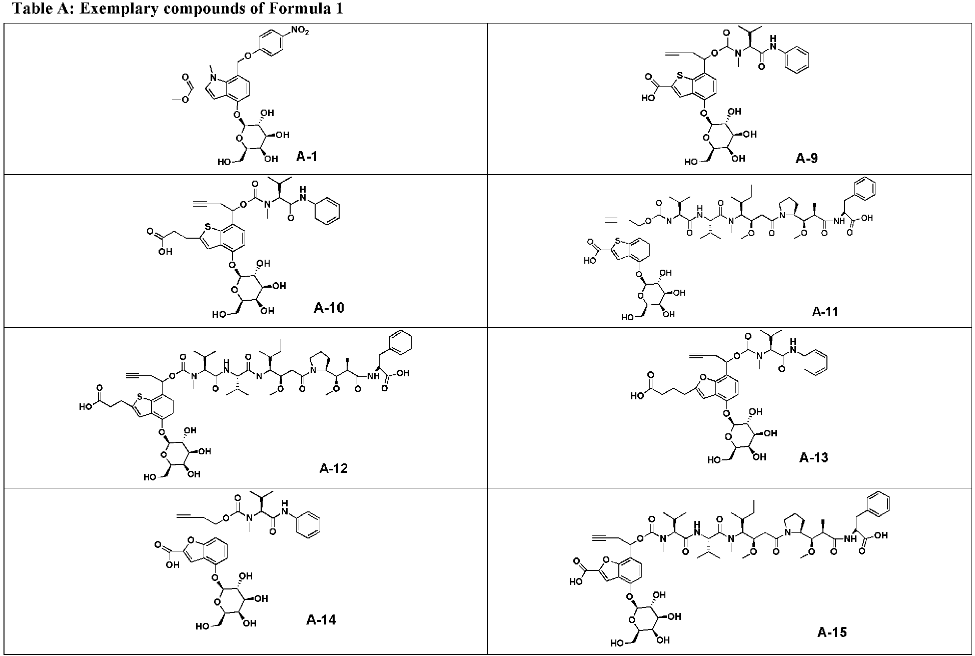

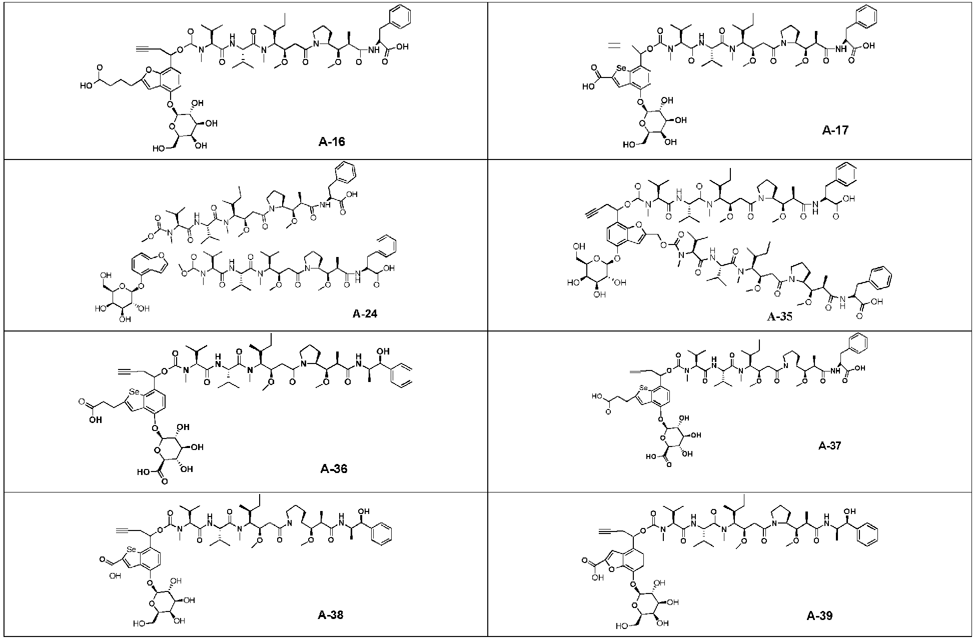

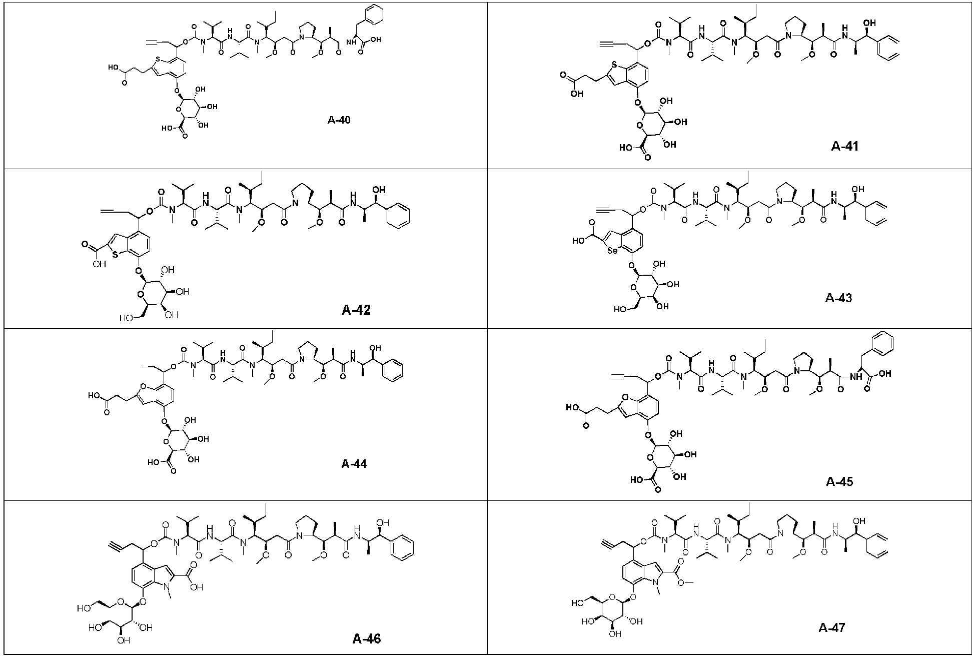

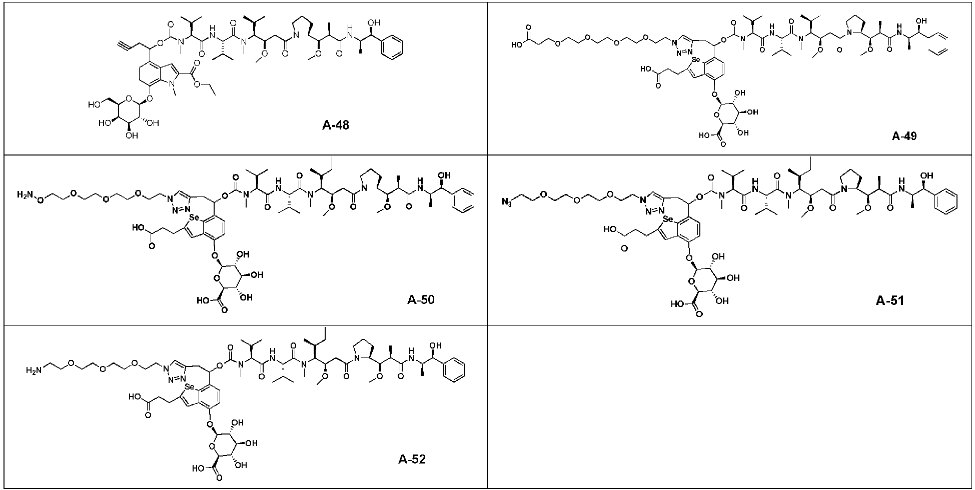

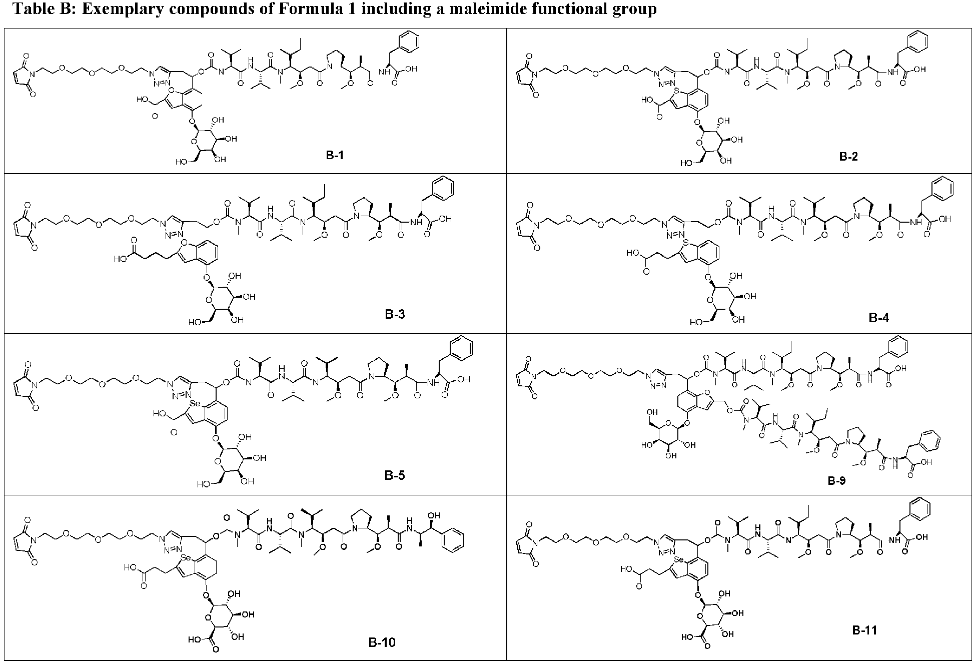

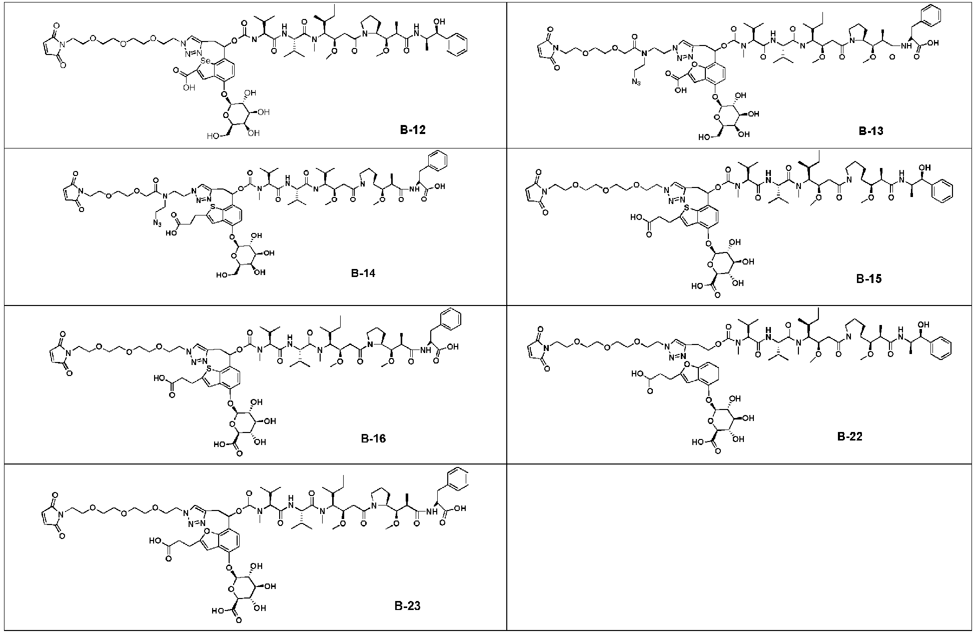

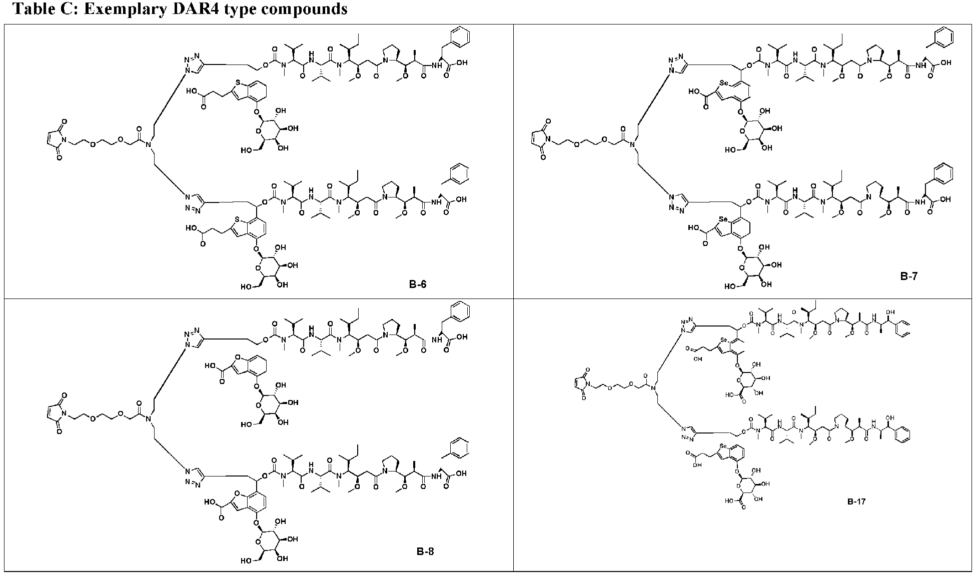

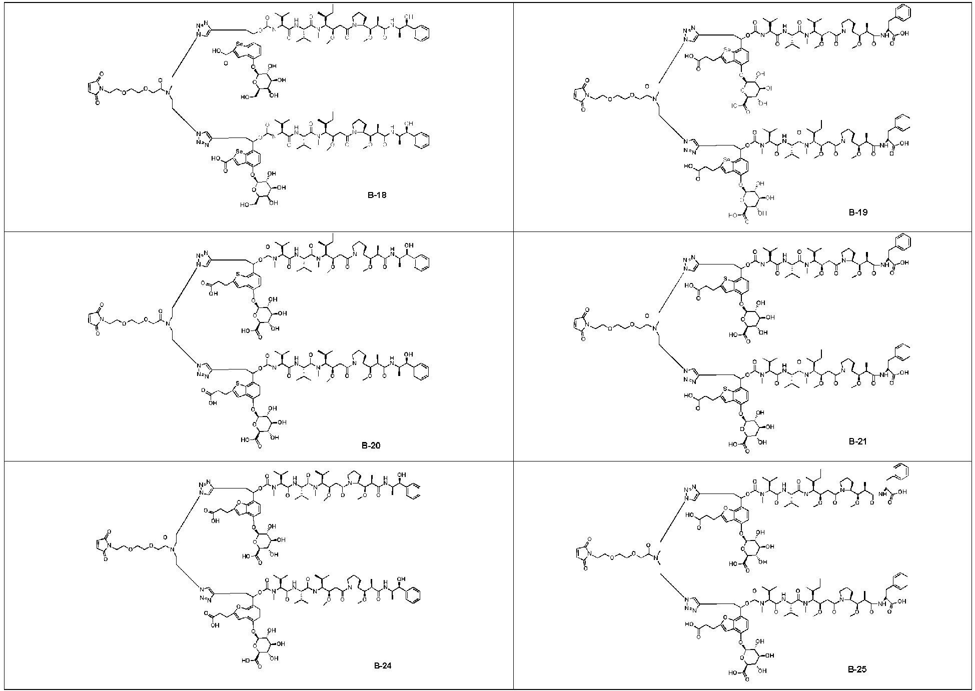

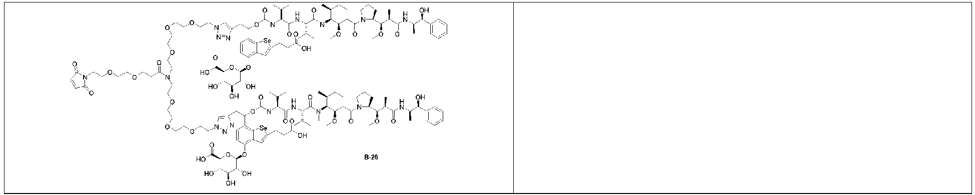

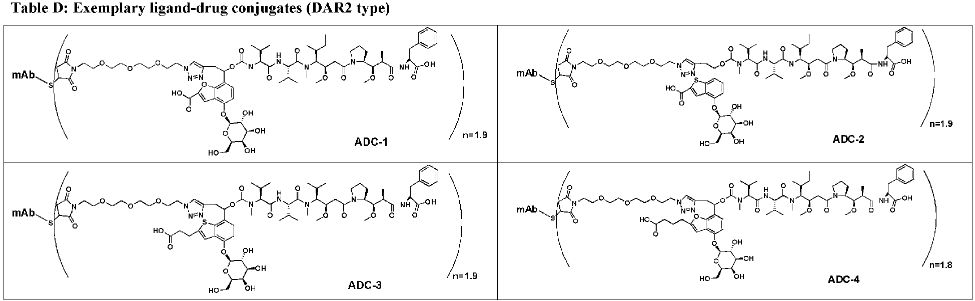

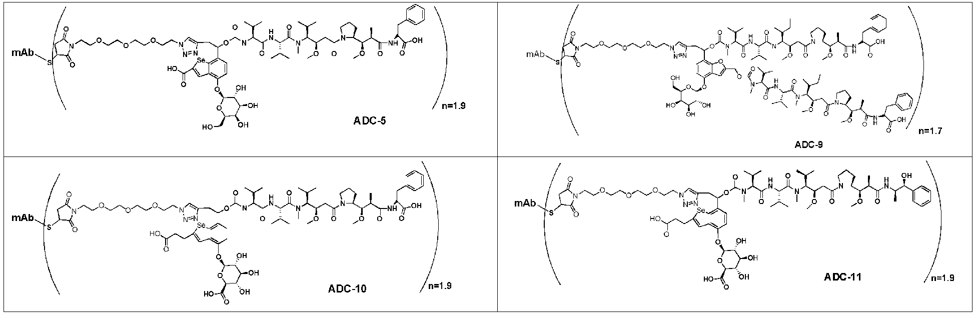

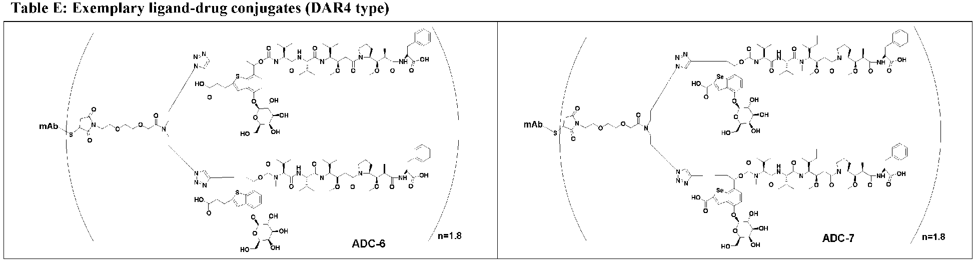

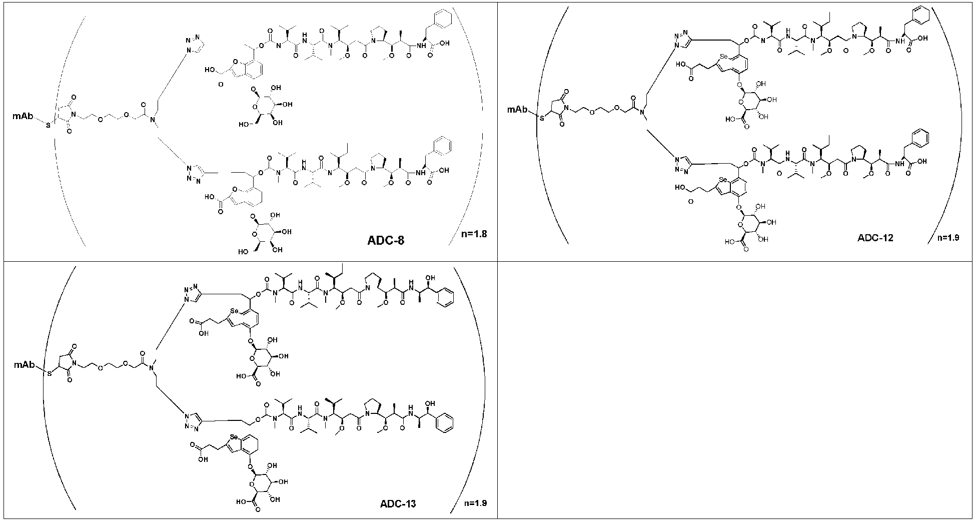

## Claims

1. A linker compound represented by Formula 1 below, or a pharmaceutically acceptable salt thereof:
[Formula 1] **A-(L¹)ₖ-Uⱼ**
wherein
L¹ is a divalent or multivalent linking group;
k is 0 or 1, and j is 1 to 10;
A is absent, H or a binding functional group; and
U is a moiety represented by Formula A below: 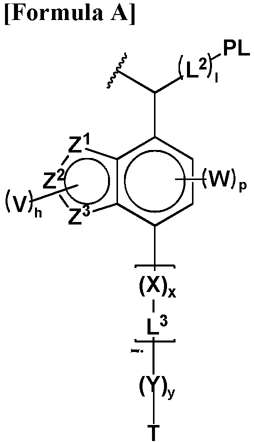 wherein
PL is an active agent linked to L² or the carbon atom to which the benzene ring and A-(L¹)ₖ- are bound, via a heteroatom selected from N, O and S;
L² is a self-eliminating linker selected such that cleavage of the bond between L² and the carbon atom to which the benzene ring and A-(L¹)ₖ- are bound promotes cleavage of the bond between L² and PL;
W is an optional substituent on the benzene ring;
one of Z¹ and Z³ is selected from the group consisting of NR³, O, S and Se, and the other one of Z¹ and Z³ and Z² are each independently CH or N, and -(V)ₕ, if present, each independently replaces H of NH or CH; 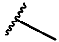
represents a bond with A-(L₁)ₖ-;
R³ is H or C₁-C₈ hydrocarbyl;
V is an electron withdrawing group, an electron donating group, or -CH₂-(L²)₁-PL-;
T is a triggering group capable of initiating the release of PL and L₂, if present, through a 1,6-elimination reaction upon cleavage;
L³ is an optional self-immolative spacer group that, if present, is cleaved sequentially upon cleavage of T;
X and Y are each independently selected from -O-, -NH-, and S; and
h, i, l, x and y are each independently 0 or 1, and p is an integer from 0 to 2.

2. The linker compound or a pharmaceutically acceptable salt thereof, according to claim 1, wherein
the ring 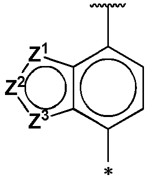 in Formula A above is selected from the following group:

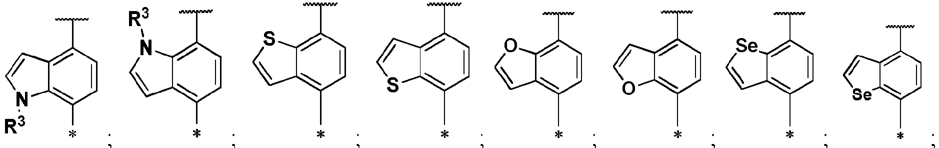

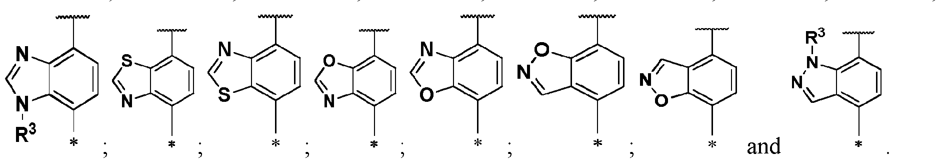

3. The linker compound according to claim 1, wherein
V is selected from the group consisting of halogen, CN, NO₂, formyl, C₁-C₈ alkylcarbonyl, carboxy, C₁-C₈ alkoxycarbonyl, carbamoyl, mono-C₁-C₈ alkylcarbamoyl, di-C₁-C₈ alkylcarbamoyl, C₁-C₈ alkyl, C₁-C₈ alkenyl, OH, C₁-C₈ alkoxy, SH, C₁-C₈ alkylsulfanyl, NH₂, mono-C₁-C₈ alkylamino, di-C₁-C₈ alkylamino, C₆-C₁₈ aryl, -CH₂-PL and -CH₂-L²-PL.

4. The linker compound according to claim 1, wherein
W is selected from the group consisting of H, C₁-C₁₂ saturated or unsaturated hydrocarbyl, halogen, halo-C₁-C₈ alkyl, CN, NO₂, OH, C₁-C₈ alkoxy, hydroxy-C₁-C₈ alkyl, C₁-C₈ alkoxy- C₁-C₈ alkyl, SH, C₁-C₈ alkylthio, mercapto-C₁-C₈ alkyl, amino, mono-C₁-C₈ alkylamino, di-C₁-C₈ alkylamino, amino-C₁-C₈ alkyl, C₁-C₈ monoalkylamino-C₁-C₈ alkyl, C₁-C₈ dialkylamino-C₁-C₈ alkyl, carboxy, C₁-C₈ alkoxycarbonyl, C₁-C₈ alkoxycarbonyloxy, carboxy-C₁-C₈ alkyl, C₁-C₈ alkoxycarbonyl-C₁-C₈ alkyl, carbamoyl, mono-C₁-C₈ alkylcarbamoyl, di-C₁-C₈ alkylcarbamoyl, carbamoyl-C₁-C₈ alkyl, mono-C₁-C₈ alkylcarbamoyl-C₁-C₈ alkyl and di-C₁-C₈ alkylcarbamoyl-C₁-C₈ alkyl.

5. The linker compound according to claim 1, wherein
-(Y)_{y-}T is selected from the group consisting of moieties represented by 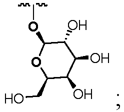 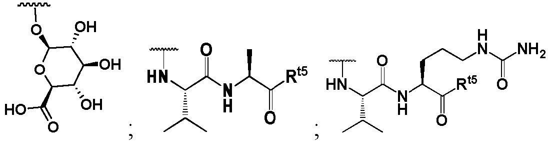 ; -O-SO₃⁻; -NO₂; -OC(O)(CH₂)ᵣCOR^{t1}; - O(CH₂)-Ar¹-NO₂; -S-C(O)(CH₂)ₛCOR^{t2}; -S(CH₂)-Ar²-NO₂ and -BR^{t3}R^{t4};
wherein 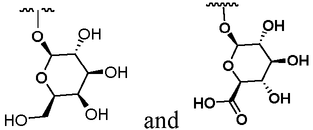 include a form in which the -OH group is protected by a protecting group or substituted with a substituent;
R^{t1} and R^{t2} are each C₁-C₈ alkyl, and Ar¹ and Ar² are each C₅-C₂₀ arylene or heteroarylene;
R^{t3} and R^{t4} are each independently hydrogen, C₁-C₈ alkoxy or hydroxy;
R^{t5} is OH, mono-C₁-C₈ alkylamino, di-C₁-C₈ alkylamino or -NH(CH₂CH₂O)_{f}R^{t6}, wherein R^{t6} is H or C₁-C₄ alkyl;
f is an integer from 1 to 10, and r and s are each an integer from 1 to 5.

6. The linker compound according to claim 1, wherein
-(X)ₓ-L³- is 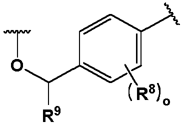 , and
R⁸ and R⁹ are each independently selected from the group consisting of H, halogen, C₁-C₈ alkyl, CN and NO₂, and o is an integer from 0 to 2.

7. The linker compound according to claim 1, wherein
PL is an active agent selected from a drug, a toxin, a fluorophore, an affinity ligand, a diagnostic substance or a detection probe.

8. The linker compound according to claim 7, wherein
the drug is selected from cytokines, immunomodulatory compounds, anticancer agents, antiviral agents, antibacterial agents, antifungal agents, anthelmintic agents or a combination thereof.

9. The linker compound according to claim 1, wherein
PL is selected from the group consisting of:

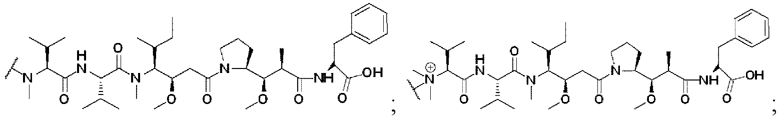

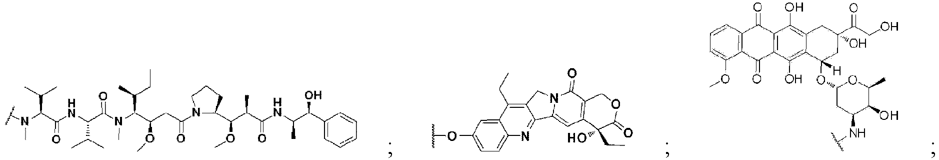

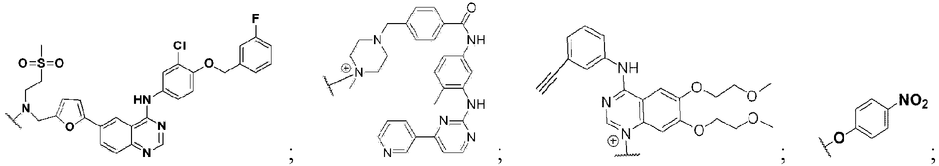

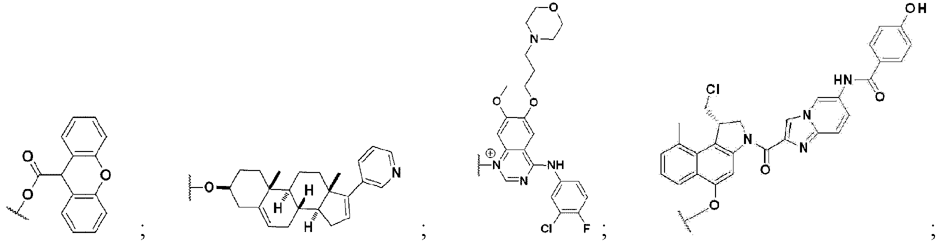

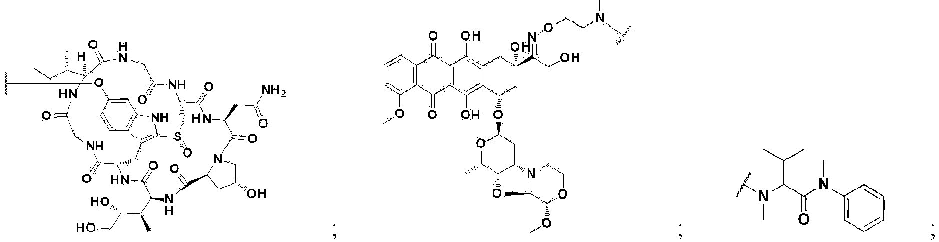

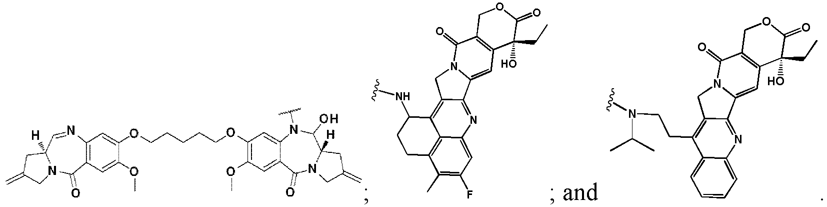

10. The linker compound according to claim 1, wherein
L² is selected from the group consisting of -OC(=O)-, -S(=O)₂-, 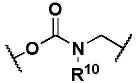 and 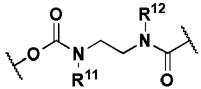 , and
R¹⁰ to R¹² are each independently H, C₁-C₈ alkyl, amino-C₁-C₈ alkyl, C₁-C₈ alkyl substituted with mono- or di-(C₁-C₈ alkyl)amino, or -(CH₂CH₂O)_{g}R¹³, wherein
R¹³ is H or C₁-C₄ alkyl, and g is an integer from 1 to 10.

11. The linker compound according to claim 1, wherein
the binding functional group is a functional group capable of binding to a ligand or protein having a receptor binding property, or a linker precursor, by a click chemistry reaction.

12. The linker compound according to claim 1, wherein
the binding functional group includes a functional group selected from the group consisting of halogen, OH, C₁-C₈ alkoxy, hydroxylamino, COH, C₁-C₈ alkylcarbonyl, carboxy, C₁-C₈ alkoxycarbonyl, tosyl, tosylate, amino, mono-C₁-C₈ alkylamino, di-C₁-C₈ alkylamino, NHNH₂, N₃, haloacetamide, maleimidyl, succinimidyl, SH, SO₃H, C₁-C₈ alkylsulfonyl, 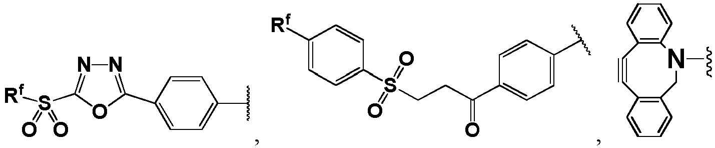 , C₁-C₈ alkoxysulfonyl, 2-pyridyl disulfide, PO₃H₂, OPO₃H₂, -N≡C, -NCS, C₄-C₁₀ dienyl, C₂-C₈ alkenyl, C₂-C₈ alkynyl, C₄-C₁₀ cycloalkynyl and C₂-C₈ alkynylcarbonyl, and
R^{f} is H or C₁-C₈ alkyl.

13. The linker compound according to claim 1, wherein
A-L¹ is formed by binding between the precursor of A and the precursor of L¹, the precursor of A includes at least one functional group selected from the group consisting of hydroxy, amino, azido, alkynyl, conjugated dienyl, alkenyl, cyclooctynyl, maleimidyl, SO₂N₃, alkoxysulfinyl, oxiranyl, aziridinyl, oxo, hydrazinyl, hydroxyamino, mercapto and 1,3-dicarbonyl, and the precursor of L¹ includes a functional group that chemically reacts with the precursor of A to form an A-L¹ bond.

14. The linker compound according to claim 1, wherein
L¹ is a C₁-C₂₀₀ alkylene optionally comprising a divalent or multivalent functional group selected from the group consisting of amide, sulfonamide, amino, ether, carbonyl, triazole, tetrazole, a sugar-derived group, sulfo ester and dendrimer in the middle of the chain.

15. The linker compound according to claim 1, wherein
L¹ includes any one selected from the group consisting of -(CH₂)ₙₐ-; -(CH₂CH₂O)ₘₐ-; - (CH₂OCH₂)_{mb}-; -(OCH₂CH₂)_{mc}-; -C(=O)-; 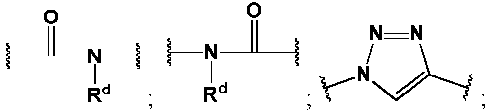 and 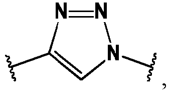 or a combination thereof, and
R^{d} is H or C₁-C₈ alkyl, and
na and ma to mc are each independently an integer from 0 to 10.

16. The linker compound according to claim 1, wherein
A-L¹- is selected from the group consisting of R^{a1}C≡C-(CH₂)ₙ₁-; N₃-(CH₂)ₙ₂-; 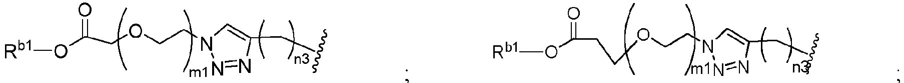 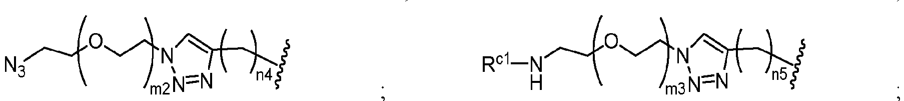 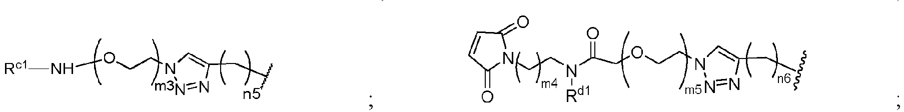 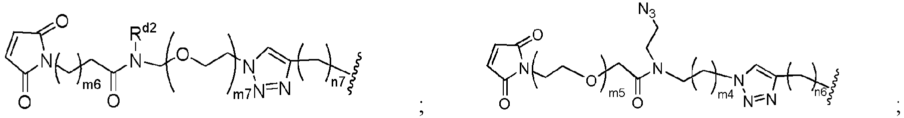 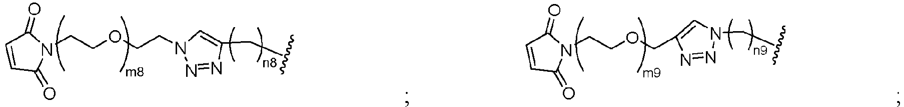 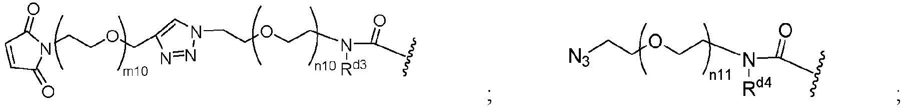 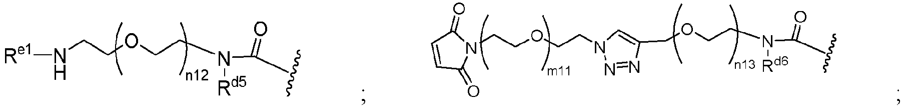 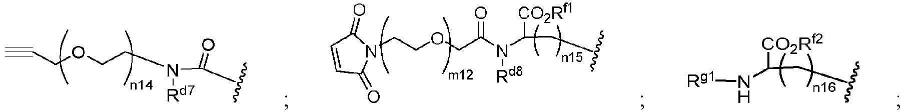 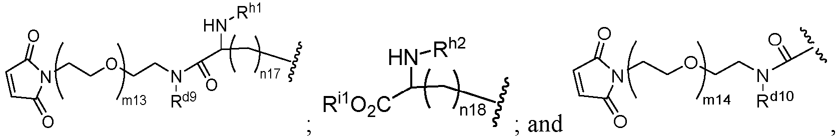
R^{a1}, R^{b1}, R^{f1}, R^{f2}, R^{h1}, R^{h2}, Rⁱ¹ and R^{d1} to R^{d10} are each independently H or C₁-C₈ alkyl, and
R^{c1}, R^{e1}, R^{g1}, R^{h1} and R^{h2} are each independently H, C₁-C₈ alkyl, C₁-C₈ alkylcarbonyl, C₁-C₈ alkoxycarbonyl, or C₆-C₁₂ aryl-C₁-C₄ alkoxycarbonyl, ,
n1 to n18 and m1 to m14 are each independently an integer from 0 to 10.

17. The linker compound according to claim 1, wherein
the compound represented by Formula 1 is selected from the group consisting of compounds represented by the following formulas: 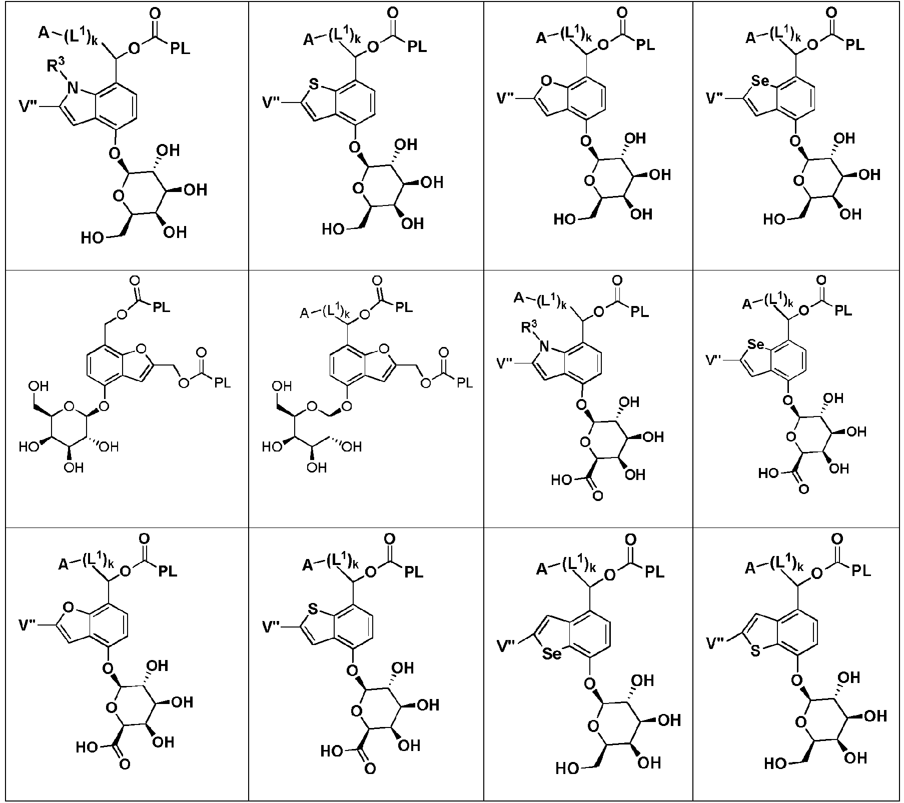 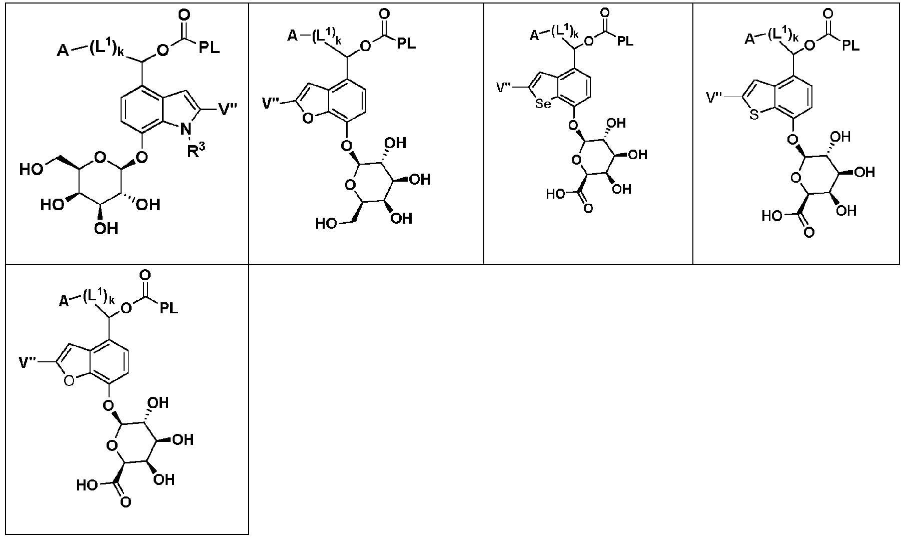
wherein A, L¹, k, R³ and PL are as defined in claim 1, and V" indicates an electron withdrawing group.

18. The linker compound according to claim 1, wherein
Formula A is represented by Formula A-1 below: 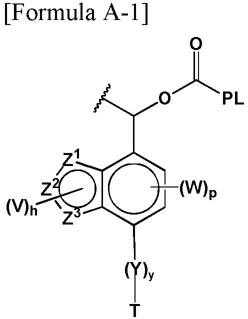
wherein the definitions of Z¹, Z², Z³, V, Y, T, W, PL, h, y and p are as described in claim 1.

19. The linker compound according to claim 1, wherein
the compound represented by Formula 1 is a compound represented by Formula 1-1 below: 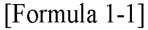 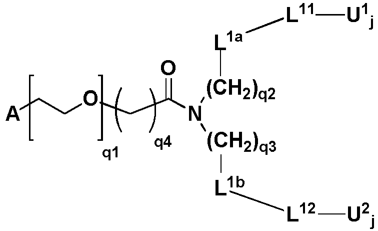 wherein
A has the same meaning as A defined in claim 1;
U¹ and U² each have the same meaning as U defined in claim 1, wherein U¹ and U² are the same as or different from each other;
j is 1 to 10;
L¹¹ and L¹² each have the same meaning as L¹ defined in claim 1, wherein L¹¹ and L¹² are the same as or different from each other;
L^{1a} and L^{1b} are each independently selected from a direct bond; 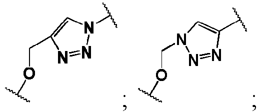 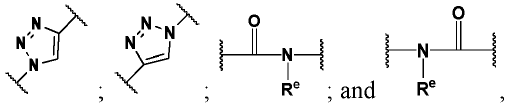 and R^{e} is H or C₁-C₈ alkyl;
q1, q2 and q3 are each independently an integer from 0 to 10, and q4 is an integer from 1 to 10;
provided that, if L^{1a} is 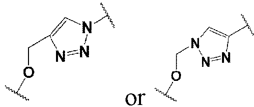 , q2 is not 0, and if L^{1b} is 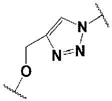 or 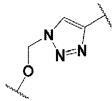 , q3 is not 0.

20. The linker compound according to claim 19, wherein 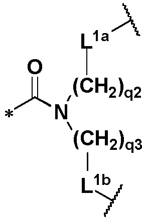 in Formula 1-1 above is selected from the following structures: 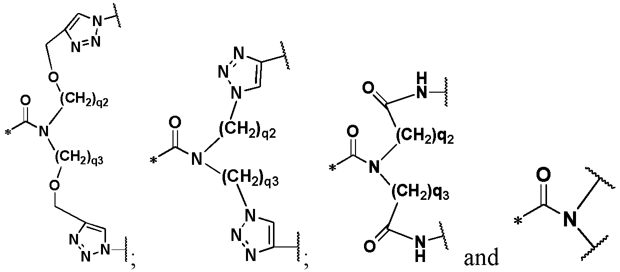 wherein the definitions of q2 and q3 are as defined in claim 19.

21. The linker compound according to claim 19, wherein
L¹¹ and L¹² are each independently selected from -(CH₂)ₙ₁-; -(CH₂CH₂O)ₘ₁-(CH₂)ₙ₂-; - (CH₂CH₂O)ₘ₂-(CH₂)ₙ₃-NR^{d1}CO-(CH₂)ₙ₄-; -(CH₂CH₂O)ₘ₁₀-(CH₂)ₙ₁₅-CONR^{d5}-(CH₂)ₙ₁₄-; and 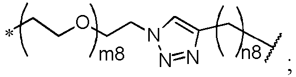
R^{d1} and R^{d5} are each independently H or C₁-C₈ alkyl, and
n1, n2, n3, n4, n8, n14 and n15, and m1, m2, m8 and m10 are each independently an integer from 1 to 8.

22. The linker compound according to claim 19, wherein
the compound represented by Formula 1-1 is selected from the group consisting of compounds represented by the following formulas: 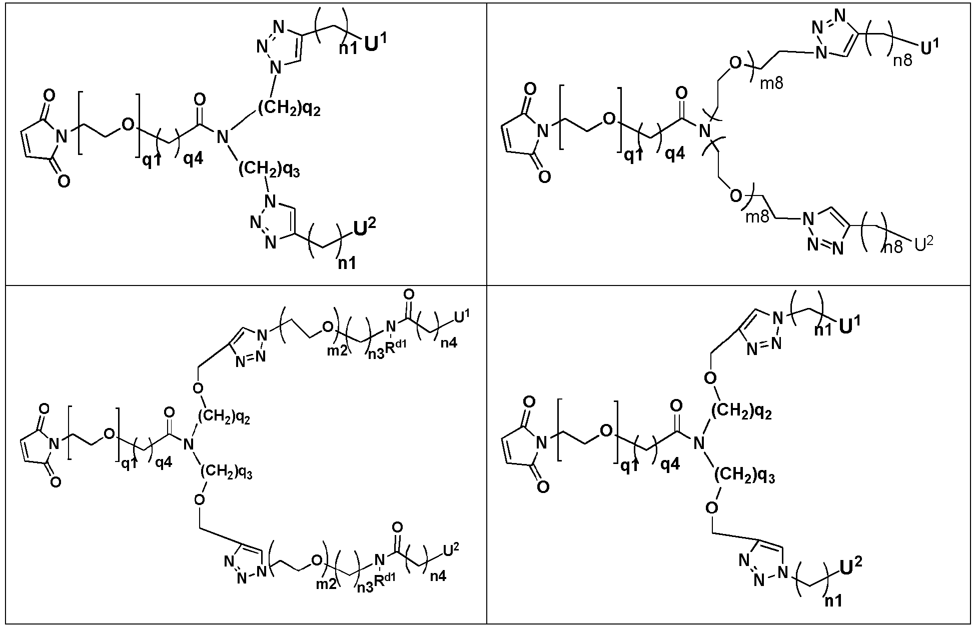 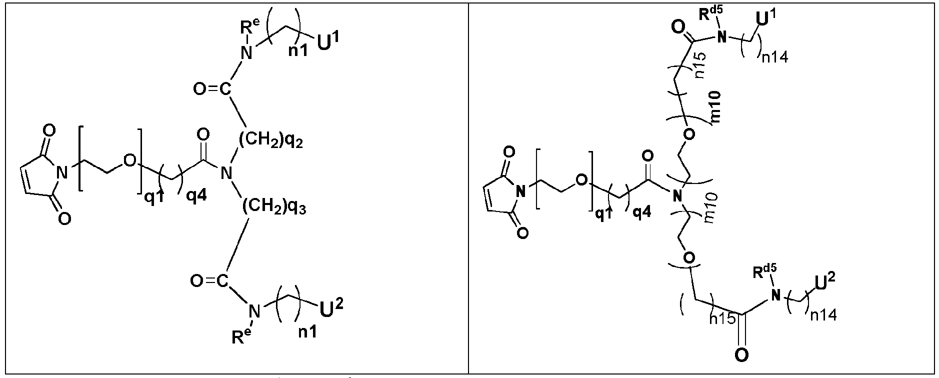
wherein q1 to q4, U¹ and U² are as defined in claim 19,
R^{d1}, R^{d5} and R^{e} are each independently H or C₁-C₈ alkyl, and
n1, n3, n4, n8, n14 and n15, m2, m8 and m10 are each independently an integer from 1 to 8.

23. A linker compound represented by Formula B below, or a pharmaceutically acceptable salt thereof: 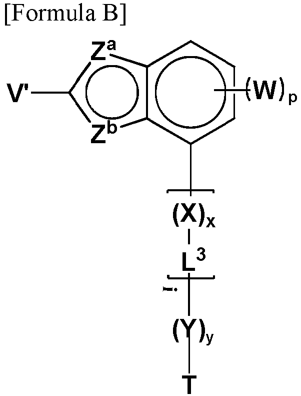 wherein
V' is -CH₂-(L²)₁-PL;
L² is a self-eliminating linker selected such that cleavage of the bond between the -CH₂-of V' and L² promotes cleavage of the bond between L² and PL;
PL is an active agent linked to L² or -CH²- of V' via a heteroatom selected from N, O and S;
one of Z^{a} and Z^{b} is selected from the group consisting of N, NR³, O, S and Se, the other one of Z^{a} and Z^{b} is CH or N;
R³ is H or C₁-C₈ hydrocarbyl;
T is a triggering group capable of initiating the release of PL and L₂, if present, through a 1,6-elimination reaction upon cleavage;
L³ is an optional self-immolative spacer group that, if present, is cleaved sequentially upon cleavage of T;
X and Y are each independently selected from -O-, -NH-, and S; and
i, l, x and y are each independently 0 or 1, and p is an integer of 0 to 2.

24. The linker compound according to claim 23, wherein
the compound is selected from the group consisting of the compounds represented by the following formulas:

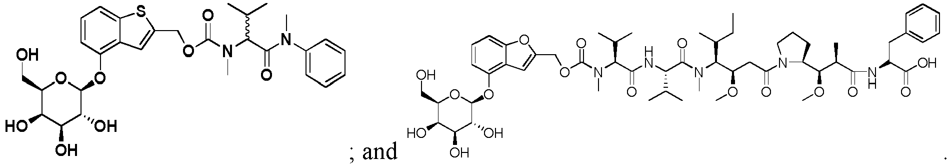

25. A ligand-drug conjugate represented by Formula 2 below, or a pharmaceutically acceptable salt thereof: 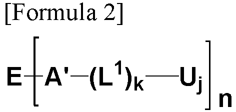 wherein
E is a ligand or protein having a receptor binding property;
A' is a divalent linking group derived from the binding functional group of A;
n is a real number from 1 to 10; and
the definitions of A, U, L¹, k and j are as defined in claim 1.

26. The ligand-drug conjugate according to claim 25, wherein
the ligand is selected from the group consisting of peptides, tumor cell-specific peptides, tumor cell-specific aptamers, tumor cell-specific carbohydrates, tumor cell-specific monoclonal or polyclonal antibodies, and antibody fragments; and
the protein is selected from the group consisting of C₁-C₂₀ hydrocarbyl, oligopeptide, polypeptide, fragment of antigenic polypeptide, and artificial antibody (Repebody).

27. The ligand-drug conjugate according to claim 26, wherein
the antibody is selected from the group consisting of an intact polyclonal antibody, an intact monoclonal antibody, an antibody fragment, a single chain Fv (scFv) mutant, a multispecific antibody, a bispecific antibody, a chimeric antibody, a humanized antibody, a human antibody, a fusion protein comprising an antigen determination portion of an antibody, and a modified immunoglobulin molecule comprising an antigen recognition site.

28. The ligand-drug conjugate according to claim 26, wherein
the antibody is selected from the group consisting of Muromonab-CD3, Abciximab, Rituximab, Daclizumab, Palivizumab, Infliximab, Trastuzumab (herceptin), Etanercept, Basiliximab, Gemtuzumab, Alemtuzumab, Ibritumomab, Adalimumab, Alefacept, Omalizumab, Efalizumab, Tositumomob-I131, Cetuximab, Bevacizumab, Natalizumab, Ranibizumab, Panitumumab, Eculizumab, Rilonacept, Certolizumab pegol, Romiplostim, AMG-531 (Romiplostim), CNTO-148 (Golimumab), CNTO-1275 (Ustekinumab), ABT874 (Briakinumab), LEA-29Y (Belatacept), Belimumab, TACI-Ig (transmembrane activator and calcium modulator and cyclophilin ligand interactor-immunoglobulin), second generation anti-CD20, ACZ-885 (Canakinumab), Tocilizumab, Atlizumab, Mepolizumab, Pertuzumab, Humax CD20 (Ofatumumab), Tremelimumab (CP-675 206), Ticilimumab, MDX-010 (Ipilimumab), IDEC-114 (Galiximab), Inotuzumab, HuMax EGFR (Zalutumumab), Aflibercept (VEGF Trap-Eye), HuMax-CD4 (Zanolimumab), Ala-Ala (hOKT3gamma1), Otelixizumab (ChAglyCD3; TRX4), Catumaxomab, MT-201 (Adecatumumab), Pregovomab, CH-14.18 (Dinutuximab), WXG250 (Girentuximab), AMG-162 (Denosumab), AAB-001 (Bapineuzumab), Motavizumab, MEDI524 (Motavizumab), Efumgumab, Aurograb^{®}, Raxibacumab, third generation anti-CD20, LY2469298 (Ocaratuzumab), and Veltuzumab.

29. The ligand-drug conjugate according to claim 25, wherein
E is an antibody, and the E-A' binding structure in Formula 2 includes 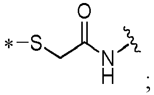 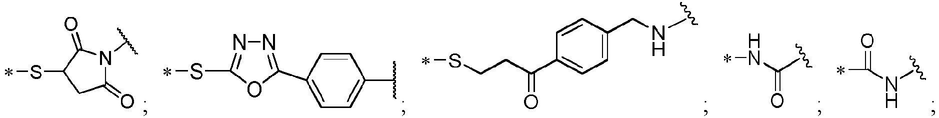 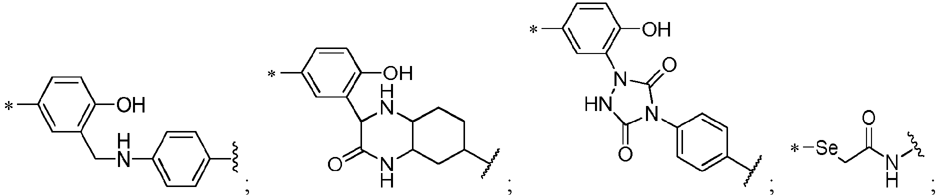 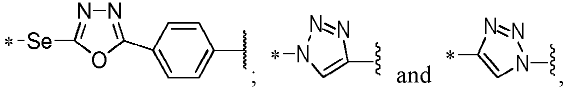 wherein * is the remaining moiety of the antibody.

30. The ligand-drug conjugate according to claim 25, wherein
the conjugate represented by Formula 2 is selected from the following conjugates: 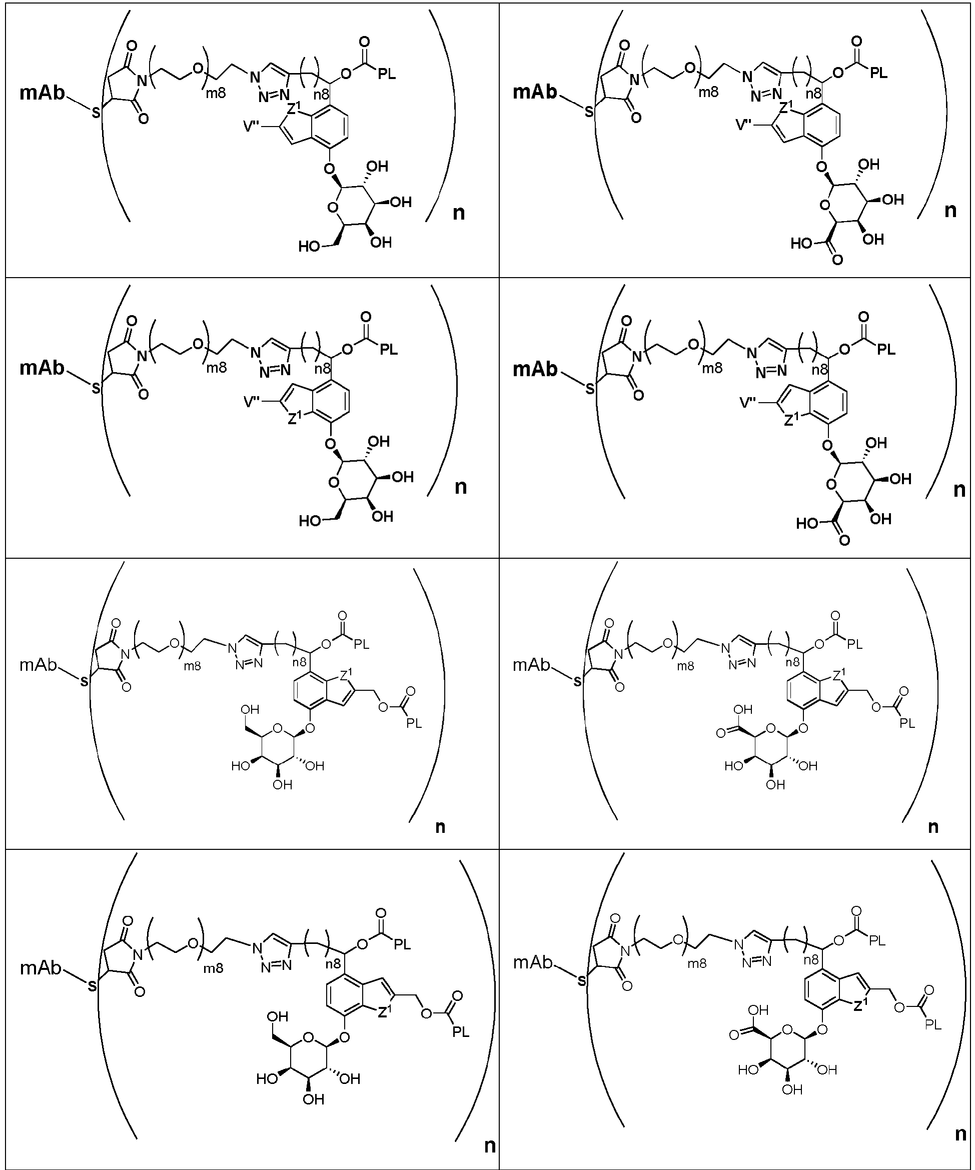
wherein mAb represents the moiety of the antibody;
V'' is an electron withdrawing group;
m8 and n8 are each independently an integer from 1 to 10;
Z¹ is a heteroatom selected from NR³, O, S and Se, and R³ is H or C₁-C₈ hydrocarbyl;
PL is the moiety of the active agent; and
n is a real number from 1 to 10.

31. The ligand-drug conjugate according to claim 30, wherein
the ligand-drug conjugate represented by Formula 2 is selected from the group consisting of conjugates represented by the following formulas: 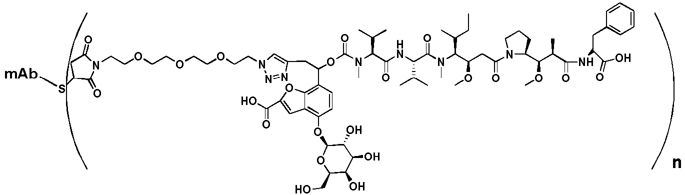 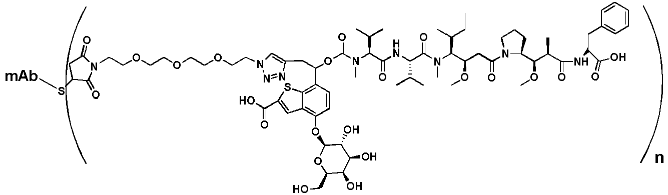 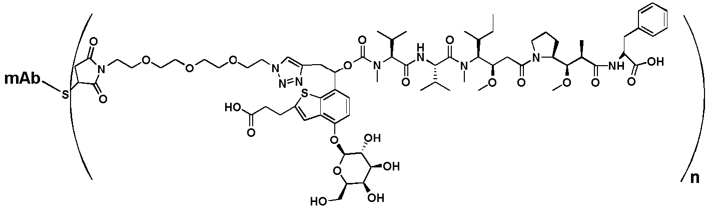 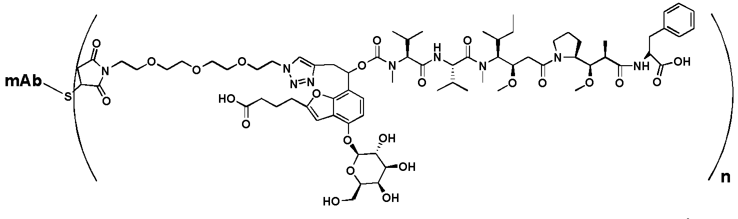 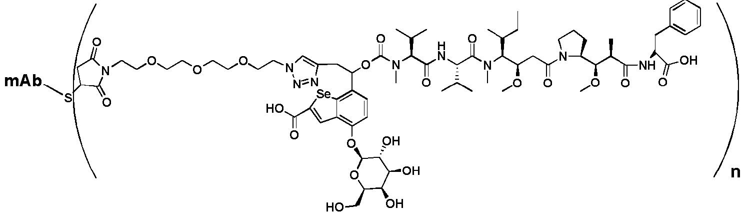 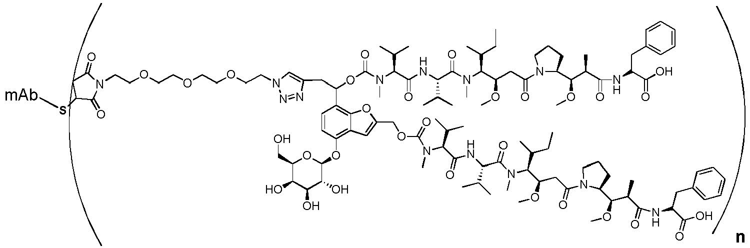 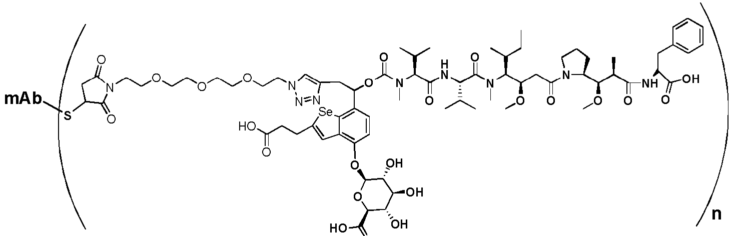 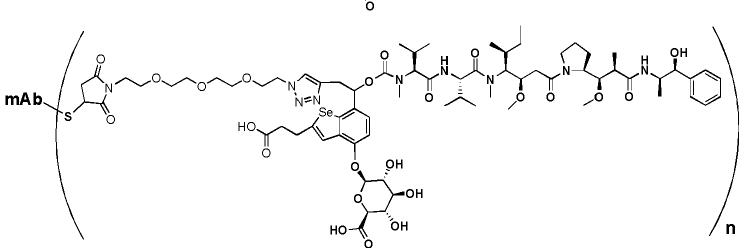 wherein mAb is the moiety of the antibody, and n is a real number from 1 to 10.

32. The ligand-drug conjugate according to claim 25, wherein
the compound represented by Formula 2 is a compound represented by Formula 2-1 below: 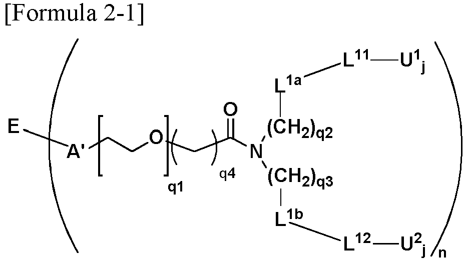 wherein
E is a ligand or protein having a receptor binding property;
A' is a divalent linking group derived from the binding functional group of A defined in claim 1;
U¹ and U² each have the same meaning as U defined in claim 1, wherein U¹ and U² are the same as or different from each other;
j is 1 to 10;
L¹¹ and L¹² each have the same meaning as L¹ defined in claim 1, wherein L¹¹ and L¹² are the same as or different from each other;
L^{1a} and L^{1b} are each independently selected from 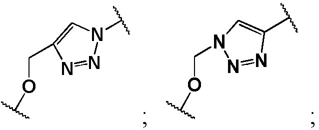 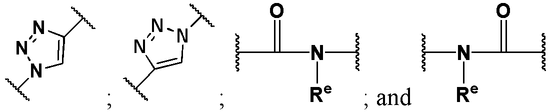 , and R^{e} is H or C₁-C₈ alkyl; and
q1, q2 and q3 are each independently an integer from 0 to 10, and q4 is an integer from 1 to 10;
provided that, if L^{1a} is 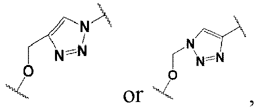 q2 is not 0, and if L^{1b} is 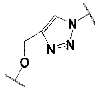 or 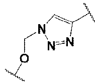 , q3 is not 0;
n is a real number from 1 to 10.

33. The ligand-drug conjugate according to claim 32, wherein
the conjugate represented by Formula 2-1 is selected from the group consisting of conjugates represented by the following formulas: 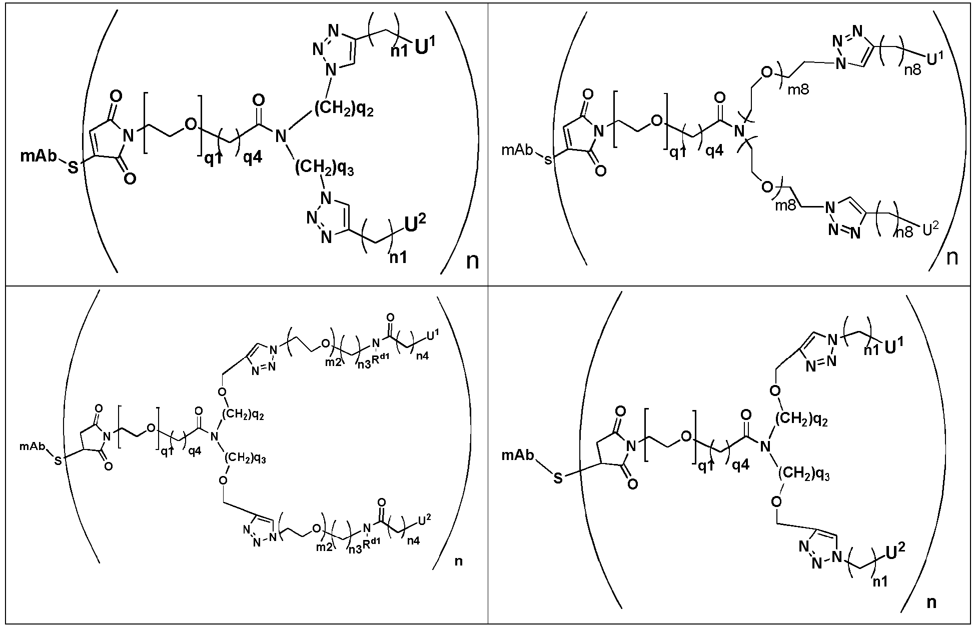 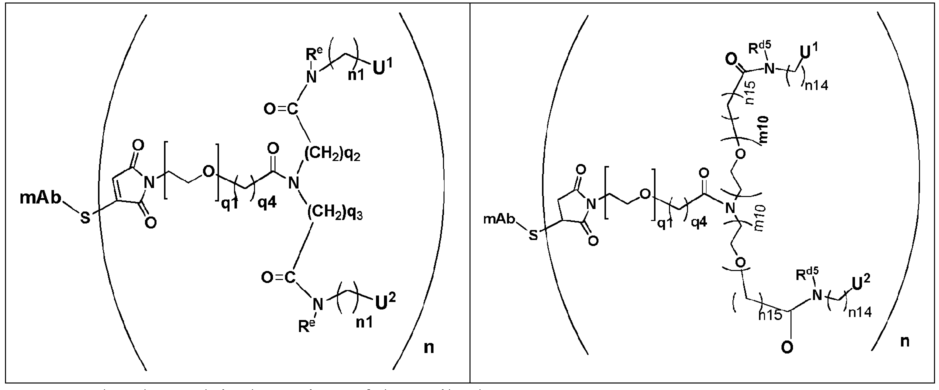
wherein mAb is the moiety of the antibody;
q1 to q4, n, U¹ and U² are as defined in claim 32;
R^{d1}, R^{d5} and R^{e} are each independently H or C₁-C₈ alkyl; and
n1, n3, n4, n8, n14 and n15, m2, m8 and m10 are each independently an integer from 1 to 8.

34. The ligand-drug conjugate according to claim 32, wherein
the conjugate represented by Formula 2-1 is selected from the group consisting of conjugates represented by the following formulas: 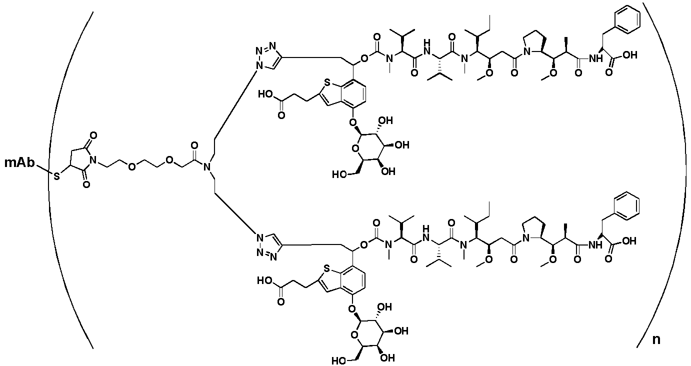 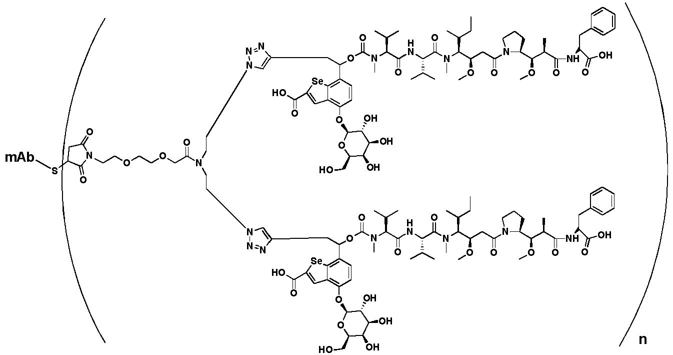 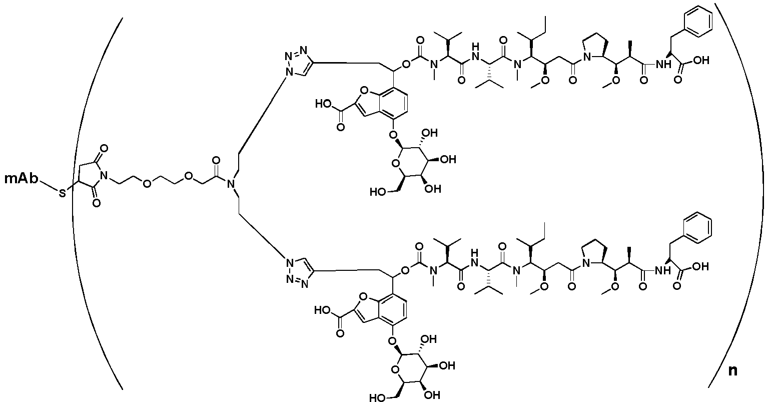 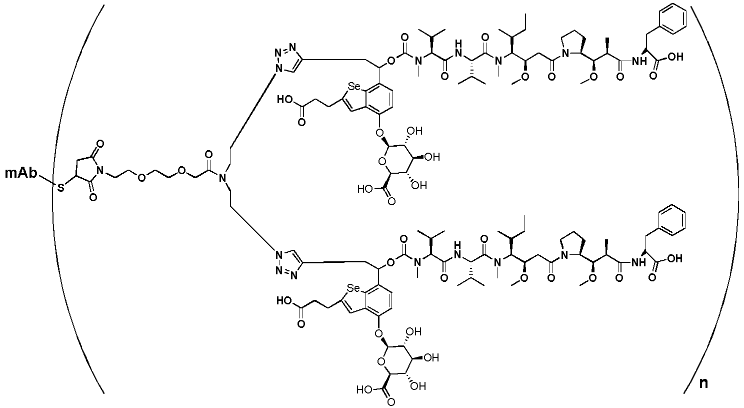 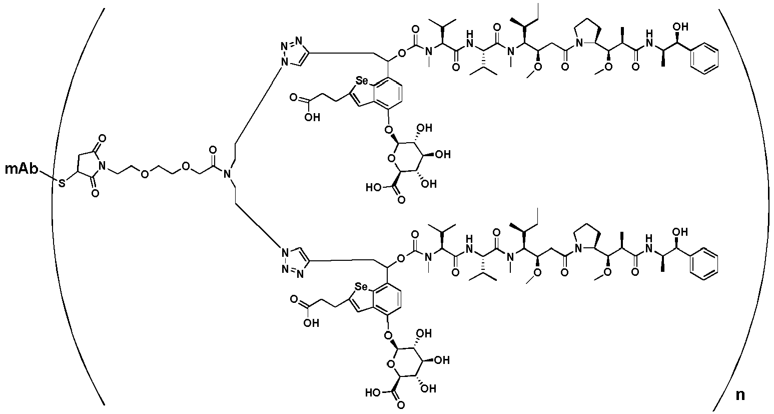 wherein mAb is the moiety of the antibody; and n is a real number from 1 to 10.

35. A pharmaceutical composition comprising the ligand-drug conjugate according to any one of claims 25 to 34, and a pharmaceutically acceptable carrier or excipient.

36. The pharmaceutical composition according to claim 35, wherein
the pharmaceutical composition is for the treatment or prevention of proliferative diseases, autoimmune diseases or infectious diseases.

37. An imaging composition comprising the ligand-drug conjugate according to any one of claims 25 to 34.

38. A composition for detection comprising the ligand-drug conjugate according to any one of claims 25 to 34.
